# EUROPEAN PATENT SPECIFICATION

(11) **EP 2 494 351 B1**
(45) Date of publication and mention of the grant of the patent: **08.06.2016**
(21) Application number: 10778592.5
(22) Date of filing: 26.10.2010
(51) Int. Cl.: G01N 33/50, G01N 33/574

(54) **COLON AND RECTAL TUMOR MARKERS AND METHODS OF USE THEREOF**
KOLON- UND REKTALTUMORMARKER SOWIE VERFAHREN ZU IHRER VERWENDUNG
MARQUEURS DE TUMEURS DU RECTUM ET DU CÔLON, ET LEURS MÉTHODES D'UTILISATION

(30) Priority: 26.10.2009 EP 09174059
(43) Date of publication of application: 05.09.2012
(73) Proprietor: Externautics S.p.A., 53100 Siena (IT)
(72) Inventor: GRIFANTINI, Renata, 53100 Siena (IT); PILERI, Piero, 53100 Siena (IT); CAMPAGNOLI, Susanna, 53100 Siena (IT); GRANDI, Alberto, 53100 Siena (IT); PARRI, Matteo, 53100 Siena (IT); PIERLEONI, Andrea, 53100 Siena (IT); NOGAROTTO, Renzo, 53100 Siena (IT)
(74) Representative: Banfi, Paolo
(86) International application number: PCT/EP2010/066144
(87) International publication number: WO 2011/051276

(56) References cited:
- EP-A1- 1 696 029
- WO-A1-99/32639
- WO-A1-2008/004719
- US-A1- 2007 237 770
- AUSCH C ET AL: "Caspase-cleaved cytokeratin 18 fragment (M30) as marker of postoperative residual tumor load in colon cancer patients" EUROPEAN JOURNAL OF SURGICAL ONCOLOGY, LONDON, GB, vol. 35, no. 11, 28 February 2009 (2009-02-28), pages 1164-1168, XP026681636 ISSN: 0748-7983 DOI: DOI:10.1016/J.EJSO.2009.02.007 [retrieved on 2009-02-28]

## Description

The present invention relates to newly identified proteins as markers for the detection of colon and rectal tumors, or as targets for their treatment. Also provided are affinity ligands capable of selectively interacting with the newly identified markers, as well as methods for tumor diagnosis and therapy using such ligands.

### Background of the invention

### Tumor markers (or biomarkers)

Tumor markers are substances that can be produced by tumor cells or by other cells of the body in response to cancer. In particular, a protein biomarker is either a single protein or a panel of different proteins that could be used to unambiguously distinguish a disease state. Ideally, a biomarker would have both a high specificity and sensitivity, being represented in a significant percentage of the cases of given disease and not in healthy state.

Biomarkers can be identified in different biological samples, like tissue biopsies or preferably biological fluids (saliva, urine, blood-derivatives and other body fluids), whose collection does not necessitate invasive treatments. Tumor marker levels may be categorized in three major classes on the basis of their clinical use. Diagnostic markers can be used in the detection and diagnosis of cancer. Prognostics markers are indicative of specific outcomes of the disease and can be used to define predictive models that allow the clinicians to predict the likely prognosis of the disease at time of diagnosis. Moreover, prognosis markers are helpful to monitor the patient response to a drug therapy and facilitate a more personalized patient management. A decrease or return to a normal level may indicate that the cancer is responding to therapy, whereas an increase may indicate that the cancer is not responding. After treatment has ended, tumor marker levels may be used to check for recurrence of the tumor. Finally, therapeutic markers can be used to develop tumor-specific drugs or affinity ligand (i.e. antibodies) for a prophylactic intervention.

Currently, although an abnormal tumor marker level may suggest cancer, this alone is usually not enough to accurately diagnose cancer and their measurement in body fluids is frequently combined with other tests, such as a biopsy and radioscopic examination. Frequently, tumor marker levels are not altered in all of people with a certain cancer disease, especially if the cancer is at early stage. Some tumor marker levels can also be altered in patients with noncancerous conditions. Most biomarkers commonly used in clinical practice do not reach a sufficiently high level of specificity and sensitivity to unambiguously distinguish a tumor from a normal state.

To date the number of markers that are expressed abnormally is limited to certain types/subtypes of cancer, some of which are also found in other diseases. (http://www.cancer.gov/cancertopics/factsheet).

For example, prostate-specific antigen (PSA) levels are often used to screen men for prostate cancer, but this is controversial since elevated PSA levels can be caused by both prostate cancer or benign conditions, and most men with elevated PSA levels turn out not to have prostate cancer.

Another tumor marker, Cancer Antigen 125, (CA 125), is sometimes used to screen women who have an increased risk for ovarian cancer. Scientists are studying whether measurement of CA 125, along with other tests and exams, is useful to find ovarian cancer before symptoms develop. So far, CA 125 measurement is not sensitive or specific enough to be used to screen all women for ovarian cancer. Mostly, CA 125 is used to monitor response to treatment and check for recurrence in women with ovarian cancer. Finally, human epidermal growth factor receptor (HER2) is a marker protein overproduced in about 20% of breast cancers, whose expression is typically associated with a more aggressive and recurrent tumors of this class.

### Routine screening test for tumor diagnosis

Screening tests are a way of detecting cancer early, before there are any symptoms. For a screening test to be helpful, it should have high sensitivity and specificity. Sensitivity refers to the test's ability to identify people who have the disease. Specificity refers to the test's ability to identify people who do not have the disease. Different molecular biology approaches such as analysis of DNA sequencing, small nucleotide polymorphyms, in situ hybridization and whole transcriptional profile analysis have done remarkable progresses to discriminate a tumor state from a normal state and are accelerating the knowledge process in the tumor field. However so far different reasons are delaying their use in the common clinical practice, including the higher analysis complexity and their expensiveness. Other diagnosis tools whose application is increasing in clinics include in situ hybridization and gene sequencing.

Currently, Immuno-HistoChemistry (IHC), a technique that allows the detection of proteins expressed in tissues and cells using specific antibodies, is the most commonly used method for the clinical diagnosis of tumor samples. This technique enables the analysis of cell morphology and the classification of tissue samples on the basis of their immunoreactivity. However, at present, IHC can be used in clinical practice to detect cancerous cells of tumor types for which protein markers and specific antibodies are available. In this context, the identification of a large panel of markers for the most frequent cancer classes would have a great impact in the clinical diagnosis of the disease.

### Anti-cancer therapies

In the last decades, an overwhelming number of studies remarkably contributed to the comprehension of the molecular mechanisms leading to cancer. However, this scientific progress in the molecular oncology field has not been paralleled by a comparable progress in cancer diagnosis and therapy. Surgery and/or radiotherapy are the still the main modality of local treatment of cancer in the majority of patients. However, these treatments are effective only at initial phases of the disease and in particular for solid tumors of epithelial origin, as is the case of colon, lung, breast, prostate and others, while they are not effective for distant recurrence of the disease. In some tumor classes, chemotherapy treatments have been developed, which generally relies on drugs, hormones and antibodies, targeting specific biological processes used by cancers to grow and spread. However, so far many cancer therapies had limited efficacy due to severity of side effects and overall toxicity. Indeed, a major effort in cancer therapy is the development of treatments able to target specifically tumor cells causing limited damages to surrounding normal cells thereby decreasing adverse side effects. Recent developments in cancer therapy in this direction are encouraging, indicating that in some cases a cancer specific therapy is feasible. In particular, the development and commercialization of humanized monoclonal antibodies that recognize specifically tumor-associated markers and promote the elimination of cancer is one of the most promising solutions that appears to be an extremely favorable market opportunity for pharmaceutical companies. However, at present the number of therapeutic antibodies available on the market or under clinical studies is very limited and restricted to specific cancer classes. So far licensed monoclonal antibodies currently used in clinics for the therapy of specific tumor classes, show only a partial efficacy and are frequently associated with chemotherapies to increase their therapeutic effect. Administration of Trastuzumab (Herceptin), a commercial monoclonal antibody targeting HER2, a protein overproduced in about 20% of breast cancers, in conjunction with Taxol adjuvant chemotherapy induces tumor remission in about 42% of the cases. Bevacizumab (Avastin) and Cetuximab (Erbitux) are two monoclonal antibodies recently licensed for use in humans, targeting the endothelial and epithelial growth factors respectively that, combined with adjuvant chemotherapy, proved to be effective against different tumor diseases. Bevacizumab proved to be effective in prolonging the life of patients with metastatic colorectal, breast and lung cancers. Cetuximab demostrated efficacy in patients with tumor types refractory to standard chemotherapeutic treatments (Adams G.P. and Weiner L.M. (2005) Monoclonal antibody therapy cancer. Nat Biotechnol. 23:1147-57).

In summary, available screening tests for tumor diagnosis are uncomfortable or invasive and this sometimes limits their applications. Moreover tumor markers available today have a limited utility in clinics due to either their incapability to detect all tumor subtypes of the defined cancers types and/or to distinguish unambiguously tumor vs. normal tissues. Similarly, licensed monoclonal antibodies combined with standard chemotherapies are not effective against the majority of cases. Therefore, there is a great demand for new tools to advance the diagnosis and treatment of cancer.

### Experimental approaches used to discover new tumor markers are based

Most popular approaches used to discover new tumor markers are based on genome-wide transcription profile or total protein content analyses of tumor. These studies usually lead to the identification of groups of mRNAs and proteins which are differentially expressed in tumors. Validation experiments then follow to eventually single out, among the hundreds of RNAs/proteins identified, the very few that have the potential to become useful markers. Although often successful, these approaches have several limitations and often, do not provide firm indications on the association of protein markers with tumor. A first limitation is that, since frequently mRNA levels not always correlate with corresponding protein abundance (approx. 50% correlation), studies based on transcription profile do not provide solid information regarding the expression of protein markers in tumor (1, 2, 3, 4).

A second limitation is that neither transcription profiles nor analysis of total protein content discriminate post-translation modifications, which often occur during oncogenesis. These modifications, including phosphorylations, acetylations, and glycosylations, or protein cleavages influence significantly protein stability, localization, interactions, and functions (5).

As a consequence, large scale studies generally result in long lists of differentially expressed genes that would require complex experimental paths in order to validate the potential markers. However, large scale genomic/proteomic studies reporting novel tumor markers frequently lack of confirmation data on the reported potential novel markers and thus do not provide solid demonstration on the association of the described protein markers with tumor.

The approach that we used to identify the protein markers included in the present invention is based on an innovative immuno-proteomic technology. In essence, a library of recombinant human proteins has been produced from E. coli and is being used to generate polyclonal antibodies against each of the recombinant proteins.

The screening of the antibodies library on TMAs carrying clinical samples from different patients affected by the tumor under investigation leads to the identification of specific tumor marker proteins. Therefore, by screening TMAs with the antibody library, the tumor markers are visualized by immuno-histochemistry, the classical technology applied in all clinical pathology laboratories. Since TMAs also include healthy tissues, the specificity of the antibodies for the tumors can be immediately appreciated and information on the relative level of expression and cellular localization of the markers can be obtained. In our approach the markers are subjected to a validation process consisting in a molecular and cellular characterization.

Altogether, the detection of the marker proteins disclosed in the present invention selectively in tumor samples and the subsequent validation experiments lead to an unambiguous confirmation of the marker identity and confirms its association with defined tumor classes. Moreover this process provides an indication of the possible use of the proteins as tools for diagnostic or therapeutic intervention. For instance, markers showing a surface cellular localization could be both diagnostic and therapeutic markers against which both chemical and antibody therapies can be developed. Differently, markers showing a cytoplasmic expression could be more likely considered for the development of tumor diagnostic tests and chemotherapy/small molecules treatments.

### Prior art

WO99/32639 (Regeneron Pharma) discloses the identification of receptor tyrosine kinase AR-1 and antibodies directed against it for its detection and treatment of tumors. The antibodies may be used to measure the amount of AR-1 in a sample taken from a patient for purposes of monitoring the course of therapy. Therein disclosed is also a method for identifying a cell which expresses human AR-1 receptor and methods for detecting expression of human AR-1 in cell or tissue samples.

EP1696029 discloses isolated nucleic acid molecules encoding TIE ligand homologues and the TIE ligand proteins encoded by such nucleic acid molecules. Therein disclosed is also a method of diagnosing tumor by detecting the level of expression of a TIE ligand encoding gene and the use of anti-TIE ligand antibodies as diagnostic agents, to detect disease states associated with the expression of a TIE receptor. The examples show the amplification of TIE ligand NL8 DNA in a variety of primary lung tumors

### Summary of the invention

The present invention provides new means for the detection and treatment of colo-rectal tumors based on the identification of a protein marker specific for these tumor types, namely the Angiopoietin-like 7 (ANGPTL7). 17)

The invention also provides a method for the diagnosis of these cancer types, comprising a step of detecting the above-identified marker in a biological sample, e.g. in a tissue sample of a subject suspected of having or at risk of developing malignancies or susceptible to cancer recurrences.

### Detailed disclosure of the invention

The present invention is based on the surprising finding of antibodies that are able to specifically stain tumor tissues from patients, while negative or very poor staining is observed in normal tissues from the same patients. These antibodies have been found to specifically bind to proteins for which no previous association with tumor has been reported. Hence, in a first aspect, the invention provides a tumor marker, which can be used alone or in combination in the detection of colo-rectal tumor and which is selected from ANGPTL7, SEQ ID NO:1, or a different isoform having sequence identity of at least 80%, preferably at least 90%, more preferably at least 95% to SEQ ID NO:1; or a nucleic acid molecule containing a sequence coding for a angiopoietin-like 7 protein, said encoding sequence being preferably SEQ ID NO: 2.

As used herein, "Percent (%) amino acid sequence identity" with respect to the marker protein sequences identified herein indicates the percentage of amino acid residues in a full-length protein variant or isoform according to the invention, or in a portion thereof, that are identical with the amino acid residues in the specific marker sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. Identity between nucleotide sequences is preferably determined by the Smith-Waterman homology search algorithm as implemented in the MPSRCH program (Oxford Molecular), using an affine gap search with parameters gap open penalty = 12 and gap extension penalty = 1.

Angiopoietin-like 7 (ANGPTL7, synonyms: Angiopoietin-related protein 7 Precursor, Angiopoietin-like factor, Cornea-derived transcript 6 protein; Gene ID: ENSG00000171819; Transcript ID: ENST00000376819; Protein ID:ENSP00000366015) is a member of the angiopoietin like family, whose role either in promoting or inhibiting angiogenesis is still under investigation (6). So far ANGPTL7 mRNA has been detected in some tissues including neural tissues, keratoconus cornea, trabecular meshwork, melanotic melanoma and uterus endometrial cancer, while evidences on protein expression are limited to corneal stroma (7). Based on available data, ANGPTL7 is a protein without previous known association with colon tumor classes and is preferably used as a marker for colon cancers. As described below, an antibody generated towards the ANGPTL7 protein shows a selective immunoreactivity in histological preparation of colo-rectal cancer tissues, which indicates the presence of this protein in these cancer samples and makes ANGPTL7 and its antibody highly interesting tools for specifically distinguishing colorectal cancer from a normal state. Moreover expression analysis showed that this protein is secreted by tumor cells lines. Therefore ANGPTL7 can be detected in body fluids of oncologic patients and can be conveniently used to develop diagnostic tools.

A further aspect of this invention is a method of screening a tissue sample for malignancy, which comprises determining the presence in said sample of at least one of the above-mentioned tumor markers. This method includes detecting either the marker protein, e.g. by means of labeled monoclonal or polyclonal antibodies that specifically bind to the target protein, or the respective mRNA, e.g. by means of polymerase chain reaction techniques such as RT-PCR. The methods for detecting proteins in a tissue sample are known to one skilled in the art and include immunoradiometric, immunoenzymatic or immunohistochemical techniques, such as radioimmunoassays, immunofluorescent assays or enzyme-linked immunoassays. Other known protein analysis techniques, such as polyacrylamide gel electrophoresis (PAGE), Western blot (WB) or Dot blot are suitable as well. Preferably, the detection of the protein marker is carried out with the immune-histochemistry technology, particularly by means of High Through-Put methods that allow the analyses of the antibody immune-reactivity simultaneously on different tissue samples immobilized on a microscope slide. Briefly, each Tissue Micro Array (TMA) slide includes tissue samples suspected of malignancy taken from different patients, and an equal number of normal tissue samples from the same patients as controls. The direct comparison of samples by qualitative or quantitative measurement, e.g. by enzimatic or colorimetric reactions, allows the identification of tumors.

In one embodiment, the invention provides a method of screening a sample of colon or colo-rectal tissue for malignancy, which comprises determining the presence in said sample of the tumor marker ANGPTL7, protein, variants or isoforms thereof as described above.

A further aspect of the invention is a method *in vitro* for determining the presence of a tumor in a subject, which comprises the steps of:
(1) providing a sample of the tissue suspected of containing tumor cells;
(2) determining the presence of the tumor marker as above defined, in said tissue sample by detecting the expression of the marker protein or the presence of the respective mRNA transcript;
wherein the detection of the tumor marker in the tissue sample is indicative of the presence of tumor in said subject.

The methods and techniques for carrying out the assay are known to one skilled in the art and are preferably based on immunoreactions for detecting proteins and on PCR methods for the detection of mRNAs. The same methods for detecting proteins or mRNAs from a tissue sample as disclosed above can be applied.

The antibodies to the tumor markers of the invention can be used to detect the presence of the marker in histologic preparations or to distinguish tumor cells from normal cells. To that purpose, the antibodies may be labeled with radiocative, fluorescent or enzyme labels.

The term "antibody" as used herein refers to all types of immunoglobulins, including IgG, IgM, IgA, IgD and IgE. Such antibodies may include polyclonal, monoclonal, chimeric, single chain, antibodies or fragments such as Fab or scFv. The antibodies may be of various origin, including human, mouse, rat, rabbit and horse, or chimeric antibodies. The production of antibodies is well known to one skilled in the art. For the production of antibodies in experimental animals, various hosts including goats, rabbits, rats, mice, and others, may be immunized by injection with polypeptides of the present invention or any fragment or oligopeptide or derivative thereof which has immunogenic properties or forms a suitable epitope. Monoclonal antibodies may be produced following the procedures described in Kohler and Milstein, Nature 265:495 (1975) or other techniques known in the art.

Herein disclosed is also a diagnostic kit containing suitable means for detection, in particular the polypeptides or polynucleotides, antibodies or fragments or derivatives thereof described above, reagents, buffers, solutions and materials needed for setting up and carrying out the immunoassays, nucleic acid hybridization or PCR assays described above. Parts of the kit of the invention can be packaged individually in vials or bottles or in combination in containers or multicontainer units.

### Description of the Figures

**Figure 1****. Analysis of purified ANGPTL7 recombinant protein expressed in *E. coli***
   Left panel: Comassie staining of purified His-tag ANGPTL7 fusion protein separated by SDS-PAGE; Right panel: WB on the recombinant ANGPTL7 protein stained with anti- ANGPTL7 antibody. Arrow marks the protein band of the expected size.
   Molecular weight markers are reported on the left.
**Figure 2****. Staining of colon tumor TMA with anti-ANGPTL7 antibodies**
   Staining of colon tumor TMA with anti-ANGPTL7 antibodies. Examples of TMA of tumor (lower panel) and normal tissue samples (upper panel) stained with anti-ANGPTL7 antibodies. The antibody stains specifically tumor cells (in dark gray).
**Figure 3****. Expression and secretion of ANGPTL7 in transiently transfected HeLa cells**
   Detection of ANGPTL7 in total protein extracts and cell culture supernatant from HeLa cells transfected with the empty pcDNA3 vector (lane 1, total extract; lane 3, supernatant) or the plasmid construct encoding the ANGPTL7 gene (lanes 2, total extract; lane 4, supernatant), stained with anti-ANGPTL7 antibody. Arrow marks the protein band of the expected size. Molecular weight markers are reported on the left.
   The following examples further illustrate the invention.

### EXAMPLES

### Example 1. Generation of recombinant human protein antigens and antibodies to identify tumor markers

### Methods

The entire coding region or suitable fragments of the genes encoding the target proteins, were designed for cloning and expression using bioinformatic tools with the human genome sequence as template (Lindskog M et al (2005). Where present, the leader sequence for secretion was replaced with the ATG codon to drive the expression of the recombinant proteins in the cytoplasm of E. coli. For cloning, genes were PCR-amplified from templates derived from Mammalian Gene Collection (http://mgc.nci.nih.gov/) clones or from cDNAs mixtures generated from pools of total RNA derived from Human testis, Human placenta, Human bone marrow, Human fetal brain, using specific primers. Clonings were designed so as to fuse a 10 histidine tag sequence at the 5' end, annealed to in house developed vectors, derivatives of vector pSP73 (Promega) adapted for the T4 ligation independent cloning method (Nucleic Acids Res. 1990 October 25; 18(20): 6069-6074) and used to transform *E.coli* NovaBlue cells recipient strain. *E. coli* tranformants were plated onto selective LB plates containing 100 µe E.coli clones were identified by restriction enzyme analysis of purified plasmid followed by DNA sequence analysis. For expression, plasmids were used to transform BL21-(DE3) *E.coli* cells and BL21-(DE3) E. coli cells harbouring the plasmid were inoculated in ZYP-5052 growth medium (Studier, 2005) and grown at 37°C for 24 hours. Afterwards, bacteria were collected by centrifugation, lysed into B-Per Reagent containing 1 mM MgCl2, 100 units DNAse I (Sigma), and 1 mg/ml lysozime (Sigma). After 30 min at room temperature under gentle shaking, the lysate was clarified by centrifugation at 30.000 g for 40 min at 4°C. All proteins were purified from the inclusion bodies by resuspending the pellet coming from lysate centrifugation in 40 mM TRIS-HCl, 1 mM TCEP {Tris(2-carboxyethyl)-phosphine hydrochloride, Pierce} and 6M guanidine hydrochloride, pH 8 and performing an IMAC in denaturing conditions. Briefly, the resuspended material was clarified by centrifugation at 30.000 g for 30 min and the supernatant was loaded on 0.5 ml columns of Ni-activated Chelating Sepharose Fast Flow (Pharmacia). The column was washed with 50 mM TRIS-HCl buffer, 1 mM TCEP, 6M urea, 60 mM imidazole, 0.5M NaCl, pH 8. Recombinant proteins were eluted with the same buffer containing 500 mM imidazole. Proteins were analysed by SDS-Page and their concentration was determined by Bradford assay using the BIORAD reagent (BIORAD) with a bovine serum albumin standard according to the manufacturer's recommendations. The identity of recombinant affinity purified proteins was further confirmed by tandem mass spectrometry (MS/MS), using standard procedures.

To generate antisera, the purified proteins were used to immunize CD1 mice (6 week-old females, Charles River laboratories, 5 mice per group) intraperitoneally, with 3 protein doses of 20 micrograms each, at 2 week-interval. Freund's complete adjuvant was used for the first immunization, while Freund's incomplete adjuvant was used for the two booster doses. Two weeks after the last immunization animals were bled and sera collected from each animal was pooled.

### Results

Gene fragments of the expected size were obtained by PCR from specific clones of the Mammalian Gene Collection or, alternatively, from cDNA generated from pools of total RNA derived from Human testis, Human placenta, Human bone marrow, Human fetal brain, using primers specific for each gene.

For the ANGPTL7 gene, a fragment corresponding to a fragment corresponding to nucleotides 318 to 1277 of the transcript SEQ ID ENST00000376819 and encoding a protein of 320 residues, corresponding to the amino acid region from 26 to 346 of ENSP00000366015 sequence was obtained.

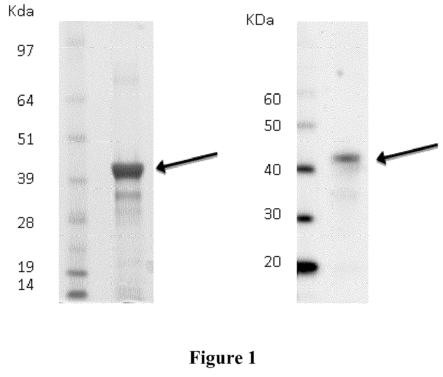

A clone encoding the correct amino acid sequence was identified for the gene/gene fragment and, upon expression in *E. coli,* a protein of the correct size was produced and subsequently purified using affinity chromatography (Figure 1 left panel). Antibodies generated by immunization specifically recognized the target protein in Western blot (WB) (Figure 1 right panel).

### Example 2. Tissue profiling by immune-histochemistry

### Methods

The analysis of the antibodies' capability to recognize their target proteins in tumor samples was carried out by Tissue Micro Array (TMA), a miniaturized immuno-histochemistry technology suitable for HTP analysis that allows to analyse the antibody immuno-reactivity simultaneously on different tissue samples immobilized on a microscope slide.

A tissue microarray was prepared containing formalin-fixed paraffin-embedded cores of human tissues from patients affected by colo-rectal cancer and corresponding normal tissues as controls and analyzed using the specific antibody sample. In total, the TMA design consisted in 10 colon tumor samples and 10 normal tissues from 5 well pedigreed patients (equal to two tumor samples and 2 normal tissues from each patient) to identify promising target molecules differentially expressed in cancer and normal cells. The direct comparison between tumor and normal tissues of each patient allowed the identification of antibodies that stain specifically tumor cells and provided indication of target expression in colo-rectal tumor.

In addition, to further confirm the data, a second TMA was used containing 100 formalin-fixed paraffin-embedded cores of colon tumor tissues from 50 patients (equal to two tissue samples from each patient).

All formalin fixed, paraffin embedded tissues used as donor blocks for TMA production were selected from the archives at the IEO (Istituto Europeo Oncologico, Milan). Corresponding whole tissue sections were examined to confirm diagnosis and tumour classification, and to select representative areas in donor blocks. Normal tissues were defined as microscopically normal (non-neoplastic) and were generally selected from specimens collected from the vicinity of surgically removed tumors. The TMA production was performed essentially as previously described (Kononen J et al (1998) Nature Med. 4:844-847; Kallioniemi OP et a/ (2001) Hum. MoI. Genet. 10:657-662). Briefly, a hole was made in the recipient TMA block. A cylindrical core tissue sample (1 mm in diameter) from the donor block was acquired and deposited in the recipient TMA block. This was repeated in an automated tissue arrayer "Galileo TMA CK 3500"( BioRep, Milan) until a complete TMA design was produced. TMA recipient blocks were baked at 42 <0>C for 2 h prior to sectioning. The TMA blocks were sectioned with 2-3 µm thickness using a waterfall microtome (Leica), and placed onto poli-L-lysinated glass slides for immuno-histochemical analysis. Automated immunohistochemistry was performed as previously described (Kampf C. et al (2004) Clin. Proteomics 1:285-300). In brief, the glass slides were incubated for 30' min in 60°C, de-paraffinized in xylene (2 x 15 min) using the Bio-Clear solution (Midway. Scientific, Melbourne, Australia), and re-hydrated in graded alcohols. For antigen retrieval, slides were immersed 0.01 M Na-citrate buffer, pH 6.0 at 99°C for 30 min Slides were placed in the Autostainer (R) (DakoCytomation) and endogenous peroxidase was initially blocked with 3% H2O2, for 5 min. Slides were then blocked in Dako Cytomation Wash Buffer containing 5% Bovine serum albumin (BSA) and subsequently incubated with mouse antibodies for 30' (dilution 1:200 in Dako Real ^{™} dilution buffer). After washing with DakoCytomation wash buffer, slides were incubated with the goat anti-mouse peroxidase conjugated Envision(R) for 30 min each at room temperature (DakoCytomation). Finally, diaminobenzidine (DakoCytomation) was used as chromogen and Harris hematoxylin (Sigma- Aldrich) was used for counterstaining. The slides were mounted with Pertex(R) (Histolab).

The staining results have been evaluated by a trained pathologist at the light microscope, and scored according to both the percentage of immunostained cells and the intensity of staining. The individual values and the combined score (from 0 to 300) were recorded in a custom-tailored database. Digital images of the immunocytochemical findings have been taken at a Leica DM LB light microscope, equipped with a Leica DFC289 color camera.

### Results

TMA analysis showed that the antibodies specific for the recombinant protein (see Example 1) are strongly immune-reactive on colon cancer tissues from patients with varying frequencies, indicating the presence of the target protein in colon tumors tissues, while no or poor reactivity was detected in normal tissues. Based on this finding, the detection of the target protein in tissue samples can be associated with the colon-rectum tumor/s.

The capability of target-specific antibodies to stain tumor tissues is summarized in Table I (the table reports the percentage of positive tumor tissue samples after staining with the target-specific antibodies).

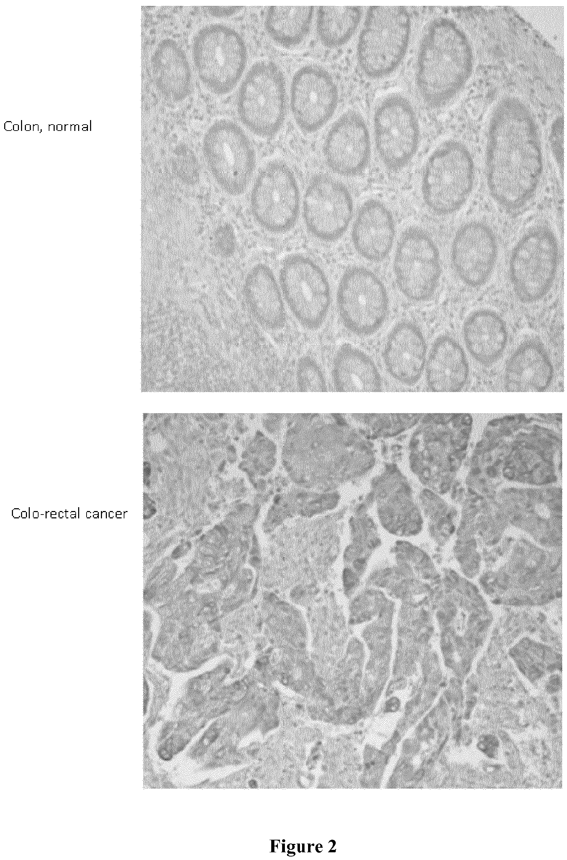

Representative examples of microscopic enlargements of tissue samples stained by the antibody are reported in Figure 2.

| **Table I.** | | |
|---|---|---|
| | **Gene** | **Percentage of colon tumor samples showing positive IHC staining** |
| **1** | **ANGPTL7** | **70*** |

| | | |
|---|---|---|
| (*) The antibody stains both colon tumor cells and secretion products, indicating that the corresponding proteins are specifically released by tumor cells. | | |

### Example 3. Expression and cell localization of target protein in transfected mammalian cells

### Methods

The expression of target proteins was assessed by Western blot analysis on total protein extracts from eukaryotic cells transiently transfected with plasmid constructs containing the complete coding sequences of the genes encoding the target proteins. Where indicated, expression and localization of target proteins were investigated by confocal microscopy analysis of transfected cells.

Examples of this type of experiments are given for ANGPTL7 (corresponding to Transcript ID ENST00000376819).

To this aim, cDNA were generated from pools of total RNA derived from Human testis, Human placenta, Human bone marrow, Human fetal brain, in reverse transcription reactions and the entire coding regions were PCR-amplified with specific primers pairs. PCR products were cloned into plasmid pcDNA3 (Invitrogen). HeLa and HEK293T cell lines were grown in DMEM-10% FCS supplemented with 1 mM Glutamine were transiently transfected with preparation of the resulting plasmid and with the empty vector as negative control using the Lipofectamine-2000 transfection reagent (Invitrogen). After 48 hours, cells were collected, lysed with PBS buffer containing 1% Triton X100 and expression of target proteins was assessed by Western blot analysis on total cell extracts (corresponding to 1x10⁶ cells) using marker-specific antibodies. In the case of ANGPTL7, cell culture supernatants were also collected and used for the analysis.

Western blot was performed by separation of the protein extracts on pre-cast SDS-PAGE gradient gels (NuPage 4-12% Bis-Tris gel, Invitrogen) under reducing conditions, followed by electro-transfer to nitrocellulose membranes (Invitrogen) according to the manufacturer's recommendations. The membranes were blocked in blocking buffer composed of 1x PBS-0.1% Tween 20 (PBST) added with 10% dry milk, for 1 h at room temperature, incubated with the antibody diluted 1:2500 in blocking buffer containing 1% dry milk and washed in PBST-1%. The secondary HRP-conjugated antibody (goat anti-mouse immunoglobulin/HRP, Perkin Elmer) was diluted 1:5000 in blocking buffer and chemiluminescence detection was carried out using a Chemidoc-IT UVP CCD camera (UVP) and the Western lightning^{Tm} cheminulescence Reagent Plus (Perkin Elmer), according to the manufacturer's protocol.

Surface localization of target proteins was assessed in transfected cells by cell surface staining and confocal microscopy.

Cells were plated on glass cover slips and after 48 h were washed with PBS and fixed with 3% *p*-formaldheyde solution in PBS for 20 min at RT. Cells were incubated overnight at 4°C with marker-specific polyclonal antibodies (1:200). Cells were then stained with Alexafluor 488-labeled goat anti-mouse antibodies (Molecular Probes). DAPI (Molecular Probes) was used to visualize nuclei; Live/Dead® red fixable (Molecular Probes) was used to visualize membrane. The cells were mounted with glycerol plastine and observed under a laser-scanning confocal microscope (LeicaSP5).

### Results

The complete coding sequences for the target protein ANGPTL7, were cloned in a eukaryotic expression vector and the derived plasmids were used for transient transfection of HeLa or HEK293T cells.

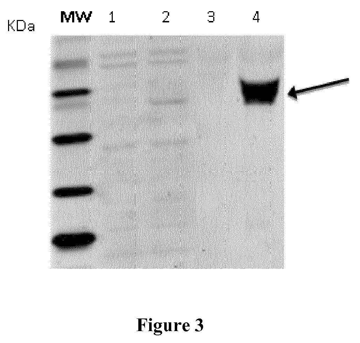

Western blot analysis confirmed that the marker-specific antibodies recognized specifically their target proteins. Concerning C9orf46, a band of the expected size was visible detected by the specific antibodies in total extracts from HeLa cells transfected with plasmid encoding the proteins while the same band was very faintly detected in HeLa cells transfected with the empty pcDNA3 plasmid. In the case of ANGPTL7, a weak specific protein band was visible in the cell extracts of transfected HeLa cells, while a very intense protein band was detected in the cell supernatant, indicating that the protein is almost completely secreted by transfected cells (Figure 3).

### Example 4. Detection of target protein in colon tumor tissue homogenates

The presence of protein bands corresponding to the marker protein was also investigated in tissue homogenates of colon tumor biopsies as compared to normal tissues from patients. Homogenates were prepared by mechanic tissue disruption in buffer containing 40 mM TRIS-HCl, 1 mM TCEP {Tris(2-carboxyethyl)-phosphine hydrochloride, Pierce} and 6M guanidine hydrochloride, pH 8. Western blot was performed by separation of the total protein extracts (20 µg/lane) proteins were detected by specific antibodies.

### Example 5. Expression and localization of target protein in tumor cell lines

Expression of target proteins was assessed by WB and/or flow cytometry analysis of tumor cell lines, including the and the cell lines OVCAR-5 and OVCAR-8 and the colon tumor cell lines HCT-15, COLO-205, HCC-2998. Cells were cultured under ATCC recommended conditions, and sub-confluent cell mono-layers were detached with PBS-0.5 mM EDTA for subsequent analysis. For Western blot, cells were lysed by several freeze-thaw passages in PBS-1% Triton. Total protein extracts were loaded on SDS-PAGE (2x10⁵ cells/lane), and subjected to WB with specific antibodies as described above.

For flow cytometry analysis cells (2x10⁴ per well) were pelletted in 96 U-bottom microplates by centrifugation at 200 x g for 5 min at 4°C and incubated for 1 hour at 4°C with the appropriate dilutions of the marker-specific antibodies. The cells were washed twice in PBS-5% FCS and incubated for 20 min with the appropriate dilution of R-Phycoerythrin (PE)-conjugated secondary antibodies (Jackson Immuno Research, PA, USA) at 4°C. After washing, cells were analysed by a FACS Canto II flow cytometer (Becton Dickinson). Data were analyzed with FlowJo 8.3.3 program.

### Example 6. Expression of the marker proteins confers malignant cell phenotype

To verify that the proteins included in the present invention can be exploited as targets for therapeutic applications, the effect of marker depletion was evaluated *in vitro* in cellular studies generally used to define the role of newly discovered proteins in tumor development. Marker-specific knock-down and control tumor cell lines were assayed for proliferation and the migration/invasiveness properties using the MTT and the Boyden in vitro invasion assays, respectively.

### Method

Expression of marker genes were silenced in tumor cell lines by the siRNA technology and the influence of the reduction of marker expression on cell parameters relevant for tumor development was assessed in *in vitro* assays. The expression of marker genes was knocked down in a panel of epithelial tumor cell lines previously shown to express the tumor markers using a panel of marker-specific siRNAs (whose target sequences are reported in Table II) using the HiPerfect transfection reagent (QIAGEN) following the manufacturer's protocol. As control, cells treated with irrelevant siRNA (scrambled siRNA) were analysed in parallel. At different time points (ranging from 24 to 72 hours) post transfection, the reduction of gene transcription was assessed by quantitative RT-PCR (Q-RT-PCR) on total RNA, by evaluating the relative marker transcript level, using the beta-actin, GAPDH or MAPK genes as internal normalization control. Afterwards, cell proliferation and migration/invasiveness assays were carried out to assess the effect of the reduced marker expression. Cell proliferation was determined using the MTT assay, a colorimetric assay based on the cellular conversion of a tetrazolium salt into a purple colored formazan product. Absorbance of the colored solution can be quantified using a spectrophotometer to provide an estimate of the number of attached living cells. Approximately 5 x 10³ cells/100µl were seeded in 96-well plates in DMEM with 10% FCS to allow cell attachment. After overnight incubation with DMEM without FCS, the cells were treated with 2,5% FBS for 72 hours. Four hours before harvest 15 µL of the MTT dye solution (Promega) were added to each well. After 4-hour incubation at 37°C, the formazan precipitates were solubilized by the addition of 100 µL of solubilization solution (Promega) for 1h at 37°C,. Absorbance at 570 nm was determined on a multiwell plate reader (SpectraMax, Molecular Devices).

Cell migration/invasiveness was tested using the Boyden *in vitro* invasion assay, as compared to control cell lines treated with a scramble siRNA. This assay is based on a chamber of two medium-filled compartments separated by a microporous membrane. Cells are placed in the upper compartment and are allowed to migrate through the pores of the membrane into the lower compartment, in which chemotactic agents are present. After an appropriate incubation time, the membrane between the two compartments is fixed and stained, and the number of cells that have migrated to the lower side of the membrane is determined. For this assay, a transwell system, equipped with 8-µm pore polyvinylpirrolidone-free polycarbonate filters, was used. The upper sides of the porous polycarbonate filters were coated with 50 µg/cm² of reconstituted Matrigel basement membrane and placed into six-well culture dishes containing complete growth medium. Cells (1x10⁴ cells/well) were loaded into the upper compartment in serum-free growth medium. After 16 h of incubation at 37°C, non invading cells were removed mechanically using cotton swabs, and the microporous membrane was stained with Diff-Quick solution. Chemotaxis was evaluated by counting the cells migrated to the lower surface of the polycarbonate filters (six randomly chosen fields, mean ± SD).

### References

1) Anderson, L., and Seilhamer, J. (1997). A comparison of selected mRNA and protein abundances in human liver. Electrophoresis 18, 533 -537;
2) Chen, G., Gharib, T. G., Wang, H., Huang, C. C., Kuick, R., Thomas, D. G., Shedden, K. A., Misek, D. E., Taylor, J. M., Giordano, T. J., Kardia, S. L., Iannettoni, M. D., Yee, J., Hogg, P. J., Orringer, M. B., Hanash, S. M., and Beer, D. G. (2003) Protein profiles associated with survival in lung adenocarcinoma. Proc. Natl. Acad. Sci. U. S. A 100, 13537 - 13542;
3) Ginestier, C., Charafe-Jauffret, E., Bertucci, F., Eisinger, F., Geneix, J., Bechlian, D., Conte, N., Adelaide, J., Toiron, Y., Nguyen, C., Viens, P., Mozziconacci, M. J., Houlgatte, R., Birnbaum, D., and Jacquemier, J. (2002) Distinct and complementary information provided by use of tissue and DNA microarrays in the study of breast tumor markers. Am. J. Pathol. 161, 1223 - 1233;
4) Gygi, S. P., Rochon, Y., Franza, B. R., and Aebersold, R. (1999) Correlation between protein and mRNA abundance in yeast. Mol. Cell. Biol. 19, 1720 -1730; Nishizuka, S., Charboneau, L., Young, L., Major, S., Reinhold, W. C., Waltham, M., Kouros-Mehr, H., Bussey, K. J., Lee, J. K., Espina, V., Munson, P. J., Petricoin, E., III, Liotta, L. A., and Weinstein, J. N. (2003) Proteomic profiling of the NCI-60 cancer cell lines using new high-density reverse-phase lysate microarrays. Proc. Natl. Acad. Sci. U. S. A 100, 14229 - 14234
5) Tyers, M., and Mann, M. (2003) From genomics to proteomics. Nature 422, 193 - 197;
6) Bouïs D, Hospers GA, Meijer C, Dam W, Peek R, Mulder NH., (2007) Effects of the CDT6/ANGX gene on tumour growth in immune competent mice, In Vivo, 2003 17:157-61; Bouïs DR, Dam WA, Meijer C, Mulder NH, Hospers GA. Effect of CDT6 on factors of angiogenic balance in tumour cell lines. Anticancer Res., 27:2325-2329);
7) Peek R, Kammerer RA, Frank S, Otte-Höller I, Westphal JR. (2002) The angiopoietin-like factor cornea-derived transcript 6 is a putative morphogen for human cornea. J Biol Chem, 277:686-693);

## Claims

1. The use of:
ANGPTL7, SEQ ID NO:1, or a different isoform having sequence identity of at least 80% to SEQ ID NO:1; or a nucleic acid molecule containing a sequence coding for a angiopoietin-like 7 protein,
as a tumor marker for *in vitro* detection of colon or rectal tumors.

2. The use according to claim 1, wherein said isoform has at least 90% sequence identity to SEQ ID NO:1.

3. The use according to claim 2, wherein said isoform has at least 95% seqeunce identity to SEQ ID NO:1.

4. The use according to claim 1, wherein said sequence coding for a angiopoietin-like 7 protein is SEQ ID NO: 2.

5. A method of screening a colon or rectal tissue sample for malignancy, said method comprising determining the presence in said sample of the tumor marker of claims 1-4.

6. A method according to claim 5, wherein the tumor marker is a protein, said method being based on immunoradiometric, immunoenzymatic or immunohistochemical techniques.

7. A method according to claim 5, wherein the tumor marker is a nucleic acid molecule, said method being based on polymerase chain reaction techniques.

8. A method *in vitro* for determining the presence of a colon or rectal tumor in a subject, which comprises the steps of:
(a) providing a sample of the tissue suspected of containing tumor cells;
(b) determining the presence of the tumor marker according to claims 1-4 in said tissue sample by detecting the expression of the marker protein or the presence of the respective mRNA transcript;
wherein the detection of the tumor marker in the tissue sample is indicative of the presence of tumor in said subject.

## Patentansprüche

1. Verwendung von:
ANGPTL7, SEQ ID NO:1 oder einer unterschiedlichen Isoform, welche eine Sequenzidentität von mindestens 80% zu SEQ ID NO:1 aufweist oder eines Nucleinsäuremoleküls, das eine Sequenz enthält, die für ein Angiopoietin-like 7 Protein codiert,
als Tumormarker für die In-vitro-Detektion von Kolontumoren oder rektalen Tumoren.

2. Verwendung gemäß Anspruch 1, wobei die Isoform mindestens 90% Sequenzidentität mit SEQ ID NO:1 aufweist.

3. Verwendung gemäß Anspruch 2, wobei die Isoform mindestens 95% Sequenzidentität mit SEQ ID NO:1 aufweist.

4. Verwendung gemäß Anspruch 1, wobei die Sequenz, welche für ein Angiopoietin-like 7 Protein codiert, SEQ ID NO:2 ist.

5. Verfahren zur Duchmusterung von Kolon- oder Rektalgewebeproben auf Tumorbösartigkeit, wobei das Verfahren die Bestimmung der Anwesenheit der Tumormarker gemäß Anspruch 1-4 in der Probe umfasst.

6. Verfahren gemäß Anspruch 5, wobei der Tumormarker ein Protein ist und das Verfahren auf immunradiometrischen, immunenzymatischen oder immunhistochemischen Techniken basiert.

7. Verfahren gemäß Anspruch 5, wobei der Tumormarker ein Nucleinsäuremolekül ist, wobei das Verfahren auf Polymerasekettenreaktionstechniken basiert.

8. *In-vitro*-Verfahren zur Bestimmung der Anwesenheit eines Kolontumors oder rektalen Tumors in einem Patienten, umfassend die Schritte:
(a) Zurverfügungstellung der Probe eines Gewebes von dem vermutet wird, dass es Tumorzellen enthält,
(b) Bestimmung der Anwesenheit des Tumormarkers gemäß Anspruch 1-4 in der Gewebeprobe durch die Detektion der Expression des Markerproteins oder der Anwesenheit des entsprechenden mRNA-Transkripts,
wobei die Detektion des Tumormarkers in der Gewebeprobe die Anwesenheit eines Tumors im Patienten indiziert.

## Revendications

1. Utilisation de :
ANGPTL7, SEQ ID NO: 1, ou d'un isoforme différent possédant une identité de séquence d'au moins 80 % par rapport à la SEQ ID NO: 1, ou d'une molécule d'acide nucléique contenant une séquence codant pour une protéine 7 analogue de l'angiopoïétine,
à titre de marqueur tumoral pour la détection *in vitro* de tumeurs du côlon ou de tumeurs rectales.

2. Utilisation selon la revendication 1, dans laquelle ledit isoforme possède une identité de séquence d'au moins 90 % par rapport à la SEQ ID NO: 1.

3. Utilisation selon la revendication 2, dans laquelle ledit isoforme possède une identité de séquence d'au moins 95 % par rapport à la SEQ ID NO: 1.

4. Utilisation selon la revendication 1, dans laquelle ladite séquence codant pour une protéine 7 analogue de l'angiopoïétine est la SEQ ID NO: 2.

5. Procédé de dépistage d'une malignité sur un échantillon de tissu de côlon ou de tissu rectal, ledit procédé comprenant la détermination de la présence dans ledit échantillon du marqueur tumoral selon les revendications 1 à 4.

6. Procédé selon la revendication 5, dans lequel le marqueur tumoral est une protéine, ledit procédé se basant sur des techniques immunoradiométriques, immunoenzymatiques ou immunohistochimiques.

7. Procédé selon la revendication 5, dans lequel le marqueur tumoral est une molécule d'acide nucléique, ledit procédé se basant sur des techniques de réactions en chaîne par polymérase.

8. Procédé in vitro pour la détermination de la présence d'une tumeur du côlon ou d'une tumeur rectale dans un sujet, qui comprend les étapes dans lesquelles :
(a) Procuration d'un échantillon du tissu suspecté de contenir des cellules tumorales ;
(b) Détermination de la présence du marqueur tumoral selon les revendications 1 à 4 dans ledit échantillon de tissu en détectant l'expression de la protéine marqueur ou la présence du transcrit d'ARNm respectif ;
la détection du marqueur tumoral dans l'échantillon de tissu étant le signe de la présence d'une tumeur dans ledit sujet.


### SEQUENCE LISTING

<110> EXTERNAUTICS S.P.A
<120> colon and rectal tumor markers and methos of use thereof
<130> 1297PCT
<160> 86
<170> PatentIn version 3.3
<210> 1
   <211> 346
   <212> PRT
   <213> Homo sapiens
<400> 1
   Met Leu Lys Lys Pro Leu Ser Ala Val Thr Trp Leu Cys Ile Phe Ile
   1               5                   10                  15
   Val Ala Phe Val Ser His Pro Ala Trp Leu Gln Lys Leu Ser Lys His
               20                  25                  30
   Lys Thr Pro Ala Gln Pro Gln Leu Lys Ala Ala Asn Cys Cys Glu Glu
           35                  40                  45
   Val Lys Glu Leu Lys Ala Gln Val Ala Asn Leu Ser Ser Leu Leu Ser
       50                  55                  60
   Glu Leu Asn Lys Lys Gln Glu Arg Asp Trp Val Ser Val Val Met Gln
   65                  70                  75                  80
   Val Met Glu Leu Glu Ser Asn Ser Lys Arg Met Glu Ser Arg Leu Thr
                   85                  90                  95
   Asp Ala Glu Ser Lys Tyr Ser Glu Met Asn Asn Gln Ile Asp Ile Met
               100                 105                 110
   Gln Leu Gln Ala Ala Gln Thr Val Thr Gln Thr Ser Ala Asp Ala Ile
           115                 120                 125
   Tyr Asp Cys Ser Ser Leu Tyr Gln Lys Asn Tyr Arg Ile Ser Gly Val
       130                 135                 140
   Tyr Lys Leu Pro Pro Asp Asp Phe Leu Gly Ser Pro Glu Leu Glu Val
   145                 150                 155                 160
   Phe Cys Asp Met Glu Thr Ser Gly Gly Gly Trp Thr Ile Ile Gln Arg
                   165                 170                 175
   Arg Lys Ser Gly Leu Val Ser Phe Tyr Arg Asp Trp Lys Gln Tyr Lys
               180                 185                 190
   Gln Gly Phe Gly Ser Ile Arg Gly Asp Phe Trp Leu Gly Asn Glu His
           195                 200                 205
   Ile His Arg Leu Ser Arg Gln Pro Thr Arg Leu Arg Val Glu Met Glu
       210                 215                 220
   Asp Trp Glu Gly Asn Leu Arg Tyr Ala Glu Tyr Ser His Phe Val Leu
   225                 230                 235                 240
   Gly Asn Glu Leu Asn Ser Tyr Arg Leu Phe Leu Gly Asn Tyr Thr Gly
                   245                 250                 255
   Asn Val Gly Asn Asp Ala Leu Gln Tyr His Asn Asn Thr Ala Phe Ser
               260                 265                 270
   Thr Lys Asp Lys Asp Asn Asp Asn Cys Leu Asp Lys Cys Ala Gln Leu
           275                 280                 285
   Arg Lys Gly Gly Tyr Trp Tyr Asn Cys Cys Thr Asp Ser Asn Leu Asn
       290                 295                 300
   Gly Val Tyr Tyr Arg Leu Gly Glu His Asn Lys His Leu Asp Gly Ile
   305                 310                 315                 320
   Thr Trp Tyr Gly Trp His Gly Ser Thr Tyr Ser Leu Lys Arg Val Glu
                   325                 330                 335
   Met Lys Ile Arg Pro Glu Asp Phe Lys Pro
               340                 345
<210> 2
   <211> 2237
   <212> DNA
   <213> Homo sapiens
<210> 3
   <211> 147
   <212> PRT
   <213> Homo sapiens
<400> 3
   Met Gly Phe Ile Phe Ser Lys Ser Met Asn Glu Ser Met Lys Asn Gln
   1               5                   10                  15
   Lys Glu Phe Met Leu Met Asn Ala Arg Leu Gln Leu Glu Arg Gln Leu
               20                  25                  30
   Ile Met Gln Ser Glu Met Arg Glu Arg Gln Met Ala Met Gln Ile Ala
           35                  40                  45
   Trp Ser Arg Glu Phe Leu Lys Tyr Phe Gly Thr Phe Phe Gly Leu Ala
       50                  55                  60
   Ala Ile Ser Leu Thr Ala Gly Ala Ile Lys Lys Lys Lys Pro Ala Phe
   65                  70                  75                  80
   Leu Val Pro Ile Val Pro Leu Ser Phe Ile Leu Thr Tyr Gln Tyr Asp
                   85                  90                  95
   Leu Gly Tyr Gly Thr Leu Leu Glu Arg Met Lys Gly Glu Ala Glu Asp
               100                 105                 110
   Ile Leu Glu Thr Glu Lys Ser Lys Leu Gln Leu Pro Arg Gly Met Ile
           115                 120                 125
   Thr Phe Glu Ser Ile Glu Lys Ala Arg Lys Glu Gln Ser Arg Phe Phe
       130                 135                 140
   Ile Asp Lys
   145
<210> 4
   <211> 932
   <212> DNA
   <213> Homo sapiens
<210> 5
   <211> 831
   <212> PRT
   <213> Homo sapiens
<400> 5
   Met Lys Val His Met His Thr Lys Phe Cys Leu Ile Cys Leu Leu Thr
   1               5                   10                  15
   Phe Ile Phe His His Cys Asn His Cys His Glu Glu His Asp His Gly
               20                  25                  30
   Pro Glu Ala Leu His Arg Gln His Arg Gly Met Thr Glu Leu Glu Pro
           35                  40                  45
   Ser Lys Phe Ser Lys Gln Ala Ala Glu Asn Glu Lys Lys Tyr Tyr Ile
       50                  55                  60
   Glu Lys Leu Phe Glu Arg Tyr Gly Glu Asn Gly Arg Leu Ser Phe Phe
   65                  70                  75                  80
   Gly Leu Glu Lys Leu Leu Thr Asn Leu Gly Leu Gly Glu Arg Lys Val
                   85                  90                  95
   Val Glu Ile Asn His Glu Asp Leu Gly His Asp His Val Ser His Leu
               100                 105                 110
   Asp Ile Leu Ala Val Gln Glu Gly Lys His Phe His Ser His Asn His
           115                 120                 125
   Gln His Ser His Asn His Leu Asn Ser Glu Asn Gln Thr Val Thr Ser
       130                 135                 140
   Val Ser Thr Lys Arg Asn His Lys Cys Asp Pro Glu Lys Glu Thr Val
   145                 150                 155                 160
   Glu Val Ser Val Lys Ser Asp Asp Lys His Met His Asp His Asn His
                   165                 170                 175
   Arg Leu Arg His His His Arg Leu His His His Leu Asp His Asn Asn
               180                 185                 190
   Thr His His Phe His Asn Asp Ser Ile Thr Pro Ser Glu Arg Gly Glu
           195                 200                 205
   Pro Ser Asn Glu Pro Ser Thr Glu Thr Asn Lys Thr Gln Glu Gln Ser
       210                 215                 220
   Asp Val Lys Leu Pro Lys Gly Lys Arg Lys Lys Lys Gly Arg Lys Ser
   225                 230                 235                 240
   Asn Glu Asn Ser Glu Val Ile Thr Pro Gly Phe Pro Pro Asn His Asp
                   245                 250                 255
   Gln Gly Glu Gln Tyr Glu His Asn Arg Val His Lys Pro Asp Arg Val
               260                 265                 270
   His Asn Pro Gly His Ser His Val His Leu Pro Glu Arg Asn Gly His
           275                 280                 285
   Asp Pro Gly Arg Gly His Gln Asp Leu Asp Pro Asp Asn Glu Gly Glu
       290                 295                 300
   Leu Arg His Thr Arg Lys Arg Glu Ala Pro His Val Lys Asn Asn Ala
   305                 310                 315                 320
   Ile Ile Ser Leu Arg Lys Asp Leu Asn Glu Asp Asp His His His Glu
                   325                 330                 335
   Cys Leu Asn Val Thr Gln Leu Leu Lys Tyr Tyr Gly His Gly Ala Asn
               340                 345                 350
   Ser Pro Ile Ser Thr Asp Leu Phe Thr Tyr Leu Cys Pro Ala Leu Leu
           355                 360                 365
   Tyr Gln Ile Asp Ser Arg Leu Cys Ile Glu His Phe Asp Lys Leu Leu
       370                 375                 380
   Val Glu Asp Ile Asn Lys Asp Lys Asn Leu Val Pro Glu Asp Glu Ala
   385                 390                 395                 400
   Asn Ile Gly Ala Ser Ala Trp Ile Cys Gly Ile Ile Ser Ile Thr Val
                   405                 410                 415
   Ile Ser Leu Leu Ser Leu Leu Gly Val Ile Leu Val Pro Ile Ile Asn
               420                 425                 430
   Gln Gly Cys Phe Lys Phe Leu Leu Thr Phe Leu Val Ala Leu Ala Val
           435                 440                 445
   Gly Thr Met Ser Gly Asp Ala Leu Leu His Leu Leu Pro His Ser Gln
       450                 455                 460
   Gly Gly His Asp His Ser His Gln His Ala His Gly His Gly His Ser
   465                 470                 475                 480
   His Gly His Glu Ser Asn Lys Phe Leu Glu Glu Tyr Asp Ala Val Leu
                   485                 490                 495
   Lys Gly Leu Val Ala Leu Gly Gly Ile Tyr Leu Leu Phe Ile Ile Glu
               500                 505                 510
   His Cys Ile Arg Met Phe Lys His Tyr Lys Gln Gln Arg Gly Lys Gln
           515                 520                 525
   Lys Trp Phe Met Lys Gln Asn Thr Glu Glu Ser Thr Ile Gly Arg Lys
       530                 535                 540
   Leu Ser Asp His Lys Leu Asn Asn Thr Pro Asp Ser Asp Trp Leu Gln
   545                 550                 555                 560
   Leu Lys Pro Leu Ala Gly Thr Asp Asp Ser Val Val Ser Glu Asp Arg
                   565                 570                 575
   Leu Asn Glu Thr Glu Leu Thr Asp Leu Glu Gly Gln Gln Glu Ser Pro
               580                 585                 590
   Pro Lys Asn Tyr Leu Cys Ile Glu Glu Glu Lys Ile Ile Asp His Ser
           595                 600                 605
   His Ser Asp Gly Leu His Thr Ile His Glu His Asp Leu His Ala Ala
       610                 615                 620
   Ala His Asn His His Gly Glu Asn Lys Thr Val Leu Arg Lys His Asn
   625                 630                 635                 640
   His Gln Trp His His Lys His Ser His His Ser His Gly Pro Cys His
                   645                 650                 655
   Ser Gly Ser Asp Leu Lys Glu Thr Gly Ile Ala Asn Ile Ala Trp Met
               660                 665                 670
   Val Ile Met Gly Asp Gly Ile His Asn Phe Ser Asp Gly Leu Ala Ile
           675                 680                 685
   Gly Ala Ala Phe Ser Ala Gly Leu Thr Gly Gly Ile Ser Thr Ser Ile
       690                 695                 700
   Ala Val Phe Cys His Glu Leu Pro His Glu Leu Gly Asp Phe Ala Val
   705                 710                 715                 720
   Leu Leu Lys Ala Gly Met Thr Val Lys Gln Ala Ile Val Tyr Asn Leu
                   725                 730                 735
   Leu Ser Ala Met Met Ala Tyr Ile Gly Met Leu Ile Gly Thr Ala Val
               740                 745                 750
   Gly Gln Tyr Ala Asn Asn Ile Thr Leu Trp Ile Phe Ala Val Thr Ala
           755                 760                 765
   Gly Met Phe Leu Tyr Val Ala Leu Val Asp Met Leu Pro Glu Met Leu
       770                 775                 780
   His Gly Asp Gly Asp Asn Glu Glu His Gly Phe Cys Pro Val Gly Gln
   785                 790                 795                 800
   Phe Ile Leu Gln Asn Leu Gly Leu Leu Phe Gly Phe Ala Ile Met Leu
                   805                 810                 815
   Val Ile Ala Leu Tyr Glu Asp Lys Ile Val Phe Asp Ile Gln Phe
               820                 825                 830
<210> 6
   <211> 831
   <212> PRT
   <213> Homo sapiens
<400> 6
   Met Lys Val His Met His Thr Lys Phe Cys Leu Ile Cys Leu Leu Thr
   1               5                   10                  15
   Phe Ile Phe His His Cys Asn His Cys His Glu Glu His Asp His Gly
               20                  25                  30
   Pro Glu Ala Leu His Arg Gln His Arg Gly Met Thr Glu Leu Glu Pro
           35                  40                  45
   Ser Lys Phe Ser Lys Gln Ala Ala Glu Asn Glu Lys Lys Tyr Tyr Ile
       50                  55                  60
   Glu Lys Leu Phe Glu Arg Tyr Gly Glu Asn Gly Arg Leu Ser Phe Phe
   65                  70                  75                  80
   Gly Leu Glu Lys Leu Leu Thr Asn Leu Gly Leu Gly Glu Arg Lys Val
                   85                  90                  95
   Val Glu Ile Asn His Glu Asp Leu Gly His Asp His Val Ser His Leu
               100                 105                 110
   Asp Ile Leu Ala Val Gln Glu Gly Lys His Phe His Ser His Asn His
           115                 120                 125
   Gln His Ser His Asn His Leu Asn Ser Glu Asn Gln Thr Val Thr Ser
       130                 135                 140
   Val Ser Thr Lys Arg Asn His Lys Cys Asp Pro Glu Lys Glu Thr Val
   145                 150                 155                 160
   Glu Val Ser Val Lys Ser Asp Asp Lys His Met His Asp His Asn His
                   165                 170                 175
   Arg Leu Arg His His His Arg Leu His His His Leu Asp His Asn Asn
               180                 185                 190
   Thr His His Phe His Asn Asp Ser Ile Thr Pro Ser Glu Arg Gly Glu
           195                 200                 205
   Pro Ser Asn Glu Pro Ser Thr Glu Thr Asn Lys Thr Gln Glu Gln Ser
       210                 215                 220
   Asp Val Lys Leu Pro Lys Gly Lys Arg Lys Lys Lys Gly Arg Lys Ser
   225                 230                 235                 240
   Asn Glu Asn Ser Glu Val Ile Thr Pro Gly Phe Pro Pro Asn His Asp
                   245                 250                 255
   Gln Gly Glu Gln Tyr Glu His Asn Arg Val His Lys Pro Asp Arg Val
               260                 265                 270
   His Asn Pro Gly His Ser His Val His Leu Pro Glu Arg Asn Gly His
           275                 280                 285
   Asp Pro Gly Arg Gly His Gln Asp Leu Asp Pro Asp Asn Glu Gly Glu
       290                 295                 300
   Leu Arg His Thr Arg Lys Arg Glu Ala Pro His Val Lys Asn Asn Ala
   305                 310                 315                 320
   Ile Ile Ser Leu Arg Lys Asp Leu Asn Glu Asp Asp His His His Glu
                   325                 330                 335
   Cys Leu Asn Val Thr Gln Leu Leu Lys Tyr Tyr Gly His Gly Ala Asn
               340                 345                 350
   Ser Pro Ile Ser Thr Asp Leu Phe Thr Tyr Leu Cys Pro Ala Leu Leu
           355                 360                 365
   Tyr Gln Ile Asp Ser Arg Leu Cys Ile Glu His Phe Asp Lys Leu Leu
       370                 375                 380
   Val Glu Asp Ile Asn Lys Asp Lys Asn Leu Val Pro Glu Asp Glu Ala
   385                 390                 395                 400
   Asn Ile Gly Ala Ser Ala Trp Ile Cys Gly Ile Ile Ser Ile Thr Val
                   405                 410                 415
   Ile Ser Leu Leu Ser Leu Leu Gly Val Ile Leu Val Pro Ile Ile Asn
               420                 425                 430
   Gln Gly Cys Phe Lys Phe Leu Leu Thr Phe Leu Val Ala Leu Ala Val
           435                 440                 445
   Gly Thr Met Ser Gly Asp Ala Leu Leu His Leu Leu Pro His Ser Gln
       450                 455                 460
   Gly Gly His Asp His Ser His Gln His Ala His Gly His Gly His Ser
   465                 470                 475                 480
   His Gly His Glu Ser Asn Lys Phe Leu Glu Glu Tyr Asp Ala Val Leu
                   485                 490                 495
   Lys Gly Leu Val Ala Leu Gly Gly Ile Tyr Leu Leu Phe Ile Ile Glu
               500                 505                 510
   His Cys Ile Arg Met Phe Lys His Tyr Lys Gln Gln Arg Gly Lys Gln
           515                 520                 525
   Lys Trp Phe Met Lys Gln Asn Thr Glu Glu Ser Thr Ile Gly Arg Lys
       530                 535                 540
   Leu Ser Asp His Lys Leu Asn Asn Thr Pro Asp Ser Asp Trp Leu Gln
   545                 550                 555                 560
   Leu Lys Pro Leu Ala Gly Thr Asp Asp Ser Val Val Ser Glu Asp Arg
                   565                 570                 575
   Leu Asn Glu Thr Glu Leu Thr Asp Leu Glu Gly Gln Gln Glu Ser Pro
               580                 585                 590
   Pro Lys Asn Tyr Leu Cys Ile Glu Glu Glu Lys Ile Ile Asp His Ser
           595                 600                 605
   His Ser Asp Gly Leu His Thr Ile His Glu His Asp Leu His Ala Ala
       610                 615                 620
   Ala His Asn His His Gly Glu Asn Lys Thr Val Leu Arg Lys His Asn
   625                 630                 635                 640
   His Gln Trp His His Lys His Ser His His Ser His Gly Pro Cys His
                   645                 650                 655
   Ser Gly Ser Asp Leu Lys Glu Thr Gly Ile Ala Asn Ile Ala Trp Met
               660                 665                 670
   Val Ile Met Gly Asp Gly Ile His Asn Phe Ser Asp Gly Leu Ala Ile
           675                 680                 685
   Gly Ala Ala Phe Ser Ala Gly Leu Thr Gly Gly Ile Ser Thr Ser Ile
       690                 695                 700
   Ala Val Phe Cys His Glu Leu Pro His Glu Leu Gly Asp Phe Ala Val
   705                 710                 715                 720
   Leu Leu Lys Ala Gly Met Thr Val Lys Gln Ala Ile Val Tyr Asn Leu
                   725                 730                 735
   Leu Ser Ala Met Met Ala Tyr Ile Gly Met Leu Ile Gly Thr Ala Val
               740                 745                 750
   Gly Gln Tyr Ala Asn Asn Ile Thr Leu Trp Ile Phe Ala Val Thr Ala
           755                 760                 765
   Gly Met Phe Leu Tyr Val Ala Leu Val Asp Met Leu Pro Glu Met Leu
       770                 775                 780
   His Gly Asp Gly Asp Asn Glu Glu His Gly Phe Cys Pro Val Gly Gln
   785                 790                 795                 800
   Phe Ile Leu Gln Asn Leu Gly Leu Leu Phe Gly Phe Ala Ile Met Leu
                   805                 810                 815
   Val Ile Ala Leu Tyr Glu Asp Lys Ile Val Phe Asp Ile Gln Phe
               820                 825                 830
<210> 7
   <211> 5227
   <212> DNA
   <213> Homo sapiens
<400> 7
   cacgatttgg tgcagccggg gtttggtacc gagcggagag gagatgcaca cggcactcga      60
   gtgtgaggaa aaatagaaat gaaggtacat atgcacacaa aattttgcct catttgtttg     120
   ctgacattta tttttcatca ttgcaaccat tgccatgaag aacatgacca tggccctgaa     180
   gcgcttcaca gacagcatcg tggaatgaca gaattggagc caagcaaatt ttcaaagcaa     240
   gctgctgaaa atgaaaaaaa atactatatt gaaaaacttt ttgagcgtta tggtgaaaat     300
   ggaagattat ccttttttgg tttggagaaa cttttaacaa acttgggcct tggagagaga     360
   aaagtagttg agattaatca tgaggatctt ggccacgatc atgtttctca tttagatatt     420
   ttggcagttc aagagggaaa gcattttcac tcacataacc accagcattc ccataatcat     480
   ttaaattcag aaaatcaaac tgtgaccagt gtatccacaa aaagaaacca taaatgtgat     540
   ccagagaaag agacagttga agtgtctgta aaatctgatg ataaacatat gcatgaccat     600
   aatcaccgcc tacgtcatca ccatcgtttg catcatcatc ttgatcataa caacactcac     660
   cattttcata atgattccat tactcccagt gagcgtgggg agcctagcaa tgaaccttca     720
   acagagacca ataaaaccca ggaacaatct gatgttaaac taccgaaagg aaagaggaag     780
   aaaaaaggga ggaaaagtaa tgaaaattct gaggttatta caccaggttt tccccctaac     840
   catgatcagg gtgaacagta tgagcataat cgggtccaca aacctgatcg tgtacataac     900
   ccaggtcatt ctcatgtaca tcttccagaa cgtaatggtc atgatcctgg tcgtggacac     960
   caagatcttg atcctgataa tgaaggtgaa cttcgacata ctagaaagag agaagcacca    1020
   catgttaaaa ataatgcaat aatttctttg agaaaagatc taaatgaaga tgaccatcat    1080
   catgaatgtt tgaacgtcac tcagttatta aaatactatg gtcatggtgc caactctccc    1140
   atctcaactg atttatttac atacctttgc cctgcattgt tatatcaaat cgacagcaga    1200
   ctttgtattg agcattttga caaactttta gttgaagata taaataagga taaaaacctg    1260
   gttcctgaag atgaggcaaa tataggggca tcagcctgga tttgtggtat catttctatc    1320
   actgtcatta gcctgctttc cttgctaggc gtgatcttgg ttcctatcat taaccaagga   1380
   tgcttcaaat tccttcttac attccttgtt gcattagctg taggaacaat gagtggagac   1440
   gcccttcttc atctactgcc ccattctcag ggtggacatg atcacagtca ccaacatgca   1500
   catgggcatg gacattctca tggacatgaa tctaacaagt ttttggaaga atatgatgct   1560
   gtattgaaag gacttgttgc tctaggaggc atttacttgc tatttatcat tgaacactgc   1620
   attagaatgt ttaagcacta caaacaacaa agaggaaaac agaaatggtt tatgaaacag   1680
   aacacagaag aatcaactat tggaagaaag ctttcagatc acaagttaaa caatacacca   1740
   gattctgact ggcttcaact caagcctctt gccggaactg atgactcggt tgtttctgaa   1800
   gatcgactta atgaaactga actgacagat ttagaaggcc aacaagaatc ccctcctaaa   1860
   aattaccttt gtatagaaga ggagaaaatc atagaccatt ctcacagtga tggattacat   1920
   accattcatg agcatgatct ccatgctgct gcacataacc accacggcga gaacaaaact   1980
   gtgctgagga agcataatca ccagtggcac cacaagcatt ctcatcattc ccatggcccc   2040
   tgtcattctg gatccgatct gaaagaaaca ggaatagcta atatagcctg gatggtgatc   2100
   atgggggatg gcatccacaa cttcagtgat gggctcgcaa ttggtgcagc tttcagtgct   2160
   ggattgacag gaggaatcag tacttctata gccgtcttct gtcatgaact gccacatgaa   2220
   ttaggagatt ttgcagttct tcttaaagca ggcatgactg taaagcaagc aattgtatac   2280
   aacctcctct ctgccatgat ggcttacata ggcatgctca taggcacagc tgttggtcag   2340
   tatgccaata acatcacact ttggatcttt gcagtcactg caggcatgtt cctctatgta   2400
   gccttggtgg atatgcttcc agaaatgttg catggtgatg gtgacaatga agaacatggc   2460
   ttttgtcctg tggggcaatt catccttcag aatttaggat tgctctttgg atttgccatt   2520
   atgctggtga ttgccctcta tgaagataaa attgtgtttg acatccagtt ttgacctttc   2580
   ccagtaatca ctgttgatta cgagaatgtt accatgcagc tttgcatctg ttccttgtac   2640
   tgtatgcaca ttgctcaaag gaaagtcagt ggcttgcact acttacaagt ttcatagatt   2700
   tgagcctaac cacaagaggc tggtgcttag tactgttttc cctgcacgta ggggtctttt   2760
   aaaaatataa agcttgtgat aaagagagga gaatatggga ctccatgaac cagtgttgat   2820
   atgtttgatt aagacttttc acaaaataat catataaaac actagtctct ttattagtag   2880
   aaacttctgt ggctatgcag aaatagagat cgaaccaaaa aaaatcattt aaactttaaa   2940
   aatattttaa atggactttg gggagacatt ttttgtgtgt tttaagaatg aattgtagtg   3000
   ctctttaatt cagctacata tattcatgtg gtgataggga tcaacttgac acaactttga   3060
   aactgcataa agtagacata ggaactagag gaaagctcag gctgcattag agtatgaatt   3120
   tagcattggg aaaagccctt attcttgaat ctagagttac tatttttgta tatatttgca   3180
   tagtgtttaa acctgcagcc taaactactg aaatttgtga ttgtatgttt gtgtgagctt 3240
   cagtttaatg aaagattcat aatggttctt tgtattatta taatacttgg tgttggggtg 3300
   ttctttctgt tttgtttttt actttaattt tgttttgatt tttttttttt ttttttggcg 3360
   ggggtaggtg agggtttgga gcatgtggtc tttttaaaaa attgtaaccc tctagaaaat 3420
   atcaaagaaa tgaaccagac gtggtttaaa tagttgattt tcctatttta acagtaccaa 3480
   ctagttaatt gggaaatgta agttctgaat gttcacattg ctttaccagt ttggcactgg 3540
   aaccaagagc acatgtcgtg gctggctaca aggttgtaaa gcagaaaatc gaagtttacc 3600
   atgtctgtaa tgtgtacatg aagtgtcaat ttagaacagt tactaggata aactccatta 3660
   ttgccatggc tgtcatggta cccaagtgac ttggaagatg catttaaatt actcagctga 3720
   aatcacttga tcatcttgtg ccaagatatg ctgttggtgc ctgataggga ttagtctttt 3780
   aggtgccctg ttctcctacc ataattgtga atgatttgtg agaagtgcaa gccatgttta 3840
   tcctgaattt ttacttaata atttgtatta ctagtcatat gcatgtagct ttctgtttac 3900
   atcctatgcc acatggtctt catttatgcc aggtaaactg tatttgaact atgtgcagct 3960
   agctttgttt taatctgctt ggcaaccagt gtagctgctg taacaatcta tcttattgtt 4020
   caaatatata agagccaaac tcttttccat tccatctaaa atgttttcat ttagtactct 4080
   tctttcctcc tactctatga acttcaaaac aaaaacaaaa ctttgagagc agcacatgca 4140
   tccaggtatt tatagattat tgccagtgtc ttttctgtat gctataagca agggagctta 4200
   ggtgttattt ctttaattta tgcttgaatc tgaaaaatta tttctgactt actccatggc 4260
   ctccttataa taagtagaag ttttatatat aattaatttt cagcattggg cactgaatta 4320
   ggacagtcct catctcattg cttggccctt caagcaacct agctaaaagg tgctgatatt 4380
   ttatttagta ctgccaactt caagtgattt agatatctat ctatctagat ttctgaacca 4440
   agatatattt atagttcact tttgggtttt tatacccacg gtaggattct gcattccagc 4500
   attaaatctg cttcatttta gaacctttat aaaagcaata gctggaatat actcccagtt 4560
   ttaaaataaa tgcctgattg atttaaagca agtaggttat gctgaagtat ataaagaagt 4620
   tttatattct ctcaaaaatg gtattatctt tctttatttg ctagattctt acaaatcttt 4680
   taagagggct gtaacagttg ctgctagtat tagggttcca catcattcta atgtatagtt 4740
   tcaagtctta atagacaatc tgaattccac tacatttctt ttggctccaa cattcctttt 4800
   agcttgacca gtctaattta aaatgtgttt gttggaggtc attaacgtta cttgtacaat 4860
   gctgtcactg tgtgacatcc atatgaattt tggtatatat caatcaatca atcaatcaca 4920
   ttgcattcaa tcaatcagct gtgattgatt gattatgctt agaaatacta tagtaactag 4980
   atgcagtgtg aattttttcc attaacaaac aaacaagtca gtggcttaaa tgtgattatg 5040
   gtcctgcaag gtgattcttg ctaaaatatc taaacttttg ttttgtttta actgaatcat 5100
   tttttaactt aaaaagctgg aaaatatcaa atgctgtttt ttttttttca ttgtcaacag 5160
   tggtgtgtca ttttatgtat gttcctaatg cttatggaac tcctccaaaa taaagttact 5220
   caaagag 5227
<210> 8
   <211> 5432
   <212> DNA
   <213> Homo sapiens
<400> 8
   agttgatcac tctgaagctt tttggctaaa gcgtttgggt ttagagcttc cattactcat     60
   tcgccttgcc caaggcctca gcaaccgacg ttcgaaagcc aggagaaaag gcgaatgata    120
   aagggcgctc cacgcatgcg ttaagaagcc gccccaactc ccccgcggcg ttctttcttg    180
   gaacaaaact agcgcggagc cacggaactc cgcagtttgc gtagacttga atttcctatt    240
   cctcggacga tccatgtgga atccgaaaaa tagaaatgaa ggtacatatg cacacaaaat    300
   tttgcctcat ttgtttgctg acatttattt ttcatcattg caaccattgc catgaagaac    360
   atgaccatgg ccctgaagcg cttcacagac agcatcgtgg aatgacagaa ttggagccaa    420
   gcaaattttc aaagcaagct gctgaaaatg aaaaaaaata ctatattgaa aaactttttg    480
   agcgttatgg tgaaaatgga agattatcct tttttggttt ggagaaactt ttaacaaact    540
   tgggccttgg agagagaaaa gtagttgaga ttaatcatga ggatcttggc cacgatcatg    600
   tttctcattt agatattttg gcagttcaag agggaaagca ttttcactca cataaccacc    660
   agcattccca taatcattta aattcagaaa atcaaactgt gaccagtgta tccacaaaaa    720
   gaaaccataa atgtgatcca gagaaagaga cagttgaagt gtctgtaaaa tctgatgata    780
   aacatatgca tgaccataat caccgcctac gtcatcacca tcgtttgcat catcatcttg    840
   atcataacaa cactcaccat tttcataatg attccattac tcccagtgag cgtggggagc    900
   ctagcaatga accttcaaca gagaccaata aaacccagga acaatctgat gttaaactac    960
   cgaaaggaaa gaggaagaaa aaagggagga aaagtaatga aaattctgag gttattacac   1020
   caggttttcc ccctaaccat gatcagggtg aacagtatga gcataatcgg gtccacaaac   1080
   ctgatcgtgt acataaccca ggtcattctc atgtacatct tccagaacgt aatggtcatg   1140
   atcctggtcg tggacaccaa gatcttgatc ctgataatga aggtgaactt cgacatacta   1200
   gaaagagaga agcaccacat gttaaaaata atgcaataat ttctttgaga aaagatctaa   1260
   atgaagatga ccatcatcat gaatgtttga acgtcactca gttattaaaa tactatggtc   1320
   atggtgccaa ctctcccatc tcaactgatt tatttacata cctttgccct gcattgttat   1380
   atcaaatcga cagcagactt tgtattgagc attttgacaa acttttagtt gaagatataa   1440
   ataaggataa aaacctggtt cctgaagatg aggcaaatat aggggcatca gcctggattt   1500
   gtggtatcat ttctatcact gtcattagcc tgctttcctt gctaggcgtg atcttggttc   1560
   ctatcattaa ccaaggatgc ttcaaattcc ttcttacatt ccttgttgca ttagctgtag   1620
   gaacaatgag tggagacgcc cttcttcatc tactgcccca ttctcagggt ggacatgatc   1680
   acagtcacca acatgcacat gggcatggac attctcatgg acatgaatct aacaagtttt   1740
   tggaagaata tgatgctgta ttgaaaggac ttgttgctct aggaggcatt tacttgctat   1800
   ttatcattga acactgcatt agaatgttta agcactacaa acaacaaaga ggaaaacaga   1860
   aatggtttat gaaacagaac acagaagaat caactattgg aagaaagctt tcagatcaca   1920
   agttaaacaa tacaccagat tctgactggc ttcaactcaa gcctcttgcc ggaactgatg   1980
   actcggttgt ttctgaagat cgacttaatg aaactgaact gacagattta gaaggccaac   2040
   aagaatcccc tcctaaaaat tacctttgta tagaagagga gaaaatcata gaccattctc   2100
   acagtgatgg attacatacc attcatgagc atgatctcca tgctgctgca cataaccacc   2160
   acggcgagaa caaaactgtg ctgaggaagc ataatcacca gtggcaccac aagcattctc   2220
   atcattccca tggcccctgt cattctggat ccgatctgaa agaaacagga atagctaata   2280
   tagcctggat ggtgatcatg ggggatggca tccacaactt cagtgatggg ctcgcaattg   2340
   gtgcagcttt cagtgctgga ttgacaggag gaatcagtac ttctatagcc gtcttctgtc   2400
   atgaactgcc acatgaatta ggagattttg cagttcttct taaagcaggc atgactgtaa   2460
   agcaagcaat tgtatacaac ctcctctctg ccatgatggc ttacataggc atgctcatag   2520
   gcacagctgt tggtcagtat gccaataaca tcacactttg gatctttgca gtcactgcag   2580
   gcatgttcct ctatgtagcc ttggtggata tgcttccaga aatgttgcat ggtgatggtg   2640
   acaatgaaga acatggcttt tgtcctgtgg ggcaattcat ccttcagaat ttaggattgc   2700
   tctttggatt tgccattatg ctggtgattg ccctctatga agataaaatt gtgtttgaca   2760
   tccagttttg acctttccca gtaatcactg ttgattacga gaatgttacc atgcagcttt   2820
   gcatctgttc cttgtactgt atgcacattg ctcaaaggaa agtcagtggc ttgcactact   2880
   tacaagtttc atagatttga gcctaaccac aagaggctgg tgcttagtac tgttttccct   2940
   gcacgtaggg gtcttttaaa aatataaagc ttgtgataaa gagaggagaa tatgggactc   3000
   catgaaccag tgttgatatg tttgattaag acttttcaca aaataatcat ataaaacact   3060
   agtctcttta ttagtagaaa cttctgtggc tatgcagaaa tagagatcga accaaaaaaa   3120
   atcatttaaa ctttaaaaat attttaaatg gactttgggg agacattttt tgtgtgtttt   3180
   aagaatgaat tgtagtgctc tttaattcag ctacatatat tcatgtggtg atagggatca   3240
   acttgacaca actttgaaac tgcataaagt agacatagga actagaggaa agctcaggct   3300
   gcattagagt atgaatttag cattgggaaa agcccttatt cttgaatcta gagttactat 3360
   ttttgtatat atttgcatag tgtttaaacc tgcagcctaa actactgaaa tttgtgattg 3420
   tatgtttgtg tgagcttcag tttaatgaaa gattcataat ggttctttgt attattataa 3480
   tacttggtgt tggggtgttc tttctgtttt gttttttact ttaattttgt tttgattttt 3540
   tttttttttt tttggcgggg gtaggtgagg gtttggagca tgtggtcttt ttaaaaaatt 3600
   gtaaccctct agaaaatatc aaagaaatga accagacgtg gtttaaatag ttgattttcc 3660
   tattttaaca gtaccaacta gttaattggg aaatgtaagt tctgaatgtt cacattgctt 3720
   taccagtttg gcactggaac caagagcaca tgtcgtggct ggctacaagg ttgtaaagca 3780
   gaaaatcgaa gtttaccatg tctgtaatgt gtacatgaag tgtcaattta gaacagttac 3840
   taggataaac tccattattg ccatggctgt catggtaccc aagtgacttg gaagatgcat 3900
   ttaaattact cagctgaaat cacttgatca tcttgtgcca agatatgctg ttggtgcctg 3960
   atagggatta gtcttttagg tgccctgttc tcctaccata attgtgaatg atttgtgaga 4020
   agtgcaagcc atgtttatcc tgaattttta cttaataatt tgtattacta gtcatatgca 4080
   tgtagctttc tgtttacatc ctatgccaca tggtcttcat ttatgccagg taaactgtat 4140
   ttgaactatg tgcagctagc tttgttttaa tctgcttggc aaccagtgta gctgctgtaa 4200
   caatctatct tattgttcaa atatataaga gccaaactct tttccattcc atctaaaatg 4260
   ttttcattta gtactcttct ttcctcctac tctatgaact tcaaaacaaa aacaaaactt 4320
   tgagagcagc acatgcatcc aggtatttat agattattgc cagtgtcttt tctgtatgct 4380
   ataagcaagg gagcttaggt gttatttctt taatttatgc ttgaatctga aaaattattt 4440
   ctgacttact ccatggcctc cttataataa gtagaagttt tatatataat taattttcag 4500
   cattgggcac tgaattagga cagtcctcat ctcattgctt ggcccttcaa gcaacctagc 4560
   taaaaggtgc tgatatttta tttagtactg ccaacttcaa gtgatttaga tatctatcta 4620
   tctagatttc tgaaccaaga tatatttata gttcactttt gggtttttat acccacggta 4680
   ggattctgca ttccagcatt aaatctgctt cattttagaa cctttataaa agcaatagct 4740
   ggaatatact cccagtttta aaataaatgc ctgattgatt taaagcaagt aggttatgct 4800
   gaagtatata aagaagtttt atattctctc aaaaatggta ttatctttct ttatttgcta 4860
   gattcttaca aatcttttaa gagggctgta acagttgctg ctagtattag ggttccacat 4920
   cattctaatg tatagtttca agtcttaata gacaatctga attccactac atttcttttg 4980
   gctccaacat tccttttagc ttgaccagtc taatttaaaa tgtgtttgtt ggaggtcatt 5040
   aacgttactt gtacaatgct gtcactgtgt gacatccata tgaattttgg tatatatcaa 5100
   tcaatcaatc aatcacattg cattcaatca atcagctgtg attgattgat tatgcttaga 5160
   aatactatag taactagatg cagtgtgaat tttttccatt aacaaacaaa caagtcagtg   5220
   gcttaaatgt gattatggtc ctgcaaggtg attcttgcta aaatatctaa acttttgttt   5280
   tgttttaact gaatcatttt ttaacttaaa aagctggaaa atatcaaatg ctgttttttt   5340
   tttttcattg tcaacagtgg tgtgtcattt tatgtatgtt cctaatgctt atggaactcc   5400
   tccaaaataa agttactcaa agagagcaaa ta                                 5432
<210> 9
   <211> 752
   <212> PRT
   <213> Homo sapiens
<400> 9
   Met Ala Glu Pro Gln Ala Glu Ser Glu Pro Leu Leu Gly Gly Ala Arg
   1               5                   10                  15
   Gly Gly Gly Gly Asp Trp Pro Ala Gly Leu Thr Thr Tyr Arg Ser Ile
               20                  25                  30
   Gln Val Gly Pro Gly Ala Ala Ala Arg Trp Asp Leu Cys Ile Asp Gln
           35                  40                  45
   Ala Val Val Phe Ile Glu Asp Ala Ile Gln Tyr Arg Ser Ile Asn His
       50                  55                  60
   Arg Val Asp Ala Ser Ser Met Trp Leu Tyr Arg Arg Tyr Tyr Ser Asn
   65                  70                  75                  80
   Val Cys Gln Arg Thr Leu Ser Phe Thr Ile Phe Leu Ile Leu Phe Leu
                   85                  90                  95
   Ala Phe Ile Glu Thr Pro Ser Ser Leu Thr Ser Thr Ala Asp Val Arg
               100                 105                 110
   Tyr Arg Ala Ala Pro Trp Glu Pro Pro Cys Gly Leu Thr Glu Ser Val
           115                 120                 125
   Glu Val Leu Cys Leu Leu Val Phe Ala Ala Asp Leu Ser Val Lys Gly
       130                 135                 140
   Tyr Leu Phe Gly Trp Ala His Phe Gln Lys Asn Leu Trp Leu Leu Gly
   145                 150                 155                 160
   Tyr Leu Val Val Leu Val Val Ser Leu Val Asp Trp Thr Val Ser Leu
                   165                 170                 175
   Ser Leu Val Cys His Glu Pro Leu Arg Ile Arg Arg Leu Leu Arg Pro
               180                 185                 190
   Phe Phe Leu Leu Gln Asn Ser Ser Met Met Lys Lys Thr Leu Lys Cys
           195                 200                 205
   Ile Arg Trp Ser Leu Pro Glu Met Ala Ser Val Gly Leu Leu Leu Ala
       210                 215                 220
   Ile His Leu Cys Leu Phe Thr Met Phe Gly Met Leu Leu Phe Ala Gly
   225                 230                 235                 240
   Gly Lys Gln Asp Asp Gly Gln Asp Arg Glu Arg Leu Thr Tyr Phe Gln
                   245                 250                 255
   Asn Leu Pro Glu Ser Leu Thr Ser Leu Leu Val Leu Leu Thr Thr Ala
               260                 265                 270
   Asn Asn Pro Asp Val Met Ile Pro Ala Tyr Ser Lys Asn Arg Ala Tyr
           275                 280                 285
   Ala Ile Phe Phe Ile Val Phe Thr Val Ile Gly Ser Leu Phe Leu Met
       290                 295                 300
   Asn Leu Leu Thr Ala Ile Ile Tyr Ser Gln Phe Arg Gly Tyr Leu Met
   305                 310                 315                 320
   Lys Ser Leu Gln Thr Ser Leu Phe Arg Arg Arg Leu Gly Thr Arg Ala
                   325                 330                 335
   Ala Phe Glu Val Leu Ser Ser Met Val Gly Glu Gly Gly Ala Phe Pro
               340                 345                 350
   Gln Ala Val Gly Val Lys Pro Gln Asn Leu Leu Gln Val Leu Gln Lys
           355                 360                 365
   Val Gln Leu Asp Ser Ser His Lys Gln Ala Met Met Glu Lys Val Arg
       370                 375                 380
   Ser Tyr Gly Ser Val Leu Leu Ser Ala Glu Glu Phe Gln Lys Leu Phe
   385                 390                 395                 400
   Asn Glu Leu Asp Arg Ser Val Val Lys Glu His Pro Pro Arg Pro Glu
                   405                 410                 415
   Tyr Gln Ser Pro Phe Leu Gln Ser Ala Gln Phe Leu Phe Gly His Tyr
               420                 425                 430
   Tyr Phe Asp Tyr Leu Gly Asn Leu Ile Ala Leu Ala Asn Leu Val Ser
             435                 440                 445
   Ile Cys Val Phe Leu Val Leu Asp Ala Asp Val Leu Pro Ala Glu Arg
       450                 455                 460
   Asp Asp Phe Ile Leu Gly Ile Leu Asn Cys Val Phe Ile Val Tyr Tyr
   465                 470                 475                 480
   Leu Leu Glu Met Leu Leu Lys Val Phe Ala Leu Gly Leu Arg Gly Tyr
                   485                 490                 495
   Leu Ser Tyr Pro Ser Asn Val Phe Asp Gly Leu Leu Thr Val Val Leu
               500                 505                 510
   Leu Val Leu Glu Ile Ser Thr Leu Ala Val Tyr Arg Leu Pro His Pro
           515                 520                 525
   Gly Trp Arg Pro Glu Met Val Gly Leu Leu Ser Leu Trp Asp Met Thr
       530                 535                 540
   Arg Met Leu Asn Met Leu Ile Val Phe Arg Phe Leu Arg Ile Ile Pro
   545                 550                 555                 560
   Ser Met Lys Leu Met Ala Val Val Ala Ser Thr Val Leu Gly Leu Val
                   565                 570                 575
   Gln Asn Met Arg Ala Phe Gly Gly Ile Leu Val Val Val Tyr Tyr Val
               580                 585                 590
   Phe Ala Ile Ile Gly Ile Asn Leu Phe Arg Gly Val Ile Val Ala Leu
           595                 600                 605
   Pro Gly Asn Ser Ser Leu Ala Pro Ala Asn Gly Ser Ala Pro Cys Gly
       610                 615                 620
   Ser Phe Glu Gln Leu Glu Tyr Trp Ala Asn Asn Phe Asp Asp Phe Ala
   625                 630                 635                 640
   Ala Ala Leu Val Thr Leu Trp Asn Leu Met Val Val Asn Asn Trp Gln
                   645                 650                 655
   Val Phe Leu Asp Ala Tyr Arg Arg Tyr Ser Gly Pro Trp Ser Lys Ile
               660                 665                 670
   Tyr Phe Val Leu Trp Trp Leu Val Ser Ser Val Ile Trp Val Asn Leu
           675                 680                 685
   Phe Leu Ala Leu Ile Leu Glu Asn Phe Leu His Lys Trp Asp Pro Arg
       690                 695                 700
   Ser His Leu Gln Pro Leu Ala Gly Thr Pro Glu Ala Thr Tyr Gln Met
   705                 710                 715                 720
   Thr Val Glu Leu Leu Phe Arg Asp Ile Leu Glu Glu Pro Gly Glu Asp
                   725                 730                 735
   Glu Leu Thr Glu Arg Leu Ser Gln His Pro His Leu Trp Leu Cys Arg
               740                 745                 750
<210> 10
   <211> 538
   <212> PRT
   <213> Homo sapiens
<400> 10
   Met Ala Glu Pro Gln Ala Glu Ser Glu Pro Leu Leu Gly Gly Ala Arg
   1               5                   10                  15
   Gly Gly Gly Gly Asp Trp Pro Ala Gly Leu Thr Thr Tyr Arg Ser Ile
               20                  25                  30
   Gln Val Gly Pro Gly Ala Ala Ala Arg Trp Asp Leu Cys Ile Asp Gln
           35                  40                  45
   Ala Val Val Phe Ile Glu Asp Ala Ile Gln Tyr Arg Ser Ile Asn His
       50                  55                  60
   Arg Val Asp Ala Ser Ser Met Trp Leu Tyr Arg Arg Tyr Tyr Ser Asn
   65                  70                  75                  80
   Val Cys Gln Arg Thr Leu Ser Phe Thr Ile Phe Leu Ile Leu Phe Leu
                   85                  90                  95
   Ala Phe Ile Glu Thr Pro Ser Ser Leu Thr Ser Thr Ala Asp Val Arg
               100                 105                 110
   Tyr Arg Ala Ala Pro Trp Glu Pro Pro Cys Gly Leu Thr Glu Ser Val
           115                 120                 125
   Glu Val Leu Cys Leu Leu Val Phe Ala Ala Asp Leu Ser Val Lys Gly
       130                 135                 140
   Tyr Leu Phe Gly Trp Ala His Phe Gln Lys Asn Leu Trp Leu Leu Gly
   145                 150                 155                 160
   Tyr Leu Val Val Leu Val Val Ser Leu Val Asp Trp Thr Val Ser Leu
                   165                 170                 175
   Ser Leu Val Cys His Glu Pro Leu Arg Ile Arg Arg Leu Leu Arg Pro
               180                 185                 190
   Phe Phe Leu Leu Gln Asn Ser Ser Met Met Lys Lys Thr Leu Lys Cys
           195                 200                 205
   Ile Arg Trp Ser Leu Pro Glu Met Ala Ser Val Gly Leu Leu Leu Ala
       210                 215                 220
   Ile His Leu Cys Leu Phe Thr Met Phe Gly Met Leu Leu Phe Ala Gly
   225                 230                 235                 240
   Gly Lys Gln Asp Asp Gly Gln Asp Arg Glu Arg Leu Thr Tyr Phe Gln
                   245                 250                 255
   Asn Leu Pro Glu Ser Leu Thr Ser Leu Leu Val Leu Leu Thr Thr Ala
               260                 265                 270
   Asn Asn Pro Asp Val Met Ile Pro Ala Tyr Ser Lys Asn Arg Ala Tyr
           275                 280                 285
   Ala Ile Phe Phe Ile Val Phe Thr Val Ile Gly Ser Leu Phe Leu Met
       290                 295                 300
   Asn Leu Leu Thr Ala Ile Ile Tyr Ser Gln Phe Arg Gly Tyr Leu Met
   305                 310                 315                 320
   Lys Ser Leu Gln Thr Ser Leu Phe Arg Arg Arg Leu Gly Thr Arg Ala
                   325                 330                 335
   Ala Phe Glu Val Leu Ser Ser Met Val Gly Glu Gly Gly Ala Phe Pro
               340                 345                 350
   Gln Ala Thr Arg Arg Gly Pro Ser Thr Ser Leu Arg Phe Cys Arg Ala
           355                 360                 365
   Pro Ser Ser Ser Ser Ala Thr Thr Thr Leu Thr Thr Trp Gly Thr Ser
       370                 375                 380
   Ser Pro Trp Gln Thr Trp Cys Pro Phe Ala Cys Ser Trp Cys Trp Met
   385                 390                 395                 400
   Gln Met Cys Cys Leu Leu Ser Val Met Thr Ser Ser Trp Gly Phe Ser
                   405                 410                 415
   Thr Ala Ser Ser Leu Cys Thr Thr Cys Trp Arg Cys Cys Ser Arg Ser
               420                 425                 430
   Leu Pro Trp Ala Cys Glu Gly Thr Cys Pro Thr Pro Ala Thr Cys Leu
           435                 440                 445
   Thr Gly Ser Ser Pro Leu Ser Cys Trp Arg Pro Glu Met Val Gly Leu
       450                 455                 460
   Leu Ser Leu Trp Asp Met Thr Arg Met Leu Asn Met Leu Ile Val Phe
   465                 470                 475                 480
   Arg Phe Leu Arg Ile Ile Pro Ser Met Lys Leu Met Ala Val Val Ala
                   485                 490                 495
   Ser Thr Val Leu Gly Leu Val Gln Asn Met Arg Ala Phe Gly Gly Ile
               500                 505                 510
   Leu Val Pro Gly Pro Cys Gln Trp Leu Gly Ala Leu Trp Glu Leu Arg
           515                 520                 525
   Ala Ala Gly Val Leu Gly Gln Gln Leu Arg
       530                 535
<210> 11
   <211> 5026
   <212> DNA
   <213> Homo sapiens
<400> 11
   tgccgtgcgc gcgtcagcaa aaacgccagg gacggggtct ccgcgcctgc gcagtgaagc      60
   tgggcgcctt cggggcttga gcttctgagg gtcgggtcca gcgcgtgggc tgctggatgg     120
   cggaacccca ggcggagtcg gagcccctgc tgggcggggc ccgcggcggt ggcggcgact     180
   ggccggcggg gctgaccact taccgcagca tccaagtcgg ccctggtgcc gcggccaggt     240
   gggacctctg cattgatcag gctgtggtct tcatcgaaga tgctattcag taccgctcca     300
   tcaaccaccg ggtggatgcc agctcgatgt ggctttaccg acggtattac tcgaacgtat     360
   gccaacggac tttgagcttc accatcttct tgatcctgtt tttggctttt atcgagaccc     420
   catcctcact caccagcacg gcggacgtgc gctaccgcgc tgctccctgg gagccgccct     480
   gcggcctgac cgagagtgtc gaggtgctct gcctgctggt ctttgcggcc gacctctctg     540
   tgaagggtta cctgttcggg tgggcccatt tccagaaaaa cctttggctg ctgggctacc     600
   tcgtggtgct ggtggtgtct ctggtggact ggaccgtgtc cctgagtctc gtgtgtcatg     660
   agcccctgcg gatccgccgg cttctccgtc ccttcttcct gctgcagaac tcctctatga     720
   tgaagaagac cttgaaatgc atccgctggt cgctgccgga aatggccagc gtcgggctgc    780
   tgctggccat ccacctgtgc ctcttcacca tgttcggaat gctgctgttc gctggtggga    840
   agcaggatga tgggcaggac agggagaggc tgacctactt ccagaacctg cctgagtctc    900
   tgacttccct cctggtgctg ctgaccacgg ccaacaaccc cgatgtgatg attcctgcgt    960
   attccaagaa ccgggcctat gccatcttct tcatagtctt cactgtgata ggaagcctgt   1020
   ttctgatgaa cctgctgaca gccatcatct acagtcagtt ccggggctac ctgatgaaat   1080
   ctctccagac ctcgctgttt cggaggcggc tgggaacccg ggctgccttt gaagtcctat   1140
   cctccatggt gggggaggga ggagccttcc ctcaggcagt tggggtgaag ccccagaact   1200
   tgctgcaggt gcttcagaag gtccagctgg acagctccca caaacaggcc atgatggaga   1260
   aggtgcgttc ctatggcagt gttctgctct cagctgagga gtttcagaag ctcttcaacg   1320
   agcttgacag aagtgtggtt aaagagcacc cgccgaggcc cgagtaccag tctccgtttc   1380
   tgcagagcgc ccagttcctc ttcggccact actactttga ctacctgggg aacctcatcg   1440
   ccctggcaaa cctggtgtcc atttgcgtgt tcctggtgct ggatgcagat gtgctgcctg   1500
   ctgagcgtga tgacttcatc ctggggattc tcaactgcgt cttcattgtg tactacctgt   1560
   tggagatgct gctcaaggtc tttgccctgg gcctgcgagg gtacctgtcc taccccagca   1620
   acgtgtttga cgggctcctc accgttgtcc tgctggtttt ggagatctca actctggctg   1680
   tgtaccgatt gccacaccca ggctggaggc cggagatggt gggcctgctg tcgctgtggg   1740
   acatgacccg catgctgaac atgctcatcg tgttccgctt cctgcgtatc atccccagca   1800
   tgaagctgat ggccgtggtg gccagtaccg tcctgggcct ggtgcagaac atgcgtgcgt   1860
   ttggcgggat cctggtggtg gtctactacg tatttgccat cattgggatc aacttgttta   1920
   gaggcgtcat tgtggctctt cctggaaaca gcagcctggc ccctgccaat ggctcggcgc   1980
   cctgtgggag cttcgagcag ctggagtact gggccaacaa cttcgatgac tttgcggctg   2040
   ccctggtcac tctgtggaac ttgatggtgg tgaacaactg gcaggtgttt ctggatgcat   2100
   atcggcgcta ctcaggcccg tggtccaaga tctattttgt attgtggtgg ctggtgtcgt   2160
   ctgtcatctg ggtcaacctg tttctggccc tgattctgga gaacttcctt cacaagtggg   2220
   acccccgcag ccacctgcag ccccttgctg ggaccccaga ggccacctac cagatgactg   2280
   tggagctcct gttcagggat attctggagg agcccgggga ggatgagctc acagagaggc   2340
   tgagccagca cccgcacctg tggctgtgca ggtgacgtcc gggctgccgt cccagcaggg   2400
   gcggcaggag agagaggctg gcctacacag gtgcccgtca tggaagaggc ggccatgctg   2460
   tggccagcca ggcaggaaga gacctttcct ctgacggacc actaagctgg ggacaggaac   2520
   caagtccttt gcgtgtggcc caacaaccat ctacagaaca gctgctggtg cttcagggag   2580
   gcgccgtgcc ctccgctttc ttttatagct gcttcagtga gaattccctc gtcgactcca   2640
   cagggacctt tcagacaaaa atgcaagaag cagcggcctc ccctgtcccc tgcagcttcc   2700
   gtggtgcctt tgctgccggc agcccttggg gaccacaggc ctgaccaggg cctgcacagg   2760
   ttaaccgtca gacttccggg gcattcaggt ggggatgctg gtggtttgac atggagagaa   2820
   ccttgactgt gttttattat ttcatggctt gtatgagtgt gactgggtgt gtttctttag   2880
   ggttctgatt gccagttatt ttcatcaata agtcttgcaa agaatgggat tgtcattctt   2940
   cacttcagca cagttctagt cctgcttctc tggagtaggg ttgttgagta aggttgcttg   3000
   ggttgtgcat tgcacaaggg cacatggctg tgaggtgtat cctggcgggg ggctgtctac   3060
   ctgcagtgag gggcaccttt tctgttttgc tcaaaggcat gtataagcca atgggtgacc   3120
   ttatttcctg tgtcttcagg tgtgtggcag gggcctgggg gtggggaggt ggggcgagcg   3180
   agcagtgtgt ggaaagcctt gttgtcacct gaagcacgcc aggtccagat tgaccaatgg   3240
   ttttctcact tcaggggcca acccacgccc cctttctgct gaggtttggg tgccatctag   3300
   tggtgggatg ggacttggtt gactacattt aaggtaaggt ggacccagca actcccagaa   3360
   acaactccgg ggacaccact ccccatcaca ctccacaccg agcctggtgc ccggtctgtg   3420
   cccgagctca gcgggaccag gaagggatgg gccctgccag ggttgcccct gcactgtgca   3480
   ttctcgcctg ggaggcacaa gttctttcat ctgcttttcc ttcagaggtg ctgagcccac   3540
   gccatagccc ctgtgggatg gtgggggagg gggcgacccg aacaacagtg cagtcggtat   3600
   cgagattggg gagaggagcg agtccaagga gaaggtcatg agtttctttt tactcgtgtt   3660
   gaataataac aataacaata acaataacaa tatggaaacc accgcaaact tggagaaaag   3720
   ttgtaagcac agtaaagaga agcttccttc tgagtcactt gagtggttgc cgttctggcc   3780
   ctgcaccctc tgtgctttgg gacggcgtcc aacccgcatt catgtcagga gtgagtcgca   3840
   cgtggctttg tggtcatggc gacttaatcc gcctggacgg tggctccgtc tccctgggct   3900
   tagacgacct tggcacttct ggagataagc ccatggctcc caggttgtgt tcatgtgacg   3960
   tttccttgtg gtaggttctg ggtctgcgtt tcgtctagga gtgtcacagg atggacactg   4020
   cctcctggca ggggctgccc aatgcagtta gcctcctgct ggtgttctct cttgttgctt   4080
   ggtgaaggtg gccctggtca gcttctccac tgcccagtga acgacccctt tgtaatgaat   4140
   gagtggggag gtagtgtgaa gcgatgccaa tatcccatcc ctgtcaaact gcctttactt   4200
   tttccttcct tccttgctcc cacctgtgtg gatcctggtc ccttcttgta ttcagggctg   4260
   tggtctgtta tgacatttac tctcaggctc aggtcctgct tgtttggccc gtgggagccc   4320
   cttcttctgc cttttgtgtt ttttttgtta tgtacctaca gttctttagc tgtttcttta   4380
   ctttctggtg caaaaagatg ttcaagcctt attttatact tgcctgcccc tttctctttc   4440
   atttattgga gtgagctgca gctctaagaa gacctgttct tttgaatgga gagtagcatc   4500
   aggaaccagg atgtgggtgc gaggcgtgct cctggctgtt gcagattgct gcacccggga   4560
   gctcttagtg gacagagcta gaggatatgt gcacgtactt ccatctctct ctctgtctca   4620
   tctattcaca gctgggaatg atactaatac ctccgatttt agcccagcac cacagggtac   4680
   gttccagttt ttctctcttt ccatagctgt aaggcccttt ctgggaatgg ttctcattct   4740
   ccttaatcta ttattgggtc agttttcctg catgtcccca gcctcccatc actgccaccc   4800
   actccccaca gagatgccct gctcatccga ctggggcttt gactcccaca ctgtgtaccc   4860
   ctcttgtgtg gacgccctgc tgccaaaacc ttcagcaaac agctttccaa atggaagttg   4920
   tcactgtcag ggcctttaca atcagcaaca gcaaaatcta catgctgctg agggtcctgc   4980
   ctcattaaga tgcaataaat atgtaagtac ataaaaacag caatag                  5026
<210> 12
   <211> 4730
   <212> DNA
   <213> Homo sapiens
<400> 12
   tgccgtgcgc gcgtcagcaa aaacgccagg gacggggtct ccgcgcctgc gcagtgaagc      60
   tgggcgcctt cggggcttga gcttctgagg gtcgggtcca gcgcgtgggc tgctggatgg     120
   cggaacccca ggcggagtcg gagcccctgc tgggcggggc ccgcggcggt ggcggcgact     180
   ggccggcggg gctgaccact taccgcagca tccaagtcgg ccctggtgcc gcggccaggt     240
   gggacctctg cattgatcag gctgtggtct tcatcgaaga tgctattcag taccgctcca     300
   tcaaccaccg ggtggatgcc agctcgatgt ggctttaccg acggtattac tcgaacgtat     360
   gccaacggac tttgagcttc accatcttct tgatcctgtt tttggctttt atcgagaccc     420
   catcctcact caccagcacg gcggacgtgc gctaccgcgc tgctccctgg gagccgccct     480
   gcggcctgac cgagagtgtc gaggtgctct gcctgctggt ctttgcggcc gacctctctg     540
   tgaagggtta cctgttcggg tgggcccatt tccagaaaaa cctttggctg ctgggctacc     600
   tcgtggtgct ggtggtgtct ctggtggact ggaccgtgtc cctgagtctc gtgtgtcatg     660
   agcccctgcg gatccgccgg cttctccgtc ccttcttcct gctgcagaac tcctctatga     720
   tgaagaagac cttgaaatgc atccgctggt cgctgccgga aatggccagc gtcgggctgc     780
   tgctggccat ccacctgtgc ctcttcacca tgttcggaat gctgctgttc gctggtggga     840
   agcaggatga tgggcaggac agggagaggc tgacctactt ccagaacctg cctgagtctc     900
   tgacttccct cctggtgctg ctgaccacgg ccaacaaccc cgatgtgatg attcctgcgt     960
   attccaagaa ccgggcctat gccatcttct tcatagtctt cactgtgata ggaagcctgt    1020
   ttctgatgaa cctgctgaca gccatcatct acagtcagtt ccggggctac ctgatgaaat    1080
   ctctccagac ctcgctgttt cggaggcggc tgggaacccg ggctgccttt gaagtcctat 1140
   cctccatggt gggggaggga ggagccttcc ctcaggccac ccgccgaggc ccgagtacca 1200
   gtctccgttt ctgcagagcg cccagttcct cttcggccac tactactttg actacctggg 1260
   gaacctcatc gccctggcaa acctggtgtc catttgcgtg ttcctggtgc tggatgcaga 1320
   tgtgctgcct gctgagcgtg atgacttcat cctggggatt ctcaactgcg tcttcattgt 1380
   gtactacctg ttggagatgc tgctcaaggt ctttgccctg ggcctgcgag ggtacctgtc 1440
   ctaccccagc aacgtgtttg acgggctcct caccgttgtc ctgctggagg ccggagatgg 1500
   tgggcctgct gtcgctgtgg gacatgaccc gcatgctgaa catgctcatc gtgttccgct 1560
   tcctgcgtat catccccagc atgaagctga tggccgtggt ggccagtacc gtcctgggcc 1620
   tggtgcagaa catgcgtgcg tttggcggga tcctggtgcc tggcccctgc caatggctcg 1680
   gcgccctgtg ggagcttcga gcagctggag tactgggcca acaacttcga tgactttgcg 1740
   gctgccctgg tcactctgtg gaacttgatg gtggtgaaca actggcaggt gtttctggat 1800
   gcatatcggc gctactcagg cccgtggtcc aagatctatt ttgtattgtg gtggctggtg 1860
   tcgtctgtca tctgggtcaa cctgtttctg gccctgattc tggagaactt ccttcacaag 1920
   tgggaccccc gcagccacct gcagcccctt gctgggaccc cagaggccac ctaccagatg 1980
   actgtggagc tcctgttcag ggatattctg gaggagcccg gggaggatga gctcacagag 2040
   aggctgagcc agcacccgca cctgtggctg tgcaggtgac gtccgggctg ccgtcccagc 2100
   aggggcggca ggagagagag gctggcctac acaggtgccc gtcatggaag aggcggccat 2160
   gctgtggcca gccaggcagg aagagacctt tcctctgacg gaccactaag ctggggacag 2220
   gaaccaagtc ctttgcgtgt ggcccaacaa ccatctacag aacagctgct ggtgcttcag 2280
   ggaggcgccg tgccctccgc tttcttttat agctgcttca gtgagaattc cctcgtcgac 2340
   tccacaggga cctttcagac aaaaatgcaa gaagcagcgg cctcccctgt cccctgcagc 2400
   ttccgtggtg cctttgctgc cggcagccct tggggaccac aggcctgacc agggcctgca 2460
   caggttaacc gtcagacttc cggggcattc aggtggggat gctggtggtt tgacatggag 2520
   agaaccttga ctgtgtttta ttatttcatg gcttgtatga gtgtgactgg gtgtgtttct 2580
   ttagggttct gattgccagt tattttcatc aataagtctt gcaaagaatg ggattgtcat 2640
   tcttcacttc agcacagttc tagtcctgct tctctggagt agggttgttg agtaaggttg 2700
   cttgggttgt gcattgcaca agggcacatg gctgtgaggt gtatcctggc ggggggctgt 2760
   ctacctgcag tgaggggcac cttttctgtt ttgctcaaag gcatgtataa gccaatgggt 2820
   gaccttattt cctgtgtctt caggtgtgtg gcagggggcc tggggtgggg aggtggggcg 2880
   agcgagcagt gtgtggaaag ccttgttgtc acctgaagca cgccaggtcc agattgacca 2940
   atggttttct cacttcaggg gccaacccac gccccctttc tgctgaggtt tgggtgccat 3000
   ctagtggtgg gatgggactt ggttgactac atttaaggta aggtggaccc agcaactccc 3060
   agaaacaact ccggggacac cactccccat cacactccac accgagcctg gtgcccggtc 3120
   tgtgcccgag ctcagcggga ccaggaaggg atgggccctg ccagggttgc ccctgcactg 3180
   tgcattctcg cctgggaggc acaagttctt tcatctgctt ttccttcaga ggtgctgagc 3240
   ccacgccata gcccctgtgg gatggtgggg gagggggcga cccgaacaac agtgcagtcg 3300
   gtatcgagat tggggagagg agcgagtcca aggagaaggt catgagtttc tttttactcg 3360
   tgttgaataa taacaataac aataacaata acaatatgga aaccaccgca aacttggaga 3420
   aaagttgtaa gcacagtaaa gagaagcttc cttctgagtc acttgagtgg ttgccgttct 3480
   ggccctgcac cctctgtgct ttgggacggc gtccaacccg cattcatgtc aggagtgagt 3540
   cgcacgtggc tttgtggtca tggcgactta atccgcctgg acggtggctc cgtctccctg 3600
   ggcttagacg accttggcac ttctggagat aagcccatgg ctcccaggtt gtgttcatgt 3660
   gacgtttcct tgtggtaggt tctgggtctg cgtttcgtct aggagtgtca caggatggac 3720
   actgcctcct ggcaggggct gcccaatgca gttagcctcc tgctggtgtt ctctcttgtt 3780
   gcttggtgaa ggtggccctg gtcagcttct ccactgccca gtgaacgacc cctttgtaat 3840
   gaatgagtgg ggaggtagtg tgaagcgatg ccaatatccc atccctgtca aactgccttt 3900
   actttttcct tccttccttg ctcccacctg tgtggatcct ggtcccttct tgtattcagg 3960
   gctgtggtct gttatgacat ttactctcag gctcaggtcc tgcttgtttg gcccgtggga 4020
   gccccttctt ctgccttttg tgtttttttt gttatgtacc tacagttctt tagctgtttc 4080
   tttactttct ggtgcaaaaa gatgttcaag ccttatttta tacttgcctg cccctttctc 4140
   tttcatttat tggagtgagc tgcagctcta agaagacctg ttcttttgaa tggagagtag 4200
   catcaggaac caggatgtgg gtgcgaggcg tgctcctggc tgttgcagat tgctgcaccc 4260
   gggagctctt agtggacaga gctagaggat atgtgcacgt acttccatct ctctctctgt 4320
   ctcatctatt cacagctggg aatgatacta atacctccga ttttagccca gcaccacagg 4380
   gtacgttcca gtttttctct ctttccatag ctgtaaggcc ctttctggga atggttctca 4440
   ttctccttaa tctattattg ggtcagtttt cctgcatgtc cccagcctcc catcactgcc 4500
   acccactccc cacagagatg ccctgctcat ccgactgggg ctttgactcc cacactgtgt 4560
   acccctcttg tgtggacgcc ctgctgccaa aaccttcagc aaacagcttt ccaaatggaa 4620
   gttgtcactg tcagggcctt tacaatcagc aacagcaaaa tctacatgct gctgagggtc 4680
   ctgcctcatt aagatgcaat aaatatgtaa gtacataaaa acagcaatag 4730
<210> 13
   <211> 279
   <212> PRT
   <213> Homo sapiens
<400> 13
   Met Met Leu Met Gln Ala Leu Val Leu Phe Thr Leu Asp Ser Leu Asp
   1               5                   10                  15
   Met Leu Pro Ala Val Lys Ala Thr Trp Leu Tyr Gly Ile Gln Ile Thr
               20                  25                  30
   Ser Leu Leu Leu Val Cys Ile Leu Gln Phe Phe Asn Ser Met Ile Leu
           35                  40                  45
   Gly Ser Leu Leu Ile Ser Phe Asn Leu Ser Val Phe Ile Ala Arg Lys
       50                  55                  60
   Leu Gln Lys Asn Leu Lys Thr Gly Ser Phe Leu Asn Arg Leu Gly Lys
   65                  70                  75                  80
   Leu Leu Leu His Leu Phe Met Val Leu Cys Leu Thr Leu Phe Leu Asn
                   85                  90                  95
   Asn Ile Ile Lys Lys Ile Leu Asn Leu Lys Ser Asp Glu His Ile Phe
               100                 105                 110
   Lys Phe Leu Lys Ala Lys Phe Gly Leu Gly Ala Thr Arg Asp Phe Asp
           115                 120                 125
   Ala Asn Leu Tyr Leu Cys Glu Glu Ala Phe Gly Leu Leu Pro Phe Asn
       130                 135                 140
   Thr Phe Gly Arg Leu Ser Asp Thr Leu Leu Phe Tyr Ala Tyr Ile Phe
   145                 150                 155                 160
   Val Leu Ser Ile Thr Val Ile Val Ala Phe Val Val Ala Phe His Asn
                   165                 170                 175
   Leu Ser Asp Ser Thr Asn Gln Gln Ser Val Gly Lys Met Glu Lys Gly
               180                 185                 190
   Thr Val Asp Leu Lys Pro Glu Thr Ala Tyr Asn Leu Ile His Thr Ile
           195                 200                 205
   Leu Phe Gly Phe Leu Ala Leu Ser Thr Met Arg Met Lys Tyr Leu Trp
       210                 215                 220
   Thr Ser His Met Cys Val Phe Ala Ser Phe Gly Leu Cys Ser Pro Glu
   225                 230                 235                 240
   Ile Trp Glu Leu Leu Leu Lys Ser Val His Leu Tyr Asn Pro Lys Arg
                   245                 250                 255
   Ile Cys Ile Met Arg Tyr Ser Val Pro Ile Leu Ile Leu Leu Tyr Leu
               260                 265                 270
   Cys Tyr Lys Asn Gln Lys Ser
           275
<210> 14
   <211> 102
   <212> PRT
   <213> Homo sapiens
<400> 14
   Met Met Ser Ile Arg Gln Arg Arg Glu Ile Arg Ala Thr Glu Val Ser
   1               5                   10                  15
   Glu Asp Phe Pro Ala Gln Glu Glu Asn Val Lys Leu Glu Asn Lys Leu
               20                  25                  30
   Pro Ser Gly Cys Thr Ser Arg Arg Leu Trp Lys Ile Leu Ser Leu Thr
           35                  40                  45
   Ile Gly Gly Thr Ile Ala Leu Cys Ile Gly Leu Leu Thr Ser Val Tyr
       50                  55                  60
   Leu Ala Thr Leu His Glu Asn Asp Leu Trp Phe Ser Asn Ile Lys Val
   65                  70                  75                  80
   Trp Ser Phe Phe Asp His Cys Ile Ile His Ser Val Gly Ser Pro Val
                   85                  90                  95
   Val Ser His Val Asp Glu
               100
<210> 15
   <211> 716
   <212> PRT
   <213> Homo sapiens
<400> 15
   Met Met Ser Ile Arg Gln Arg Arg Glu Ile Arg Ala Thr Glu Val Ser
   1               5                   10                  15
   Glu Asp Phe Pro Ala Gln Glu Glu Asn Val Lys Leu Glu Asn Lys Leu
               20                  25                  30
   Pro Ser Gly Cys Thr Ser Arg Arg Leu Trp Lys Ile Leu Ser Leu Thr
           35                  40                  45
   Ile Gly Gly Thr Ile Ala Leu Cys Ile Gly Leu Leu Thr Ser Val Tyr
       50                  55                  60
   Leu Ala Thr Leu His Glu Asn Asp Leu Trp Phe Ser Asn Ile Lys Glu
   65                  70                  75                  80
   Val Glu Arg Glu Ile Ser Phe Arg Thr Glu Cys Gly Leu Tyr Tyr Ser
                   85                  90                  95
   Tyr Tyr Lys Gln Met Leu Gln Ala Pro Thr Leu Val Gln Gly Phe His
               100                 105                 110
   Gly Leu Ile Tyr Asp Asn Lys Thr Glu Ser Met Lys Thr Ile Asn Leu
           115                 120                 125
   Leu Gln Arg Met Asn Ile Tyr Gln Glu Val Phe Leu Ser Ile Leu Tyr
       130                 135                 140
   Arg Val Leu Pro Ile Gln Lys Tyr Leu Glu Pro Val Tyr Phe Tyr Ile
   145                 150                 155                 160
   Tyr Thr Leu Phe Gly Leu Gln Ala Ile Tyr Val Thr Ala Leu Tyr Ile
                   165                 170                 175
   Thr Ser Trp Leu Leu Ser Gly Thr Trp Leu Ser Gly Leu Leu Ala Ala
               180                 185                 190
   Phe Trp Tyr Val Thr Asn Arg Ile Asp Thr Thr Arg Val Glu Phe Thr
           195                 200                 205
   Ile Pro Leu Arg Glu Asn Trp Ala Leu Pro Phe Phe Ala Ile Gln Ile
       210                 215                 220
   Ala Ala Ile Thr Tyr Phe Leu Arg Pro Asn Leu Gln Pro Leu Ser Glu
   225                 230                 235                 240
   Arg Leu Thr Leu Leu Ala Ile Phe Ile Ser Thr Phe Leu Phe Ser Leu
                   245                 250                 255
   Thr Trp Gln Phe Asn Gln Phe Met Met Leu Met Gln Ala Leu Val Leu
               260                 265                 270
   Phe Thr Leu Asp Ser Leu Asp Met Leu Pro Ala Val Lys Ala Thr Trp
           275                 280                 285
   Leu Tyr Gly Ile Gln Ile Thr Ser Leu Leu Leu Val Cys Ile Leu Gln
       290                 295                 300
   Phe Phe Asn Ser Met Ile Leu Gly Ser Leu Leu Ile Ser Phe Asn Leu
   305                 310                 315                 320
   Ser Val Phe Ile Ala Arg Lys Leu Gln Lys Asn Leu Lys Thr Gly Ser
                   325                 330                 335
   Phe Leu Asn Arg Leu Gly Lys Leu Leu Leu His Leu Phe Met Val Leu
               340                 345                 350
   Cys Leu Thr Leu Phe Leu Asn Asn Ile Ile Lys Lys Ile Leu Asn Leu
           355                 360                 365
   Lys Ser Asp Glu His Ile Phe Lys Phe Leu Lys Ala Lys Phe Gly Leu
       370                 375                 380
   Gly Ala Thr Arg Asp Phe Asp Ala Asn Leu Tyr Leu Cys Glu Glu Ala
   385                 390                 395                 400
   Phe Gly Leu Leu Pro Phe Asn Thr Phe Gly Arg Leu Ser Asp Thr Leu
                   405                 410                 415
   Leu Phe Tyr Ala Tyr Ile Phe Val Leu Ser Ile Thr Val Ile Val Ala
               420                 425                 430
   Phe Val Val Ala Phe His Asn Leu Ser Asp Ser Thr Asn Gln Gln Ser
           435                 440                 445
   Val Gly Lys Met Glu Lys Gly Thr Val Asp Leu Lys Pro Glu Thr Ala
       450                 455                 460
   Tyr Asn Leu Ile His Thr Ile Leu Phe Gly Phe Leu Ala Leu Ser Thr
   465                 470                 475                 480
   Met Arg Met Lys Tyr Leu Trp Thr Ser His Met Cys Val Phe Ala Ser
                   485                 490                 495
   Phe Gly Leu Cys Ser Pro Glu Ile Trp Glu Leu Leu Leu Lys Ser Val
               500                 505                 510
   His Leu Tyr Asn Pro Lys Arg Ile Cys Ile Met Arg Tyr Ser Val Pro
           515                 520                 525
   Ile Leu Ile Leu Leu Tyr Leu Cys Tyr Lys Phe Trp Pro Gly Met Met
       530                 535                 540
   Asp Glu Leu Ser Glu Leu Arg Glu Phe Tyr Asp Pro Asp Thr Val Glu
   545                 550                 555                 560
   Leu Met Asn Trp Ile Asn Ser Asn Thr Pro Arg Lys Ala Val Phe Ala
                   565                 570                 575
   Gly Ser Met Gln Leu Leu Ala Gly Val Lys Leu Cys Thr Gly Arg Thr
               580                 585                 590
   Leu Thr Asn His Pro His Tyr Glu Asp Ser Ser Leu Arg Glu Arg Thr
           595                 600                 605
   Arg Ala Val Tyr Gln Ile Tyr Ala Lys Arg Ala Pro Glu Glu Val His
       610                 615                 620
   Ala Leu Leu Arg Ser Phe Gly Thr Asp Tyr Val Ile Leu Glu Asp Ser
   625                 630                 635                 640
   Ile Cys Tyr Glu Arg Arg His Arg Arg Gly Cys Arg Leu Arg Asp Leu
                   645                 650                 655
   Leu Asp Ile Ala Asn Gly His Met Met Asp Gly Pro Gly Glu Asn Asp
               660                 665                 670
   Pro Asp Leu Lys Pro Ala Asp His Pro Arg Phe Cys Glu Glu Ile Lys
           675                 680                 685
   Arg Asn Leu Pro Pro Tyr Val Ala Tyr Phe Thr Arg Val Phe Gln Asn
       690                 695                 700
   Lys Thr Phe His Val Tyr Lys Leu Ser Arg Asn Lys
   705                 710                 715
<210> 16
   <211> 112
   <212> PRT
   <213> Homo sapiens
<400> 16
   Met Met Ser Ile Arg Gln Arg Arg Glu Ile Arg Ala Thr Glu Val Ser
   1               5                   10                  15
   Glu Asp Phe Pro Ala Gln Glu Glu Asn Val Lys Leu Glu Asn Lys Leu
               20                  25                  30
   Pro Ser Gly Cys Thr Ser Arg Arg Leu Trp Lys Ile Leu Ser Leu Thr
           35                  40                  45
   Ile Gly Gly Thr Pro Phe Ala Leu Asp Phe Leu His Leu Ser Thr Leu
       50                  55                  60
   Pro Arg Tyr Met Lys Met Ile Tyr Gly Phe Leu Ile Leu Arg Lys Trp
   65                  70                  75                  80
   Ser Glu Lys Ser His Ser Glu Gln Ser Val Ala Cys Ile Thr Pro Thr
                   85                  90                  95
   Thr Ser Arg Cys Cys Arg Leu Gln Pro Ser Cys Lys Val Ile Thr Thr
               100                 105                 110
<210> 17
   <211> 1841
   <212> DNA
   <213> Homo sapiens
<400> 17
   aagtttgcgg agcggcttct gctcgtcggc cgtgcggcga ggcagggcct gggctgcgac 60
   cccggcggcc gctcgcggtc ttgggagagc tggggcgcgt gcctgaactt cccggctgcc 120
   cctgtccttg gagacctacc tgatggggac gccaggtgtg caggggcgtg gcgcgtagga 180
   gtgatttgga gaacaatgca tgtaagtctg acatcatgat gtccatccgg caaagaagag 240
   aaataagagc cacagaagtt tctgaagact ttccagccca agaagaaaat gtgaagttgg 300
   aaaataaatt gccatctggt tgtaccagta gaagattatg gaagattttg tcattgacaa 360
   ttggtggaac cattgccctt tgcattggac ttcttacatc tgtctacctt gccacgttac 420
   atgaaaatga tttatggttt tctaatatta aggaagtgga gcgagaaatc tcattcagaa 480
   cagagtgtgg cctgtattac tcctactaca agcagatgct gcaggctcca accctcgtgc 540
   aaggttttca tggcctaata tatgataata aaactgaatc tatgaagaca attaacctcc 600
   ttcagcgaat gaatatttac caagaggttt ttctcagtat tttatataga gttctaccca 660
   tacagaaata tttagagcca gtttattttt atatttacac cttatttggg ctccaggcga 720
   tctatgtcac agctctctac ataaccagct ggctactcag tggtacatgg ctgtcaggac 780
   tgttggcagc tttctggtat gtcacaaata gaatagatac cacaagagtt gagtttacca 840
   tcccactgag ggagaactgg gcgctgccat tctttgcaat tcagatagca gcaattacat 900
   atttcctgag accaaactta cagcctcttt ctgaaaggct gacacttctt gccattttca 960
   tatcaacttt tctctttagt ctgacatggc aatttaatca atttatgatg ctgatgcaag 1020
   cattagtgct gttcacactg gactccctgg acatgctgcc agcagtgaag gcgacatggc 1080
   tgtatggaat acagataaca agtttactcc tggtctgcat tcttcagttt tttaattcca   1140
   tgattcttgg atcactgctt atcagtttta acctttcagt attcattgca agaaaacttc   1200
   agaaaaatct gaaaactgga agcttcctta ataggcttgg gaaacttttg ttacatttat   1260
   ttatggtttt atgtttgaca ctttttctca acaacataat taagaaaatt cttaacctga   1320
   agtcagatga acacatattt aaatttctga aggcaaaatt tgggcttgga gcaacaaggg   1380
   attttgatgc aaatctctat ctgtgtgaag aagcttttgg cctcctgcct tttaatacat   1440
   ttggaaggct ttcagatact ctgctttttt atgcttacat attcgttctg tccatcacag   1500
   tgattgtagc attcgttgtt gcctttcata atctcagtga ttctacaaat caacaatccg   1560
   tgggtaaaat ggaaaaaggc acagttgacc tgaaaccaga aactgcctac aacttaatac   1620
   ataccattct gtttggattc ttggcattga gtacaatgag aatgaagtac ctctggacgt   1680
   cacacatgtg tgtgttcgca tcattcggcc tatgtagccc tgaaatatgg gagttacttc   1740
   tgaagtcagt ccatctttat aacccaaaga ggatatgtat aatgcgatat tcagtaccga   1800
   tattaatact gctgtatcta tgctataaga atcagaaatc t                       1841
<210> 18
   <211> 2206
   <212> DNA
   <213> Homo sapiens
<400> 18
   cggttctgcc ctccttgtac ccgcggcgcg ctgcggcccg tggcgcggcc ccgttcccgc      60
   ctagccccgt cggcctcctt cccctcccgg agccgcgcgt gaggacggct gaggccgcag     120
   gagtgatttg gagaacaatg catgtaagtc tgacatcatg atgtccatcc ggcaaagaag     180
   agaaataaga gccacagaag tttctgaaga ctttccagcc caagaagaaa atgtgaagtt     240
   ggaaaataaa ttgccatctg gttgtaccag tagaagatta tggaagattt tgtcattgac     300
   aattggtgga accattgccc tttgcattgg acttcttaca tctgtctacc ttgccacgtt     360
   acatgaaaat gatttatggt tttctaatat taaggtatgg agtttctttg accattgtat     420
   cattcactca gtgggatctc cagtagtaag ccatgtggat gaatgaccaa ggcaacacag     480
   ttttgccata aagaatccaa tctctagaaa ggttggacta tagagtgaaa taacttttgt     540
   gtttattatt ttaaaataac atattagaat ctttttttaa atttttcttt attatttatt     600
   tatttttgag atggagtctc actctgtcac ccaggctgga gtgcggtggc gcaatcttgg     660
   ctcactacaa cctctgcctc gcaggttcag gtgattcttc tggcttagcc tcccaagtag     720
   ctgggactat aggtgcgtgc caccacaccc agctaatttt tgtattttta ctagagacgg     780
   ggtttcagca tattgaccag gctgatctcg aactcctgac cttgtgatct gcctgtctca     840
   gcctcccaaa gtgctgggat tacaggcgtg agccactgcg tccagccaga atctttattt     900
   ttcattttaa ttttttgaga tagggtattg ctctgtcacc caggctagaa tgcagtggtg 960
   caaacatggg tcactgcagc ctcaacctcc tgggctcaag tgagtatcct gcctaagctt 1020
   cctgtgtcac tgggacccca ggcatgcacc acctcaccaa gctaaatttg atttttttgt 1080
   agagacaggg tctcactttg ttgcccatgc tggtctcgaa ctcctgggct caagcgatcc 1140
   tactgccctg gtcttccaaa atatgagaat gagccatagc acccagccca gaatttttat 1200
   aatcaagtga gtttttcttt tttcattaac ttattccatt tatttagcag ttattctaaa 1260
   ttagtatttt tcaagttata gattgtgaaa ttagtgcagt aggtcatgag taacattttt 1320
   cttaatgaaa tcaaaaagaa agaatactat cacatctagt agggttgagg attgttttgt 1380
   gaaactttta attttatata tatatatata tgcacaaact gggtcacagt atacaaggta 1440
   cttccttttc tttttttctt tgttggctac aacaggaaaa aaaaaaaaca gaaaaggaaa 1500
   taaaaaagcc actgctttaa atcatggggt ctaaatgtgg ctccacagag ggtcctcagc 1560
   atgttcatga ctatctaata ctctgtgcaa gtggttttgc agggcatagg gcgatgggga 1620
   agccatatgt ttccagggaa aggaactgta attttaatca gattttcagg agggttagcc 1680
   gggcgtcacg cctgtaatcc cagcactttg ggaggtcgag gcgggcagat cacttgaagt 1740
   caggagttca agaccagcct ggccaacatg gtggaaccct atctctacta aaaatacaaa 1800
   aattagccgg gcatggtgac acacacctgt aatctcagct actcaggagg ctgaggcaca 1860
   agaatcactt gaactcggga ggaagaggtt gcagtgagct gagatcccac cactgcactc 1920
   cagcctgggc aacagagcaa tactctttat caaaaaaaaa aagaaaaaag ttgagggggt 1980
   ggtctgtgac tctttaaaca cgtttccttg ttttctttct ctctctcttt ttcaacattt 2040
   ctagaactcc tcttggcatt gttttcagaa ctcgtatata acttacatgt ggaaatttgc 2100
   atccaaatat accttacatt ttaatctaat atgtcatgat ctttaaccta aactgtggtg 2160
   tctaatgact agttgcttgt aaaaataaac aaacaccttc aaagcc 2206
<210> 19
   <211> 4456
   <212> DNA
   <213> Homo sapiens
<400> 19
   aagtttgcgg agcggcttct gctcgtcggc cgtgcggcga ggcagggcct gggctgcgac     60
   cccggcggcc gctcgcggtc ttgggagagc tggggcgcgt gcctgaactt cccggctgcc    120
   cctgtccttg gagacctacc tgatggggac gccaggtgtg caggggcgtg gcgcgtagga    180
   gtgatttgga gaacaatgca tgtaagtctg acatcatgat gtccatccgg caaagaagag    240
   aaataagagc cacagaagtt tctgaagact ttccagccca agaagaaaat gtgaagttgg    300
   aaaataaatt gccatctggt tgtaccagta gaagattatg gaagattttg tcattgacaa    360
   ttggtggaac cattgccctt tgcattggac ttcttacatc tgtctacctt gccacgttac 420
   atgaaaatga tttatggttt tctaatatta aggaagtgga gcgagaaatc tcattcagaa 480
   cagagtgtgg cctgtattac tcctactaca agcagatgct gcaggctcca accctcgtgc 540
   aaggttttca tggcctaata tatgataata aaactgaatc tatgaagaca attaacctcc 600
   ttcagcgaat gaatatttac caagaggttt ttctcagtat tttatataga gttctaccca 660
   tacagaaata tttagagcca gtttattttt atatttacac cttatttggg ctccaggcga 720
   tctatgtcac agctctctac ataaccagct ggctactcag tggtacatgg ctgtcaggac 780
   tgttggcagc tttctggtat gtcacaaata gaatagatac cacaagagtt gagtttacca 840
   tcccactgag ggagaactgg gcgctgccat tctttgcaat tcagatagca gcaattacat 900
   atttcctgag accaaactta cagcctcttt ctgaaaggct gacacttctt gccattttca 960
   tatcaacttt tctctttagt ctgacatggc aatttaatca atttatgatg ctgatgcaag 1020
   cattagtgct gttcacactg gactccctgg acatgctgcc agcagtgaag gcgacatggc 1080
   tgtatggaat acagataaca agtttactcc tggtctgcat tcttcagttt tttaattcca 1140
   tgattcttgg atcactgctt atcagtttta acctttcagt attcattgca agaaaacttc 1200
   agaaaaatct gaaaactgga agcttcctta ataggcttgg gaaacttttg ttacatttat 1260
   ttatggtttt atgtttgaca ctttttctca acaacataat taagaaaatt cttaacctga 1320
   agtcagatga acacatattt aaatttctga aggcaaaatt tgggcttgga gcaacaaggg 1380
   attttgatgc aaatctctat ctgtgtgaag aagcttttgg cctcctgcct tttaatacat 1440
   ttggaaggct ttcagatact ctgctttttt atgcttacat attcgttctg tccatcacag 1500
   tgattgtagc attcgttgtt gcctttcata atctcagtga ttctacaaat caacaatccg 1560
   tgggtaaaat ggaaaaaggc acagttgacc tgaaaccaga aactgcctac aacttaatac 1620
   ataccattct gtttggattc ttggcattga gtacaatgag aatgaagtac ctctggacgt 1680
   cacacatgtg tgtgttcgca tcattcggcc tatgtagccc tgaaatatgg gagttacttc 1740
   tgaagtcagt ccatctttat aacccaaaga ggatatgtat aatgcgatat tcagtaccga 1800
   tattaatact gctgtatcta tgctataagt tctggccagg aatgatggat gaactctccg 1860
   agttgagaga attctatgat ccagatacag tggagctgat gaactggatt aactctaaca 1920
   ctccaagaaa ggctgtgttt gcgggaagca tgcagttgct ggccggagtc aagctgtgca 1980
   cgggaaggac cctaaccaac cacccgcact atgaagacag cagcctgaga gagcggacca 2040
   gagcggttta tcagatatat gccaagaggg caccagagga agtgcatgcc ctcctaaggt 2100
   ccttcggcac tgactacgta atcctggaag acagcatctg ctacgagcgg aggcaccgcc 2160
   ggggctgccg actccgggac ctgctggaca ttgccaacgg ccacatgatg gatggcccag 2220
   gagagaatga tcctgatttg aaacctgcag accaccctcg cttctgtgaa gagatcaaaa   2280
   gaaacctgcc tccctacgtg gcctacttca ccagagtgtt ccagaacaaa accttccacg   2340
   tttacaagct gtccagaaac aagtagcgca gatttctgcc cagtgtctat ttttgatacg   2400
   gagaaactgc atcatgatga aactcaatag atgacgtttc ctatgtaagt aggtagccca   2460
   aaccttcaag ctgtgatatg agtaagttct acagatgttt acacaagtgt tgccatcttt   2520
   gaaagcatct tctacaagca gaagtctttt tcgttgtgtg tctatctttc tcattaatgt   2580
   tctttagcct aaatgttaac aactttctaa gagtgaccta gaattatgtt gttggagaga   2640
   atgatgtgtg ttccatggat acctggatag gcacataaca tgttggaaga tgagcacctg   2700
   ctcaggattt gaaatacgtt taattttcag gtgacttaag acagctatga ttgaatcaac   2760
   tagagatgat gatcgactta tttaatatga tttcactggt gaagaccaat tggtagcttt   2820
   ttaaaaagca ctttagtgtc ctgttttacc ttaaaatgtt ataatatttt ccagttgtca   2880
   tgctgtcaac attaacaaaa aaaatcatgt taaggctttg tatcaaacat tttgttacac   2940
   tctgtctgaa atgtaatgtg gagtacttca gcagtatgtg tcatgtattg tgtgtgtctg   3000
   tgtgtgtgca tgtgcacaca tgtgttttaa tgctgggcac agaaaagtgt tacaagttcc   3060
   atatcgtaag tccttaaagg ggcagaaata tatgtagcca agtagaattt attacatttt   3120
   agtgttatta ttttaaaact tactgatact ctttaacctc tcctgcagta atagttttgc   3180
   tttatttctt actcatttca atttattggg tttgcaaaat tttgtaaact ttttgtgttt   3240
   ttagcctttg tattttttac agcctagaat cttgcaaagt ctgaatattt tttaaatgtt   3300
   ctatcttaac tagttcacta atacagtatt tttagcagac agcattttca gacagcattt   3360
   tcataccaag tttgacttgt ggtctccaat cttactggga aggccctggt agtgtaattc   3420
   ttttccttat taaaaggtaa ccaagtgcct ctaagtcatg cttatttgta aacaacaaag   3480
   aagagtatat gtacctgctc aaaatttttt tgataatcgc ttatataatt aatttctaat   3540
   gatgaggaca tgtaaaagtt gccagtaaga acatagtatg catttaatta aatcaagatg   3600
   gctaatggaa ttaactttct cccctgttct tgccaggtgg aaatgattta agcatttctc   3660
   cttgcagttg tattgaagta aattaccata ggcatcaaga tggctgcatc acattttcaa   3720
   atgattttat attcagttgc tacttataaa gcagcattca aaaagtcttt tacactgtca   3780
   tgttggacac aagcagactc agcttttatc aaaacttgtt taaataaaaa attgacagta   3840
   gctgggttat taaattatgc aactgaaact cctgaattat atcttttctg tatcccttaa   3900
   taagattgga gaccactgcc gtttaggata atacaataat aaaacgtttt aatcagtact   3960
   aaaactttaa ttaagccaat aatgatgcat gcctgttgta gctgacagca tgggtcagta   4020
   catccttcag cgagtgcctt actctaattg aaaccaagca cacgtaaggt acaatatgtt   4080
   agactctgtg attttgtttt caaaatcctc tgttatggct atatttaaat ttattttaaa 4140
   tattcctgta tgtattcatc taagcatttg ggcatttgga gtcttaatat acaagaaaca 4200
   cgtacttaaa tttttatgct tatcaccgca atgatggcaa acagtgattt tttttttcat 4260
   agtttaggtg tcattgttgc cagcaccttt agtgctcagt cttcagtgaa aaatataaag 4320
   tgccaaaaaa atcttgcaag acagaatcca tacttaacac tctttccaag acactgtgac 4380
   catgtacagt agctatttcc tgatgaccaa atctctcaac gaatcatgtt attaataaat 4440
   atttttagca ctcatc 4456
<210> 20
   <211> 336
   <212> DNA
   <213> Homo sapiens
<400> 20
   atgatgtcca tccggcaaag aagagaaata agagccacag aagtttctga agactttcca 60
   gcccaagaag aaaatgtgaa gttggaaaat aaattgccat ctggttgtac cagtagaaga 120
   ttatggaaga ttttgtcatt gacaattggt ggaaccccct ttgcattgga cttcttacat 180
   ctgtctacct tgccacgtta catgaaaatg atttatggtt ttctaatatt aaggaagtgg 240
   agcgagaaat ctcattcaga acagagtgtg gcctgtatta ctcctactac aagcagatgc 300
   tgcaggctcc aaccctcgtg caaggtaatt acaact 336
<210> 21
   <211> 173
   <212> PRT
   <213> Homo sapiens
<400> 21
   Met Thr Leu Cys His Arg Asp Ser Phe Gly Ser Trp His Leu Phe His
   1               5                   10                  15
   Leu Leu Leu Leu Glu Tyr Met Ile His Ile Leu Gln Ser Cys Leu Glu
               20                  25                  30
   Glu Glu Glu Glu Glu Glu Asp Met Gly Thr Val Lys Glu Met Leu Pro
           35                  40                  45
   Asp Asp Pro Thr Leu Gly Gln Pro Asp Gln Ala Leu Phe His Ser Leu
       50                  55                  60
   Asn Ser Ser Leu Ser Gln Ala Cys Ala Ser Pro Ser Met Glu Pro Leu
   65                  70                  75                  80
   Gly Val Met Pro Thr His Met Gly Gln Gly Arg Tyr Pro Val Gly Val
                   85                  90                  95
   Ser Asn Met Val Leu Arg Ile Leu Gly Phe Leu Val Asp Thr Ala Met
               100                 105                 110
   Gly Asn Lys Leu Ile Gln Val Leu Leu Glu Asp Glu Thr Thr Glu Ser
           115                 120                 125
   Ala Val Lys Leu Ser Leu Pro Met Gly Gln Glu Ala Leu Ile Thr Leu
       130                 135                 140
   Lys Asp Gly Gln Gln Phe Val Ile Gln Ile Ser Asp Val Pro Gln Ser
   145                 150                 155                 160
   Ser Glu Asp Ile Tyr Phe Arg Glu Asn Asn Ala Asn Val
                   165                 170
<210> 22
   <211> 1907
   <212> DNA
   <213> Homo sapiens
<400> 22
   ataactccaa cgctcaagca agtcaaggac acccacggac tcaacaccgc gaccagattg     60
   gaaaaggtgt tggtcgacaa cttctgcatt tgcgaagagt gcagcgtccc tcgctgtctc    120
   atgtatgaga tttacgtgga gacctgtggg caaaacactg agaaccaagt caacccggcc    180
   acctttggga agcttgtgag attggttttt ccggaccttg gcacccggag gctgggcact    240
   agaggaagtg ccaggtatca ttatgatgga atctgtatca agaaaagctc tttcttctat    300
   gcccagtatt gctacctgat aggtgaaaaa aggtatcaca gtggagatgc cattgccttt    360
   gaaaaatcta ctaattataa cagcattatc caacaagaag caacatgtga agatcattca    420
   ccgatgaaga cagacccagt tggatcccct ttgtctgaat tcaggagatg tccatttctg    480
   gagcaagaac aggcaaagaa atactcctgt aatatgatgg ccttccttgc tgacgaatac    540
   tgcaactatt gtcgagacat tttacgaaat gtgaggaact gagaacttga gagggtggag    600
   gacttgctta cttccttctg gaagtctctg cagcaagaca cagtcatgct gatgtcattg    660
   cctgacgtgt gccagctctt taaatgctac gacgtccagc tgtacaaggg aattgaggat    720
   gttctccttc atgacttctt ggaagatgtt tctattcagt acctgaaatc tgtgcagtta    780
   tttagtaaga aatttaagct gtggctcctt aatgctttgg aaggtgttcc agccctcttg    840
   cagatctcca aactcaaaga ctatgcgaat ggtattgaaa agtaagaggc gtgtcagcgt    900
   tttgaagtca gatctacagg ccatcatcaa tcaaggcact ttggctactt ctaagaaagc    960
   cctggcaagt gaccggagtg gcgcagatga actggagaac aacccagaga tgaaatgttt   1020
   aagaaactta atttctttgc tgggaacatc aacagatctc agggtattcc tcagctgtct   1080
   gtcttcacat ctccaagcat ttgtgttcca gacaagcaga agcaaagaag agtttaccaa   1140
   attggccgcc agcttccagc tgagatggaa tcttcttctc actgctgtaa gcaaagccat   1200
   gaccctctgc cacagagata gttttggctc ctggcatctg tttcacttgt tgcttttgga   1260
   atatatgatt catatacttc agtcatgcct agaggaggaa gaggaggagg aggacatggg   1320
   gactgtcaag gaaatgctac cagatgaccc gactctcggc cagccagacc aggcactttt   1380
   ccattctctg aattcctcac tgtcgcaggc gtgtgccagc cccagcatgg agccactggg   1440
   ggtgatgccc acacacatgg gccagggccg atatcccgtg ggtgtgagca acatggtcct   1500
   caggatcctg ggcttcctgg tggacactgc catgggcaat aagctcatcc aggtgctgtt   1560
   ggaagatgaa accactgaaa gcgcagttaa actcagcctt cctatgggac aagaagccct   1620
   cataacccta aaagatggac aacaatttgt gattcagata tcagatgtac cccaaagctc   1680
   tgaagatatt tatttcagag aaaacaatgc taatgtgtga gattatttat ttgaatagag   1740
   aataagaaaa ctgatagact tgcattctta aaaatattaa atactaaagt ttttctattg   1800
   acgaaagatg atgttatgta tataatagat gtagcattgt ctattttatg tttatatgta   1860
   tttcaaggag gtggtttcga taaaatatgt aaactgattt ggagaat                 1907
<210> 23
   <211> 272
   <212> PRT
   <213> Homo sapiens
<400> 23
   Met Gln Trp Asn Val Pro Arg Thr Val Ser Arg Leu Ala Arg Arg Thr
   1               5                   10                  15
   Cys Leu Glu Pro His Asn Ala Gly Leu Phe Gly His Cys Gln Asn Val
               20                  25                  30
   Lys Gly Pro Leu Leu Leu Tyr Asn Ala Glu Ser Lys Val Val Leu Val
           35                  40                  45
   Gln Gly Pro Gln Lys Gln Trp Leu His Leu Ser Ala Ala Gln Cys Val
       50                  55                  60
   Ala Lys Glu Arg Arg Pro Leu Asp Ala His Pro Pro Gln Pro Gly Val
   65                  70                  75                  80
   Leu Arg His Lys Gln Gly Lys Gln His Val Ser Phe Arg Arg Val Phe
                   85                  90                  95
   Ser Ser Ser Ala Thr Ala Gln Gly Thr Pro Glu Lys Lys Glu Glu Pro
               100                 105                 110
   Asp Pro Leu Gln Asp Lys Ser Ile Ser Leu Tyr Gln Arg Phe Lys Lys
           115                 120                 125
   Thr Phe Arg Gln Tyr Gly Lys Val Leu Ile Pro Val His Leu Ile Thr
       130                 135                 140
   Ser Gly Val Trp Phe Gly Thr Phe Tyr Tyr Ala Ala Leu Lys Gly Val
   145                 150                 155                 160
   Asn Val Val Pro Phe Leu Glu Leu Ile Gly Leu Pro Asp Ser Val Val
                   165                 170                 175
   Ser Ile Leu Lys Asn Ser Gln Ser Gly Asn Ala Leu Thr Ala Tyr Ala
               180                 185                 190
   Leu Phe Lys Ile Ala Thr Pro Ala Arg Tyr Thr Val Thr Leu Gly Gly
           195                 200                 205
   Thr Ser Val Thr Val Lys Tyr Leu Arg Ser His Gly Tyr Met Ser Thr
       210                 215                 220
   Pro Pro Pro Val Lys Glu Tyr Leu Gln Asp Arg Met Glu Glu Thr Lys
   225                 230                 235                 240
   Glu Leu Ile Thr Glu Lys Met Glu Glu Thr Lys Asp Arg Leu Thr Glu
                   245                 250                 255
   Lys Leu Gln Glu Thr Lys Glu Lys Val Ser Phe Lys Lys Lys Val Glu
               260                 265                 270
<210> 24
   <211> 272
   <212> PRT
   <213> Homo sapiens
<400> 24
   Met Gln Trp Asn Val Pro Arg Thr Val Ser Arg Leu Ala Arg Arg Thr
   1               5                   10                  15
   Cys Leu Glu Pro His Asn Ala Gly Leu Phe Gly His Cys Gln Asn Val
               20                  25                  30
   Lys Gly Pro Leu Leu Leu Tyr Asn Ala Glu Ser Lys Val Val Leu Val
           35                  40                  45
   Gln Gly Pro Gln Lys Gln Trp Leu His Leu Ser Ala Ala Gln Cys Val
       50                  55                  60
   Ala Lys Glu Arg Arg Pro Leu Asp Ala His Pro Pro Gln Pro Gly Val
   65                  70                  75                  80
   Leu Arg His Lys Gln Gly Lys Gln His Val Ser Phe Arg Arg Val Phe
                   85                  90                  95
   Ser Ser Ser Ala Thr Ala Gln Gly Thr Pro Glu Lys Lys Glu Glu Pro
               100                 105                 110
   Asp Pro Leu Gln Asp Lys Ser Ile Ser Leu Tyr Gln Arg Phe Lys Lys
           115                 120                 125
   Thr Phe Arg Gln Tyr Gly Lys Val Leu Ile Pro Val His Leu Ile Thr
       130                 135                 140
   Ser Gly Val Trp Phe Gly Thr Phe Tyr Tyr Ala Ala Leu Lys Gly Val
   145                 150                 155                 160
   Asn Val Val Pro Phe Leu Glu Leu Ile Gly Leu Pro Asp Ser Val Val
                   165                 170                 175
   Ser Ile Leu Lys Asn Ser Gln Ser Gly Asn Ala Leu Thr Ala Tyr Ala
               180                 185                 190
   Leu Phe Lys Ile Ala Thr Pro Ala Arg Tyr Thr Val Thr Leu Gly Gly
           195                 200                 205
   Thr Ser Val Thr Val Lys Tyr Leu Arg Ser His Gly Tyr Met Ser Thr
       210                 215                 220
   Pro Pro Pro Val Lys Glu Tyr Leu Gln Asp Arg Met Glu Glu Thr Lys
   225                 230                 235                 240
   Glu Leu Ile Thr Glu Lys Met Glu Glu Thr Lys Asp Arg Leu Thr Glu
                   245                 250                 255
   Lys Leu Gln Glu Thr Lys Glu Lys Val Ser Phe Lys Lys Lys Val Glu
               260                 265                 270
<210> 25
   <211> 4340
   <212> DNA
   <213> Homo sapiens
<400> 25
   gcagaagctt cactgagcgg gcactggagg tcgcgagcac gttgctagtg ggcgcggctg      60
   gcggggccgg gggcggcagg gggctagcgt ccggctgagc ccggcgggtt cctgcacgcg     120
   gacccacgct ttgtcgggct gctctgctga gtgacaccgc cgtggcatcc gaggagctgt    180
   ggtccagcct gcgcggcggg aagcggcggc ggcggctcca gcgtcctaag agagtctcac    240
   tctgtctccc aggctgcaac atccacctcc caggttcaag tgattcttgt gcctcagcct    300
   ctccagtagc tgggattaca ggtgtgtgcc agcatgcctg gccaattgtt ggattgtatg    360
   aagctgaaac ctatcaacac tcttcaaaat gcaatggaat gtaccacgga ctgtatctcg    420
   actggcacgc aggacatgct tggaaccaca taatgctggt ctctttggac actgtcaaaa    480
   tgtaaaggga cctttacttt tatacaatgc tgaatccaaa gtggttttgg tacaaggccc    540
   tcaaaaacaa tggttgcatt tatctgctgc ccagtgtgtt gcaaaggaaa ggaggccatt    600
   ggatgctcat ccaccccaac caggagtcct tcgccataag caagggaagc aacatgtttc    660
   attcaggagg gttttttcat ccagtgccac agctcaggga actccggaaa aaaaggaaga    720
   gcctgatcct ttgcaagaca aatctattag tctttatcaa cgattcaaga agacatttag    780
   acagtatgga aaagttctga ttccagtgca tctaataact tctggtgttt ggtttggaac    840
   attttattat gcagccttga aaggagtgaa tgtcgttcct tttctagaac tcattgggtt    900
   acctgacagt gtggtaagca tcctgaaaaa ctcccagagt ggaaatgccc tcacagcata    960
   tgccttgttt aagattgcaa cacctgctcg gtataccgtg actttgggag gaacatctgt   1020
   cactgtgaag tatctgcgca gtcatggcta catgtccacg ccgccacccg tcaaggagta   1080
   tctgcaggac aggatggaag agacaaagga gcttatcaca gagaaaatgg aagaaacaaa   1140
   agatagactc actgaaaagt tacaagaaac caaagaaaaa gtttccttta agaaaaaagt   1200
   ggaataaggt gccttatata gcagtataga aaattcctgc actttaaccc tttggaaact   1260
   atgggcaaag atacatgtgt ctgattattt ttttggttag ttgccgaaat atactagttc   1320
   tctgagggtt aaagaagtaa aatacctttt taaagttaaa tatcactaga aaaatcagtg   1380
   ttattacaag ggaagaaatg aacccagttt aagaatttgc catcagtagc agtattaagc   1440
   agtggttaat gtcttagaag tcagacttct ttttcaaggt cttcagaacc acacttgatt   1500
   tctgttttgt tgcagctgta attgacacat actaggcagc tgactccttg aatatccagt   1560
   gtgacccata aaatagtctg ttaataccgg atcttaattt ttatgttatt cattaagatt   1620
   ttaactatat tcagtacgta atttggagac aaactagcat catcaaaact gcctgtaaat   1680
   aaggtgttta gtctttctat aaaaacagaa tagagcagtt acctaccagt taaaatatct   1740
   tatatgaaga aaatagaata aagatccagt catatatgta aataagatgt actgattgta   1800
   cgtaaatgaa aaatggaccc tttaaaaatt atttttacct gaagcttgtc ataatttttt   1860
   taaagcaaat atatatatgg tgatggtact tttcaaagtg tgtattagtg gtgatcacct   1920
   caaacataaa cctctgttgt gaatcatttg tgtccttttc aactgtcttt cagaggaaag   1980
   gtaaaaaatc attaaacctt aaattcattg ttaaaatcaa atatttgtca gcagtaactc 2040
   aagctcatgg ttctcaagca gaaaaaggtt tgggagacta aaaatggagt caggttttca 2100
   tggagactgc taactccttg gggtagacat gggccttgcc tcagcaaacc agtgcaattt 2160
   cccaatgtct tagtttcaga attcatgctg atttcattat ggagtaaagt tttaaattgt 2220
   ggctgctgct ttttgtctta cattgaaggg aaatatcctt gtttgagcat tctacttgat 2280
   tttttttttt tttttttttt tttttgagac ggagcctccc tctctctctc ccagactgga 2340
   gtgcaatggt acaatctcgg ctcactgcat cctctgcctc ccaggttcaa gtgatcctcc 2400
   cgcctcagct ttccaagtag ctgggagtat aagcgtgtgc caccacactc agctaatttt 2460
   tgtattttta gtggaggtgg agtttcacca tgttagccag gctagtctcc aactcctgac 2520
   ctcagcctcc cgagtagctg ggactacagg cgcccaccac cacgcccagc taattttttt 2580
   gtatttttag tagagatggg gtttcaccat cttaacaaag ctatcattaa atcatttgct 2640
   tgtaatgaaa ttcagccgtt gtcagctgtg agcgttgcgg ggctggtggg gtgtgtttga 2700
   gtatgtaagt gtctatttcc tgtgctctaa cagtgactat ttcagttcta acccttcaat 2760
   tgctaattgg atgagggaat ggcctcttag attgtccttg ttttgactta tctgctaagg 2820
   cgagagaatg tctgggtttg ccacacagtc ccgcagggac ccctgctctt tgccaggatt 2880
   tttatatcaa gtacttagtt tggccaaatt tagagtatag tttaaagggg aaaaaaagtt 2940
   tgtatttgat gagtctaatt actatattag aatgcttttt aaatgaatat gctgtgattg 3000
   gaatctcttc ttgggggccg gggagagtgc tgagagatgc agctgtgtct ctgctctctg 3060
   cagtcttggg caggcagcac cgctgtctcc tgatacagag ggttgaatgc actgcttcac 3120
   attgatgcca cattttcaaa tctaaacatg attaacatgt tttttaagta tttatcaaat 3180
   aattcacatt tttgcaggtt taagtgctgt tttttaagat attaaacatg ttccttgttt 3240
   cttttttctt tttaaccact ggagactatg tgaaaatctt gtgttatatg gaatttttct 3300
   aaaacaattt ccttttactt ttcatgttgc atattttcca gtggggataa attggaggtt 3360
   cttattacta ataatcctga aactaaaatt tgcttttcct gcatagacat cttttgttag 3420
   tccatttgag ttttgttgcc atccagtttc atgatcatgt tgtgttagtc taatagacaa 3480
   tgagttactg cttgaatgat atgaaattct ctagtaaaaa atgctctgaa gatgtgagaa 3540
   gctctgttgt attacttggc accaggaatg ttacaaaaat gttgttgcta gcatattgct 3600
   aacctagtcc ttaagtttca gggattcctg taatatgttg agtttggagt attaatagca 3660
   ggctaacaga atacagtacc aatatgccaa gttaaataat tgagaatata tacagtatag 3720
   tttgctatgt ataatagacc tcatttcatt tacagtacac ctcacacttg ggattaattc 3780
   acctataacc tctgtgaact gcgagtgctc ggcagaagca gccctgtgtc ttacatgtct 3840
   acatttatat ttatatttat atttattttt ttgagacaga gtctcactct gtcacccagg 3900
   ctagagtgca gtggcatgat catagctcac ttgcgtcttg aactcctggc tcaagtgatc 3960
   ctcccacctc agcctgctga gtagctggga ctacaggtgc acaccaccat gcctggctaa 4020
   aggcatgtct gcatttatgc gaggtgtgtt gaccgggtgg ggagggagct aggactgaga 4080
   ttcgcctgtg taaaatgcac aaaaagggtt tgctaagcaa attgatattt gaaatgaaag 4140
   atggattagg tgagagactt agtttattca ggaataagag caaaaccaac cacatcaaaa 4200
   aatgtatttc actgagacag ccttttgtac atacagcagg gtgctgttta cagatttcaa 4260
   gaaaggagaa aaagaatgct tatactgaga ttacctacct cccagtagtg aaaataataa 4320
   atttatccct tgaaatataa 4340
<210> 26
   <211> 4240
   <212> DNA
   <213> Homo sapiens
<400> 26
   aactacgagt cccgacgtgc cgcgcgcggc atgcagaagc ttcactgagc gggcactgga     60
   ggtcgcgagc acgttgctag tgggcgcggc tggcggggcc gggggcggca gggggctagc    120
   gtccggctga gcccggcggg ttcctgcacg cggacccacg ctttgtcggg ctgctctgct    180
   gagtgacacc gccgtggcat ccgaggagct gtggtccagc ctgcgcggcg ggaagcggcg    240
   gcggcggctc cagcgtccta agagaagctg aaacctatca acactcttca aaatgcaatg    300
   gaatgtacca cggactgtat ctcgactggc acgcaggaca tgcttggaac cacataatgc    360
   tggtctcttt ggacactgtc aaaatgtaaa gggaccttta cttttataca atgctgaatc    420
   caaagtggtt ttggtacaag gccctcaaaa acaatggttg catttatctg ctgcccagtg    480
   tgttgcaaag gaaaggaggc cattggatgc tcatccaccc caaccaggag tccttcgcca    540
   taagcaaggg aagcaacatg tttcattcag gagggttttt tcatccagtg ccacagctca    600
   gggaactccg gaaaaaaagg aagagcctga tcctttgcaa gacaaatcta ttagtcttta    660
   tcaacgattc aagaagacat ttagacagta tggaaaagtt ctgattccag tgcatctaat    720
   aacttctggt gtttggtttg gaacatttta ttatgcagcc ttgaaaggag tgaatgtcgt    780
   tccttttcta gaactcattg ggttacctga cagtgtggta agcatcctga aaaactccca    840
   gagtggaaat gccctcacag catatgcctt gtttaagatt gcaacacctg ctcggtatac    900
   cgtgactttg ggaggaacat ctgtcactgt gaagtatctg cgcagtcatg gctacatgtc    960
   cacgccgcca cccgtcaagg agtatctgca ggacaggatg gaagagacaa aggagcttat   1020
   cacagagaaa atggaagaaa caaaagatag actcactgaa aagttacaag aaaccaaaga   1080
   aaaagtttcc tttaagaaaa aagtggaata aggtgcctta tatagcagta tagaaaattc   1140
   ctgcacttta accctttgga aactatgggc aaagatacat gtgtctgatt atttttttgg   1200
   ttagttgccg aaatatacta gttctctgag ggttaaagaa gtaaaatacc tttttaaagt   1260
   taaatatcac tagaaaaatc agtgttatta caagggaaga aatgaaccca gtttaagaat   1320
   ttgccatcag tagcagtatt aagcagtggt taatgtctta gaagtcagac ttctttttca   1380
   aggtcttcag aaccacactt gatttctgtt ttgttgcagc tgtaattgac acatactagg   1440
   cagctgactc cttgaatatc cagtgtgacc cataaaatag tctgttaata ccggatctta   1500
   atttttatgt tattcattaa gattttaact atattcagta cgtaatttgg agacaaacta   1560
   gcatcatcaa aactgcctgt aaataaggtg tttagtcttt ctataaaaac agaatagagc   1620
   agttacctac cagttaaaat atcttatatg aagaaaatag aataaagatc cagtcatata   1680
   tgtaaataag atgtactgat tgtacgtaaa tgaaaaatgg accctttaaa aattattttt   1740
   acctgaagct tgtcataatt tttttaaagc aaatatatat atggtgatgg tacttttcaa   1800
   agtgtgtatt agtggtgatc acctcaaaca taaacctctg ttgtgaatca tttgtgtcct   1860
   tttcaactgt ctttcagagg aaaggtaaaa aatcattaaa ccttaaattc attgttaaaa   1920
   tcaaatattt gtcagcagta actcaagctc atggttctca agcagaaaaa ggtttgggag   1980
   actaaaaatg gagtcaggtt ttcatggaga ctgctaactc cttggggtag acatgggcct   2040
   tgcctcagca aaccagtgca atttcccaat gtcttagttt cagaattcat gctgatttca   2100
   ttatggagta aagttttaaa ttgtggctgc tgctttttgt cttacattga agggaaatat   2160
   ccttgtttga gcattctact tgattttttt tttttttttt tttttttttg agacggagcc   2220
   tccctctctc tctcccagac tggagtgcaa tggtacaatc tcggctcact gcatcctctg   2280
   cctcccaggt tcaagtgatc ctcccgcctc agctttccaa gtagctggga gtataagcgt   2340
   gtgccaccac actcagctaa tttttgtatt tttagtggag gtggagtttc accatgttag   2400
   ccaggctagt ctccaactcc tgacctcagc ctcccgagta gctgggacta caggcgccca   2460
   ccaccacgcc cagctaattt ttttgtattt ttagtagaga tggggtttca ccatcttaac   2520
   aaagctatca ttaaatcatt tgcttgtaat gaaattcagc cgttgtcagc tgtgagcgtt   2580
   gcggggctgg tggggtgtgt ttgagtatgt aagtgtctat ttcctgtgct ctaacagtga   2640
   ctatttcagt tctaaccctt caattgctaa ttggatgagg gaatggcctc ttagattgtc   2700
   cttgttttga cttatctgct aaggcgagag aatgtctggg tttgccacac agtcccgcag   2760
   ggacccctgc tctttgccag gatttttata tcaagtactt agtttggcca aatttagagt   2820
   atagtttaaa ggggaaaaaa agtttgtatt tgatgagtct aattactata ttagaatgct   2880
   ttttaaatga atatgctgtg attggaatct cttcttgggg gccggggaga gtgctgagag   2940
   atgcagctgt gtctctgctc tctgcagtct tgggcaggca gcaccgctgt ctcctgatac   3000
   agagggttga atgcactgct tcacattgat gccacatttt caaatctaaa catgattaac 3060
   atgtttttta agtatttatc aaataattca catttttgca ggtttaagtg ctgtttttta 3120
   agatattaaa catgttcctt gtttcttttt tctttttaac cactggagac tatgtgaaaa 3180
   tcttgtgtta tatggaattt ttctaaaaca atttcctttt acttttcatg ttgcatattt 3240
   tccagtgggg ataaattgga ggttcttatt actaataatc ctgaaactaa aatttgcttt 3300
   tcctgcatag acatcttttg ttagtccatt tgagttttgt tgccatccag tttcatgatc 3360
   atgttgtgtt agtctaatag acaatgagtt actgcttgaa tgatatgaaa ttctctagta 3420
   aaaaatgctc tgaagatgtg agaagctctg ttgtattact tggcaccagg aatgttacaa 3480
   aaatgttgtt gctagcatat tgctaaccta gtccttaagt ttcagggatt cctgtaatat 3540
   gttgagtttg gagtattaat agcaggctaa cagaatacag taccaatatg ccaagttaaa 3600
   taattgagaa tatatacagt atagtttgct atgtataata gacctcattt catttacagt 3660
   acacctcaca cttgggatta attcacctat aacctctgtg aactgcgagt gctcggcaga 3720
   agcagccctg tgtcttacat gtctacattt atatttatat ttatatttat ttttttgaga 3780
   cagagtctca ctctgtcacc caggctagag tgcagtggca tgatcatagc tcacttgcgt 3840
   cttgaactcc tggctcaagt gatcctccca cctcagcctg ctgagtagct gggactacag 3900
   gtgcacacca ccatgcctgg ctaaaggcat gtctgcattt atgcgaggtg tgttgaccgg 3960
   gtggggaggg agctaggact gagattcgcc tgtgtaaaat gcacaaaaag ggtttgctaa 4020
   gcaaattgat atttgaaatg aaagatggat taggtgagag acttagttta ttcaggaata 4080
   agagcaaaac caaccacatc aaaaaatgta tttcactgag acagcctttt gtacatacag 4140
   cagggtgctg tttacagatt tcaagaaagg agaaaaagaa tgcttatact gagattacct 4200
   acctcccagt agtgaaaata ataaatttat cccttgaaat 4240
<210> 27
   <211> 402
   <212> PRT
   <213> Homo sapiens
<400> 27
   Met Met Val Ala Leu Arg Gly Ala Ser Ala Leu Leu Val Leu Phe Leu
   1 5 10 15
   Ala Ala Phe Leu Pro Pro Pro Gln Cys Thr Gln Asp Pro Ala Met Val
   20 25 30
   His Tyr Ile Tyr Gln Arg Phe Arg Val Leu Glu Gln Gly Leu Glu Lys
   35 40 45
   Cys Thr Gln Ala Thr Arg Ala Tyr Ile Gln Glu Phe Gln Glu Phe Ser
       50                  55                  60
   Lys Asn Ile Ser Val Met Leu Gly Arg Cys Gln Thr Tyr Thr Ser Glu
   65                  70                  75                  80
   Tyr Lys Ser Ala Val Gly Asn Leu Ala Leu Arg Val Glu Arg Ala Gln
                   85                  90                  95
   Arg Glu Ile Asp Tyr Ile Gln Tyr Leu Arg Glu Ala Asp Glu Cys Ile
               100                 105                 110
   Glu Ser Glu Asp Lys Thr Leu Ala Glu Met Leu Leu Gln Glu Ala Glu
           115                 120                 125
   Glu Glu Lys Lys Ile Arg Thr Leu Leu Asn Ala Ser Cys Asp Asn Met
       130                 135                 140
   Leu Met Gly Ile Lys Ser Leu Lys Ile Val Lys Lys Met Met Asp Thr
   145                 150                 155                 160
   His Gly Ser Trp Met Lys Asp Ala Val Tyr Asn Ser Pro Lys Val Tyr
                   165                 170                 175
   Leu Leu Ile Gly Ser Arg Asn Asn Thr Val Trp Glu Phe Ala Asn Ile
               180                 185                 190
   Arg Ala Phe Met Glu Asp Asn Thr Lys Pro Ala Pro Arg Lys Gln Ile
           195                 200                 205
   Leu Thr Leu Ser Trp Gln Gly Thr Gly Gln Val Ile Tyr Lys Gly Phe
       210                 215                 220
   Leu Phe Phe His Asn Gln Ala Thr Ser Asn Glu Ile Ile Lys Tyr Asn
   225                 230                 235                 240
   Leu Gln Lys Arg Thr Val Glu Asp Arg Met Leu Leu Pro Gly Gly Val
                   245                 250                 255
   Gly Arg Ala Leu Val Tyr Gln His Ser Pro Ser Thr Tyr Ile Asp Leu
               260                 265                 270
   Ala Val Asp Glu His Gly Leu Trp Ala Ile His Ser Gly Pro Gly Thr
           275                 280                 285
   His Ser His Leu Val Leu Thr Lys Ile Glu Pro Gly Thr Leu Gly Val
       290                 295                 300
   Glu His Ser Trp Asp Thr Pro Cys Arg Ser Gln Asp Ala Glu Ala Ser
   305                 310                 315                 320
   Phe Leu Leu Cys Gly Val Leu Tyr Val Val Tyr Ser Thr Gly Gly Gln
                   325                 330                 335
   Gly Pro His Arg Ile Thr Cys Ile Tyr Asp Pro Leu Gly Thr Ile Ser
               340                 345                 350
   Glu Glu Asp Leu Pro Asn Leu Phe Phe Pro Lys Arg Pro Arg Ser His
           355                 360                 365
   Ser Met Ile His Tyr Asn Pro Arg Asp Lys Gln Leu Tyr Ala Trp Asn
       370                 375                 380
   Glu Gly Asn Gln Ile Ile Tyr Lys Leu Gln Thr Lys Arg Lys Leu Pro
   385                 390                 395                 400
   Leu Lys
<210> 28
   <211> 2786
   <212> DNA
   <213> Homo sapiens
<400> 28
   gggggaaggg aacgggggga agccatcttc accccccacc ccaatgcaca cacaattaag     60
   ccaggaagca gcttgcaacc actagcctgg ggagggtccg catgtgtcaa gggtgagggc    120
   aacagatgct ggacccaggg agctctctgc cacaggtcag tctacaaggc ctcagggacc    180
   aacttgccaa cagctggact tgatcactag ctggcaaact gagctcacgt atcgggtgga    240
   ataacaagcg gactttgctc tctgctgtgc aaaacgctgt ttttagagga tttgccacag    300
   cagcggatag agcaggagag caccaccgga gcccttgaga catccttgag aagagccaca    360
   gcataagaga ctgccctgct tggtgttttg caggatgatg gtggcccttc gaggagcttc    420
   tgcattgctg gttctgttcc ttgcagcttt tctgcccccg ccgcagtgta cccaggaccc    480
   agccatggtg cattacatct accagcgctt tcgagtcttg gagcaagggc tggaaaaatg    540
   tacccaagca acgagggcat acattcaaga attccaagag ttctcaaaaa atatatctgt    600
   catgctggga agatgtcaga cctacacaag tgagtacaag agtgcagtgg gtaacttggc    660
   actgagagtt gaacgtgccc aacgggagat tgactacata caataccttc gagaggctga    720
   cgagtgcatc gaatcagagg acaagacact ggcagaaatg ttgctccaag aagctgaaga    780
   agagaaaaag atccggactc tgctgaatgc aagctgtgac aacatgctga tgggcataaa    840
   gtctttgaaa atagtgaaga agatgatgga cacacatggc tcttggatga aagatgctgt 900
   ctataactct ccaaaggtgt acttattaat tggatccaga aacaacactg tttgggaatt 960
   tgcaaacata cgggcattca tggaggataa caccaagcca gctccccgga agcaaatcct 1020
   aacactttcc tggcagggaa caggccaagt gatctacaaa ggttttctat tttttcataa 1080
   ccaagcaact tctaatgaga taatcaaata taacctgcag aagaggactg tggaagatcg 1140
   aatgctgctc ccaggagggg taggccgagc attggtttac cagcactccc cctcaactta 1200
   cattgacctg gctgtggatg agcatgggct ctgggccatc cactctgggc caggcaccca 1260
   tagccatttg gttctcacaa agattgagcc gggcacactg ggagtggagc attcatggga 1320
   taccccatgc agaagccagg atgctgaagc ctcattcctc ttgtgtgggg ttctctatgt 1380
   ggtctacagt actgggggcc agggccctca tcgcatcacc tgcatctatg atccactggg 1440
   cactatcagt gaggaggact tgcccaactt gttcttcccc aagagaccaa gaagtcactc 1500
   catgatccat tacaacccca gagataagca gctctatgcc tggaatgaag gaaaccagat 1560
   catttacaaa ctccagacaa agagaaagct gcctctgaag taatgcatta cagctgtgag 1620
   aaagagcact gtggctttgg cagctgttct acaggacagt gaggctatag ccccttcaca 1680
   atatagtatc cctctaatca cacacaggaa gagtgtgtag aagtggaaat acgtatgcct 1740
   cctttcccaa atgtcactgc cttaggtatc ttccaagagc ttagatgaga gcatatcatc 1800
   aggaaagttt caacaatgtc cattactccc ccaaacctcc tggctctcaa ggatgaccac 1860
   attctgatac agcctacttc aagccttttg ttttactgct ccccagcatt tactgtaact 1920
   ctgccatctt ccctcccaca attagagttg tatgccagcc cctaatattc accactggct 1980
   tttctctccc ctggcctttg ctgaagctct tccctctttt tcaaatgtct attgatattc 2040
   tcccattttc actgcccaac taaaatacta ttaatatttc tttcttttct tttctttttt 2100
   ttgagacaag gtctcactat gttgcccagg ctggtctcaa actccagagc tcaagagatc 2160
   ctcctgcctc agcctcctaa gtacctggga ttacaggcat gtgccaccac acctggctta 2220
   aaatactatt tcttattgag gtttaacctc tatttcccct agccctgtcc ttccactaag 2280
   cttggtagat gtaataataa agtgaaaata ttaacatttg aatatcgctt tccaggtgtg 2340
   gagtgtttgc acatcattta attctcgttt cacctttgtg aaacatgcac aagtctttac 2400
   agctgtcatt ctagagttta ggtgagtaac acaattacaa agtgaaagat acagctagaa 2460
   aatactacaa atcccatagt ttttccattg cccaaggaag catcaaatac gtatgtttgt 2520
   tcacctactc ttatagtcaa tgcgttcatc gtttcagcct aaaaataata gtctgtccct 2580
   ttagccagtt ttcatgtctg cacaagacct ttcaataggc ctttcaaatg ataattcctc 2640
   cagaaaacca gtctaagggt gaggacccca actctagcct cctcttgtct tgctgtcctc 2700
   tgtttctctc tttctgcttt aaattcaata aaagtgacac tgagcaaata acctcatcag 2760
   gttatatttg cccacatacc ctaagc 2786
<210> 29
   <211> 1328
   <212> PRT
   <213> Homo sapiens
<400> 29
   Met Ser Ser Gly Asp Pro Ala His Leu Gly Leu Cys Leu Trp Leu Trp
   1               5                   10                  15
   Leu Gly Ala Thr Leu Gly Arg Glu Gln Val Gln Ala Ser Gly Leu Leu
               20                  25                  30
   Arg Leu Ala Val Leu Pro Glu Asp Arg Leu Gln Met Lys Trp Arg Glu
           35                  40                  45
   Ser Glu Gly Ser Gly Leu Gly Tyr Leu Val Gln Val Lys Pro Met Ala
       50                  55                  60
   Gly Asp Ser Glu Gln Glu Val Ile Leu Thr Thr Lys Thr Pro Lys Ala
   65                  70                  75                  80
   Thr Val Gly Gly Leu Ser Pro Ser Lys Gly Tyr Thr Leu Gln Ile Phe
                   85                  90                  95
   Glu Leu Thr Gly Ser Gly Arg Phe Leu Leu Ala Arg Arg Glu Phe Val
               100                 105                 110
   Ile Glu Asp Leu Lys Ser Ser Ser Leu Asp Arg Ser Ser Gln Arg Pro
           115                 120                 125
   Leu Gly Ser Gly Ala Pro Glu Pro Thr Pro Ser His Thr Gly Ser Pro
       130                 135                 140
   Asp Pro Glu Gln Ala Ser Glu Pro Gln Val Ala Phe Thr Pro Ser Gln
   145                 150                 155                 160
   Asp Pro Arg Thr Pro Gly Gly Ser Glu Trp Arg Glu Thr Gly Pro Gln
                   165                 170                 175
   Phe Arg Cys Leu Pro Pro Val Pro Ala Asp Met Val Phe Leu Val Asp
               180                 185                 190
   Gly Ser Trp Ser Ile Gly His Ser His Phe Gln Gln Val Lys Asp Phe
           195                 200                 205
   Leu Ala Ser Val Ile Ala Pro Phe Glu Ile Gly Pro Asp Lys Val Gln
       210                 215                 220
   Val Gly Leu Thr Gln Tyr Ser Gly Asp Ala Gln Thr Glu Trp Asp Leu
   225                 230                 235                 240
   Asn Ser Leu Ser Thr Lys Glu Gln Val Leu Ala Ala Val Arg Arg Leu
                   245                 250                 255
   Arg Tyr Lys Gly Gly Asn Thr Phe Thr Gly Leu Ala Leu Thr His Val
               260                 265                 270
   Leu Gly Gln Asn Leu Gln Pro Ala Ala Gly Leu Arg Pro Glu Ala Ala
           275                 280                 285
   Lys Val Val Ile Leu Val Thr Asp Gly Lys Ser Gln Asp Asp Val His
       290                 295                 300
   Thr Ala Ala Arg Val Leu Lys Asp Leu Gly Val Asn Val Phe Ala Val
   305                 310                 315                 320
   Gly Val Lys Asn Ala Asp Glu Ala Glu Leu Arg Leu Leu Ala Ser Pro
                   325                 330                 335
   Pro Arg Asp Ile Thr Val His Ser Val Leu Asp Phe Leu Gln Leu Gly
               340                 345                 350
   Ala Leu Ala Gly Leu Leu Ser Arg Leu Ile Cys Gln Arg Leu Gln Gly
           355                 360                 365
   Gly Ser Pro Arg Gln Gly Pro Ala Ala Ala Pro Ala Leu Asp Thr Leu
       370                 375                 380
   Pro Ala Pro Thr Ser Leu Val Leu Ser Gln Val Thr Ser Ser Ser Ile
   385                 390                 395                 400
   Arg Leu Ser Trp Thr Pro Ala Pro Arg His Pro Leu Lys Tyr Leu Ile
                   405                 410                 415
   Val Trp Arg Ala Ser Arg Gly Gly Thr Pro Arg Glu Val Val Val Glu
               420                 425                 430
   Gly Pro Ala Ala Ser Thr Glu Leu His Asn Leu Ala Ser Arg Thr Glu
           435                 440                 445
   Tyr Leu Val Ser Val Phe Pro Ile Tyr Glu Gly Gly Val Gly Glu Gly
       450                 455                 460
   Leu Arg Gly Leu Val Thr Thr Ala Pro Leu Pro Pro Pro Arg Ala Leu
   465                 470                 475                 480
   Thr Leu Ala Ala Val Thr Pro Arg Thr Val His Leu Thr Trp Gln Pro
                   485                 490                 495
   Ser Ala Gly Ala Thr His Tyr Leu Val Arg Cys Ser Pro Ala Ser Pro
               500                 505                 510
   Lys Gly Glu Glu Glu Glu Arg Glu Val Gln Val Gly Arg Pro Glu Val
           515                 520                 525
   Leu Leu Asp Gly Leu Glu Pro Gly Arg Asp Tyr Glu Val Ser Val Gln
       530                 535                 540
   Ser Leu Arg Gly Pro Glu Gly Ser Glu Ala Arg Gly Ile Arg Ala Arg
   545                 550                 555                 560
   Thr Pro Thr Leu Ala Pro Pro Arg His Leu Gly Phe Ser Asp Val Ser
                   565                 570                 575
   His Asp Ala Ala Arg Val Phe Trp Glu Gly Ala Pro Arg Pro Val Arg
               580                 585                 590
   Leu Val Arg Val Thr Tyr Val Ser Ser Glu Gly Gly His Ser Gly Gln
           595                 600                 605
   Thr Glu Ala Pro Gly Asn Ala Thr Ser Ala Thr Leu Gly Pro Leu Ser
       610                 615                 620
   Ser Ser Thr Thr Tyr Thr Val Arg Val Thr Cys Leu Tyr Pro Gly Gly
   625                 630                 635                 640
   Gly Ser Ser Thr Leu Thr Gly Arg Val Thr Thr Lys Lys Ala Pro Ser
                   645                 650                 655
   Pro Ser Gln Leu Ser Met Thr Glu Leu Pro Gly Asp Ala Val Gln Leu
               660                 665                 670
   Ala Trp Val Ala Ala Ala Pro Ser Gly Val Leu Val Tyr Gln Ile Thr
           675                 680                 685
   Trp Thr Pro Leu Gly Glu Gly Lys Ala His Glu Ile Ser Val Pro Gly
       690                 695                 700
   Asn Leu Gly Thr Ala Val Leu Pro Gly Leu Gly Arg His Thr Glu Tyr
   705                 710                 715                 720
   Asp Val Thr Ile Leu Ala Tyr Tyr Arg Asp Gly Ala Arg Ser Asp Pro
                   725                 730                 735
   Val Ser Leu Arg Tyr Thr Pro Ser Thr Val Ser Arg Ser Pro Pro Ser
               740                 745                 750
   Asn Leu Ala Leu Ala Ser Glu Thr Pro Asp Ser Leu Gln Val Ser Trp
           755                 760                 765
   Thr Pro Pro Leu Gly Arg Val Leu His Tyr Trp Leu Thr Tyr Ala Pro
       770                 775                 780
   Ala Ser Gly Leu Gly Pro Glu Lys Ser Val Ser Val Pro Gly Ala Arg
   785                 790                 795                 800
   Ser His Val Thr Leu Pro Asp Leu Gln Ala Ala Thr Lys Tyr Arg Val
                   805                 810                 815
   Leu Val Ser Ala Ile Tyr Ala Ala Gly Arg Ser Glu Ala Val Ser Ala
               820                 825                 830
   Thr Gly Gln Thr Ala Cys Pro Ala Leu Arg Pro Asp Gly Ser Leu Pro
           835                 840                 845
   Gly Phe Asp Leu Met Val Ala Phe Ser Leu Val Glu Lys Ala Tyr Ala
       850                 855                 860
   Ser Ile Arg Gly Val Ala Met Glu Pro Ser Ala Phe Gly Gly Thr Pro
   865                 870                 875                 880
   Thr Phe Thr Leu Phe Lys Asp Ala Gln Leu Thr Arg Arg Val Ser Asp
                   885                 890                 895
   Val Tyr Pro Ala Pro Leu Pro Pro Glu His Thr Ile Val Phe Leu Val
               900                 905                 910
   Arg Leu Leu Pro Glu Thr Pro Arg Glu Ala Phe Ala Leu Trp Gln Met
           915                 920                 925
   Thr Ala Glu Asp Phe Gln Pro Leu Leu Gly Val Leu Leu Asp Ala Gly
       930                 935                 940
   Lys Lys Ser Leu Thr Tyr Phe His Arg Asp Pro Arg Ala Ala Leu Gln
   945                 950                 955                 960
   Glu Ala Thr Phe Asp Pro Gln Glu Val Arg Lys Ile Phe Phe Gly Ser
                   965                 970                 975
   Phe His Lys Val His Val Ala Val Gly Arg Ser Lys Val Arg Leu Tyr
               980                 985                 990
   Val Asp Cys Arg Lys Val Ala Glu  Arg Pro Leu Gly Glu  Met Gly Ser
           995                 1000                1005
   Pro Pro  Ala Ala Gly Phe Val  Thr Leu Gly Arg Leu  Ala Lys Ala
       1010                 1015                 1020
   Arg Gly  Pro Arg Ser Ser Ser  Ala Ala Phe Gln Leu  Gln Met Leu
       1025                 1030                 1035
   Gln Ile  Val Cys Ser Asp Thr  Trp Ala Asp Glu Asp  Arg Cys Cys
       1040                 1045                 1050
   Glu Leu  Pro Ala Ser Arg Asp  Gly Glu Thr Cys Pro  Ala Phe Val
       1055                 1060                 1065
   Ser Ala  Cys Ser Cys Ser Ser  Glu Thr Pro Gly Pro  Pro Gly Pro
       1070                 1075                 1080
   Gln Gly  Pro Pro Gly Leu Pro  Gly Arg Asn Gly Thr  Pro Gly Glu
       1085                 1090                 1095
   Gln Gly  Phe Pro Gly Pro Arg  Gly Glu Pro Gly Pro  Pro Gly Gln
       1100                 1105                 1110
   Met Gly  Pro Glu Gly Pro Gly  Gly Gln Gln Gly Ser  Pro Gly Thr
       1115                 1120                 1125
   Gln Gly  Arg Ala Val Gln Gly  Pro Val Gly Pro Pro  Gly Val Lys
       1130                 1135                 1140
   Gly Glu  Lys Gly Asp His Gly  Leu Pro Gly Leu Gln  Gly His Pro
       1145                 1150                 1155
   Gly His  Gln Gly Ile Pro Gly  Arg Val Gly Leu Gln  Gly Pro Lys
       1160                 1165                 1170
   Gly Met  Arg Gly Leu Glu Gly  Thr Ala Gly Leu Pro  Gly Pro Gly
       1175                 1180                 1185
   Pro Arg  Gly Phe Gln Gly Met  Ala Gly Ala Arg Gly  Thr Ser Gly
       1190                 1195                 1200
   Glu Arg  Gly Pro Pro Gly Thr  Val Gly Pro Thr Gly  Leu Pro Gly
       1205                 1210                 1215
   Pro Lys Gly Glu Arg Gly Glu Lys Gly Glu Pro Gln Ser Leu Ala
       1220                1225                1230
   Thr Leu Tyr Gln Leu Val Ser Gln Ala Cys Glu Ser Ala Ile Gln
       1235                1240                1245
   Thr His Val Ser Lys Phe Asp Ser Phe His Glu Asn Thr Arg Pro
       1250                1255                1260
   Pro Met Pro Ile Leu Glu Gln Lys Leu Glu Pro Gly Thr Glu Pro
       1265                1270                1275
   Leu Gly Ser Pro Gly Thr Arg Ser Lys Ala Leu Val Pro Gly Glu
       1280                1285                1290
   Trp Gly Arg Gly Gly Arg His Leu Glu Gly Arg Gly Glu Pro Gly
       1295                1300                1305
   Ala Val Gly Gln Met Gly Ser Pro Gly Gln Gln Gly Ala Ser Thr
       1310                1315                1320
   Gln Gly Leu Trp Glu
       1325
<210> 30
   <211> 1278
   <212> PRT
   <213> Homo sapiens
<400> 30
   Met Ser Ser Gly Asp Pro Ala His Leu Gly Leu Cys Leu Trp Leu Trp
   1               5                   10                  15
   Leu Gly Ala Thr Leu Gly Arg Glu Gln Val Gln Ala Ser Gly Leu Leu
               20                  25                  30
   Arg Leu Ala Val Leu Pro Glu Asp Arg Leu Gln Met Lys Trp Arg Glu
           35                  40                  45
   Ser Glu Gly Ser Gly Leu Gly Tyr Leu Val Gln Val Lys Pro Met Ala
       50                  55                  60
   Gly Asp Ser Glu Gln Glu Val Ile Leu Thr Thr Lys Thr Pro Lys Ala
   65                  70                  75                  80
   Thr Val Gly Gly Leu Ser Pro Ser Lys Gly Tyr Thr Leu Gln Ile Phe
                   85                  90                  95
   Glu Leu Thr Gly Ser Gly Arg Phe Leu Leu Ala Arg Arg Glu Phe Val
               100                 105                 110
   Ile Glu Asp Leu Lys Ser Ser Ser Leu Asp Arg Ser Ser Gln Arg Pro
           115                 120                 125
   Leu Gly Ser Gly Ala Pro Glu Pro Thr Pro Ser His Thr Gly Ser Pro
       130                 135                 140
   Asp Pro Glu Gln Ala Ser Glu Pro Gln Val Ala Phe Thr Pro Ser Gln
   145                 150                 155                 160
   Asp Pro Arg Thr Pro Gly Gly Ser Glu Trp Arg Glu Thr Gly Pro Gln
                   165                 170                 175
   Phe Arg Cys Leu Pro Pro Val Pro Ala Asp Met Val Phe Leu Val Asp
               180                 185                 190
   Gly Ser Trp Ser Ile Gly His Ser His Phe Gln Gln Val Lys Asp Phe
           195                 200                 205
   Leu Ala Ser Val Ile Ala Pro Phe Glu Ile Gly Pro Asp Lys Val Gln
       210                 215                 220
   Val Gly Leu Thr Gln Tyr Ser Gly Asp Ala Gln Thr Glu Trp Asp Leu
   225                 230                 235                 240
   Asn Ser Leu Ser Thr Lys Glu Gln Val Leu Ala Ala Val Arg Arg Leu
                   245                 250                 255
   Arg Tyr Lys Gly Gly Asn Thr Phe Thr Gly Leu Ala Leu Thr His Val
               260                 265                 270
   Leu Gly Gln Asn Leu Gln Pro Ala Ala Gly Leu Arg Pro Glu Ala Ala
           275                 280                 285
   Lys Val Val Ile Leu Val Thr Asp Gly Lys Ser Gln Asp Asp Val His
       290                 295                 300
   Thr Ala Ala Arg Val Leu Lys Asp Leu Gly Val Asn Val Phe Ala Val
   305                 310                 315                 320
   Gly Val Lys Asn Ala Asp Glu Ala Glu Leu Arg Leu Leu Ala Ser Pro
                   325                 330                 335
   Pro Arg Asp Ile Thr Val His Ser Val Leu Asp Phe Leu Gln Leu Gly
               340                 345                 350
   Ala Leu Ala Gly Leu Leu Ser Arg Leu Ile Cys Gln Arg Leu Gln Gly
           355                 360                 365
   Gly Ser Pro Arg Gln Gly Pro Ala Ala Ala Pro Ala Leu Asp Thr Leu
       370                 375                 380
   Pro Ala Pro Thr Ser Leu Val Leu Ser Gln Val Thr Ser Ser Ser Ile
   385                 390                 395                 400
   Arg Leu Ser Trp Thr Pro Ala Pro Arg His Pro Leu Lys Tyr Leu Ile
                   405                 410                 415
   Val Trp Arg Ala Ser Arg Gly Gly Thr Pro Arg Glu Val Val Val Glu
               420                 425                 430
   Gly Pro Ala Ala Ser Thr Glu Leu His Asn Leu Ala Ser Arg Thr Glu
           435                 440                 445
   Tyr Leu Val Ser Val Phe Pro Ile Tyr Glu Gly Gly Val Gly Glu Gly
       450                 455                 460
   Leu Arg Gly Leu Val Thr Thr Ala Pro Leu Pro Pro Pro Arg Ala Leu
   465                 470                 475                 480
   Thr Leu Ala Ala Val Thr Pro Arg Thr Val His Leu Thr Trp Gln Pro
                   485                 490                 495
   Ser Ala Gly Ala Thr His Tyr Leu Val Arg Cys Ser Pro Ala Ser Pro
               500                 505                 510
   Lys Gly Glu Glu Glu Glu Arg Glu Val Gln Val Gly Arg Pro Glu Val
           515                 520                 525
   Leu Leu Asp Gly Leu Glu Pro Gly Arg Asp Tyr Glu Val Ser Val Gln
       530                 535                 540
   Ser Leu Arg Gly Pro Glu Gly Ser Glu Ala Arg Gly Ile Arg Ala Arg
   545                 550                 555                 560
   Thr Pro Thr Leu Ala Pro Pro Arg His Leu Gly Phe Ser Asp Val Ser
                   565                 570                 575
   His Asp Ala Ala Arg Val Phe Trp Glu Gly Ala Pro Arg Pro Val Arg
               580                 585                 590
   Leu Val Arg Val Thr Tyr Val Ser Ser Glu Gly Gly His Ser Gly Gln
           595                 600                 605
   Thr Glu Ala Pro Gly Asn Ala Thr Ser Ala Thr Leu Gly Pro Leu Ser
       610                 615                 620
   Ser Ser Thr Thr Tyr Thr Val Arg Val Thr Cys Leu Tyr Pro Gly Gly
   625                 630                 635                 640
   Gly Ser Ser Thr Leu Thr Gly Arg Val Thr Thr Lys Lys Ala Pro Ser
                   645                 650                 655
   Pro Ser Gln Leu Ser Met Thr Glu Leu Pro Gly Asp Ala Val Gln Leu
               660                 665                 670
   Ala Trp Val Ala Ala Ala Pro Ser Gly Val Leu Val Tyr Gln Ile Thr
           675                 680                 685
   Trp Thr Pro Leu Gly Glu Gly Lys Ala His Glu Ile Ser Val Pro Gly
       690                 695                 700
   Asn Leu Gly Thr Ala Val Leu Pro Gly Leu Gly Arg His Thr Glu Tyr
   705                 710                 715                 720
   Asp Val Thr Ile Leu Ala Tyr Tyr Arg Asp Gly Ala Arg Ser Asp Pro
                   725                 730                 735
   Val Ser Leu Arg Tyr Thr Pro Ser Thr Val Ser Arg Ser Pro Pro Ser
               740                 745                 750
   Asn Leu Ala Leu Ala Ser Glu Thr Pro Asp Ser Leu Gln Val Ser Trp
           755                 760                 765
   Thr Pro Pro Leu Gly Arg Val Leu His Tyr Trp Leu Thr Tyr Ala Pro
       770                 775                 780
   Ala Ser Gly Leu Gly Pro Glu Lys Ser Val Ser Val Pro Gly Ala Arg
   785                 790                 795                 800
   Ser His Val Thr Leu Pro Asp Leu Gln Ala Ala Thr Lys Tyr Arg Val
                   805                 810                 815
   Leu Val Ser Ala Ile Tyr Ala Ala Gly Arg Ser Glu Ala Val Ser Ala
               820                 825                 830
   Thr Gly Gln Thr Ala Cys Pro Ala Leu Arg Pro Asp Gly Ser Leu Pro
           835                 840                 845
   Gly Phe Asp Leu Met Val Ala Phe Ser Leu Val Glu Lys Ala Tyr Ala
       850                 855                 860
   Ser Ile Arg Gly Val Ala Met Glu Pro Ser Ala Phe Gly Gly Thr Pro
   865                 870                 875                 880
   Thr Phe Thr Leu Phe Lys Asp Ala Gln Leu Thr Arg Arg Val Ser Asp
                   885                 890                 895
   Val Tyr Pro Ala Pro Leu Pro Pro Glu His Thr Ile Val Phe Leu Val
               900                 905                 910
   Arg Leu Leu Pro Glu Thr Pro Arg Glu Ala Phe Ala Leu Trp Gln Met
           915                 920                 925
   Thr Ala Glu Asp Phe Gln Pro Leu Leu Gly Val Leu Leu Asp Ala Gly
       930                 935                 940
   Lys Lys Ser Leu Thr Tyr Phe His Arg Asp Pro Arg Ala Ala Leu Gln
   945                 950                 955                 960
   Glu Ala Thr Phe Asp Pro Gln Glu Val Arg Lys Ile Phe Phe Gly Ser
                   965                 970                 975
   Phe His Lys Val His Val Ala Val Gly Arg Ser Lys Val Arg Leu Tyr
               980                 985                 990
   Val Asp Cys Arg Lys Val Ala Glu  Arg Pro Leu Gly Glu  Met Gly Ser
           995                 1000                1005
   Pro Pro  Ala Ala Gly Phe Val  Thr Leu Gly Arg Leu  Ala Lys Ala
       1010                1015                1020
   Arg Gly  Pro Arg Ser Ser Ser  Ala Ala Phe Gln Leu  Gln Met Leu
       1025                1030                1035
   Gln Ile  Val Cys Ser Asp Thr  Trp Ala Asp Glu Asp  Arg Cys Cys
       1040                1045                1050
   Glu Leu  Pro Ala Ser Arg Asp  Gly Glu Thr Cys Pro  Ala Phe Val
       1055                1060                1065
   Ser Ala  Cys Ser Cys Ser Ser  Glu Thr Pro Gly Pro  Pro Gly Pro
       1070                1075                1080
   Gln Gly  Pro Pro Gly Pro Pro  Gly Val Lys Gly Glu  Lys Gly Asp
       1085                1090                1095
   His Gly  Leu Pro Gly Leu Gln  Gly His Pro Gly His  Gln Gly Ile
       1100                1105                1110
   Pro Gly  Arg Val Gly Leu Gln  Gly Pro Lys Gly Met  Arg Gly Leu
       1115                 1120                 1125
   Glu Gly  Thr Ala Gly Leu Pro  Gly Pro Gly Pro Arg  Gly Phe Gln
       1130                 1135                 1140
   Gly Met  Ala Gly Ala Arg Gly  Thr Ser Gly Glu Arg  Gly Pro Pro
       1145                 1150                 1155
   Gly Thr  Val Gly Pro Thr Gly  Leu Pro Gly Pro Lys  Gly Glu Arg
       1160                 1165                 1170
   Gly Glu  Lys Gly Glu Pro Gln  Ser Leu Ala Thr Leu  Tyr Gln Leu
       1175                 1180                 1185
   Val Ser  Gln Ala Cys Glu Ser  Ala Ile Gln Thr His  Val Ser Lys
       1190                 1195                 1200
   Phe Asp  Ser Phe His Glu Asn  Thr Arg Pro Pro Met  Pro Ile Leu
       1205                 1210                 1215
   Glu Gln  Lys Leu Glu Pro Gly  Thr Glu Pro Leu Gly  Ser Pro Gly
       1220                 1225                 1230
   Thr Arg  Ser Lys Ala Leu Val  Pro Gly Glu Trp Gly  Arg Gly Gly
       1235                 1240                 1245
   Arg His  Leu Glu Gly Arg Gly  Glu Pro Gly Ala Val  Gly Gln Met
       1250                 1255                 1260
   Gly Ser  Pro Gly Gln Gln Gly  Ala Ser Thr Gln Gly  Leu Trp Glu
       1265                 1270                 1275
<210> 31
   <211> 1283
   <212> PRT
   <213> Homo sapiens
<400> 31
   Met Ser Ser Gly Asp Pro Ala His Leu Gly Leu Cys Leu Trp Leu Trp
   1               5                   10                  15
   Leu Gly Ala Thr Leu Gly Arg Glu Gln Val Gln Ala Ser Gly Leu Leu
               20                  25                  30
   Arg Leu Ala Val Leu Pro Glu Asp Arg Leu Gln Met Lys Trp Arg Glu
           35                  40                  45
   Ser Glu Gly Ser Gly Leu Gly Tyr Leu Val Gln Val Lys Pro Met Ala
       50                  55                  60
   Gly Asp Ser Glu Gln Glu Val Ile Leu Thr Thr Lys Thr Pro Lys Ala
   65                  70                  75                  80
   Thr Val Gly Gly Leu Ser Pro Ser Lys Gly Tyr Thr Leu Gln Ile Phe
                   85                  90                  95
   Glu Leu Thr Gly Ser Gly Arg Phe Leu Leu Ala Arg Arg Glu Phe Val
               100                 105                 110
   Ile Glu Asp Leu Lys Ser Ser Ser Leu Asp Arg Ser Ser Gln Arg Pro
           115                 120                 125
   Leu Gly Ser Gly Ala Pro Glu Pro Thr Pro Ser His Thr Gly Ser Pro
       130                 135                 140
   Asp Pro Glu Gln Ala Ser Glu Pro Gln Val Ala Phe Thr Pro Ser Gln
   145                 150                 155                 160
   Asp Pro Arg Thr Pro Ala Gly Pro Gln Phe Arg Cys Leu Pro Pro Val
                   165                 170                 175
   Pro Ala Asp Met Val Phe Leu Val Asp Gly Ser Trp Ser Ile Gly His
               180                 185                 190
   Ser His Phe Gln Gln Val Lys Asp Phe Leu Ala Ser Val Ile Ala Pro
           195                 200                 205
   Phe Glu Ile Gly Pro Asp Lys Val Gln Val Gly Leu Thr Gln Tyr Ser
       210                 215                 220
   Gly Asp Ala Gln Thr Glu Trp Asp Leu Asn Ser Leu Ser Thr Lys Glu
   225                 230                 235                 240
   Gln Val Leu Ala Ala Val Arg Arg Leu Arg Tyr Lys Gly Gly Asn Thr
                   245                 250                 255
   Phe Thr Gly Leu Ala Leu Thr His Val Leu Gly Gln Asn Leu Gln Pro
               260                 265                 270
   Ala Ala Gly Leu Arg Pro Glu Ala Ala Lys Val Val Ile Leu Val Thr
           275                 280                 285
   Asp Gly Lys Ser Gln Asp Asp Val His Thr Ala Ala Arg Val Leu Lys
       290                 295                 300
   Asp Leu Gly Val Asn Val Phe Ala Val Gly Val Lys Asn Ala Asp Glu
   305                 310                 315                 320
   Ala Glu Leu Arg Leu Leu Ala Ser Pro Pro Arg Asp Ile Thr Val His
                   325                 330                 335
   Ser Val Leu Asp Phe Leu Gln Leu Gly Ala Leu Ala Gly Leu Leu Ser
               340                 345                 350
   Arg Leu Ile Cys Gln Arg Leu Gln Gly Gly Ser Pro Arg Gln Gly Pro
           355                 360                 365
   Ala Ala Ala Pro Ala Leu Asp Thr Leu Pro Ala Pro Thr Ser Leu Val
       370                 375                 380
   Leu Ser Gln Val Thr Ser Ser Ser Ile Arg Leu Ser Trp Thr Pro Ala
   385                 390                 395                 400
   Pro Arg His Pro Leu Lys Tyr Leu Ile Val Trp Arg Ala Ser Arg Gly
                   405                 410                 415
   Gly Thr Pro Arg Glu Val Val Val Glu Gly Pro Ala Ala Ser Thr Glu
               420                 425                 430
   Leu His Asn Leu Ala Ser Arg Thr Glu Tyr Leu Val Ser Val Phe Pro
           435                 440                 445
   Ile Tyr Glu Gly Gly Val Gly Glu Gly Leu Arg Gly Leu Val Thr Thr
       450                 455                 460
   Ala Pro Leu Pro Pro Pro Arg Ala Leu Thr Leu Ala Ala Val Thr Pro
   465                 470                 475                 480
   Arg Thr Val His Leu Thr Trp Gln Pro Ser Ala Gly Ala Thr His Tyr
                   485                 490                 495
   Leu Val Arg Cys Ser Pro Ala Ser Pro Lys Gly Glu Glu Glu Glu Arg
               500                 505                 510
   Glu Val Gln Val Gly Arg Pro Glu Val Leu Leu Asp Gly Leu Glu Pro
           515                 520                 525
   Gly Arg Asp Tyr Glu Val Ser Val Gln Ser Leu Arg Gly Pro Glu Gly
       530                 535                 540
   Ser Glu Ala Arg Gly Ile Arg Ala Arg Thr Pro Thr Leu Ala Pro Pro
   545                 550                 555                 560
   Arg His Leu Gly Phe Ser Asp Val Ser His Asp Ala Ala Arg Val Phe
                   565                 570                 575
   Trp Glu Gly Ala Pro Arg Pro Val Arg Leu Val Arg Val Thr Tyr Val
               580                 585                 590
   Ser Ser Glu Gly Gly His Ser Gly Gln Thr Glu Ala Pro Gly Asn Ala
           595                 600                 605
   Thr Ser Ala Thr Leu Gly Pro Leu Ser Ser Ser Thr Thr Tyr Thr Val
       610                 615                 620
   Arg Val Thr Cys Leu Tyr Pro Gly Gly Gly Ser Ser Thr Leu Thr Gly
   625                 630                 635                 640
   Arg Val Thr Thr Lys Lys Ala Pro Ser Pro Ser Gln Leu Ser Met Thr
                   645                 650                 655
   Glu Leu Pro Gly Asp Ala Val Gln Leu Ala Trp Val Ala Ala Ala Pro
               660                 665                 670
   Ser Gly Val Leu Val Tyr Gln Ile Thr Trp Thr Pro Leu Gly Glu Gly
           675                 680                 685
   Lys Ala His Glu Ile Ser Val Pro Gly Asn Leu Gly Thr Ala Val Leu
       690                 695                 700
   Pro Gly Leu Gly Arg His Thr Glu Tyr Asp Val Thr Ile Leu Ala Tyr
   705                 710                 715                 720
   Tyr Arg Asp Gly Ala Arg Ser Asp Pro Val Ser Leu Arg Tyr Thr Pro
                   725                 730                 735
   Ser Thr Val Ser Arg Ser Pro Pro Ser Asn Leu Ala Leu Ala Ser Glu
               740                 745                 750
   Thr Pro Asp Ser Leu Gln Val Ser Trp Thr Pro Pro Leu Gly Arg Val
           755                 760                 765
   Leu His Tyr Trp Leu Thr Tyr Ala Pro Ala Ser Gly Leu Gly Pro Glu
       770                 775                 780
   Lys Ser Val Ser Val Pro Gly Ala Arg Ser His Val Thr Leu Pro Asp
   785                 790                 795                 800
   Leu Gln Ala Ala Thr Lys Tyr Arg Val Leu Val Ser Ala Ile Tyr Ala
                   805                 810                 815
   Ala Gly Arg Ser Glu Ala Val Ser Ala Thr Gly Gln Thr Ala Cys Pro
               820                 825                 830
   Ala Leu Arg Pro Asp Gly Ser Leu Pro Gly Phe Asp Leu Met Val Ala
           835                 840                 845
   Phe Ser Leu Val Glu Lys Ala Tyr Ala Ser Ile Arg Gly Val Ala Met
       850                 855                 860
   Glu Pro Ser Ala Phe Gly Gly Thr Pro Thr Phe Thr Leu Phe Lys Asp
   865                 870                 875                 880
   Ala Gln Leu Thr Arg Arg Val Ser Asp Val Tyr Pro Ala Pro Leu Pro
                   885                 890                 895
   Pro Glu His Thr Ile Val Phe Leu Val Arg Leu Leu Pro Glu Thr Pro
               900                 905                 910
   Arg Glu Ala Phe Ala Leu Trp Gln Met Thr Ala Glu Asp Phe Gln Pro
           915                 920                 925
   Leu Leu Gly Val Leu Leu Asp Ala Gly Lys Lys Ser Leu Thr Tyr Phe
       930                 935                 940
   His Arg Asp Pro Arg Ala Ala Leu Gln Glu Ala Thr Phe Asp Pro Gln
   945                 950                 955                 960
   Glu Val Arg Lys Ile Phe Phe Gly Ser Phe His Lys Val His Val Ala
                   965                 970                 975
   Val Gly Arg Ser Lys Val Arg Leu Tyr Val Asp Cys Arg Lys Val Ala
               980                 985                 990
   Glu Arg Pro Leu Gly Glu Met Gly  Ser Pro Pro Ala Ala  Gly Phe Val
           995                 1000                1005
   Thr Leu  Gly Arg Leu Ala Lys  Ala Arg Gly Pro Arg  Ser Ser Ser
       1010                1015                1020
   Ala Ala  Phe Gln Leu Gln Met  Leu Gln Ile Val Cys  Ser Asp Thr
       1025                1030                1035
   Trp Ala  Asp Glu Asp Arg Cys  Cys Glu Leu Pro Ala  Ser Arg Asp
       1040                1045                1050
   Gly Glu  Thr Cys Pro Ala Phe  Val Ser Ala Cys Ser  Cys Ser Ser
       1055                1060                1065
   Glu Thr  Pro Gly Pro Pro Gly  Pro Gln Gly Pro Pro  Gly Leu Pro
       1070                 1075                 1080
   Gly Arg  Asn Gly Thr Pro Gly  Glu Gln Gly Phe Pro  Gly Pro Arg
       1085                 1090                 1095
   Gly Pro  Pro Gly Val Lys Gly  Glu Lys Gly Asp His  Gly Leu Pro
       1100                 1105                 1110
   Gly Leu  Gln Gly His Pro Gly  His Gln Gly Ile Pro  Gly Arg Val
       1115                 1120                 1125
   Gly Leu  Gln Gly Pro Lys Gly  Met Arg Gly Leu Glu  Gly Thr Ala
       1130                 1135                 1140
   Gly Leu  Pro Gly Pro Gly Pro  Arg Gly Phe Gln Gly  Met Ala Gly
       1145                 1150                 1155
   Ala Arg  Gly Thr Ser Gly Glu  Arg Gly Pro Pro Gly  Thr Val Gly
       1160                 1165                 1170
   Pro Thr  Gly Leu Pro Gly Pro  Lys Gly Glu Arg Gly  Glu Lys Gly
       1175                 1180                 1185
   Glu Pro  Gln Ser Leu Ala Thr  Leu Tyr Gln Leu Val  Ser Gln Ala
       1190                 1195                 1200
   Ser His  Val Ser Lys Phe Asp  Ser Phe His Glu Asn  Thr Arg Pro
       1205                 1210                 1215
   Pro Met  Pro Ile Leu Glu Gln  Lys Leu Glu Pro Gly  Thr Glu Pro
       1220                 1225                 1230
   Leu Gly  Ser Pro Gly Thr Arg  Ser Lys Ala Leu Val  Pro Gly Glu
       1235                 1240                 1245
   Trp Gly  Arg Gly Gly Arg His  Leu Glu Gly Arg Gly  Glu Pro Gly
       1250                 1255                 1260
   Ala Val  Gly Gln Met Gly Ser  Pro Gly Gln Gln Gly  Ala Ser Thr
       1265                 1270                 1275
   Gln Gly  Leu Trp Glu
       1280
<210> 32
   <211> 3987
   <212> DNA
   <213> Homo sapiens
<400> 32
   atgagctccg gagaccctgc acacctcggc ctctgcctct ggctgtggct gggcgccacc 60
   ctgggaagag agcaagttca agcaagcggt ctcctgaggc tggctgtgct gcctgaggac 120
   cggctgcaga tgaagtggag agagtcggag gggagcggcc tcggctacct ggtgcaggtg 180
   aagcccatgg caggggactc ggaacaggag gtgatactga ccaccaagac ccctaaggcc 240
   acagtggggg gcctgagccc ctccaagggc tacaccttgc agatcttcga gctcactggc 300
   tctgggcgct tcctgctagc tcggagggag tttgtgattg aggatctgaa gagtagctcc 360
   ctggacagga gcagccagag gcccctcggc tctggagccc cggagcccac cccctcccac 420
   acggggagcc cagaccctga gcaggcttct gagccccaag ttgccttcac accaagccag 480
   gatccgcgca ctcctggtgg gtcagagtgg agagagaccg gcccccagtt ccgctgcctg 540
   ccccccgtgc ctgctgacat ggtcttcctg gtggacgggt cctggagcat tggccacagt 600
   cacttccagc aggtcaagga cttcctggcc agtgtcatcg caccctttga aatcgggccg 660
   gataaggtcc aagtaggcct gactcagtac agcggggatg ctcagactga gtgggacctg 720
   aactccctca gcaccaagga acaggtgctg gcagctgtgc gccgcctccg ctacaagggg 780
   gggaacacgt tcacaggcct tgccctgacc cacgtgctgg ggcagaacct gcagccggcg 840
   gctggcctcc gtccagaggc agccaaggtg gtgattctgg tgacggacgg caagtcccag 900
   gacgatgtgc acactgctgc ccgtgtcctc aaggacctgg gcgtgaacgt cttcgctgtg 960
   ggtgtgaaga acgccgatga ggctgagctg aggctcctgg cgtccccgcc gagggacatc 1020
   accgtccaca gcgtgctgga cttcctgcag ctcggcgcgc tggctggcct gctcagccgt 1080
   ctcatctgcc agaggctcca gggtgggagc ccgcggcagg gcccagcagc ggctccagcc 1140
   ctggacaccc tccctgcccc caccagcctg gtcctgagcc aggtgacctc ctccagcatc 1200
   cgcctgtcct ggactccagc cccccggcac cccctcaagt atctgatcgt ttggcgagcc 1260
   tctagaggtg gcacccccag ggaggtggtg gtggagggac ccgccgcctc cacggagctg 1320
   cacaacctgg cctcccgcac agagtacctg gtctccgtgt tccccatcta tgagggcggg 1380
   gttggcgaag gcctgcgggg cctggtgacc acagcacctc tgcctccgcc ccgggcgctg 1440
   accctggccg cagtgacgcc cagaaccgtc cacctcacct ggcagccctc ggccggggcc 1500
   acccactacc tggtgcgatg ttctcctgct tcccccaagg gtgaagagga ggagcgagag 1560
   gtgcaggtcg ggcggcccga ggtgctgctg gatggcctgg aacctggcag ggactatgag 1620
   gtctcggtgc agagcctgcg aggccctgag ggcagcgagg cccggggcat ccgtgccagg 1680
   acccccaccc tggccccccc gagacacctg ggcttctcag acgtgagcca cgacgcggca 1740
   cgagtgttct gggagggtgc cccgaggcct gtgcgcctgg tcagggtcac ctatgtgtcc 1800
   agcgagggtg gacactcggg gcagacagag gctcctggga acgccacctc ggccacgctg 1860
   gggcctctct cttcctccac cacctacact gtccgtgtca cctgcctcta ccctgggggt 1920
   ggctcctcta cgctgactgg ccgggtgacc accaagaaag ctcccagccc aagccagctg 1980
   tccatgacgg agctgccagg ggatgcagtc cagctggcgt gggtggccgc agccccgtct 2040
   ggcgtgcttg tctaccagat cacgtggacg cccctgggag aggggaaggc tcacgagatc 2100
   tctgtcccag ggaacctcgg cacggccgtc ctgcctggcc tagggaggca cacagagtac 2160
   gacgtcacca tcttggccta ctacagggac ggggcccgca gtgaccctgt gtccctccgc 2220
   tataccccct ccacggtgag caggagccca ccctccaacc tggccctggc ctcggagacc 2280
   cccgacagcc tgcaggtcag ctggacgccc ccgcttggcc gcgtgctcca ttactggctc 2340
   acctacgccc ccgcctctgg cttgggaccc gagaaatccg tctctgtgcc aggagccagg 2400
   agccacgtga cactgcccga cctgcaggca gccacgaagt acagggtcct ggtctcagct 2460
   atctatgcag caggcaggag tgaggctgtg tctgccacgg gccagacagc ctgcccagcc 2520
   ctccgccctg acggctccct cccagggttt gacctgatgg tggccttcag cctggtggaa 2580
   aaggcttatg cgtccatccg gggcgtggcc atggagccct ctgccttcgg tgggaccccg 2640
   accttcacgc tcttcaagga cgcccagctg acaagacggg tcagtgacgt ctacccagcc 2700
   cccctacctc cagagcacac catcgtcttc cttgtgcgcc tacttcccga gacaccccgt 2760
   gaggccttcg cgctgtggca gatgacagcc gaggacttcc agcccctcct tggggttctg 2820
   ctggatgccg ggaagaagtc cctgacctac ttccaccgtg accccagggc tgccttgcag 2880
   gaggccacct tcgacccgca ggaagtgagg aagattttct tcgggagctt ccacaaggtg 2940
   cacgtggctg tgggccgctc caaggtcagg ctctatgtgg actgccggaa ggtggctgag 3000
   cggccccttg gggagatggg cagcccaccc gctgcgggct tcgtcacgct ggggaggctg 3060
   gccaaggcca ggggcccccg gagcagttcg gccgcgtttc agctccagat gctgcagatc 3120
   gtgtgcagtg acacctgggc cgatgaggac cggtgctgtg agctccctgc ctcgagggat 3180
   ggagagacct gccccgcctt cgtgtctgcc tgttcctgtt cctcagagac ccctgggccc 3240
   ccaggacctc aaggaccccc aggcctccct gggaggaatg gcaccccagg agagcagggc 3300
   ttcccagggc ccaggggaga gcccgggcca cccggacaga tgggaccaga aggtcctgga 3360
   ggccagcagg gctcgccggg gacccagggc cgtgcagtcc aggggcctgt gggtccacca 3420
   ggggtcaaag gagagaaggg agaccatggg cttccaggct tgcagggcca ccccggccac 3480
   cagggcatcc ccgggagagt tggcctccag ggaccaaagg gaatgagagg cctggaggga 3540
   actgctggcc tgcctggacc cggccccagg gggttccagg gcatggcagg ggccaggggc 3600
   actagtggag agcgaggacc tccagggacc gtggggccca caggactgcc agggcccaaa   3660
   ggggaacgag gagagaaggg cgagccgcag tcccttgcca ccctctacca gcttgtgagc   3720
   caggcctgtg agtctgccat tcagacacac gtgtcaaagt tcgactcctt ccacgagaac   3780
   accaggcccc ccatgcccat cttggagcag aagctggagc cgggcactga gcccctgggg   3840
   tcccctggca cccgcagcaa ggccctggtt cctggagaat gggggcgtgg tggccgccac   3900
   cttgagggca gaggggagcc tggagctgtt ggtcagatgg gcagccctgg gcagcagggg   3960
   gctagcaccc agggcctctg ggagtga                                        3987
<210> 33
   <211> 3837
   <212> DNA
   <213> Homo sapiens
<400> 33
   atgagctccg gagaccctgc acacctcggc ctctgcctct ggctgtggct gggcgccacc     60
   ctgggaagag agcaagttca agcaagcggt ctcctgaggc tggctgtgct gcctgaggac    120
   cggctgcaga tgaagtggag agagtcggag gggagcggcc tcggctacct ggtgcaggtg    180
   aagcccatgg caggggactc ggaacaggag gtgatactga ccaccaagac ccctaaggcc    240
   acagtggggg gcctgagccc ctccaagggc tacaccttgc agatcttcga gctcactggc    300
   tctgggcgct tcctgctagc tcggagggag tttgtgattg aggatctgaa gagtagctcc    360
   ctggacagga gcagccagag gcccctcggc tctggagccc cggagcccac cccctcccac    420
   acggggagcc cagaccctga gcaggcttct gagccccaag ttgccttcac accaagccag    480
   gatccgcgca ctcctggtgg gtcagagtgg agagagaccg gcccccagtt ccgctgcctg    540
   ccccccgtgc ctgctgacat ggtcttcctg gtggacgggt cctggagcat tggccacagt    600
   cacttccagc aggtcaagga cttcctggcc agtgtcatcg caccctttga aatcgggccg    660
   gataaggtcc aagtaggcct gactcagtac agcggggatg ctcagactga gtgggacctg    720
   aactccctca gcaccaagga acaggtgctg gcagctgtgc gccgcctccg ctacaagggg    780
   gggaacacgt tcacaggcct tgccctgacc cacgtgctgg ggcagaacct gcagccggcg    840
   gctggcctcc gtccagaggc agccaaggtg gtgattctgg tgacggacgg caagtcccag    900
   gacgatgtgc acactgctgc ccgtgtcctc aaggacctgg gcgtgaacgt cttcgctgtg    960
   ggtgtgaaga acgccgatga ggctgagctg aggctcctgg cgtccccgcc gagggacatc   1020
   accgtccaca gcgtgctgga cttcctgcag ctcggcgcgc tggctggcct gctcagccgt   1080
   ctcatctgcc agaggctcca gggtgggagc ccgcggcagg gcccagcagc ggctccagcc   1140
   ctggacaccc tccctgcccc caccagcctg gtcctgagcc aggtgacctc ctccagcatc   1200
   cgcctgtcct ggactccagc cccccggcac cccctcaagt atctgatcgt ttggcgagcc   1260
   tctagaggtg gcacccccag ggaggtggtg gtggagggac ccgccgcctc cacggagctg 1320
   cacaacctgg cctcccgcac agagtacctg gtctccgtgt tccccatcta tgagggcggg 1380
   gttggcgaag gcctgcgggg cctggtgacc acagcacctc tgcctccgcc ccgggcgctg 1440
   accctggccg cagtgacgcc cagaaccgtc cacctcacct ggcagccctc ggccggggcc 1500
   acccactacc tggtgcgatg ttctcctgct tcccccaagg gtgaagagga ggagcgagag 1560
   gtgcaggtcg ggcggcccga ggtgctgctg gatggcctgg aacctggcag ggactatgag 1620
   gtctcggtgc agagcctgcg aggccctgag ggcagcgagg cccggggcat ccgtgccagg 1680
   acccccaccc tggccccccc gagacacctg ggcttctcag acgtgagcca cgacgcggca 1740
   cgagtgttct gggagggtgc cccgaggcct gtgcgcctgg tcagggtcac ctatgtgtcc 1800
   agcgagggtg gacactcggg gcagacagag gctcctggga acgccacctc ggccacgctg 1860
   gggcctctct cttcctccac cacctacact gtccgtgtca cctgcctcta ccctgggggt 1920
   ggctcctcta cgctgactgg ccgggtgacc accaagaaag ctcccagccc aagccagctg 1980
   tccatgacgg agctgccagg ggatgcagtc cagctggcgt gggtggccgc agccccgtct 2040
   ggcgtgcttg tctaccagat cacgtggacg cccctgggag aggggaaggc tcacgagatc 2100
   tctgtcccag ggaacctcgg cacggccgtc ctgcctggcc tagggaggca cacagagtac 2160
   gacgtcacca tcttggccta ctacagggac ggggcccgca gtgaccctgt gtccctccgc 2220
   tataccccct ccacggtgag caggagccca ccctccaacc tggccctggc ctcggagacc 2280
   cccgacagcc tgcaggtcag ctggacgccc ccgcttggcc gcgtgctcca ttactggctc 2340
   acctacgccc ccgcctctgg cttgggaccc gagaaatccg tctctgtgcc aggagccagg 2400
   agccacgtga cactgcccga cctgcaggca gccacgaagt acagggtcct ggtctcagct 2460
   atctatgcag caggcaggag tgaggctgtg tctgccacgg gccagacagc ctgcccagcc 2520
   ctccgccctg acggctccct cccagggttt gacctgatgg tggccttcag cctggtggaa 2580
   aaggcttatg cgtccatccg gggcgtggcc atggagccct ctgccttcgg tgggaccccg 2640
   accttcacgc tcttcaagga cgcccagctg acaagacggg tcagtgacgt ctacccagcc 2700
   cccctacctc cagagcacac catcgtcttc cttgtgcgcc tacttcccga gacaccccgt 2760
   gaggccttcg cgctgtggca gatgacagcc gaggacttcc agcccctcct tggggttctg 2820
   ctggatgccg ggaagaagtc cctgacctac ttccaccgtg accccagggc tgccttgcag 2880
   gaggccacct tcgacccgca ggaagtgagg aagattttct tcgggagctt ccacaaggtg 2940
   cacgtggctg tgggccgctc caaggtcagg ctctatgtgg actgccggaa ggtggctgag 3000
   cggccccttg gggagatggg cagcccaccc gctgcgggct tcgtcacgct ggggaggctg 3060
   gccaaggcca ggggcccccg gagcagttcg gccgcgtttc agctccagat gctgcagatc 3120
   gtgtgcagtg acacctgggc cgatgaggac cggtgctgtg agctccctgc ctcgagggat   3180
   ggagagacct gccccgcctt cgtgtctgcc tgttcctgtt cctcagagac ccctgggccc   3240
   ccaggacctc aaggaccccc aggtccacca ggggtcaaag gagagaaggg agaccatggg   3300
   cttccaggct tgcagggcca ccccggccac cagggcatcc ccgggagagt tggcctccag   3360
   ggaccaaagg gaatgagagg cctggaggga actgctggcc tgcctggacc tggccccagg   3420
   gggttccagg gcatggcagg ggccaggggc actagtggag agcgaggacc tccagggacc   3480
   gtggggccca caggactgcc agggcccaaa ggggaacgag gagagaaggg cgagccgcag   3540
   tcccttgcca ccctctacca gcttgtgagc caggcctgtg agtctgccat tcagacacac   3600
   gtgtcaaagt tcgactcctt ccacgagaac accaggcccc ccatgcccat cttggagcag   3660
   aagctggagc cgggcactga gcccctgggg tcccctggca cccgcagcaa ggccctggtt   3720
   cctggagaat gggggcgtgg tggccgccac cttgagggca gaggggagcc tggagctgtt   3780
   ggtcagatgg gcagccctgg gcagcagggg gctagcaccc agggcctctg ggagtga      3837
<210> 34
   <211> 4168
   <212> DNA
   <213> Homo sapiens
<400> 34
   ataagctcca gccttcctgt ggccacagca ggaccagagt ggaccagcac accccaggag      60
   agaggactgg ggtcccagga gtaggaggag cccgagcacc atgagctccg gagaccctgc     120
   acacctcggc ctctgcctct ggctgtggct gggcgccacc ctgggaagag agcaagttca     180
   agcaagcggt ctcctgaggc tggctgtgct gcctgaggac cggctgcaga tgaagtggag     240
   agagtcggag gggagcggcc tcggctacct ggtgcaggtg aagcccatgg caggggactc     300
   ggaacaggag gtgatactga ccaccaagac ccctaaggcc acagtggggg gcctgagccc     360
   ctccaagggc tacaccttgc agatcttcga gctcactggc tctgggcgct tcctgctagc     420
   tcggagggag tttgtgattg aggatctgaa gagtagctcc ctggacagga gcagccagag     480
   gcccctcggc tctggagccc cggagcccac cccctcccac acggggagcc cagaccctga     540
   gcaggcttct gagccccaag ttgccttcac accaagccag gatccgcgca ctcctgccgg     600
   cccccagttc cgctgcctgc cccccgtgcc tgctgacatg gtcttcctgg tggacgggtc     660
   ctggagcatt ggccacagtc acttccagca ggtcaaggac ttcctggcca gtgtcatcgc     720
   accctttgaa atcgggccgg ataaggtcca agtaggcctg actcagtaca gcggggatgc     780
   tcagactgag tgggacctga actccctcag caccaaggaa caggtgctgg cagctgtgcg     840
   ccgcctccgc tacaaggggg ggaacacgtt cacaggcctt gccctgaccc acgtgctggg     900
   gcagaacctg cagccggcgg ctggcctccg tccagaggca gccaaggtgg tgattctggt     960
   gacggacggc aagtcccagg acgatgtgca cactgctgcc cgtgtcctca aggacctggg   1020
   cgtgaacgtc ttcgctgtgg gtgtgaagaa cgccgatgag gctgagctga ggctcctggc   1080
   gtccccgccg agggacatca ccgtccacag cgtgctggac ttcctgcagc tcggcgcgct   1140
   ggctggcctg ctcagccgtc tcatctgcca gaggctccag ggtgggagcc cgcggcaggg   1200
   cccagcagcg gctccagccc tggacaccct ccctgccccc accagcctgg tcctgagcca   1260
   ggtgacctcc tccagcatcc gcctgtcctg gactccagcc ccccggcacc ccctcaagta   1320
   tctgatcgtt tggcgagcct ctagaggtgg cacccccagg gaggtggtgg tggagggacc   1380
   cgccgcctcc acggagctgc acaacctggc ctcccgcaca gagtacctgg tctccgtgtt   1440
   ccccatctat gagggcgggg ttggcgaagg cctgcggggc ctggtgacca cagcacctct   1500
   gcctccgccc cgggcgctga ccctggccgc agtgacgccc agaaccgtcc acctcacctg   1560
   gcagccctcg gccggggcca cccactacct ggtgcgatgt tctcctgctt cccccaaggg   1620
   tgaagaggag gagcgagagg tgcaggtcgg gcggcccgag gtgctgctgg atggcctgga   1680
   acctggcagg gactatgagg tctcggtgca gagcctgcga ggccctgagg gcagcgaggc   1740
   ccggggcatc cgtgccagga cccccaccct ggcccccccg agacacctgg gcttctcaga   1800
   cgtgagccac gacgcggcac gagtgttctg ggagggtgcc ccgaggcctg tgcgcctggt   1860
   cagggtcacc tatgtgtcca gcgagggtgg acactcgggg cagacagagg ctcctgggaa   1920
   cgccacctcg gccacgctgg ggcctctctc ttcctccacc acctacactg tccgtgtcac   1980
   ctgcctctac cctgggggtg gctcctctac gctgactggc cgggtgacca ccaagaaagc   2040
   tcccagccca agccagctgt ccatgacgga gctgccaggg gatgcagtcc agctggcgtg   2100
   ggtggccgca gccccgtctg gcgtgcttgt ctaccagatc acgtggacgc ccctgggaga   2160
   ggggaaggct cacgagatct ctgtcccagg gaacctcggc acggccgtcc tgcctggcct   2220
   agggaggcac acagagtacg acgtcaccat cttggcctac tacagggacg gggcccgcag   2280
   tgaccctgtg tccctccgct ataccccctc cacggtgagc aggagcccac cctccaacct   2340
   ggccctggcc tcggagaccc ccgacagcct gcaggtcagc tggacgcccc cgcttggccg   2400
   cgtgctccat tactggctca cctacgcccc cgcctctggc ttgggacccg agaaatccgt   2460
   ctctgtgcca ggagccagga gccacgtgac actgcccgac ctgcaggcag ccacgaagta   2520
   cagggtcctg gtctcagcta tctatgcagc aggcaggagt gaggctgtgt ctgccacggg   2580
   ccagacagcc tgcccagccc tccgccctga cggctccctc ccagggtttg acctgatggt   2640
   ggccttcagc ctggtggaaa aggcttatgc gtccatccgg ggcgtggcca tggagccctc   2700
   tgccttcggt gggaccccga ccttcacgct cttcaaggac gcccagctga caagacgggt   2760
   cagtgacgtc tacccagccc ccctacctcc agagcacacc atcgtcttcc ttgtgcgcct   2820
   acttcccgag acaccccgtg aggccttcgc gctgtggcag atgacagccg aggacttcca   2880
   gcccctcctt ggggttctgc tggatgccgg gaagaagtcc ctgacctact tccaccgtga   2940
   ccccagggct gccttgcagg aggccacctt cgacccgcag gaagtgagga agattttctt   3000
   cgggagcttc cacaaggtgc acgtggctgt gggccgctcc aaggtcaggc tctatgtgga   3060
   ctgccggaag gtggctgagc ggccccttgg ggagatgggc agcccacccg ctgcgggctt   3120
   cgtcacgctg gggaggctgg ccaaggccag gggcccccgg agcagttcgg ccgcgtttca   3180
   gctccagatg ctgcagatcg tgtgcagtga cacctgggcc gatgaggacc ggtgctgtga   3240
   gctccctgcc tcgagggatg gagagacctg ccccgccttc gtgtctgcct gttcctgttc   3300
   ctcagagacc cctgggcccc caggacctca aggaccccca ggcctccctg ggaggaatgg   3360
   caccccagga gagcagggct tcccagggcc caggggtcca ccaggggtca aaggagagaa   3420
   gggagaccat gggcttccag gcttgcaggg ccaccccggc caccagggca tccccgggag   3480
   agttggcctc cagggaccaa agggaatgag aggcctggag ggaactgctg gcctgcctgg   3540
   acctggcccc agggggttcc agggcatggc aggggccagg ggcactagtg gagagcgagg   3600
   acctccaggg accgtggggc ccacaggact gccagggccc aaaggggaac gaggagagaa   3660
   gggcgagccg cagtcccttg ccaccctcta ccagcttgtg agccaggcct cacacgtgtc   3720
   aaagttcgac tccttccacg agaacaccag gccccccatg cccatcttgg agcagaagct   3780
   ggagccgggc actgagcccc tggggtcccc tggcacccgc agcaaggccc tggttcctgg   3840
   agaatggggg cgtggtggcc gccaccttga gggcagaggg gagcctggag ctgttggtca   3900
   gatgggcagc cctgggcagc agggggctag cacccagggc ctctgggagt gacaggacat   3960
   tttctgcact gccccgagga acgctgagcc ttcctccctg ggtttgtctg gacaccgaga   4020
   gcgaccacat cctggagaag ccaggagaaa agctcaggaa gagcctgcag gtggaaggag   4080
   agggaagcag cggcctcggc caaggcccac cccatactct tggctctgta gcatttccaa   4140
   gttcagataa acccctgagt gctcaccc                                       4168
<210> 35
   <211> 406
   <212> PRT
   <213> Homo sapiens
<400> 35
   Met Asp Cys Arg Thr Lys Ala Asn Pro Asp Arg Thr Phe Asp Leu Val
   1               5                   10                  15
   Leu Lys Val Lys Cys His Ala Ser Glu Asn Glu Asp Pro Val Val Leu
               20                  25                  30
   Trp Lys Phe Pro Glu Asp Phe Gly Asp Gln Glu Ile Leu Gln Ser Val
           35                  40                  45
   Pro Lys Phe Cys Phe Pro Phe Asp Val Glu Arg Val Ser Gln Asn Gln
       50                  55                  60
   Val Gly Gln His Phe Thr Phe Val Leu Thr Asp Ile Glu Ser Lys Gln
   65                  70                  75                  80
   Arg Phe Gly Phe Cys Arg Leu Thr Ser Gly Gly Thr Ile Cys Leu Cys
                   85                  90                  95
   Ile Leu Ser Tyr Leu Pro Trp Phe Glu Val Tyr Tyr Lys Leu Leu Asn
               100                 105                 110
   Thr Leu Ala Asp Tyr Leu Ala Lys Glu Leu Glu Asn Asp Leu Asn Glu
           115                 120                 125
   Thr Leu Arg Ser Leu Tyr Asn His Pro Val Pro Lys Ala Asn Thr Pro
       130                 135                 140
   Val Asn Leu Ser Val His Ser Tyr Phe Ile Ala Pro Asp Val Thr Gly
   145                 150                 155                 160
   Leu Pro Thr Ile Pro Glu Ser Arg Asn Leu Thr Glu Tyr Phe Val Ala
                   165                 170                 175
   Val Asp Val Asn Asn Met Leu Gln Leu Tyr Ala Ser Met Leu His Glu
               180                 185                 190
   Arg Arg Ile Val Ile Ile Ser Ser Lys Leu Ser Thr Leu Thr Ala Cys
           195                 200                 205
   Ile His Gly Ser Ala Ala Leu Leu Tyr Pro Met Tyr Trp Gln His Ile
       210                 215                 220
   Tyr Ile Pro Val Leu Pro Pro His Leu Leu Asp Tyr Cys Cys Ala Pro
   225                 230                 235                 240
   Met Pro Tyr Leu Ile Gly Ile His Ser Ser Leu Ile Glu Arg Val Lys
                   245                 250                 255
   Asn Lys Ser Leu Glu Asp Val Val Met Leu Asn Val Asp Thr Asn Thr
               260                 265                 270
   Leu Glu Ser Pro Phe Ser Asp Leu Asn Asn Leu Pro Ser Asp Val Val
           275                 280                 285
   Ser Ala Leu Lys Asn Lys Leu Lys Lys Gln Ser Thr Ala Thr Gly Asp
       290                 295                 300
   Gly Val Ala Arg Ala Phe Leu Arg Ala Gln Ala Ala Leu Phe Gly Ser
   305                 310                 315                 320
   Tyr Arg Asp Ala Leu Arg Tyr Lys Pro Gly Glu Pro Ile Thr Phe Cys
                   325                 330                 335
   Glu Glu Ser Phe Val Lys His Arg Ser Ser Val Met Lys Gln Phe Leu
               340                 345                 350
   Glu Thr Ala Ile Asn Leu Gln Leu Phe Lys Gln Phe Ile Asp Gly Arg
           355                 360                 365
   Leu Ala Lys Leu Asn Ala Gly Arg Gly Phe Ser Asp Val Phe Glu Glu
       370                 375                 380
   Glu Ile Thr Ser Gly Gly Phe Cys Gly Gly Lys Asp Lys Leu Gln Tyr
   385                 390                 395                 400
   Asp Tyr Pro Phe Ser Gln
                   405
<210> 36
   <211> 426
   <212> PRT
   <213> Homo sapiens
<400> 36
   Met Asp Cys Arg Thr Lys Ala Asn Pro Asp Arg Thr Phe Asp Leu Val
   1               5                   10                  15
   Leu Lys Val Lys Cys His Ala Ser Glu Asn Glu Asp Pro Val Val Leu
               20                  25                  30
   Trp Lys Phe Pro Glu Asp Phe Gly Asp Gln Glu Ile Leu Gln Ser Val
           35                  40                  45
   Pro Lys Phe Cys Phe Pro Phe Asp Val Glu Arg Val Ser Gln Asn Gln
       50                  55                  60
   Val Gly Gln His Phe Thr Phe Val Leu Thr Asp Ile Glu Ser Lys Gln
   65                  70                  75                  80
   Arg Phe Gly Phe Cys Arg Leu Thr Ser Gly Gly Thr Ile Cys Leu Cys
                   85                  90                  95
   Ile Leu Ser Tyr Leu Pro Trp Phe Glu Val Tyr Tyr Lys Leu Leu Asn
               100                 105                 110
   Thr Leu Ala Asp Tyr Leu Ala Lys Glu Leu Glu Asn Asp Leu Asn Glu
           115                 120                 125
   Thr Leu Arg Ser Leu Tyr Asn His Pro Val Pro Lys Ala Asn Thr Pro
       130                 135                 140
   Val Asn Leu Ser Val Asn Gln Glu Ile Phe Ile Ala Cys Glu Gln Val
   145                 150                 155                 160
   Leu Lys Asp Gln Pro Ala Leu Val Pro His Ser Tyr Phe Ile Ala Pro
                   165                 170                 175
   Asp Val Thr Gly Leu Pro Thr Ile Pro Glu Ser Arg Asn Leu Thr Glu
               180                 185                 190
   Tyr Phe Val Ala Val Asp Val Asn Asn Met Leu Gln Leu Tyr Ala Ser
           195                 200                 205
   Met Leu His Glu Arg Arg Ile Val Ile Ile Ser Ser Lys Leu Ser Thr
       210                 215                 220
   Leu Thr Ala Cys Ile His Gly Ser Ala Ala Leu Leu Tyr Pro Met Tyr
   225                 230                 235                 240
   Trp Gln His Ile Tyr Ile Pro Val Leu Pro Pro His Leu Leu Asp Tyr
                   245                 250                 255
   Cys Cys Ala Pro Met Pro Tyr Leu Ile Gly Ile His Ser Ser Leu Ile
               260                 265                 270
   Glu Arg Val Lys Asn Lys Ser Leu Glu Asp Val Val Met Leu Asn Val
           275                 280                 285
   Asp Thr Asn Thr Leu Glu Ser Pro Phe Ser Asp Leu Asn Asn Leu Pro
       290                 295                 300
   Ser Asp Val Val Ser Ala Leu Lys Asn Lys Leu Lys Lys Gln Ser Thr
   305                 310                 315                 320
   Ala Thr Gly Asp Gly Val Ala Arg Ala Phe Leu Arg Ala Gln Ala Ala
                   325                 330                 335
   Leu Phe Gly Ser Tyr Arg Asp Ala Leu Arg Tyr Lys Pro Gly Glu Pro
               340                 345                 350
   Ile Thr Phe Cys Glu Glu Ser Phe Val Lys His Arg Ser Ser Val Met
           355                 360                 365
   Lys Gln Phe Leu Glu Thr Ala Ile Asn Leu Gln Leu Phe Lys Gln Phe
       370                 375                 380
   Ile Asp Gly Arg Leu Ala Lys Leu Asn Ala Gly Arg Gly Phe Ser Asp
   385                 390                 395                 400
   Val Phe Glu Glu Glu Ile Thr Ser Gly Gly Phe Cys Gly Gly Lys Asp
                   405                 410                 415
   Lys Leu Gln Tyr Asp Tyr Pro Phe Ser Gln
               420                 425
<210> 37
   <211> 426
   <212> PRT
   <213> Homo sapiens
<400> 37
   Ile Glu Thr Lys Thr Arg Ala Asn Pro Asp Arg Thr Phe Asp Leu Val
   1               5                   10                  15
   Leu Lys Val Lys Cys His Ala Ser Glu Asn Glu Asp Pro Val Val Leu
               20                  25                  30
   Trp Lys Phe Pro Glu Asp Phe Gly Asp Gln Glu Ile Leu Gln Ser Val
           35                  40                  45
   Pro Lys Phe Cys Phe Pro Phe Asp Val Glu Arg Val Ser Gln Asn Gln
       50                  55                  60
   Val Gly Gln His Phe Thr Phe Val Leu Thr Asp Ile Glu Ser Lys Gln
   65                  70                  75                  80
   Arg Phe Gly Phe Cys Arg Leu Thr Ser Gly Gly Thr Ile Cys Leu Cys
                   85                  90                  95
   Ile Leu Ser Tyr Leu Pro Trp Phe Glu Val Tyr Tyr Lys Leu Leu Asn
               100                 105                 110
   Thr Leu Ala Asp Tyr Leu Ala Lys Glu Leu Glu Asn Asp Leu Asn Glu
           115                 120                 125
   Thr Leu Arg Ser Leu Tyr Asn His Pro Val Pro Lys Ala Asn Thr Pro
       130                 135                 140
   Val Asn Leu Ser Val Asn Gln Glu Ile Phe Ile Ala Cys Glu Gln Val
   145                 150                 155                 160
   Leu Lys Asp Gln Pro Ala Leu Val Pro His Ser Tyr Phe Ile Ala Pro
                   165                 170                 175
   Asp Val Thr Gly Leu Pro Thr Ile Pro Glu Ser Arg Asn Leu Thr Glu
               180                 185                 190
   Tyr Phe Val Ala Val Asp Val Asn Asn Met Leu Gln Leu Tyr Ala Ser
           195                 200                 205
   Met Leu His Glu Arg Arg Ile Val Ile Ile Ser Ser Lys Leu Ser Thr
       210                 215                 220
   Leu Thr Ala Cys Ile His Gly Ser Ala Ala Leu Leu Tyr Pro Met Tyr
   225                 230                 235                 240
   Trp Gln His Ile Tyr Ile Pro Val Leu Pro Pro His Leu Leu Asp Tyr
                   245                 250                 255
   Cys Cys Ala Pro Met Pro Tyr Leu Ile Gly Ile His Ser Ser Leu Ile
               260                 265                 270
   Glu Arg Val Lys Asn Lys Ser Leu Glu Asp Val Val Met Leu Asn Val
           275                 280                 285
   Asp Thr Asn Thr Leu Glu Ser Pro Phe Ser Asp Leu Asn Asn Leu Pro
       290                 295                 300
   Ser Asp Val Val Ser Ala Leu Lys Asn Lys Leu Lys Lys Gln Ser Thr
   305                 310                 315                 320
   Ala Thr Gly Asp Gly Val Ala Arg Ala Phe Leu Arg Ala Gln Ala Ala
                   325                 330                 335
   Leu Phe Gly Ser Tyr Arg Asp Ala Leu Arg Tyr Lys Pro Gly Glu Pro
               340                 345                 350
   Ile Thr Phe Cys Glu Glu Ser Phe Val Lys His Arg Ser Ser Val Met
           355                 360                 365
   Lys Gln Phe Leu Glu Thr Ala Ile Asn Leu Gln Leu Phe Lys Gln Phe
       370                 375                 380
   Ile Asp Gly Arg Leu Ala Lys Leu Asn Ala Gly Arg Gly Phe Ser Asp
   385                 390                 395                 400
   Val Phe Glu Glu Glu Ile Thr Ser Gly Gly Phe Cys Gly Gly Lys Asp
                   405                 410                 415
   Lys Leu Gln Tyr Asp Tyr Pro Phe Ser Gln
               420                 425
<210> 38
   <211> 396
   <212> PRT
   <213> Homo sapiens
<400> 38
   Met Ala Ala Ala Pro Arg Glu Glu Lys Arg Trp Pro Gln Pro Val Phe
   1               5                   10                  15
   Ser Asn Pro Val Val Leu Trp Lys Phe Pro Glu Asp Phe Gly Asp Gln
               20                  25                  30
   Glu Ile Leu Gln Ser Val Pro Lys Phe Cys Phe Pro Phe Asp Val Glu
           35                  40                  45
   Arg Val Ser Gln Asn Gln Val Gly Gln His Phe Thr Phe Val Leu Thr
       50                  55                  60
   Asp Ile Glu Ser Lys Gln Arg Phe Gly Phe Cys Arg Leu Thr Ser Gly
   65                  70                  75                  80
   Gly Thr Ile Cys Leu Cys Ile Leu Ser Tyr Leu Pro Trp Phe Glu Val
                   85                  90                  95
   Tyr Tyr Lys Leu Leu Asn Thr Leu Ala Asp Tyr Leu Ala Lys Glu Leu
               100                 105                 110
   Glu Asn Asp Leu Asn Glu Thr Leu Arg Ser Leu Tyr Asn His Pro Val
           115                 120                 125
   Pro Lys Ala Asn Thr Pro Val Asn Leu Ser Val His Ser Tyr Phe Ile
       130                 135                 140
   Ala Pro Asp Val Thr Gly Leu Pro Thr Ile Pro Glu Ser Arg Asn Leu
   145                 150                 155                 160
   Thr Glu Tyr Phe Val Ala Val Asp Val Asn Asn Met Leu Gln Leu Tyr
                   165                 170                 175
   Ala Ser Met Leu His Glu Arg Arg Ile Val Ile Ile Ser Ser Lys Leu
               180                 185                 190
   Ser Thr Leu Thr Ala Cys Ile His Gly Ser Ala Ala Leu Leu Tyr Pro
           195                 200                 205
   Met Tyr Trp Gln His Ile Tyr Ile Pro Val Leu Pro Pro His Leu Leu
       210                 215                 220
   Asp Tyr Cys Cys Ala Pro Met Pro Tyr Leu Ile Gly Ile His Ser Ser
   225                 230                 235                 240
   Leu Ile Glu Arg Val Lys Asn Lys Ser Leu Glu Asp Val Val Met Leu
                   245                 250                 255
   Asn Val Asp Thr Asn Thr Leu Glu Ser Pro Phe Ser Asp Leu Asn Asn
               260                 265                 270
   Leu Pro Ser Asp Val Val Ser Ala Leu Lys Asn Lys Leu Lys Lys Gln
           275                 280                 285
   Ser Thr Ala Thr Gly Asp Gly Val Ala Arg Ala Phe Leu Arg Ala Gln
       290                 295                 300
   Ala Ala Leu Phe Gly Ser Tyr Arg Asp Ala Leu Arg Tyr Lys Pro Gly
   305                 310                 315                 320
   Glu Pro Ile Thr Phe Cys Glu Glu Ser Phe Val Lys His Arg Ser Ser
                   325                 330                 335
   Val Met Lys Gln Phe Leu Glu Thr Ala Ile Asn Leu Gln Leu Phe Lys
               340                 345                 350
   Gln Phe Ile Asp Gly Arg Leu Ala Lys Leu Asn Ala Gly Arg Gly Phe
           355                 360                 365
   Ser Asp Val Phe Glu Glu Glu Ile Thr Ser Gly Gly Phe Cys Gly Gly
       370                 375                 380
   Lys Asp Lys Leu Gln Tyr Asp Tyr Pro Phe Ser Gln
   385                 390                 395
<210> 39
   <211> 2117
   <212> DNA
   <213> Homo sapiens
<400> 39
   gccgggggcg cagccgacat gggcccgccg ccacggctgc tgtgagcagc ctctttccct 60
   gtgtggccgc cggcgtgggc ggggacggcg cgaccctcgc gcggccgggc tgcgggcttc 120
   caggccagcg cgcgggggcc ggacggacag ccccacaccg acatgtaacc atggactgca 180
   ggaccaaggc aaatccagac agaacctttg acttggtgtt gaaagtgaaa tgtcatgcct 240
   ctgaaaatga agatcctgtg gtattgtgga aattcccaga ggactttgga gaccaggaaa 300
   tactacagag tgtgccaaag ttctgttttc cctttgacgt tgaaagggtg tctcagaatc 360
   aagttggaca gcactttacc tttgtactga cagacattga aagtaaacag agatttggat 420
   tctgcagact gacgtcagga ggcacaattt gtttatgcat ccttagttac cttccctggt 480
   ttgaagtgta ttacaagctt ctaaatactc ttgcagatta cttggctaag gaactggaaa 540
   atgatttgaa tgaaactctc agatcactgt ataaccaccc agtaccaaag gcaaatactc 600
   ctgtaaattt gagtgtgcat tcctacttca ttgcccctga tgtaactgga ctcccaacaa 660
   tacccgagag tagaaatctt acagaatatt ttgttgccgt ggatgtgaac aacatgctgc 720
   agctgtatgc cagtatgctg catgaaaggc gcatcgtgat tatctcgagc aaattaagca 780
   ctttaactgc ctgtatccat ggatcagctg ctcttctata cccaatgtat tggcaacaca 840
   tatacatccc agtgcttcct ccacacctgc tggactactg ctgtgcccca atgccatacc 900
   tgattggaat acactccagc ctcatagaga gagtgaaaaa caaatcattg gaagatgttg 960
   ttatgttaaa tgttgataca aacacattag aatcaccatt tagtgacttg aacaacctac 1020
   caagtgatgt ggtctcggcc ttgaaaaata aactgaagaa gcagtctaca gctacgggtg 1080
   atggagtagc tagggccttt cttagagcac aggctgcttt gtttggatcc tacagagatg 1140
   cactgagata caaacctggt gagcccatca ctttctgtga ggagagtttt gtaaagcacc 1200
   gctcaagcgt gatgaaacag ttcctggaaa ctgccattaa cctccagctt tttaagcagt 1260
   ttatcgatgg tcgactggca aaactaaatg caggaagggg tttctctgat gtatttgaag 1320
   aagagatcac ttcaggtggc ttttgtggag gtaaagacaa gttacaatat gattatccat 1380
   tttctcaata acaattttct tggtctttgc acttgtgtct gataaaacct atttcataaa 1440
   caactaatga tttcctccta aatatgtaat gtcttaaata catttttcat cttataaaag 1500
   ctatggaatt agcttatttt gcctgatacc tgttactcaa ggcattaagt tggcctcctg 1560
   aattggcagc tgttggcctc gataatctct taatattgct ggaaattagt aatacagaaa 1620
   tccaatcaac tcatatcttc ctgtctttcc ttctgaatag tagtattctc tgctagaaaa 1680
   ctactagtga tggttattac tgagtatgaa tttaagaact gaggttatga ttggtaatac 1740
   aatccaaaaa gaagggtctg aacaccaaaa ttctttatac atatttaagt aactgtatta 1800
   ttattataca gatgtcttta cctttttgac tttatagatc actgcagcat taagaaagtt 1860
   tccagtttac cattccataa gtacaattaa tccttctagt gtaaatgttc aaatactgtt 1920
   ataattatct aggcaattaa taatttacaa actgatattt ttgcacgatt gtagtggtgt 1980
   atagtcttga cttgcagagc attttgcttg agtccttgaa atgtcgtgtt cattcattat 2040
   ttgctgagtg cttacaatgt attaggcact gttctaaata ttaagtgtac taaataaaca 2100
   aaaatccttg tattctg 2117
<210> 40
   <211> 2177
   <212> DNA
   <213> Homo sapiens
<400> 40
   gccgggggcg cagccgacat gggcccgccg ccacggctgc tgtgagcagc ctctttccct      60
   gtgtggccgc cggcgtgggc ggggacggcg cgaccctcgc gcggccgggc tgcgggcttc     120
   caggccagcg cgcgggggcc ggacggacag ccccacaccg acatgtaacc atggactgca     180
   ggaccaaggc aaatccagac agaacctttg acttggtgtt gaaagtgaaa tgtcatgcct     240
   ctgaaaatga agatcctgtg gtattgtgga aattcccaga ggactttgga gaccaggaaa     300
   tactacagag tgtgccaaag ttctgttttc cctttgacgt tgaaagggtg tctcagaatc     360
   aagttggaca gcactttacc tttgtactga cagacattga aagtaaacag agatttggat     420
   tctgcagact gacgtcagga ggcacaattt gtttatgcat ccttagttac cttccctggt     480
   ttgaagtgta ttacaagctt ctaaatactc ttgcagatta cttggctaag gaactggaaa     540
   atgatttgaa tgaaactctc agatcactgt ataaccaccc agtaccaaag gcaaatactc     600
   ctgtaaattt gagtgtgaac caagagatat ttattgcctg tgagcaagtt ctgaaagatc     660
   agcctgctct agtaccgcat tcctacttca ttgcccctga tgtaactgga ctcccaacaa     720
   tacccgagag tagaaatctt acagaatatt ttgttgccgt ggatgtgaac aacatgctgc     780
   agctgtatgc cagtatgctg catgaaaggc gcatcgtgat tatctcgagc aaattaagca     840
   ctttaactgc ctgtatccat ggatcagctg ctcttctata cccaatgtat tggcaacaca     900
   tatacatccc agtgcttcct ccacacctgc tggactactg ctgtgcccca atgccatacc     960
   tgattggaat acactccagc ctcatagaga gagtgaaaaa caaatcattg gaagatgttg    1020
   ttatgttaaa tgttgataca aacacattag aatcaccatt tagtgacttg aacaacctac    1080
   caagtgatgt ggtctcggcc ttgaaaaata aactgaagaa gcagtctaca gctacgggtg    1140
   atggagtagc tagggccttt cttagagcac aggctgcttt gtttggatcc tacagagatg    1200
   cactgagata caaacctggt gagcccatca ctttctgtga ggagagtttt gtaaagcacc    1260
   gctcaagcgt gatgaaacag ttcctggaaa ctgccattaa cctccagctt tttaagcagt    1320
   ttatcgatgg tcgactggca aaactaaatg caggaagggg tttctctgat gtatttgaag    1380
   aagagatcac ttcaggtggc ttttgtggag gtaaagacaa gttacaatat gattatccat    1440
   tttctcaata acaattttct tggtctttgc acttgtgtct gataaaacct atttcataaa    1500
   caactaatga tttcctccta aatatgtaat gtcttaaata catttttcat cttataaaag   1560
   ctatggaatt agcttatttt gcctgatacc tgttactcaa ggcattaagt tggcctcctg   1620
   aattggcagc tgttggcctc gataatctct taatattgct ggaaattagt aatacagaaa   1680
   tccaatcaac tcatatcttc ctgtctttcc ttctgaatag tagtattctc tgctagaaaa   1740
   ctactagtga tggttattac tgagtatgaa tttaagaact gaggttatga ttggtaatac   1800
   aatccaaaaa gaagggtctg aacaccaaaa ttctttatac atatttaagt aactgtatta   1860
   ttattataca gatgtcttta cctttttgac tttatagatc actgcagcat taagaaagtt   1920
   tccagtttac cattccataa gtacaattaa tccttctagt gtaaatgttc aaatactgtt   1980
   ataattatct aggcaattaa taatttacaa actgatattt ttgcacgatt gtagtggtgt   2040
   atagtcttga cttgcagagc attttgcttg agtccttgaa atgtcgtgtt cattcattat   2100
   ttgctgagtg cttacaatgt attaggcact gttctaaata ttaagtgtac taaataaaca   2160
   aaaatccttg tattctg                                                  2177
<210> 41
   <211> 2007
   <212> DNA
   <213> Homo sapiens
<400> 41
   attgagacaa aaacaagggc aaatccagac agaacctttg acttggtgtt gaaagtgaaa      60
   tgtcatgcct ctgaaaatga agatcctgtg gtattgtgga aattcccaga ggactttgga     120
   gaccaggaaa tactacagag tgtgccaaag ttctgttttc cctttgacgt tgaaagggtg     180
   tctcagaatc aagttggaca gcactttacc tttgtactga cagacattga aagtaaacag     240
   agatttggat tctgcagact gacgtcagga ggcacaattt gtttatgcat ccttagttac     300
   cttccctggt ttgaagtgta ttacaagctt ctaaatactc ttgcagatta cttggctaag     360
   gaactggaaa atgatttgaa tgaaactctc agatcactgt ataaccaccc agtaccaaag     420
   gcaaatactc ctgtaaattt gagtgtgaac caagagatat ttattgcctg tgagcaagtt     480
   ctgaaagatc agcctgctct agtaccgcat tcctacttca ttgcccctga tgtaactgga     540
   ctcccaacaa tacccgagag tagaaatctt acagaatatt ttgttgccgt ggatgtgaac     600
   aacatgctgc agctgtatgc cagtatgctg catgaaaggc gcatcgtgat tatctcgagc     660
   aaattaagca ctttaactgc ctgtatccat ggatcagctg ctcttctata cccaatgtat     720
   tggcaacaca tatacatccc agtgcttcct ccacacctgc tggactactg ctgtgcccca     780
   atgccatacc tgattggaat acactccagc ctcatagaga gagtgaaaaa caaatcattg     840
   gaagatgttg ttatgttaaa tgttgataca aacacattag aatcaccatt tagtgacttg     900
   aacaacctac caagtgatgt ggtctcggcc ttgaaaaata aactgaagaa gcagtctaca     960
   gctacgggtg atggagtagc tagggccttt cttagagcac aggctgcttt gtttggatcc 1020
   tacagagatg cactgagata caaacctggt gagcccatca ctttctgtga ggagagtttt 1080
   gtaaagcacc gctcaagcgt gatgaaacag ttcctggaaa ctgccattaa cctccagctt 1140
   tttaagcagt ttatcgatgg tcgactggca aaactaaatg caggaagggg tttctctgat 1200
   gtatttgaag aagagatcac ttcaggtggc ttttgtggag gtaaagacaa gttacaatat 1260
   gattatccat tttctcaata acaattttct tggtctttgc acttgtgtct gataaaacct 1320
   atttcataaa caactaatga tttcctccta aatatgtaat gtcttaaata catttttcat 1380
   cttataaaag ctatggaatt agcttatttt gcctgatacc tgttactcaa ggcattaagt 1440
   tggcctcctg aattggcagc tgttggcctc gataatctct taatattgct ggaaattagt 1500
   aatacagaaa tccaatcaac tcatatcttc ctgtctttcc ttctgaatag tagtattctc 1560
   tgctagaaaa ctactagtga tggttattac tgagtatgaa tttaagaact gaggttatga 1620
   ttggtaatac aatccaaaaa gaagggtctg aacaccaaaa ttctttatac atatttaagt 1680
   aactgtatta ttattataca gatgtcttta cctttttgac tttatagatc actgcagcat 1740
   taagaaagtt tccagtttac cattccataa gtacaattaa tccttctagt gtaaatgttc 1800
   aaatactgtt ataattatct aggcaattaa taatttacaa actgatattt ttgcacgatt 1860
   gtagtggtgt atagtcttga cttgcagagc attttgcttg agtccttgaa atgtcgtgtt 1920
   cattcattat ttgctgagtg cttacaatgt attaggcact gttctaaata ttaagtgtac 1980
   taaataaaca aaaatccttg tattctg 2007
<210> 42
   <211> 2197
   <212> DNA
   <213> Homo sapiens
<400> 42
   gcgggggccg gacggacagc cccacaccga catgtaacca tggactgcag gaccaaggca      60
   aatccagaca gaacctttga cttggtgttg aaagtgaaat gtcatgcctc tgaaaatgaa     120
   gaggacagtc cagcttatct gccgaggatt ccccctggaa aagtacgccg attcgcattt     180
   tgcattaaga aactggaaaa ctttcctgtc ggtcctggcg tagcgcctcc cgtgtccggg     240
   gtagaccttg taccggctga aaccgcatag ctcgaccttc atggcggcag ctccacggga     300
   ggagaaaaga tggccccaac ctgtattttc gaatcctgtg gtattgtgga aattcccaga     360
   ggactttgga gaccaggaaa tactacagag tgtgccaaag ttctgttttc cctttgacgt     420
   tgaaagggtg tctcagaatc aagttggaca gcactttacc tttgtactga cagacattga     480
   aagtaaacag agatttggat tctgcagact gacgtcagga ggcacaattt gtttatgcat     540
   ccttagttac cttccctggt ttgaagtgta ttacaagctt ctaaatactc ttgcagatta     600
   cttggctaag gaactggaaa atgatttgaa tgaaactctc agatcactgt ataaccaccc    660
   agtaccaaag gcaaatactc ctgtaaattt gagtgtgcat tcctacttca ttgcccctga    720
   tgtaactgga ctcccaacaa tacccgagag tagaaatctt acagaatatt ttgttgccgt    780
   ggatgtgaac aacatgctgc agctgtatgc cagtatgctg catgaaaggc gcatcgtgat    840
   tatctcgagc aaattaagca ctttaactgc ctgtatccat ggatcagctg ctcttctata    900
   cccaatgtat tggcaacaca tatacatccc agtgcttcct ccacacctgc tggactactg    960
   ctgtgcccca atgccatacc tgattggaat acactccagc ctcatagaga gagtgaaaaa   1020
   caaatcattg gaagatgttg ttatgttaaa tgttgataca aacacattag aatcaccatt   1080
   tagtgacttg aacaacctac caagtgatgt ggtctcggcc ttgaaaaata aactgaagaa   1140
   gcagtctaca gctacgggtg atggagtagc tagggccttt cttagagcac aggctgcttt   1200
   gtttggatcc tacagagatg cactgagata caaacctggt gagcccatca ctttctgtga   1260
   ggagagtttt gtaaagcacc gctcaagcgt gatgaaacag ttcctggaaa ctgccattaa   1320
   cctccagctt tttaagcagt ttatcgatgg tcgactggca aaactaaatg caggaagggg   1380
   tttctctgat gtatttgaag aagagatcac ttcaggtggc ttttgtggag gtaaagacaa   1440
   gttacaatat gattatccat tttctcaata acaattttct tggtctttgc acttgtgtct   1500
   gataaaacct atttcataaa caactaatga tttcctccta aatatgtaat gtcttaaata   1560
   catttttcat cttataaaag ctatggaatt agcttatttt gcctgatacc tgttactcaa   1620
   ggcattaagt tggcctcctg aattggcagc tgttggcctc gataatctct taatattgct   1680
   ggaaattagt aatacagaaa tccaatcaac tcatatcttc ctgtctttcc ttctgaatag   1740
   tagtattctc tgctagaaaa ctactagtga tggttattac tgagtatgaa tttaagaact   1800
   gaggttatga ttggtaatac aatccaaaaa gaagggtctg aacaccaaaa ttctttatac   1860
   atatttaagt aactgtatta ttattataca gatgtcttta cctttttgac tttatagatc   1920
   actgcagcat taagaaagtt tccagtttac cattccataa gtacaattaa tccttctagt   1980
   gtaaatgttc aaatactgtt ataattatct aggcaattaa taatttacaa actgatattt   2040
   ttgcacgatt gtagtggtgt atagtcttga cttgcagagc attttgcttg agtccttgaa   2100
   atgtcgtgtt cattcattat ttgctgagtg cttacaatgt attaggcact gttctaaata   2160
   ttaagtgtac taaataaaca aaaatccttg tattctg                             2197
<210> 43
   <211> 211
   <212> PRT
   <213> Homo sapiens
<400> 43
   Met Gly Pro Gln His Leu Arg Leu Val Gln Leu Phe Cys Leu Leu Gly
   1               5                   10                  15
   Ala Ile Ser Thr Leu Pro Arg Met Ser Cys Gly Ala Gly Cys Tyr Lys
               20                  25                  30
   Thr Gln Lys Gly Thr Ala Arg Gly Val Val Gly Phe Lys Gly Cys Ser
           35                  40                  45
   Ser Ser Ser Ser Tyr Pro Ala Gln Ile Ser Tyr Leu Val Ser Pro Pro
       50                  55                  60
   Gly Val Ser Ile Ala Ser Tyr Ser Arg Val Cys Arg Ser Tyr Leu Cys
   65                  70                  75                  80
   Asn Asn Leu Thr Asn Leu Glu Pro Phe Val Lys Leu Lys Ala Ser Thr
                   85                  90                  95
   Pro Lys Ser Ile Thr Ser Ala Ser Cys Ser Cys Pro Thr Cys Val Gly
               100                 105                 110
   Glu His Met Lys Asp Cys Leu Pro Asn Phe Val Thr Thr Asn Ser Cys
           115                 120                 125
   Pro Leu Ala Ala Ser Thr Cys Tyr Ser Ser Thr Leu Lys Phe Gln Ala
       130                 135                 140
   Gly Phe Leu Asn Thr Thr Phe Leu Leu Met Gly Cys Ala Arg Glu His
   145                 150                 155                 160
   Asn Gln Leu Leu Ala Asp Phe His His Ile Gly Ser Ile Lys Val Thr
                   165                 170                 175
   Glu Val Leu Asn Ile Leu Glu Lys Ser Gln Ile Val Gly Ala Ala Ser
               180                 185                 190
   Ser Arg Gln Asp Pro Ala Trp Gly Val Val Leu Gly Leu Leu Phe Ala
           195                 200                 205
   Phe Arg Asp
       210
<210> 44
   <211> 246
   <212> PRT
   <213> Homo sapiens
<400> 44
   Met Gly Pro Gln His Leu Arg Leu Val Gln Leu Phe Cys Leu Leu Gly
   1               5                   10                  15
   Ala Ile Ser Thr Leu Pro Arg Ala Gly Ala Leu Leu Cys Tyr Glu Ala
               20                  25                  30
   Thr Ala Ser Arg Phe Arg Ala Val Ala Phe His Asn Trp Lys Trp Leu
           35                  40                  45
   Leu Met Arg Asn Met Val Cys Lys Leu Gln Glu Gly Cys Glu Glu Thr
       50                  55                  60
   Leu Val Phe Ile Glu Thr Gly Thr Ala Arg Gly Val Val Gly Phe Lys
   65                  70                  75                  80
   Gly Cys Ser Ser Ser Ser Ser Tyr Pro Ala Gln Ile Ser Tyr Leu Val
                   85                  90                  95
   Ser Pro Pro Gly Val Ser Ile Ala Ser Tyr Ser Arg Val Cys Arg Ser
               100                 105                 110
   Tyr Leu Cys Asn Asn Leu Thr Asn Leu Glu Pro Phe Val Lys Leu Lys
           115                 120                 125
   Ala Ser Thr Pro Lys Ser Ile Thr Ser Ala Ser Cys Ser Cys Pro Thr
       130                 135                 140
   Cys Val Gly Glu His Met Lys Asp Cys Leu Pro Asn Phe Val Thr Thr
   145                 150                 155                 160
   Asn Ser Cys Pro Leu Ala Ala Ser Thr Cys Tyr Ser Ser Thr Leu Lys
                   165                 170                 175
   Phe Gln Ala Gly Phe Leu Asn Thr Thr Phe Leu Leu Met Gly Cys Ala
               180                 185                 190
   Arg Glu His Asn Gln Leu Leu Ala Asp Phe His His Ile Gly Ser Ile
           195                 200                 205
   Lys Val Thr Glu Val Leu Asn Ile Leu Glu Lys Ser Gln Ile Val Gly
       210                 215                 220
   Ala Ala Ser Ser Arg Gln Asp Pro Ala Trp Gly Val Val Leu Gly Leu
   225                 230                 235                 240
   Leu Phe Ala Phe Arg Asp
                   245
<210> 45
   <211> 705
   <212> DNA
   <213> Homo sapiens
<400> 45
   atgggacccc agcatttgag acttgtgcag ctgttctgcc ttctaggggc catctccact     60
   ctgcctcgta tgtcctgtgg ggctggatgc tataagaccc agaaagggac tgcaagggga    120
   gttgtgggct ttaaaggctg cagctcgtct tcgtcttacc ctgcgcaaat ctcctacctt    180
   gtttccccac ccggagtgtc cattgcctcc tacagtcgcg tctgccggtc ttatctctgc    240
   aacaacctca ccaatttgga gccttttgtg aaactcaagg ccagcactcc taagtctatc    300
   acatctgcgt cctgtagctg cccgacctgt gtgggcgagc acatgaagga ttgcctccca    360
   aattttgtca ccactaattc ttgccccttg gctgcttcta cgtgttacag ttccacctta    420
   aaatttcagg cagggtttct caataccacc ttcctcctca tggggtgtgc tcgtgaacat    480
   aaccagcttt tagcagattt tcatcatatt gggagcatca aagtgactga ggtcctcaac    540
   atcttagaga agtctcagat tgttggtgca gcatcctcca ggcaagatcc tgcttggggt    600
   gtcgtcttag gcctcctgtt tgccttcagg gactgaccat ctagctgcac ccgacaagca    660
   cccagactct ttcacataac aaataaaata gcagagttcc ctttc                    705
<210> 46
   <211> 2022
   <212> DNA
   <213> Homo sapiens
<400> 46
   ggtctgtgca cgggttgcct cgtttctggc cttcccttgc cttgtacctc gacttcgcgc      60
   gtggcttgtt tttgctgagt ggaggcctgc gttttctgtt atgtctcttc cggtgcccca     120
   caactcctga gctacccttc ttttatgcgt tctctcccag cgctgtgatt accgggttgc     180
   ttaccgggcc ctccttcctc agattgcacc cctttccttg tgtctcttct tgtctttgtg     240
   tcggttgtga ttttcctaat ctctgatttt ccttttctct cggacgctct ccctcttcgg     300
   acccattttc tcccgtgctt catgccctga tagcctggcc ccttcccggc ttccttcgct     360
   accggggacg cctctagttt ttctgaattt ctggctggct ccaccctccg cgttcatctt     420
   cctcaagagt tcgcccctct gggggctcct ctgtgtaatc gtcgccttct ctgggtattt     480
   ctgtgaactc cgtctcacac catcccgcca tcttctctgc cttggcccct tttctctgta     540
   cagccagctc tgtgtccttt tcttctcccc ctctaaaatc gactcctctt ctccctgaga     600
   gccccacctt tgtgccccac tcctcatttt cctacgcctc cctctctctg ctggtcctct     660
   ctctccctgc aaggttccat tccatcaatt tgtttgtctt ttgtaggggt ggcatcccct     720
   ctgactactg ctccatcctt tttttttttt tttttttttt tttgcttgag gatttcactt     780
   caatcttttc tggttgcgtc tccacttgta ctcagcttgt taggtccagg tccagttgtt    840
   ctgcatctga ggctggcgtg tgctgtcttc tctgattggc ctaatctccc tcacccccgt    900
   gagatctgtt gtcagccttc gtttctcttt cctgtgtccc agcttttctg cgggtcttgg    960
   cacctttctt ggccacagat ttctgggtta cagagcatgt gtgtctgagg cattgcaggc   1020
   agaaaagggt ggccgacgtg acctctagct ggactgctgg gcaggggagc tgtcctagat   1080
   aaaattggaa agaaacagtg acccagagac aggtggacaa agaattcggg gactgatggg   1140
   aactgagctt gggatccaga ctgaaactga ttccagactg acctctagca cccaggaccc   1200
   agacacaggg ccatgggacc ccagcatttg agacttgtgc agctgttctg ccttctaggg   1260
   gccatctcca ctctgcctcg ggctggagct cttttgtgct atgaagcaac agcctcaaga   1320
   ttcagagctg ttgctttcca taactggaag tggcttctga tgaggaacat ggtgtgtaag   1380
   ctgcaagagg gctgcgagga gacgctagtg ttcattgaga cagggactgc aaggggagtt   1440
   gtgggcttta aaggctgcag ctcgtcttcg tcttaccctg cgcaaatctc ctaccttgtt   1500
   tccccacccg gagtgtccat tgcctcctac agtcgcgtct gccggtctta tctctgcaac   1560
   aacctcacca atttggagcc ttttgtgaaa ctcaaggcca gcactcctaa gtctatcaca   1620
   tctgcgtcct gtagctgccc gacctgtgtg ggcgagcaca tgaaggattg cctcccaaat   1680
   tttgtcacca ctaattcttg ccccttggct gcttctacgt gttacagttc caccttaaaa   1740
   tttcaggcag ggtttctcaa taccaccttc ctcctcatgg ggtgtgctcg tgaacataac   1800
   cagcttttag cagattttca tcatattggg agcatcaaag tgactgaggt cctcaacatc   1860
   ttagagaagt ctcagattgt tggtgcagca tcctccaggc aagatcctgc ttggggtgtc   1920
   gtcttaggcc tcctgtttgc cttcagggac tgaccatcta gctgcacccg acaagcaccc   1980
   agactctttc acataacaaa taaaatagca gagttccctt tc                      2022
<210> 47
   <211> 131
   <212> PRT
   <213> Homo sapiens
<400> 47
   Met Phe Leu Gly Leu Val Gly Leu Arg Thr Lys Gly Arg Arg Trp Ile
   1               5                   10                  15
   Ser Ser Trp Ser Glu Gly Glu Asp Arg Gly Gln Ser Pro Glu Gly Val
               20                  25                  30
   Leu Leu Thr Trp Val Phe Gly Thr Lys Cys Val Met His Pro Cys Glu
           35                  40                  45
   Glu Thr Thr Lys Gln Ala Leu Cys Glu Gln Gln Gly Cys Leu Phe His
       50                  55                  60
   Leu Gly Ala Asp Glu Leu Ser Pro Lys Arg Glu Ser Ala Gln Ser Ile
   65                  70                  75                  80
   Ser Phe Lys Trp Glu Asn Ser Ile Tyr Leu His Ala Thr Leu Phe Leu
                   85                  90                  95
   Ile Gly Glu Tyr Leu His Leu Ala Phe Tyr Tyr Phe Leu Leu Val Leu
               100                 105                 110
   Tyr Ile Leu Cys Ser Phe Leu Ser Tyr Cys Leu Leu Leu Trp Leu Gly
           115                 120                 125
   Ser Phe Leu
       130
<210> 48
   <211> 2950
   <212> DNA
   <213> Homo sapiens
<400> 48
   attatctagg tctcggagga tggagaaatc aaaagtgcca ttttctggcc atttagaacc      60
   attgtcgagt ttgtattggg gccaagcagt gttgcagaag aaaataagac atttagattt     120
   tagttcaggt gatagttgaa gaaattttaa gttcttgaga acacaggcta agggagaaga     180
   aggaggaatg gagggtggaa gtttgcccat agtgaaggag gcaagtttaa agagaaaggt     240
   agagacatgg agaaagggtt ggggagcagc cctgggctgc aatgtgggtg agcagccaaa     300
   gcaggcatcc ccgcaattga cttgccacca agggaatgtg gttgaatgac caaggcaggc     360
   atccctgaag atatcagacg ccaatggaat gtgggtgaat aatcaggcag gcatccccgg     420
   aatgattaaa cactaaggga aggctgcctt cctgagtaca tgaccagcac cagagttttg     480
   ggtccatgga taaaatgtgt ctcctttgtc tctactagaa aatgaaagga attgaaatta     540
   agagaagaga gggagtgaag ggtggcacca agaatgaaag gagaaagagg ttgagggata     600
   gtgagaaagg ttggagaaga gagtaaaaag aggccactta cccgatttaa aatttgtgag     660
   atgttccttg ggctggttgg tctgaggacc aaaggtcgta ggtggatctc ttcatggagt     720
   gagggtgagg acaggggaca gtctcctgaa ggagtcctgc tgacctgggt ctttggcacc     780
   aaatgtgtca tgcatccatg tgaagagacc accaaacagg ctttgtgtga gcaacagggc     840
   tgtttgtttc acctgggtgc agacgagttg agtccaaaaa gagagtcagc ccagtctata     900
   tcttttaaat gggaaaattc aatctattta catgcaacgt tattcttgat aggtgagtac     960
   ttacatctag cattctatta ttttctgctt gttttgtata tcctttgttc cttcctctct   1020
   tactgtttac ttttgtggtt gggcagtttt ctgtagggat aagatttgag tcttatctct   1080
   ttctccctat gtgttagctt taccagtgag tttttatagt ttcacatatt tttatgatgc   1140
   tggttatcat cttctctgtg gggaacaggc cccccaaaac ctggccataa actggcccca   1200
   aaactggcca taaacaaaat ctctgcagca ctgtgacatg tacatgatgg tcttaacgcc   1260
   cacgctggaa ggttgtgggt ttaccagaat gagggcaagg aacacctggc ccacccaggg   1320
   tggaaaaccg cttaaaggca ttcttaaacc acaaacaata gcatgagtga tctgtgcctt   1380
   aaggccatgc tcctgctgca gatagctagt ccaacccatc cctttatttc agcccatctc   1440
   ttcatttccc ataaggaata attttagtta atctaatatc tatagaaaga atgctaatga   1500
   ctagcttgct gttaataaat acatgggtaa acctctgttg gaggctctca gctctgaagg   1560
   ctgtgagacc cttgatttcc tacttcactc ctctatattt ctgtgtcttt aattcctcta   1620
   gtgccactgg gttagagtct ccccgaccaa gctggtctca gcaagtggtc tccatcatgg   1680
   gggctcgaat ccaggttgaa gggtcaccag agtgatggtt ggagaacatg gaactagctg   1740
   gaggacacct gagtactctt aaagcaaacc ccgtggtgag taagaagggg agctcagaag   1800
   catcagggta acaatgggac aagtgtgggg tctggttcgt tccatcttgg aactttttca   1860
   cactgatgat gaggaagaag gagagtataa tgaagtaaca gaagaggtta tagagcaggt   1920
   ttatttgcca gctaaagcta aagtggcaaa ggagggagag gttcatccct acccttctgc   1980
   accccctcat tattattttg aagaaaaaga gtggcctgac cctccagatc tttcttttcc   2040
   agaggacagt gggcaaaaat tagttgcccc agtgactgtt caagcagcac ctcgagcgac   2100
   tgctcttagt tctattcagt caggaattca gcaagctaga tgagaaggtg attaagaggc   2160
   ttggcagttc cctgttagac tacactgccc agaccaacag ggaaatattg tagctacatt   2220
   tgagcctttt tgttttaaat tactcaaaga atttaaacaa gctattaatc agtatggacc   2280
   aggttctcct tttgtaatgg gactattaaa gaacattgct gtttccagtc agatgattcc   2340
   tactgactgg gacgctctta ctcaagcttg tctaactcct gcttagttct tacaatttaa   2400
   aacttggtgg gcagatgaag cttccattca ggcttctcac aacacgcagg accaacctca   2460
   aattaatata actgcagacc aacttttggg ggttggcagt tgggctggtt tagatgcaca   2520
   aatggtcatg caggatgatg ccatagaaca gcttagagga gcgtgcatta gagcttgggg   2580
   aaaaaaaatc acttcaagtg gagaacaata ccctttcttt agtgctataa aacagggacc    2640
   agaagaatca tatgtggatt ttatagctca gttacaggag tctcttaaaa agatgactgc   2700
   agatttggct gctcaggata tagtgttgca attattagct ttcaacaatg ctaatcctga   2760
   ttgccaggct gctctgtgac ctatcagagg gaaagcacat ttagttgatt atatcaaggc   2820
   ctgtggtggt atcagaggta atctgcatca ggccacctgc tagcacgggc aatggcagga   2880
   ctgagagtgg atacagaaag tactccattt cctggagctt gttttaactg tgggaagcat   2940
   ggtcatactg                                                          2950
<210> 49
   <211> 132
   <212> PRT
   <213> Homo sapiens
<400> 49
   Met Ser Gln Gly Arg His Leu Leu Glu Phe Leu Pro Leu Tyr Ile Ala
   1               5                   10                  15
   Phe Met Leu Arg Gly Val Cys Arg Ile Asp Ala Gly Ser Leu Asn Pro
               20                  25                  30
   Glu Leu Phe Leu Pro Met Leu His Glu Glu Asp Trp Cys Trp Glu Ile
           35                  40                  45
   Ala Gly His Val Asp Ser Gln Glu Leu Phe Val Gly Leu Phe Ser Ser
       50                  55                  60
   Thr Ser Thr Gly His Ala Glu Leu Asp Lys Lys Val Asn Gly Leu Tyr
   65                  70                  75                  80
   Tyr Asp Ser Val Phe Gln Leu Ser Leu Asp Arg Met Arg His Thr Arg
                   85                  90                  95
   Ser Met Ala Arg Val Glu Arg Leu Arg His Arg Lys Ala Ile Gln Lys
               100                 105                 110
   Lys Thr Gln Leu Val His His Leu Leu Phe Lys Gly Trp Ala Ser Asp
           115                 120                 125
   Glu Thr Glu Ile
       130
<210> 50
   <211> 399
   <212> DNA
   <213> Homo sapiens
<400> 50
   atgtcacaag gcaggcatct tcttgagttt cttccattgt acatagcttt catgttacgt 60
   ggggtttgta ggatagacgc tggaagcctt aatccagaac tgtttttgcc aatgttacat 120
   gaagaggatt ggtgttggga gatagctggc catgtggact cccaagagtt attcgttggt 180
   ttgttttcta gtacctctac tgggcatgca gagctggaca aaaaggttaa tggactttat 240
   tatgactctg tattccagtt gtctctggac cgtatgcgtc atacaaggag tatggctaga 300
   gtagagaggc tgagacacag gaaagcgatc cagaaaaaga ctcagttagt ccatcatctg 360
   ctatttaaag gatgggcttc tgatgaaact gaaatttag 399
<210> 51
   <211> 431
   <212> PRT
   <213> Homo sapiens
<400> 51
   Met Arg Arg Leu Arg Arg Leu Ala His Leu Val Leu Phe Cys Pro Phe
   1               5                   10                  15
   Ser Lys Arg Leu Gln Gly Arg Leu Pro Gly Leu Arg Val Arg Cys Ile
               20                  25                  30
   Phe Leu Ala Trp Leu Gly Val Phe Ala Gly Ser Trp Leu Val Tyr Val
           35                  40                  45
   His Tyr Ser Ser Tyr Ser Glu Arg Cys Arg Gly His Val Cys Gln Val
       50                  55                  60
   Val Ile Cys Asp Gln Tyr Arg Lys Gly Ile Ile Ser Gly Ser Val Cys
   65                  70                  75                  80
   Gln Asp Leu Cys Glu Leu His Met Val Glu Trp Arg Thr Cys Leu Ser
                   85                  90                  95
   Val Ala Pro Gly Gln Gln Val Tyr Ser Gly Leu Trp Arg Asp Lys Asp
               100                 105                 110
   Val Thr Ile Lys Cys Gly Ile Glu Glu Thr Leu Asp Ser Lys Ala Arg
           115                 120                 125
   Ser Asp Ala Ala Pro Arg Arg Glu Leu Val Leu Phe Asp Lys Pro Thr
       130                 135                 140
   Arg Gly Thr Ser Ile Lys Glu Phe Arg Glu Met Thr Leu Ser Phe Leu
   145                 150                 155                 160
   Lys Ala Asn Leu Gly Asp Leu Pro Ser Leu Pro Ala Leu Val Gly Gln
                   165                 170                 175
   Val Leu Leu Met Ala Asp Phe Asn Lys Asp Asn Arg Val Ser Leu Ala
               180                 185                 190
   Glu Ala Lys Ser Val Trp Ala Leu Leu Gln Arg Asn Glu Phe Leu Leu
           195                 200                 205
   Leu Leu Ser Leu Gln Glu Lys Glu His Ala Ser Arg Leu Leu Gly Tyr
       210                 215                 220
   Cys Gly Asp Leu Tyr Leu Thr Glu Gly Val Pro His Gly Ala Trp His
   225                 230                 235                 240
   Ala Ala Ala Leu Pro Pro Leu Leu Arg Pro Leu Leu Pro Pro Ala Leu
                   245                 250                 255
   Gln Gly Ala Leu Gln Gln Trp Leu Gly Pro Ala Trp Pro Trp Arg Ala
               260                 265                 270
   Lys Ile Ala Ile Gly Leu Leu Glu Phe Val Glu Glu Leu Phe His Gly
           275                 280                 285
   Ser Tyr Gly Thr Phe Tyr Met Cys Glu Thr Thr Leu Ala Asn Val Gly
       290                 295                 300
   Tyr Thr Ala Thr Tyr Asp Phe Lys Met Ala Asp Leu Gln Gln Val Ala
   305                 310                 315                 320
   Pro Glu Ala Thr Val Arg Arg Phe Leu Gln Gly Arg Arg Cys Glu His
                   325                 330                 335
   Ser Thr Asp Cys Thr Tyr Gly Arg Asp Cys Arg Ala Pro Cys Asp Arg
               340                 345                 350
   Leu Met Arg Gln Cys Lys Gly Asp Leu Ile Gln Pro Asn Leu Ala Lys
           355                 360                 365
   Val Cys Ala Leu Leu Arg Gly Tyr Leu Leu Pro Gly Ala Pro Ala Asp
       370                 375                 380
   Leu Arg Glu Glu Leu Gly Thr Gln Leu Arg Thr Cys Thr Thr Leu Ser
   385                 390                 395                 400
   Gly Leu Ala Ser Gln Val Glu Ala His His Ser Leu Val Leu Ser His
                   405                 410                 415
   Leu Lys Thr Leu Leu Trp Lys Lys Ile Ser Asn Thr Lys Tyr Ser
               420                 425                 430
<210> 52
   <211> 344
   <212> PRT
   <213> Homo sapiens
<400> 52
   Met Val Glu Trp Arg Thr Cys Leu Ser Val Ala Pro Gly Gln Gln Val
   1               5                   10                  15
   Tyr Ser Gly Leu Trp Arg Asp Lys Asp Val Thr Ile Lys Cys Gly Ile
               20                  25                  30
   Glu Glu Thr Leu Asp Ser Lys Ala Arg Ser Asp Ala Ala Pro Arg Arg
           35                  40                  45
   Glu Leu Val Leu Phe Asp Lys Pro Thr Arg Gly Thr Ser Ile Lys Glu
       50                  55                  60
   Phe Arg Glu Met Thr Leu Ser Phe Leu Lys Ala Asn Leu Gly Asp Leu
   65                  70                  75                  80
   Pro Ser Leu Pro Ala Leu Val Gly Gln Val Leu Leu Met Ala Asp Phe
                   85                  90                  95
   Asn Lys Asp Asn Arg Val Ser Leu Ala Glu Ala Lys Ser Val Trp Ala
               100                 105                 110
   Leu Leu Gln Arg Asn Glu Phe Leu Leu Leu Leu Ser Leu Gln Glu Lys
           115                 120                 125
   Glu His Ala Ser Arg Leu Leu Gly Tyr Cys Gly Asp Leu Tyr Leu Thr
       130                 135                 140
   Glu Gly Val Pro His Gly Ala Trp His Ala Ala Ala Leu Pro Pro Leu
   145                 150                 155                 160
   Leu Arg Pro Leu Leu Pro Pro Ala Leu Gln Gly Ala Leu Gln Gln Trp
                   165                 170                 175
   Leu Gly Pro Ala Trp Pro Trp Arg Ala Lys Ile Ala Ile Gly Leu Leu
               180                 185                 190
   Glu Phe Val Glu Glu Leu Phe His Gly Ser Tyr Gly Thr Phe Tyr Met
           195                 200                 205
   Cys Glu Thr Thr Leu Ala Asn Val Gly Tyr Thr Ala Thr Tyr Asp Phe
       210                 215                 220
   Lys Met Ala Asp Leu Gln Gln Val Ala Pro Glu Ala Thr Val Arg Arg
   225                 230                 235                 240
   Phe Leu Gln Gly Arg Arg Cys Glu His Ser Thr Asp Cys Thr Tyr Gly
                   245                 250                 255
   Arg Asp Cys Arg Ala Pro Cys Asp Arg Leu Met Arg Gln Cys Lys Gly
               260                 265                 270
   Asp Leu Ile Gln Pro Asn Leu Ala Lys Val Cys Ala Leu Leu Arg Gly
           275                 280                 285
   Tyr Leu Leu Pro Gly Ala Pro Ala Asp Leu Arg Glu Glu Leu Gly Thr
       290                 295                 300
   Gln Leu Arg Thr Cys Thr Thr Leu Ser Gly Leu Ala Ser Gln Val Glu
   305                 310                 315                 320
   Ala His His Ser Leu Val Leu Ser His Leu Lys Thr Leu Leu Trp Lys
                   325                 330                 335
   Lys Ile Ser Asn Thr Lys Tyr Ser
               340
<210> 53
   <211> 1645
   <212> DNA
   <213> Homo sapiens
<400> 53
   agggagcggc ggccgctgcg ggccgggccg ggccggggct gaggccgagc gagccgcggg 60
   gcccgcgcag ccccggccgg agcccaccat gcggcggctg cggcgcctgg cgcacctggt 120
   gctcttctgc cccttctcca agcgcctgca gggccggctc ccaggcctca gggtccgctg 180
   catcttcctg gcctggctgg gcgtctttgc aggcagctgg ctggtgtacg tgcactactc 240
   gtcctactcg gagcgctgtc gcggccatgt ctgccaggtg gtcatttgtg accagtaccg 300
   caaggggatc atctcgggct ccgtctgcca ggacctgtgt gagctgcata tggtggagtg 360
   gaggacctgc ctctcggtgg ccccgggcca gcaggtgtac agcgggctct ggcgggacaa 420
   ggatgtaacc atcaagtgtg gcattgagga gaccctcgac tccaaggccc ggtcggatgc 480
   ggccccccgg cgggagctgg tactgtttga caagcccacc cggggcacct ccatcaagga 540
   attccgggag atgaccctca gcttcctcaa ggcgaacctg ggagacctgc cttccctgcc 600
   ggcgctggtt ggccaggtcc tgctcatggc tgacttcaac aaggacaacc gggtgtccct 660
   ggcggaagcc aagtccgtgt gggccctgct gcagcgtaac gagttcctgc tgctgctgtc 720
   cctgcaggag aaggagcacg cctccagact gctgggctac tgtggggacc tctacctcac 780
   cgagggcgtg ccgcatggcg cctggcacgc ggccgccctt ccacccctgt tgcgcccact 840
   gctgccgcct gccctgcagg gtgctctcca gcagtggctg gggcctgcgt ggccttggcg 900
   ggccaagatc gccatcggcc tgctggagtt cgtggaggag ctcttccacg gctcttacgg 960
   gactttctac atgtgtgaga ccacactggc caacgtgggc tacacagcca cctacgactt 1020
   caagatggcc gacctgcagc aggtggcacc cgaggccacc gtgcgccgct tcctgcaggg 1080
   ccgccgctgc gagcacagca ccgactgcac ctacgggcgc gactgcaggg ccccgtgtga 1140
   caggctcatg aggcagtgca agggcgacct catccagccc aacctggcca aggtgtgcgc 1200
   actgctacgg ggctacctgc tgcctggcgc gcccgccgac ctccgcgagg agctgggcac 1260
   acagctgcgc acctgtacca cgctgagcgg gctggccagc caggtggagg cccatcactc 1320
   gctggtgctc agccacctca agactctgct ctggaagaag atctccaaca ccaagtactc 1380
   ttgatggggc agtgaggggc ctggccaccc ttcctggagc tggccaggtg ccagggtcca 1440
   accctccctc aaggaatcct gtcagaagat gtgaaatgca actgtgttgc aaaatcactc 1500
   ccctaccgtc agggctctgg attccagcac cacagacatg agaccccagc tcggagcaaa 1560
   ggcggacatg gacatcccgg caggagagtc ctccaagggg gtttgttact ctgaagaacg 1620
   taatgtcaat aaacagcttt tatgt 1645
<210> 54
   <211> 2410
   <212> DNA
   <213> Homo sapiens
<400> 54
   ggccgcagcc tgtcctcctg cctcagcctg gggaaggctg ggccgggcca aagagggagg      60
   ccaagtcctt gggacaggag gagacccaca cctgagatta gtggaaaccc agccagaagc     120
   tgccatgcag gtgtggctct gggcatcagg atctgtcgtg agagccccca tgagtgccca     180
   gtgcagaatg gctggcagcc cgccctgacg gggagcagag gggctggacg cggtgccctt     240
   cacgggatga cacccagctg tttgccctgt gtccaggggg ttgcttctct gacagaggcc     300
   ctatggctcg tgtctgactc ctgtccaggt tctgccagcc tgaccatcca tcgctctggc     360
   accaagagcc cacccttttg ttcttcctgg cgtcccaggg aaagccctgc ctgggtgggg     420
   cagctcctgg cccttcagat ggaagacgca gtccagtcag caccatcata ggaaacaagt     480
   tcagaaatgt ctcacttact attccgggca gggagggcgc catgagtcag ggggtgcatc     540
   ctccctcctg gcgtcacccg aggcaggaat gaagagtcag gcagagagcg cgcgtgtggc     600
   agctggtggt gtagatatta gggactagtg tgaattctag ttcaccggcc aatgcctgga     660
   tggtccagag ctgggtcggc tgggcggaga gctgcctcca ggttcctgcc tctggccctg     720
   gcgtggggtc gacactgggt gtggtgtgtg tctcatgtcc aggcagtggc ctttgctgtg     780
   ccgtcctgtt acaggagcca ggatggtggg gacgggaccg gaccggaggg ttggcggggc     840
   tgcccctgca gccgacagcc ccatcctgca gccaccaatg gcatgaccca gggccccggc     900
   actgcctgtg tgaggggctg gcagctttcc aactgcagca agtggaggcc cctgccagct     960
   tcgggcctgt gggcaggggc tcagtggggc aggggtgtgg ctgccccgcc cggcacgcct    1020
   gcacctgtct cctcagtgtg accagtaccg caaggggatc atctcgggct ccgtctgcca    1080
   ggacctgtgt gagctgcata tggtggagtg gaggacctgc ctctcggtgg ccccgggcca    1140
   gcaggtgtac agcgggctct ggcgggacaa ggatgtaacc atcaagtgtg gcattgagga    1200
   gaccctcgac tccaaggccc ggtcggatgc ggcccccggg cgggagctgg tactgtttga    1260
   caagcccacc cggggcacct ccatcaagga attccgggag atgaccctca gcttcctcaa    1320
   ggcgaacctg ggagacctgc cttccctgcc ggcgctggtt ggccaggtcc tgctcatggc    1380
   tgacttcaac aaggacaacc gggtgtccct ggcggaagcc aagtccgtgt gggccctgct    1440
   gcagcgtaac gagttcctgc tgctgctgtc cctgcaggag aaggagcacg cctccagact    1500
   gctgggctac tgtggggacc tctacctcac cgagggcgtg ccgcatggcg cctggcacgc    1560
   ggccgccctt ccacccctgt tgcgcccact gctgccgcct gccctgcagg gtgctctcca    1620
   gcagtggctg gggcctgcgt ggccttggcg ggccaagatc gccatcggcc tgctggagtt    1680
   cgtggaggag ctcttccacg gctcttacgg gactttctac atgtgtgaga ccacactggc    1740
   caacgtgggc tacacagcca cctacgactt caagatggcc gacctgcagc aggtggcacc    1800
   cgaggccacc gtgcgccgct tcctgcaggg ccgccgctgc gagcacagca ccgactgcac    1860
   ctacgggcgc gactgcaggg ccccgtgtga caggctcatg aggcagtgca agggcgacct    1920
   catccagccc aacctggcca aggtgtgcgc actgctacgg ggctacctgc tgcctggcgc    1980
   gcccgccgac ctccgcgagg agctgggcac acagctgcgc acctgtacca cgctgagcgg    2040
   gctggccagc caggtggagg cccatcactc gctggtgctc agccacctca agactctgct    2100
   ctggaagaag atctccaaca ccaagtactc ttgatggggc agtgaggggc ctggccaccc    2160
   ttcctggagc tggccaggtg ccagggtcca accctccctc aaggaatcct gtcagaagat    2220
   gtgaaatgca actgtgttgc aaaatcactc ccctaccgtc agggctctgg attccagcac    2280
   cacagacatg agaccccagc tcggagcaaa ggcggacatg gacatcccgg caggagagtc    2340
   ctccaagggg gtttgttact ctgaagaacg taatgtcaat aaacagcttt tatgtaatgc    2400
   ccagggctga                                                           2410
<210> 55
   <211> 2778
   <212> PRT
   <213> Homo sapiens
<400> 55
   Met Ala Leu Gly Lys Val Leu Ala Met Ala Leu Val Leu Ala Leu Ala
   1               5                   10                  15
   Val Leu Gly Ser Leu Ser Pro Gly Ala Arg Ala Gly Asp Cys Lys Gly
               20                  25                  30
   Gln Arg Gln Val Leu Arg Glu Ala Pro Gly Phe Val Thr Asp Gly Ala
           35                  40                  45
   Gly Asn Tyr Ser Val Asn Gly Asn Cys Glu Trp Leu Ile Glu Ala Pro
       50                  55                  60
   Ser Pro Gln His Arg Ile Leu Leu Asp Phe Leu Phe Leu Asp Thr Glu
   65                  70                  75                  80
   Cys Thr Tyr Asp Tyr Leu Phe Val Tyr Asp Gly Asp Ser Pro Arg Gly
                   85                  90                  95
   Pro Leu Leu Ala Ser Leu Ser Gly Ser Thr Arg Pro Pro Pro Ile Glu
               100                 105                 110
   Ala Ser Ser Gly Lys Met Leu Leu His Leu Phe Ser Asp Ala Asn Tyr
           115                 120                 125
   Asn Leu Leu Gly Phe Asn Ala Ser Phe Arg Phe Ser Leu Cys Pro Gly
       130                 135                 140
   Gly Cys Gln Ser His Gly Gln Cys Gln Pro Pro Gly Val Cys Ala Cys
   145                 150                 155                 160
   Glu Pro Gly Trp Gly Gly Pro Asp Cys Gly Leu Gln Glu Cys Ser Ala
                   165                 170                 175
   Tyr Cys Gly Ser His Gly Thr Cys Ala Ser Pro Leu Gly Pro Cys Arg
               180                 185                 190
   Cys Glu Pro Gly Phe Leu Gly Arg Ala Cys Asp Leu His Leu Trp Glu
           195                 200                 205
   Asn Gln Gly Ala Gly Trp Trp His Asn Val Ser Ala Arg Asp Pro Ala
       210                 215                 220
   Phe Ser Ala Arg Ile Gly Ala Ala Gly Ala Phe Leu Ser Pro Pro Gly
   225                 230                 235                 240
   Leu Leu Ala Val Phe Gly Gly Gln Asp Leu Asn Asn Ala Leu Gly Asp
                   245                 250                 255
   Leu Val Leu Tyr Asn Phe Ser Ala Asn Thr Trp Glu Ser Trp Asp Leu
               260                 265                 270
   Ser Pro Ala Pro Ala Ala Arg His Ser His Val Ala Val Ala Trp Ala
           275                 280                 285
   Gly Ser Leu Val Leu Met Gly Gly Glu Leu Ala Asp Gly Ser Leu Thr
       290                 295                 300
   Asn Asp Val Trp Ala Phe Ser Pro Leu Gly Arg Gly His Trp Glu Leu
   305                 310                 315                 320
   Leu Ala Pro Pro Ala Ser Ser Ser Ser Gly Pro Pro Gly Leu Ala Gly
                   325                 330                 335
   His Ala Ala Ala Leu Val Asp Asp Val Trp Leu Tyr Val Ser Gly Gly
               340                 345                 350
   Arg Thr Pro His Asp Leu Phe Ser Ser Gly Leu Phe Arg Phe Arg Leu
           355                 360                 365
   Asp Ser Thr Ser Gly Gly Tyr Trp Glu Gln Val Ile Pro Ala Gly Gly
       370                 375                 380
   Arg Pro Pro Ala Ala Thr Gly His Ser Met Val Phe His Ala Pro Ser
   385                 390                 395                 400
   Arg Ala Leu Leu Val His Gly Gly His Arg Pro Ser Thr Ala Arg Phe
                   405                 410                 415
   Ser Val Arg Val Asn Ser Thr Glu Leu Phe His Val Asp Arg His Val
               420                 425                 430
   Trp Thr Thr Leu Lys Gly Arg Asp Gly Leu Gln Gly Pro Arg Glu Arg
           435                 440                 445
   Ala Phe His Thr Ala Ser Val Leu Gly Asn Tyr Met Val Val Tyr Gly
       450                 455                 460
   Gly Asn Val His Thr His Tyr Gln Glu Glu Lys Cys Tyr Glu Asp Gly
   465                 470                 475                 480
   Ile Phe Phe Tyr His Leu Gly Cys His Gln Trp Val Ser Gly Ala Glu
                   485                 490                 495
   Leu Ala Pro Pro Gly Thr Pro Glu Gly Arg Ala Ala Pro Pro Ser Gly
               500                 505                 510
   Arg Tyr Ser His Val Ala Ala Val Leu Gly Gly Ser Val Leu Leu Val
           515                 520                 525
   Ala Gly Gly Tyr Ser Gly Arg Pro Arg Gly Asp Leu Met Ala Tyr Lys
       530                 535                 540
   Val Pro Pro Phe Val Phe Gln Ala Pro Ala Pro Asp Tyr His Leu Asp
   545                 550                 555                 560
   Tyr Cys Ser Met Tyr Thr Asp His Ser Val Cys Ser Arg Asp Pro Glu
                   565                 570                 575
   Cys Ser Trp Cys Gln Gly Ala Cys Gln Ala Ala Pro Pro Pro Gly Thr
               580                 585                 590
   Pro Leu Gly Ala Cys Pro Ala Ala Ser Cys Leu Gly Leu Gly Arg Leu
           595                 600                 605
   Leu Gly Asp Cys Gln Ala Cys Leu Ala Phe Ser Ser Pro Thr Ala Pro
       610                 615                 620
   Pro Arg Gly Pro Gly Thr Leu Gly Trp Cys Val His Asn Glu Ser Cys
   625                 630                 635                 640
   Leu Pro Arg Pro Glu Gln Ala Arg Cys Arg Gly Glu Gln Ile Ser Gly
                   645                 650                 655
   Thr Val Gly Trp Trp Gly Pro Ala Pro Val Phe Val Thr Ser Leu Glu
               660                 665                 670
   Ala Cys Val Thr Gln Ser Phe Leu Pro Gly Leu His Leu Leu Thr Phe
           675                 680                 685
   Gln Gln Pro Pro Asn Thr Ser Gln Pro Asp Lys Glu Glu Val Gly Arg
       690                 695                 700
   Trp Val Ala His Gln Glu Lys Glu Thr Arg Arg Leu Gln Arg Pro Gly
   705                 710                 715                 720
   Ser Ala Arg Leu Phe Pro Leu Pro Gly Arg Asp His Lys Tyr Ala Val
                   725                 730                 735
   Glu Ile Gln Gly Gln Leu Asn Gly Ser Ala Gly Pro Gly His Ser Glu
               740                 745                 750
   Leu Thr Leu Leu Trp Asp Arg Thr Gly Val Pro Gly Gly Ser Glu Ile
           755                 760                 765
   Ser Phe Phe Phe Leu Glu Pro Tyr Arg Ser Ser Ser Cys Thr Ser Tyr
       770                 775                 780
   Ser Ser Cys Leu Gly Cys Leu Ala Asp Gln Gly Cys Gly Trp Cys Leu
   785                 790                 795                 800
   Thr Ser Ala Thr Cys His Leu Arg Gln Gly Gly Ala His Cys Gly Asp
                   805                 810                 815
   Asp Gly Ala Gly Gly Ser Leu Leu Val Leu Val Pro Thr Leu Cys Pro
               820                 825                 830
   Leu Cys Glu Glu His Arg Asp Cys His Ala Cys Thr Gln Asp Pro Phe
           835                 840                 845
   Cys Glu Trp His Gln Ser Thr Ser Arg Lys Gly Asp Ala Ala Cys Ser
       850                 855                 860
   Arg Arg Gly Arg Gly Arg Gly Ala Leu Lys Ser Pro Glu Glu Cys Pro
   865                 870                 875                 880
   Pro Leu Cys Ser Gln Arg Leu Thr Cys Glu Asp Cys Leu Ala Asn Ser
                   885                 890                 895
   Ser Gln Cys Ala Trp Cys Gln Ser Thr His Thr Cys Phe Leu Phe Ala
               900                 905                 910
   Ala Tyr Leu Ala Arg Tyr Pro His Gly Gly Cys Arg Gly Trp Asp Asp
           915                 920                 925
   Ser Val His Ser Glu Pro Arg Cys Arg Ser Cys Asp Gly Phe Leu Thr
       930                 935                 940
   Cys His Glu Cys Leu Gln Ser His Glu Cys Gly Trp Cys Gly Asn Glu
   945                 950                 955                 960
   Asp Asn Pro Thr Leu Gly Arg Cys Leu Gln Gly Asp Phe Ser Gly Pro
                   965                 970                 975
   Leu Gly Gly Gly Asn Cys Ser Leu Trp Val Gly Glu Gly Leu Gly Leu
               980                 985                 990
   Pro Val Ala Leu Pro Ala Arg Trp  Ala Tyr Ala Arg Cys  Pro Asp Val
           995                 1000                 1005
   Asp Glu  Cys Arg Leu Gly Leu  Ala Arg Cys His Pro  Arg Ala Thr
       1010                 1015                 1020
   Cys Leu  Asn Thr Pro Leu Ser  Tyr Glu Cys His Cys  Gln Arg Gly
       1025                 1030                 1035
   Tyr Gln  Gly Asp Gly Ile Ser  His Cys Asn Arg Thr  Cys Leu Glu
       1040                 1045                 1050
   Asp Cys  Gly His Gly Val Cys  Ser Gly Pro Pro Asp  Phe Thr Cys
       1055                 1060                 1065
   Val Cys  Asp Leu Gly Trp Thr  Ser Asp Leu Pro Pro  Pro Thr Pro
       1070                 1075                 1080
   Ala Pro  Gly Pro Pro Ala Pro  Arg Cys Ser Arg Asp  Cys Gly Cys
       1085                 1090                 1095
   Ser Phe  His Ser His Cys Arg  Lys Arg Gly Pro Gly  Phe Cys Asp
       1100                 1105                 1110
   Glu Cys  Gln Asp Trp Thr Trp  Gly Glu His Cys Glu  Arg Cys Arg
       1115                 1120                 1125
   Pro Gly  Ser Phe Gly Asn Ala  Thr Gly Ser Arg Gly  Cys Arg Pro
       1130                 1135                 1140
   Cys Gln  Cys Asn Gly His Gly  Asp Pro Arg Arg Gly  His Cys Asp
       1145                 1150                 1155
   Asn Leu  Ser Gly Leu Cys Phe  Cys Gln Asp His Thr  Glu Gly Ala
       1160                 1165                 1170
   His Cys  Gln Leu Cys Ser Pro  Gly Tyr Tyr Gly Asp  Pro Arg Ala
       1175                 1180                 1185
   Gly Gly  Ser Cys Phe Arg Glu  Cys Gly Gly Arg Ala  Leu Leu Thr
       1190                 1195                 1200
   Asn Val  Ser Ser Val Ala Leu  Gly Ser Arg Arg Val  Gly Gly Leu
       1205                 1210                 1215
   Leu Pro  Pro Gly Gly Gly Ala  Ala Arg Ala Gly Pro  Gly Leu Ser
       1220                 1225                 1230
   Tyr Cys  Val Trp Val Val Ser  Ala Thr Glu Glu Leu  Gln Pro Cys
       1235                 1240                 1245
   Ala Pro  Gly Thr Leu Cys Pro  Pro Leu Thr Leu Thr  Phe Ser Pro
       1250                 1255                 1260
   Asp Ser Ser Thr Pro Cys Thr Leu Ser Tyr Val Leu Ala Phe Asp
   1265 1270 1275
   Gly Phe Pro Arg Phe Leu Asp Thr Gly Val Val Gln Ser Asp Arg
   1280 1285 1290
   Ser Leu Ile Ala Ala Phe Cys Gly Gln Arg Arg Asp Arg Pro Leu
   1295 1300 1305
   Thr Val Gln Ala Leu Ser Gly Leu Leu Val Leu His Trp Glu Ala
   1310 1315 1320
   Asn Gly Ser Ser Ser Trp Gly Phe Asn Ala Ser Val Gly Ser Ala
   1325 1330 1335
   Arg Cys Gly Ser Gly Gly Pro Gly Ser Cys Pro Val Pro Gln Glu
   1340 1345 1350
   Cys Val Pro Gln Asp Gly Ala Ala Gly Ala Gly Leu Cys Arg Cys
   1355 1360 1365
   Pro Gln Gly Trp Ala Gly Pro His Cys Arg Met Ala Leu Cys Pro
   1370 1375 1380
   Glu Asn Cys Asn Ala His Thr Gly Ala Gly Thr Cys Asn Gln Ser
   1385 1390 1395
   Leu Gly Val Cys Ile Cys Ala Glu Gly Phe Gly Gly Pro Asp Cys
   1400 1405 1410
   Ala Thr Lys Leu Asp Gly Gly Gln Leu Val Trp Glu Thr Leu Met
   1415 1420 1425
   Asp Ser Arg Leu Ser Ala Asp Thr Ala Ser Arg Phe Leu His Arg
   1430 1435 1440
   Leu Gly His Thr Met Val Asp Gly Pro Asp Ala Thr Leu Trp Met
   1445 1450 1455
   Phe Gly Gly Leu Gly Leu Pro Gln Gly Leu Leu Gly Asn Leu Tyr
   1460 1465 1470
   Arg Tyr Ser Val Ser Glu Arg Arg Trp Thr Gln Met Leu Ala Gly
   1475 1480 1485
   Ala Glu Asp Gly Gly Pro Gly Pro Ser Pro Arg Ser Phe His Ala
   1490 1495 1500
   Ala Ala  Tyr Val Pro Ala Gly  Arg Gly Ala Met Tyr  Leu Leu Gly
       1505                 1510                 1515
   Gly Leu  Thr Ala Gly Gly Val  Thr Arg Asp Phe Trp  Val Leu Asn
       1520                 1525                 1530
   Leu Thr  Thr Leu Gln Trp Arg  Gln Glu Lys Ala Pro  Gln Thr Val
       1535                 1540                 1545
   Glu Leu  Pro Ala Val Ala Gly  His Thr Leu Thr Ala  Arg Arg Gly
       1550                 1555                 1560
   Leu Ser  Leu Leu Leu Val Gly  Gly Tyr Ser Pro Glu  Asn Gly Phe
       1565                 1570                 1575
   Asn Gln  Gln Leu Leu Glu Tyr  Gln Leu Ala Thr Gly  Thr Trp Val
       1580                 1585                 1590
   Ser Gly  Ala Gln Ser Gly Thr  Pro Pro Thr Gly Leu  Tyr Gly His
       1595                 1600                 1605
   Ser Ala  Val Tyr His Glu Ala  Thr Asp Ser Leu Tyr  Val Phe Gly
       1610                 1615                 1620
   Gly Phe  Arg Phe His Val Glu  Leu Ala Ala Pro Ser  Pro Glu Leu
       1625                 1630                 1635
   Tyr Ser  Leu His Cys Pro Asp  Arg Thr Trp Ser Leu  Leu Ala Pro
       1640                 1645                 1650
   Ser Gln  Gly Ala Lys Arg Asp  Arg Met Arg Asn Val  Arg Gly Ser
       1655                 1660                 1665
   Ser Arg  Gly Leu Gly Gln Val  Pro Gly Glu Gln Pro  Gly Ser Trp
       1670                 1675                 1680
   Gly Phe  Arg Glu Val Arg Lys  Lys Met Ala Leu Trp  Ala Ala Leu
       1685                 1690                 1695
   Ala Gly  Thr Gly Gly Phe Leu  Glu Glu Ile Ser Pro  His Leu Lys
       1700                 1705                 1710
   Glu Pro  Arg Pro Arg Leu Phe  His Ala Ser Ala Leu  Leu Gly Asp
       1715                 1720                 1725
   Thr Met  Val Val Leu Gly Gly  Arg Ser Asp Pro Asp  Glu Phe Ser
       1730                 1735                 1740
   Ser Asp Val Leu Leu Tyr Gln Val Asn Cys Asn Ala Trp Leu Leu
   1745 1750 1755
   Pro Asp Leu Thr Arg Ser Ala Ser Val Gly Pro Pro Met Glu Glu
   1760 1765 1770
   Ser Val Ala His Ala Val Ala Ala Val Gly Ser Arg Leu Tyr Ile
   1775 1780 1785
   Ser Gly Gly Phe Gly Gly Val Ala Leu Gly Arg Leu Leu Ala Leu
   1790 1795 1800
   Thr Leu Pro Pro Asp Pro Cys Arg Leu Leu Ser Ser Pro Glu Ala
   1805 1810 1815
   Cys Asn Gln Ser Gly Ala Cys Thr Trp Cys His Gly Ala Cys Leu
   1820 1825 1830
   Ser Gly Asp Gln Ala His Arg Leu Gly Cys Gly Gly Ser Pro Cys
   1835 1840 1845
   Ser Pro Met Pro Arg Ser Pro Glu Glu Cys Arg Arg Leu Arg Thr
   1850 1855 1860
   Cys Ser Glu Cys Leu Ala Arg His Pro Arg Thr Leu Gln Pro Gly
   1865 1870 1875
   Asp Gly Glu Ala Ser Thr Pro Arg Cys Lys Trp Cys Thr Asn Cys
   1880 1885 1890
   Pro Glu Gly Ala Cys Ile Gly Arg Asn Gly Ser Cys Thr Ser Glu
   1895 1900 1905
   Asn Asp Cys Arg Ile Asn Gln Arg Glu Val Phe Trp Ala Gly Asn
   1910 1915 1920
   Cys Ser Glu Ala Ala Cys Gly Ala Ala Asp Cys Glu Gln Cys Thr
   1925 1930 1935
   Arg Glu Gly Lys Cys Met Trp Thr Arg Gln Phe Lys Arg Thr Gly
   1940 1945 1950
   Glu Thr Arg Arg Ile Leu Ser Val Gln Pro Thr Tyr Asp Trp Thr
   1955 1960 1965
   Cys Phe Ser His Ser Leu Leu Asn Val Ser Pro Met Pro Val Glu
   1970 1975 1980
   Ser Ser Pro Pro Leu Pro Cys Pro Thr Pro Cys His Leu Leu Pro
       1985                1990                1995
   Asn Cys Thr Ser Cys Leu Asp Ser Lys Gly Ala Asp Gly Gly Trp
       2000                2005                2010
   Gln His Cys Val Trp Ser Ser Ser Leu Gln Gln Cys Leu Ser Pro
       2015                2020                2025
   Ser Tyr Leu Pro Leu Arg Cys Met Ala Gly Gly Cys Gly Arg Leu
       2030                2035                2040
   Leu Arg Gly Pro Glu Ser Cys Ser Leu Gly Cys Ala Gln Ala Thr
       2045                2050                2055
   Gln Cys Ala Leu Cys Leu Arg Arg Pro His Cys Gly Trp Cys Ala
       2060                2065                2070
   Trp Gly Gly Gln Asp Gly Gly Gly Arg Cys Met Glu Gly Gly Leu
       2075                2080                2085
   Ser Gly Pro Arg Asp Gly Leu Thr Cys Gly Arg Pro Gly Ala Ser
       2090                2095                2100
   Trp Ala Phe Leu Ser Cys Pro Pro Glu Asp Glu Cys Ala Asn Gly
       2105                2110                2115
   His His Asp Cys Asn Glu Thr Gln Asn Cys His Asp Gln Pro His
       2120                2125                2130
   Gly Tyr Glu Cys Ser Cys Lys Thr Gly Tyr Thr Met Asp Asn Met
       2135                2140                2145
   Thr Gly Leu Cys Arg Pro Val Cys Ala Gln Gly Cys Val Asn Gly
       2150                2155                2160
   Ser Cys Val Glu Pro Asp His Cys Arg Cys His Phe Gly Phe Val
       2165                2170                2175
   Gly Arg Asn Cys Ser Thr Glu Cys Arg Cys Asn Arg His Ser Glu
       2180                2185                2190
   Cys Ala Gly Val Gly Ala Arg Asp His Cys Leu Leu Cys Arg Asn
       2195                2200                2205
   His Thr Lys Gly Ser His Cys Glu Gln Cys Leu Pro Leu Phe Val
       2210                2215                2220
   Gly Ser  Ala Val Gly Gly Gly  Thr Cys Arg Pro Cys  His Ala Phe
       2225                 2230                 2235
   Cys Arg  Gly Asn Ser His Ile  Cys Ile Ser Arg Lys  Glu Leu Gln
       2240                 2245                 2250
   Met Ser  Lys Gly Glu Pro Lys  Lys Tyr Ser Leu Asp  Pro Glu Glu
       2255                 2260                 2265
   Ile Glu  Asn Trp Val Thr Glu  Gly Pro Ser Glu Asp  Glu Ala Val
       2270                 2275                 2280
   Cys Val  Asn Cys Gln Asn Asn  Ser Tyr Gly Glu Lys  Cys Glu Ser
       2285                 2290                 2295
   Cys Leu  Gln Gly Tyr Phe Leu  Leu Asp Gly Lys Cys  Thr Lys Cys
       2300                 2305                 2310
   Gln Cys  Asn Gly His Ala Asp  Thr Cys Asn Glu Gln  Asp Gly Thr
       2315                 2320                 2325
   Gly Cys  Pro Cys Gln Asn Asn  Thr Glu Thr Gly Thr  Cys Gln Gly
       2330                 2335                 2340
   Ser Ser  Pro Ser Asp Arg Arg  Asp Cys Tyr Lys Tyr  Gln Cys Ala
       2345                 2350                 2355
   Lys Cys  Arg Glu Ser Phe His  Gly Ser Pro Leu Gly  Gly Gln Gln
       2360                 2365                 2370
   Cys Tyr  Arg Leu Ile Ser Val  Glu Gln Glu Cys Cys  Leu Asp Pro
       2375                 2380                 2385
   Thr Ser  Gln Thr Asn Cys Phe  His Glu Pro Lys Arg  Arg Ala Leu
       2390                 2395                 2400
   Gly Pro  Gly Arg Thr Val Leu  Phe Gly Val Gln Pro  Lys Phe Thr
       2405                 2410                 2415
   Asn Val  Asp Ile Arg Leu Thr  Leu Asp Val Thr Phe  Gly Ala Val
       2420                 2425                 2430
   Asp Leu  Tyr Val Ser Thr Ser  Tyr Asp Thr Phe Val  Val Arg Val
       2435                 2440                 2445
   Ala Pro  Asp Thr Gly Val His  Thr Val His Ile Gln  Pro Pro Pro
       2450                 2455                 2460
   Ala Pro  Pro Pro Pro Pro Pro  Pro Ala Asp Gly Gly  Pro Arg Gly
       2465                 2470                 2475
   Ala Gly  Asp Pro Gly Gly Ala  Gly Ala Ser Ser Gly  Pro Gly Ala
       2480                 2485                 2490
   Pro Ala  Glu Pro Arg Val Arg  Glu Val Trp Pro Arg  Gly Leu Ile
       2495                 2500                 2505
   Thr Tyr  Val Thr Val Thr Glu  Pro Ser Ala Val Leu  Val Val Arg
       2510                 2515                 2520
   Gly Val  Arg Asp Arg Leu Val  Ile Thr Tyr Pro His  Glu His His
       2525                 2530                 2535
   Ala Leu  Lys Ser Ser Arg Phe  Tyr Leu Leu Leu Leu  Gly Val Gly
       2540                 2545                 2550
   Asp Pro  Ser Gly Pro Gly Ala  Asn Gly Ser Ala Asp  Ser Gln Gly
       2555                 2560                 2565
   Leu Leu  Phe Phe Arg Gln Asp  Gln Ala His Ile Asp  Leu Phe Val
       2570                 2575                 2580
   Phe Phe  Ser Val Phe Phe Ser  Cys Phe Phe Leu Phe  Leu Ser Leu
       2585                 2590                 2595
   Cys Val  Leu Leu Trp Lys Ala  Lys Gln Ala Leu Asp  Gln Arg Gln
       2600                 2605                 2610
   Glu Gln  Arg Arg His Leu Gln  Glu Met Thr Lys Met  Ala Ser Arg
       2615                 2620                 2625
   Pro Phe  Ala Lys Val Thr Val  Cys Phe Pro Pro Asp  Pro Thr Ala
       2630                 2635                 2640
   Pro Ala  Ser Ala Trp Lys Pro  Ala Gly Leu Pro Pro  Pro Ala Phe
       2645                 2650                 2655
   Arg Arg  Ser Glu Pro Phe Leu  Ala Pro Leu Leu Leu  Thr Gly Ala
       2660                 2665                 2670
   Gly Gly  Pro Trp Gly Pro Met  Gly Gly Gly Cys Cys  Pro Pro Ala
       2675                 2680                 2685
   Ile Pro  Ala Thr Thr Ala Gly  Leu Arg Ala Gly Pro  Ile Thr Leu
       2690                 2695                 2700
   Glu Pro  Thr Glu Asp Gly Met  Ala Gly Val Ala Thr  Leu Leu Leu
       2705                 2710                 2715
   Gln Leu  Pro Gly Gly Pro His  Ala Pro Asn Gly Ala  Cys Leu Gly
       2720                 2725                 2730
   Ser Ala  Leu Val Thr Leu Arg  His Arg Leu His Glu  Tyr Cys Gly
       2735                 2740                 2745
   Gly Gly  Gly Gly Ala Gly Gly  Ser Gly His Gly Thr  Gly Ala Gly
       2750                 2755                 2760
   Arg Lys  Gly Leu Leu Ser Gln  Asp Asn Leu Thr Ser  Met Ser Leu
       2765                 2770                 2775
<210> 56
   <211> 2319
   <212> PRT
   <213> Homo sapiens
<400> 56
   Met Val Val Tyr Gly Gly Asn Val His Thr His Tyr Gln Glu Glu Lys
   1               5                   10                  15
   Cys Tyr Glu Asp Gly Ile Phe Phe Tyr His Leu Gly Cys His Gln Trp
               20                  25                  30
   Val Ser Gly Ala Glu Leu Ala Pro Pro Gly Thr Pro Glu Gly Arg Ala
           35                  40                  45
   Ala Pro Pro Ser Gly Arg Tyr Ser His Val Ala Ala Val Leu Gly Gly
       50                  55                  60
   Ser Val Leu Leu Val Ala Gly Gly Tyr Ser Gly Arg Pro Arg Gly Asp
   65                  70                  75                  80
   Leu Met Ala Tyr Lys Val Pro Pro Phe Val Phe Gln Ala Pro Ala Pro
                   85                  90                  95
   Asp Tyr His Leu Asp Tyr Cys Ser Met Tyr Thr Asp His Ser Val Cys
               100                 105                 110
   Ser Arg Asp Pro Glu Cys Ser Trp Cys Gln Gly Ala Cys Gln Ala Ala
           115                 120                 125
   Pro Pro Pro Gly Thr Pro Leu Gly Ala Cys Pro Ala Ala Ser Cys Leu
       130                 135                 140
   Gly Leu Gly Arg Leu Leu Gly Asp Cys Gln Ala Cys Leu Ala Phe Ser
   145                 150                 155                 160
   Ser Pro Thr Ala Pro Pro Arg Gly Pro Gly Thr Leu Gly Trp Cys Val
                   165                 170                 175
   His Asn Glu Ser Cys Leu Pro Arg Pro Glu Gln Ala Arg Cys Arg Gly
               180                 185                 190
   Glu Gln Ile Ser Gly Thr Val Gly Trp Trp Gly Pro Ala Pro Val Phe
           195                 200                 205
   Val Thr Ser Leu Glu Ala Cys Val Thr Gln Ser Phe Leu Pro Gly Leu
       210                 215                 220
   His Leu Leu Thr Phe Gln Gln Pro Pro Asn Thr Ser Gln Pro Asp Lys
   225                 230                 235                 240
   Glu Glu Val Gly Arg Trp Val Ala His Gln Glu Lys Glu Thr Arg Arg
                   245                 250                 255
   Leu Gln Arg Pro Gly Ser Ala Arg Leu Phe Pro Leu Pro Gly Arg Asp
               260                 265                 270
   His Lys Tyr Ala Val Glu Ile Gln Gly Gln Leu Asn Gly Ser Ala Gly
           275                 280                 285
   Pro Gly His Ser Glu Leu Thr Leu Leu Trp Asp Arg Thr Gly Val Pro
       290                 295                 300
   Gly Gly Ser Glu Ile Ser Phe Phe Phe Leu Glu Pro Tyr Arg Ser Ser
   305                 310                 315                 320
   Ser Cys Thr Ser Tyr Ser Ser Cys Leu Gly Cys Leu Ala Asp Gln Gly
                   325                 330                 335
   Cys Gly Trp Cys Leu Thr Ser Ala Thr Cys His Leu Arg Gln Gly Gly
               340                 345                 350
   Ala His Cys Gly Asp Asp Gly Ala Gly Gly Ser Leu Leu Val Leu Val
           355                 360                 365
   Pro Thr Leu Cys Pro Leu Cys Glu Glu His Arg Asp Cys His Ala Cys
       370                 375                 380
   Thr Gln Asp Pro Phe Cys Glu Trp His Gln Ser Thr Ser Arg Lys Gly
   385                 390                 395                 400
   Asp Ala Ala Cys Ser Arg Arg Gly Arg Gly Arg Gly Ala Leu Lys Ser
                   405                 410                 415
   Pro Glu Glu Cys Pro Pro Leu Cys Ser Gln Arg Leu Thr Cys Glu Asp
               420                 425                 430
   Cys Leu Ala Asn Ser Ser Gln Cys Ala Trp Cys Gln Ser Thr His Thr
           435                 440                 445
   Cys Phe Leu Phe Ala Ala Tyr Leu Ala Arg Tyr Pro His Gly Gly Cys
       450                 455                 460
   Arg Gly Trp Asp Asp Ser Val His Ser Glu Pro Arg Cys Arg Ser Cys
   465                 470                 475                 480
   Asp Gly Phe Leu Thr Cys His Glu Cys Leu Gln Ser His Glu Cys Gly
                   485                 490                 495
   Trp Cys Gly Asn Glu Asp Asn Pro Thr Leu Gly Arg Cys Leu Gln Gly
               500                 505                 510
   Asp Phe Ser Gly Pro Leu Gly Gly Gly Asn Cys Ser Leu Trp Val Gly
           515                 520                 525
   Glu Gly Leu Gly Leu Pro Val Ala Leu Pro Ala Arg Trp Ala Tyr Ala
       530                 535                 540
   Arg Cys Pro Asp Val Asp Glu Cys Arg Leu Gly Leu Ala Arg Cys His
   545                 550                 555                 560
   Pro Arg Ala Thr Cys Leu Asn Thr Pro Leu Ser Tyr Glu Cys His Cys
                   565                 570                 575
   Gln Arg Gly Tyr Gln Gly Asp Gly Ile Ser His Cys Asn Arg Thr Cys
               580                 585                 590
   Leu Glu Asp Cys Gly His Gly Val Cys Ser Gly Pro Pro Asp Phe Thr
           595                 600                 605
   Cys Val Cys Asp Leu Gly Trp Thr Ser Asp Leu Pro Pro Pro Thr Pro
       610                 615                 620
   Ala Pro Gly Pro Pro Ala Pro Arg Cys Ser Arg Asp Cys Gly Cys Ser
   625                 630                 635                 640
   Phe His Ser His Cys Arg Lys Arg Gly Pro Gly Phe Cys Asp Glu Cys
                   645                 650                 655
   Gln Asp Trp Thr Trp Gly Glu His Cys Glu Arg Cys Arg Pro Gly Ser
               660                 665                 670
   Phe Gly Asn Ala Thr Gly Ser Arg Gly Cys Arg Pro Cys Gln Cys Asn
           675                 680                 685
   Gly His Gly Asp Pro Arg Arg Gly His Cys Asp Asn Leu Ser Gly Leu
       690                 695                 700
   Cys Phe Cys Gln Asp His Thr Glu Gly Ala His Cys Gln Leu Cys Ser
   705                 710                 715                 720
   Pro Gly Tyr Tyr Gly Asp Pro Arg Ala Gly Gly Ser Cys Phe Arg Glu
                   725                 730                 735
   Cys Gly Gly Arg Ala Leu Leu Thr Asn Val Ser Ser Val Ala Leu Gly
               740                 745                 750
   Ser Arg Arg Val Gly Gly Leu Leu Pro Pro Gly Gly Gly Ala Ala Arg
           755                 760                 765
   Ala Gly Pro Gly Leu Ser Tyr Cys Val Trp Val Val Ser Ala Thr Glu
       770                 775                 780
   Glu Leu Gln Pro Cys Ala Pro Gly Thr Leu Cys Pro Pro Leu Thr Leu
   785                 790                 795                 800
   Thr Phe Ser Pro Asp Ser Ser Thr Pro Cys Thr Leu Ser Tyr Val Leu
                   805                 810                 815
   Ala Phe Asp Gly Phe Pro Arg Phe Leu Asp Thr Gly Val Val Gln Ser
               820                 825                 830
   Asp Arg Ser Leu Ile Ala Ala Phe Cys Gly Gln Arg Arg Asp Arg Pro
           835                 840                 845
   Leu Thr Val Gln Ala Leu Ser Gly Leu Leu Val Leu His Trp Glu Ala
       850                 855                 860
   Asn Gly Ser Ser Ser Trp Gly Phe Asn Ala Ser Val Gly Ser Ala Arg
   865                 870                 875                 880
   Cys Gly Ser Gly Gly Pro Gly Ser Cys Pro Val Pro Gln Glu Cys Val
                   885                 890                 895
   Pro Gln Asp Gly Ala Ala Gly Ala Gly Leu Cys Arg Cys Pro Gln Gly
               900                 905                 910
   Trp Ala Gly Pro His Cys Arg Met Ala Leu Cys Pro Glu Asn Cys Asn
           915                 920                 925
   Ala His Thr Gly Ala Gly Thr Cys Asn Gln Ser Leu Gly Val Cys Ile
       930                 935                 940
   Cys Ala Glu Gly Phe Gly Gly Pro Asp Cys Ala Thr Lys Leu Asp Gly
   945                 950                 955                 960
   Gly Gln Leu Val Trp Glu Thr Leu Met Asp Ser Arg Leu Ser Ala Asp
                   965                 970                 975
   Thr Ala Ser Arg Phe Leu His Arg Leu Gly His Thr Met Val Asp Gly
               980                 985                 990
   Pro Asp Ala Thr Leu Trp Met Phe  Gly Gly Leu Gly Leu  Pro Gln Gly
           995                 1000                1005
   Leu Leu  Gly Asn Leu Tyr Arg  Tyr Ser Val Ser Glu  Arg Arg Trp
       1010                 1015                 1020
   Thr Gln  Met Leu Ala Gly Ala  Glu Asp Gly Gly Pro  Gly Pro Ser
       1025                 1030                 1035
   Pro Arg  Ser Phe His Ala Ala  Ala Tyr Val Pro Ala  Gly Arg Gly
       1040                 1045                 1050
   Ala Met  Tyr Leu Leu Gly Gly  Leu Thr Ala Gly Gly  Val Thr Arg
       1055                 1060                 1065
   Asp Phe  Trp Val Leu Asn Leu  Thr Thr Leu Gln Trp  Arg Gln Glu
       1070                 1075                 1080
   Lys Ala  Pro Gln Thr Val Glu  Leu Pro Ala Val Ala  Gly His Thr
       1085                 1090                 1095
   Leu Thr  Ala Arg Arg Gly Leu  Ser Leu Leu Leu Val  Gly Gly Tyr
       1100                 1105                 1110
   Ser Pro  Glu Asn Gly Phe Asn  Gln Gln Leu Leu Glu  Tyr Gln Leu
       1115                 1120                 1125
   Ala Thr  Gly Thr Trp Val Ser  Gly Ala Gln Ser Gly  Thr Pro Pro
       1130                 1135                 1140
   Thr Gly  Leu Tyr Gly His Ser  Ala Val Tyr His Glu  Ala Thr Asp
       1145                 1150                 1155
   Ser Leu Tyr Val Phe Gly Gly Phe Arg Phe His Val Glu Leu Ala
       1160                1165                1170
   Ala Pro Ser Pro Glu Leu Tyr Ser Leu His Cys Pro Asp Arg Thr
       1175                1180                1185
   Trp Ser Leu Leu Ala Pro Ser Gln Gly Ala Lys Arg Asp Arg Met
       1190                1195                1200
   Arg Asn Val Arg Gly Ser Ser Arg Gly Leu Gly Gln Val Pro Gly
       1205                1210                1215
   Glu Gln Pro Gly Ser Trp Gly Phe Arg Glu Val Arg Lys Lys Met
       1220                1225                1230
   Ala Leu Trp Ala Ala Leu Ala Gly Thr Gly Gly Phe Leu Glu Glu
       1235                1240                1245
   Ile Ser Pro His Leu Lys Glu Pro Arg Pro Arg Leu Phe His Ala
       1250                1255                1260
   Ser Ala Leu Leu Gly Asp Thr Met Val Val Leu Gly Gly Arg Ser
       1265                1270                1275
   Asp Pro Asp Glu Phe Ser Ser Asp Val Leu Leu Tyr Gln Val Asn
       1280                1285                1290
   Cys Asn Ala Trp Leu Leu Pro Asp Leu Thr Arg Ser Ala Ser Val
       1295                1300                1305
   Gly Pro Pro Met Glu Glu Ser Val Ala His Ala Val Ala Ala Val
       1310                1315                1320
   Gly Ser Arg Leu Tyr Ile Ser Gly Gly Phe Gly Gly Val Ala Leu
       1325                1330                1335
   Gly Arg Leu Leu Ala Leu Thr Leu Pro Pro Asp Pro Cys Arg Leu
       1340                1345                1350
   Leu Ser Ser Pro Glu Ala Cys Asn Gln Ser Gly Ala Cys Thr Trp
       1355                1360                1365
   Cys His Gly Ala Cys Leu Ser Gly Asp Gln Ala His Arg Leu Gly
       1370                1375                1380
   Cys Gly Gly Ser Pro Cys Ser Pro Met Pro Arg Ser Pro Glu Glu
       1385                1390                1395
   Cys Arg  Arg Leu Arg Thr Cys  Ser Glu Cys Leu Ala  Arg His Pro
       1400                 1405                 1410
   Arg Thr  Leu Gln Pro Gly Asp  Gly Glu Ala Ser Thr  Pro Arg Cys
       1415                 1420                 1425
   Lys Trp  Cys Thr Asn Cys Pro  Glu Gly Ala Cys Ile  Gly Arg Asn
       1430                 1435                 1440
   Gly Ser  Cys Thr Ser Glu Asn  Asp Cys Arg Ile Asn  Gln Arg Glu
       1445                 1450                 1455
   Val Phe  Trp Ala Gly Asn Cys  Ser Glu Ala Ala Cys  Gly Ala Ala
       1460                 1465                 1470
   Asp Cys  Glu Gln Cys Thr Arg  Glu Gly Lys Cys Met  Trp Thr Arg
       1475                 1480                 1485
   Gln Phe  Lys Arg Thr Gly Glu  Thr Arg Arg Ile Leu  Ser Val Gln
       1490                 1495                 1500
   Pro Thr  Tyr Asp Trp Thr Cys  Phe Ser His Ser Leu  Leu Asn Val
       1505                 1510                 1515
   Ser Pro  Met Pro Val Glu Ser  Ser Pro Pro Leu Pro  Cys Pro Thr
       1520                 1525                 1530
   Pro Cys  His Leu Leu Pro Asn  Cys Thr Ser Cys Leu  Asp Ser Lys
       1535                 1540                 1545
   Gly Ala  Asp Gly Gly Trp Gln  His Cys Val Trp Ser  Ser Ser Leu
       1550                 1555                 1560
   Gln Gln  Cys Leu Ser Pro Ser  Tyr Leu Pro Leu Arg  Cys Met Ala
       1565                 1570                 1575
   Gly Gly  Cys Gly Arg Leu Leu  Arg Gly Pro Glu Ser  Cys Ser Leu
       1580                 1585                 1590
   Gly Cys  Ala Gln Ala Thr Gln  Cys Ala Leu Cys Leu  Arg Arg Pro
       1595                 1600                 1605
   His Cys  Gly Trp Cys Ala Trp  Gly Gly Gln Asp Gly  Gly Gly Arg
       1610                 1615                 1620
   Cys Met  Glu Gly Gly Leu Ser  Gly Pro Arg Asp Gly  Leu Thr Cys
       1625                 1630                 1635
   Gly Arg  Pro Gly Ala Ser Trp  Ala Phe Leu Ser Cys  Pro Pro Glu
       1640                 1645                 1650
   Asp Glu  Cys Ala Asn Gly His  His Asp Cys Asn Glu  Thr Gln Asn
       1655                 1660                 1665
   Cys His  Asp Gln Pro His Gly  Tyr Glu Cys Ser Cys  Lys Thr Gly
       1670                 1675                 1680
   Tyr Thr  Met Asp Asn Met Thr  Gly Leu Cys Arg Pro  Val Cys Ala
       1685                 1690                 1695
   Gln Gly  Cys Val Asn Gly Ser  Cys Val Glu Pro Asp  His Cys Arg
       1700                 1705                 1710
   Cys His  Phe Gly Phe Val Gly  Arg Asn Cys Ser Thr  Glu Cys Arg
       1715                 1720                 1725
   Cys Asn  Arg His Ser Glu Cys  Ala Gly Val Gly Ala  Arg Asp His
       1730                 1735                 1740
   Cys Leu  Leu Cys Arg Asn His  Thr Lys Gly Ser His  Cys Glu Gln
       1745                 1750                 1755
   Cys Leu  Pro Leu Phe Val Gly  Ser Ala Val Gly Gly  Gly Thr Cys
       1760                 1765                 1770
   Arg Pro  Cys His Ala Phe Cys  Arg Gly Asn Ser His  Ile Cys Ile
       1775                 1780                 1785
   Ser Arg  Lys Glu Leu Gln Met  Ser Lys Gly Glu Pro  Lys Lys Tyr
       1790                 1795                 1800
   Ser Leu  Asp Pro Glu Glu Ile  Glu Asn Trp Val Thr  Glu Gly Pro
       1805                 1810                 1815
   Ser Glu  Asp Glu Ala Val Cys  Val Asn Cys Gln Asn  Asn Ser Tyr
       1820                 1825                 1830
   Gly Glu  Lys Cys Glu Ser Cys  Leu Gln Gly Tyr Phe  Leu Leu Asp
       1835                 1840                 1845
   Gly Lys  Cys Thr Lys Cys Gln  Cys Asn Gly His Ala  Asp Thr Cys
       1850                 1855                 1860
   Asn Glu  Gln Asp Gly Thr Gly  Cys Pro Cys Gln Asn  Asn Thr Glu
       1865                 1870                 1875
   Thr Gly  Thr Cys Gln Gly Ser  Ser Pro Ser Asp Arg  Arg Asp Cys
       1880                 1885                 1890
   Tyr Lys  Tyr Gln Cys Ala Lys  Cys Arg Glu Ser Phe  His Gly Ser
       1895                 1900                 1905
   Pro Leu  Gly Gly Gln Gln Cys  Tyr Arg Leu Ile Ser  Val Glu Gln
       1910                 1915                 1920
   Glu Cys  Cys Leu Asp Pro Thr  Ser Gln Thr Asn Cys  Phe His Glu
       1925                 1930                 1935
   Pro Lys  Arg Arg Ala Leu Gly  Pro Gly Arg Thr Val  Leu Phe Gly
       1940                 1945                 1950
   Val Gln  Pro Lys Phe Thr Asn  Val Asp Ile Arg Leu  Thr Leu Asp
       1955                 1960                 1965
   Val Thr  Phe Gly Ala Val Asp  Leu Tyr Val Ser Thr  Ser Tyr Asp
       1970                 1975                 1980
   Thr Phe  Val Val Arg Val Ala  Pro Asp Thr Gly Val  His Thr Val
       1985                 1990                 1995
   His Ile  Gln Pro Pro Pro Ala  Pro Pro Pro Pro Pro  Pro Pro Ala
       2000                 2005                 2010
   Asp Gly  Gly Pro Arg Gly Ala  Gly Asp Pro Gly Gly  Ala Gly Ala
       2015                 2020                 2025
   Ser Ser  Gly Pro Gly Ala Pro  Ala Glu Pro Arg Val  Arg Glu Val
       2030                 2035                 2040
   Trp Pro  Arg Gly Leu Ile Thr  Tyr Val Thr Val Thr  Glu Pro Ser
       2045                 2050                 2055
   Ala Val  Leu Val Val Arg Gly  Val Arg Asp Arg Leu  Val Ile Thr
       2060                 2065                 2070
   Tyr Pro  His Glu His His Ala  Leu Lys Ser Ser Arg  Phe Tyr Leu
       2075                 2080                 2085
   Leu Leu  Leu Gly Val Gly Asp  Pro Ser Gly Pro Gly  Ala Asn Gly
       2090                 2095                 2100
   Ser Ala  Asp Ser Gln Gly Leu  Leu Phe Phe Arg Gln  Asp Gln Ala
       2105                 2110                 2115
   His Ile Asp Leu Phe Val Phe Phe Ser Val Phe Phe Ser Cys Phe
   2120 2125 2130
   Phe Leu Phe Leu Ser Leu Cys Val Leu Leu Trp Lys Ala Lys Gln
   2135 2140 2145
   Ala Leu Asp Gln Arg Gln Glu Gln Arg Arg His Leu Gln Glu Met
   2150 2155 2160
   Thr Lys Met Ala Ser Arg Pro Phe Ala Lys Val Thr Val Cys Phe
   2165 2170 2175
   Pro Pro Asp Pro Thr Ala Pro Ala Ser Ala Trp Lys Pro Ala Gly
   2180 2185 2190
   Leu Pro Pro Pro Ala Phe Arg Arg Ser Glu Pro Phe Leu Ala Pro
   2195 2200 2205
   Leu Leu Leu Thr Gly Ala Gly Gly Pro Trp Gly Pro Met Gly Gly
   2210 2215 2220
   Gly Cys Cys Pro Pro Ala Ile Pro Ala Thr Thr Ala Gly Leu Arg
   2225 2230 2235
   Ala Gly Pro Ile Thr Leu Glu Pro Thr Glu Asp Gly Met Ala Gly
   2240 2245 2250
   Val Ala Thr Leu Leu Leu Gln Leu Pro Gly Gly Pro His Ala Pro
   2255 2260 2265
   Asn Gly Ala Cys Leu Gly Ser Ala Leu Val Thr Leu Arg His Arg
   2270 2275 2280
   Leu His Glu Tyr Cys Gly Gly Gly Gly Gly Ala Gly Gly Ser Gly
   2285 2290 2295
   His Gly Thr Gly Ala Gly Arg Lys Gly Leu Leu Ser Gln Asp Asn
   2300 2305 2310
   Leu Thr Ser Met Ser Leu
   2315
<210> 57
   <211> 2386
   <212> PRT
   <213> Homo sapiens
<400> 57
   Met Val Val Tyr Gly Gly Asn Val His Thr His Tyr Gln Glu Glu Lys
   1               5                   10                  15
   Cys Tyr Glu Asp Gly Ile Phe Phe Tyr His Leu Gly Cys His Gln Trp
               20                  25                  30
   Val Ser Gly Ala Glu Leu Ala Pro Pro Gly Thr Pro Glu Gly Arg Ala
           35                  40                  45
   Ala Pro Pro Ser Gly Arg Tyr Ser His Val Ala Ala Val Leu Gly Gly
       50                  55                  60
   Ser Val Leu Leu Val Ala Gly Gly Tyr Ser Gly Arg Pro Arg Gly Asp
   65                  70                  75                  80
   Leu Met Ala Tyr Lys Val Pro Pro Phe Val Phe Gln Ala Pro Ala Pro
                   85                  90                  95
   Asp Tyr His Leu Asp Tyr Cys Ser Met Tyr Thr Asp His Ser Val Cys
               100                 105                 110
   Ser Arg Asp Pro Glu Cys Ser Trp Cys Gln Gly Ala Cys Gln Ala Ala
           115                 120                 125
   Pro Pro Pro Gly Thr Pro Leu Gly Ala Cys Pro Ala Ala Ser Cys Leu
       130                 135                 140
   Gly Leu Gly Arg Leu Leu Gly Asp Cys Gln Ala Cys Leu Ala Phe Ser
   145                 150                 155                 160
   Ser Pro Thr Ala Pro Pro Arg Gly Pro Gly Thr Leu Gly Trp Cys Val
                   165                 170                 175
   His Asn Glu Ser Cys Leu Pro Arg Pro Glu Gln Ala Arg Cys Arg Gly
               180                 185                 190
   Glu Gln Ile Ser Gly Thr Val Gly Trp Trp Gly Pro Ala Pro Val Phe
           195                 200                 205
   Val Thr Ser Leu Glu Ala Cys Val Thr Gln Ser Phe Leu Pro Gly Leu
       210                 215                 220
   His Leu Leu Thr Phe Gln Gln Pro Pro Asn Thr Ser Gln Pro Asp Lys
   225                 230                 235                 240
   Val Ser Ile Val Arg Ser Thr Thr Ile Thr Leu Thr Pro Ser Ala Glu
                   245                 250                 255
   Thr Asp Val Ser Leu Val Tyr Arg Gly Phe Ile Tyr Pro Met Leu Pro
               260                 265                 270
   Gly Gly Pro Gly Gly Pro Gly Ala Glu Asp Val Ala Val Trp Thr Arg
           275                 280                 285
   Ala Gln Arg Leu His Val Leu Ala Arg Met Ala Arg Gly Pro Asp Thr
       290                 295                 300
   Glu Asn Met Glu Glu Val Gly Arg Trp Val Ala His Gln Glu Lys Glu
   305                 310                 315                 320
   Thr Arg Arg Leu Gln Arg Pro Gly Ser Ala Arg Leu Phe Pro Leu Pro
                   325                 330                 335
   Gly Arg Asp His Lys Tyr Ala Val Glu Ile Gln Gly Gln Leu Asn Gly
               340                 345                 350
   Ser Ala Gly Pro Gly His Ser Glu Leu Thr Leu Leu Trp Asp Arg Thr
           355                 360                 365
   Gly Val Pro Gly Gly Ser Glu Ile Ser Phe Phe Phe Leu Glu Pro Tyr
       370                 375                 380
   Arg Ser Ser Ser Cys Thr Ser Tyr Ser Ser Cys Leu Gly Cys Leu Ala
   385                 390                 395                 400
   Asp Gln Gly Cys Gly Trp Cys Leu Thr Ser Ala Thr Cys His Leu Arg
                   405                 410                 415
   Gln Gly Gly Ala His Cys Gly Asp Asp Gly Ala Gly Gly Ser Leu Leu
               420                 425                 430
   Val Leu Val Pro Thr Leu Cys Pro Leu Cys Glu Glu His Arg Asp Cys
           435                 440                 445
   His Ala Cys Thr Gln Asp Pro Phe Cys Glu Trp His Gln Ser Thr Ser
       450                 455                 460
   Arg Lys Gly Asp Ala Ala Cys Ser Arg Arg Gly Arg Gly Arg Gly Ala
   465                 470                 475                 480
   Leu Lys Ser Pro Glu Glu Cys Pro Pro Leu Cys Ser Gln Arg Leu Thr
                   485                 490                 495
   Cys Glu Asp Cys Leu Ala Asn Ser Ser Gln Cys Ala Trp Cys Gln Ser
               500                 505                 510
   Thr His Thr Cys Phe Leu Phe Ala Ala Tyr Leu Ala Arg Tyr Pro His
           515                 520                 525
   Gly Gly Cys Arg Gly Trp Asp Asp Ser Val His Ser Glu Pro Arg Cys
       530                 535                 540
   Arg Ser Cys Asp Gly Phe Leu Thr Cys His Glu Cys Leu Gln Ser His
   545                 550                 555                 560
   Glu Cys Gly Trp Cys Gly Asn Glu Asp Asn Pro Thr Leu Gly Arg Cys
                   565                 570                 575
   Leu Gln Gly Asp Phe Ser Gly Pro Leu Gly Gly Gly Asn Cys Ser Leu
               580                 585                 590
   Trp Val Gly Glu Gly Leu Gly Leu Pro Val Ala Leu Pro Ala Arg Trp
           595                 600                 605
   Ala Tyr Ala Arg Cys Pro Asp Val Asp Glu Cys Arg Leu Gly Leu Ala
       610                 615                 620
   Arg Cys His Pro Arg Ala Thr Cys Leu Asn Thr Pro Leu Ser Tyr Glu
   625                 630                 635                 640
   Cys His Cys Gln Arg Gly Tyr Gln Gly Asp Gly Ile Ser His Cys Asn
                   645                 650                 655
   Arg Thr Cys Leu Glu Asp Cys Gly His Gly Val Cys Ser Gly Pro Pro
               660                 665                 670
   Asp Phe Thr Cys Val Cys Asp Leu Gly Trp Thr Ser Asp Leu Pro Pro
           675                 680                 685
   Pro Thr Pro Ala Pro Gly Pro Pro Ala Pro Arg Cys Ser Arg Asp Cys
       690                 695                 700
   Gly Cys Ser Phe His Ser His Cys Arg Lys Arg Gly Pro Gly Phe Cys
   705                 710                 715                 720
   Asp Glu Cys Gln Asp Trp Thr Trp Gly Glu His Cys Glu Arg Cys Arg
                   725                 730                 735
   Pro Gly Ser Phe Gly Asn Ala Thr Gly Ser Arg Gly Cys Arg Pro Cys
               740                 745                 750
   Gln Cys Asn Gly His Gly Asp Pro Arg Arg Gly His Cys Asp Asn Leu
           755                 760                 765
   Ser Gly Leu Cys Phe Cys Gln Asp His Thr Glu Gly Ala His Cys Gln
       770                 775                 780
   Leu Cys Ser Pro Gly Tyr Tyr Gly Asp Pro Arg Ala Gly Gly Ser Cys
   785                 790                 795                 800
   Phe Arg Glu Cys Gly Gly Arg Ala Leu Leu Thr Asn Val Ser Ser Val
                   805                 810                 815
   Ala Leu Gly Ser Arg Arg Val Gly Gly Leu Leu Pro Pro Gly Gly Gly
               820                 825                 830
   Ala Ala Arg Ala Gly Pro Gly Leu Ser Tyr Cys Val Trp Val Val Ser
           835                 840                 845
   Ala Thr Glu Glu Leu Gln Pro Cys Ala Pro Gly Thr Leu Cys Pro Pro
       850                 855                 860
   Leu Thr Leu Thr Phe Ser Pro Asp Ser Ser Thr Pro Cys Thr Leu Ser
   865                 870                 875                 880
   Tyr Val Leu Ala Phe Asp Gly Phe Pro Arg Phe Leu Asp Thr Gly Val
                   885                 890                 895
   Val Gln Ser Asp Arg Ser Leu Ile Ala Ala Phe Cys Gly Gln Arg Arg
               900                 905                 910
   Asp Arg Pro Leu Thr Val Gln Ala Leu Ser Gly Leu Leu Val Leu His
           915                 920                 925
   Trp Glu Ala Asn Gly Ser Ser Ser Trp Gly Phe Asn Ala Ser Val Gly
       930                 935                 940
   Ser Ala Arg Cys Gly Ser Gly Gly Pro Gly Ser Cys Pro Val Pro Gln
   945                 950                 955                 960
   Glu Cys Val Pro Gln Asp Gly Ala Ala Gly Ala Gly Leu Cys Arg Cys
                   965                 970                 975
   Pro Gln Gly Trp Ala Gly Pro His Cys Arg Met Ala Leu Cys Pro Glu
               980                 985                 990
   Asn Cys Asn Ala His Thr Gly Ala  Gly Thr Cys Asn Gln  Ser Leu Gly
           995                 1000                1005
   Val Cys  Ile Cys Ala Glu Gly  Phe Gly Gly Pro Asp  Cys Ala Thr
       1010                1015                1020
   Lys Leu  Asp Gly Gly Gln Leu  Val Trp Glu Thr Leu  Met Asp Ser
       1025                 1030                 1035
   Arg Leu  Ser Ala Asp Thr Ala  Ser Arg Phe Leu His  Arg Leu Gly
       1040                 1045                 1050
   His Thr  Met Val Asp Gly Pro  Asp Ala Thr Leu Trp  Met Phe Gly
       1055                 1060                 1065
   Gly Leu  Gly Leu Pro Gln Gly  Leu Leu Gly Asn Leu  Tyr Arg Tyr
       1070                 1075                 1080
   Ser Val  Ser Glu Arg Arg Trp  Thr Gln Met Leu Ala  Gly Ala Glu
       1085                 1090                 1095
   Asp Gly  Gly Pro Gly Pro Ser  Pro Arg Ser Phe His  Ala Ala Ala
       1100                 1105                 1110
   Tyr Val  Pro Ala Gly Arg Gly  Ala Met Tyr Leu Leu  Gly Gly Leu
       1115                 1120                 1125
   Thr Ala  Gly Gly Val Thr Arg  Asp Phe Trp Val Leu  Asn Leu Thr
       1130                 1135                 1140
   Thr Leu  Gln Trp Arg Gln Glu  Lys Ala Pro Gln Thr  Val Glu Leu
       1145                 1150                 1155
   Pro Ala  Val Ala Gly His Thr  Leu Thr Ala Arg Arg  Gly Leu Ser
       1160                 1165                 1170
   Leu Leu  Leu Val Gly Gly Tyr  Ser Pro Glu Asn Gly  Phe Asn Gln
       1175                 1180                 1185
   Gln Leu  Leu Glu Tyr Gln Leu  Ala Thr Gly Thr Trp  Val Ser Gly
       1190                 1195                 1200
   Ala Gln  Ser Gly Thr Pro Pro  Thr Gly Leu Tyr Gly  His Ser Ala
       1205                 1210                 1215
   Val Tyr  His Glu Ala Thr Asp  Ser Leu Tyr Val Phe  Gly Gly Phe
       1220                 1225                 1230
   Arg Phe  His Val Glu Leu Ala  Ala Pro Ser Pro Glu  Leu Tyr Ser
       1235                 1240                 1245
   Leu His  Cys Pro Asp Arg Thr  Trp Ser Leu Leu Ala  Pro Ser Gln
       1250                 1255                 1260
   Gly Ala  Lys Arg Asp Arg Met  Arg Asn Val Arg Gly  Ser Ser Arg
       1265                 1270                 1275
   Gly Leu  Gly Gln Val Pro Gly  Glu Gln Pro Gly Ser  Trp Gly Phe
       1280                 1285                 1290
   Arg Glu  Val Arg Lys Lys Met  Ala Leu Trp Ala Ala  Leu Ala Gly
       1295                 1300                 1305
   Thr Gly  Gly Phe Leu Glu Glu  Ile Ser Pro His Leu  Lys Glu Pro
       1310                 1315                 1320
   Arg Pro  Arg Leu Phe His Ala  Ser Ala Leu Leu Gly  Asp Thr Met
       1325                 1330                 1335
   Val Val  Leu Gly Gly Arg Ser  Asp Pro Asp Glu Phe  Ser Ser Asp
       1340                 1345                 1350
   Val Leu  Leu Tyr Gln Val Asn  Cys Asn Ala Trp Leu  Leu Pro Asp
       1355                 1360                 1365
   Leu Thr  Arg Ser Ala Ser Val  Gly Pro Pro Met Glu  Glu Ser Val
       1370                 1375                 1380
   Ala His  Ala Val Ala Ala Val  Gly Ser Arg Leu Tyr  Ile Ser Gly
       1385                 1390                 1395
   Gly Phe  Gly Gly Val Ala Leu  Gly Arg Leu Leu Ala  Leu Thr Leu
       1400                 1405                 1410
   Pro Pro  Asp Pro Cys Arg Leu  Leu Ser Ser Pro Glu  Ala Cys Asn
       1415                 1420                 1425
   Gln Ser  Gly Ala Cys Thr Trp  Cys His Gly Ala Cys  Leu Ser Gly
       1430                 1435                 1440
   Asp Gln  Ala His Arg Leu Gly  Cys Gly Gly Ser Pro  Cys Ser Pro
       1445                 1450                 1455
   Met Pro  Arg Ser Pro Glu Glu  Cys Arg Arg Leu Arg  Thr Cys Ser
       1460                 1465                 1470
   Glu Cys  Leu Ala Arg His Pro  Arg Thr Leu Gln Pro  Gly Asp Gly
       1475                 1480                 1485
   Glu Ala  Ser Thr Pro Arg Cys  Lys Trp Cys Thr Asn  Cys Pro Glu
       1490                 1495                 1500
   Gly Ala Cys Ile Gly Arg Asn Gly Ser Cys Thr Ser Glu Asn Asp
   1505 1510 1515
   Cys Arg Ile Asn Gln Arg Glu Val Phe Trp Ala Gly Asn Cys Ser
   1520 1525 1530
   Glu Ala Ala Cys Gly Ala Ala Asp Cys Glu Gln Cys Thr Arg Glu
   1535 1540 1545
   Gly Lys Cys Met Trp Thr Arg Gln Phe Lys Arg Thr Gly Glu Thr
   1550 1555 1560
   Arg Arg Ile Leu Ser Val Gln Pro Thr Tyr Asp Trp Thr Cys Phe
   1565 1570 1575
   Ser His Ser Leu Leu Asn Val Ser Pro Met Pro Val Glu Ser Ser
   1580 1585 1590
   Pro Pro Leu Pro Cys Pro Thr Pro Cys His Leu Leu Pro Asn Cys
   1595 1600 1605
   Thr Ser Cys Leu Asp Ser Lys Gly Ala Asp Gly Gly Trp Gln His
   1610 1615 1620
   Cys Val Trp Ser Ser Ser Leu Gln Gln Cys Leu Ser Pro Ser Tyr
   1625 1630 1635
   Leu Pro Leu Arg Cys Met Ala Gly Gly Cys Gly Arg Leu Leu Arg
   1640 1645 1650
   Gly Pro Glu Ser Cys Ser Leu Gly Cys Ala Gln Ala Thr Gln Cys
   1655 1660 1665
   Ala Leu Cys Leu Arg Arg Pro His Cys Gly Trp Cys Ala Trp Gly
   1670 1675 1680
   Gly Gln Asp Gly Gly Gly Arg Cys Met Glu Gly Gly Leu Ser Gly
   1685 1690 1695
   Pro Arg Asp Gly Leu Thr Cys Gly Arg Pro Gly Ala Ser Trp Ala
   1700 1705 1710
   Phe Leu Ser Cys Pro Pro Glu Asp Glu Cys Ala Asn Gly His His
   1715 1720 1725
   Asp Cys Asn Glu Thr Gln Asn Cys His Asp Gln Pro His Gly Tyr
   1730 1735 1740
   Glu Cys  Ser Cys Lys Thr Gly  Tyr Thr Met Asp Asn  Met Thr Gly
       1745                 1750                 1755
   Leu Cys  Arg Pro Val Cys Ala  Gln Gly Cys Val Asn  Gly Ser Cys
       1760                 1765                 1770
   Val Glu  Pro Asp His Cys Arg  Cys His Phe Gly Phe  Val Gly Arg
       1775                 1780                 1785
   Asn Cys  Ser Thr Glu Cys Arg  Cys Asn Arg His Ser  Glu Cys Ala
       1790                 1795                 1800
   Gly Val  Gly Ala Arg Asp His  Cys Leu Leu Cys Arg  Asn His Thr
       1805                 1810                 1815
   Lys Gly  Ser His Cys Glu Gln  Cys Leu Pro Leu Phe  Val Gly Ser
       1820                 1825                 1830
   Ala Val  Gly Gly Gly Thr Cys  Arg Pro Cys His Ala  Phe Cys Arg
       1835                 1840                 1845
   Gly Asn  Ser His Ile Cys Ile  Ser Arg Lys Glu Leu  Gln Met Ser
       1850                 1855                 1860
   Lys Gly  Glu Pro Lys Lys Tyr  Ser Leu Asp Pro Glu  Glu Ile Glu
       1865                 1870                 1875
   Asn Trp  Val Thr Glu Gly Pro  Ser Glu Asp Glu Ala  Val Cys Val
       1880                 1885                 1890
   Asn Cys  Gln Asn Asn Ser Tyr  Gly Glu Lys Cys Glu  Ser Cys Leu
       1895                 1900                 1905
   Gln Gly  Tyr Phe Leu Leu Asp  Gly Lys Cys Thr Lys  Cys Gln Cys
       1910                 1915                 1920
   Asn Gly  His Ala Asp Thr Cys  Asn Glu Gln Asp Gly  Thr Gly Cys
       1925                 1930                 1935
   Pro Cys  Gln Asn Asn Thr Glu  Thr Gly Thr Cys Gln  Gly Ser Ser
       1940                 1945                 1950
   Pro Ser  Asp Arg Arg Asp Cys  Tyr Lys Tyr Gln Cys  Ala Lys Cys
       1955                 1960                 1965
   Arg Glu  Ser Phe His Gly Ser  Pro Leu Gly Gly Gln  Gln Cys Tyr
       1970                 1975                 1980
   Arg Leu Ile Ser Val Glu Gln Glu Cys Cys Leu Asp Pro Thr Ser
   1985 1990 1995
   Gln Thr Asn Cys Phe His Glu Pro Lys Arg Arg Ala Leu Gly Pro
   2000 2005 2010
   Gly Arg Thr Val Leu Phe Gly Val Gln Pro Lys Phe Thr Asn Val
   2015 2020 2025
   Asp Ile Arg Leu Thr Leu Asp Val Thr Phe Gly Ala Val Asp Leu
   2030 2035 2040
   Tyr Val Ser Thr Ser Tyr Asp Thr Phe Val Val Arg Val Ala Pro
   2045 2050 2055
   Asp Thr Gly Val His Thr Val His Ile Gln Pro Pro Pro Ala Pro
   2060 2065 2070
   Pro Pro Pro Pro Pro Pro Ala Asp Gly Gly Pro Arg Gly Ala Gly
   2075 2080 2085
   Asp Pro Gly Gly Ala Gly Ala Ser Ser Gly Pro Gly Ala Pro Ala
   2090 2095 2100
   Glu Pro Arg Val Arg Glu Val Trp Pro Arg Gly Leu Ile Thr Tyr
   2105 2110 2115
   Val Thr Val Thr Glu Pro Ser Ala Val Leu Val Val Arg Gly Val
   2120 2125 2130
   Arg Asp Arg Leu Val Ile Thr Tyr Pro His Glu His His Ala Leu
   2135 2140 2145
   Lys Ser Ser Arg Phe Tyr Leu Leu Leu Leu Gly Val Gly Asp Pro
   2150 2155 2160
   Ser Gly Pro Gly Ala Asn Gly Ser Ala Asp Ser Gln Gly Leu Leu
   2165 2170 2175
   Phe Phe Arg Gln Asp Gln Ala His Ile Asp Leu Phe Val Phe Phe
   2180 2185 2190
   Ser Val Phe Phe Ser Cys Phe Phe Leu Phe Leu Ser Leu Cys Val
   2195 2200 2205
   Leu Leu Trp Lys Ala Lys Gln Ala Leu Asp Gln Arg Gln Glu Gln
   2210 2215 2220
   Arg Arg  His Leu Gln Glu Met  Thr Lys Met Ala Ser  Arg Pro Phe
       2225                 2230                 2235
   Ala Lys  Val Thr Val Cys Phe  Pro Pro Asp Pro Thr  Ala Pro Ala
       2240                 2245                 2250
   Ser Ala  Trp Lys Pro Ala Gly  Leu Pro Pro Pro Ala  Phe Arg Arg
       2255                 2260                 2265
   Ser Glu  Pro Phe Leu Ala Pro  Leu Leu Leu Thr Gly  Ala Gly Gly
       2270                 2275                 2280
   Pro Trp  Gly Pro Met Gly Gly  Gly Cys Cys Pro Pro  Ala Ile Pro
       2285                 2290                 2295
   Ala Thr  Thr Ala Gly Leu Arg  Ala Gly Pro Ile Thr  Leu Glu Pro
       2300                 2305                 2310
   Thr Glu  Asp Gly Met Ala Gly  Val Ala Thr Leu Leu  Leu Gln Leu
       2315                 2320                 2325
   Pro Gly  Gly Pro His Ala Pro  Asn Gly Ala Cys Leu  Gly Ser Ala
       2330                 2335                 2340
   Leu Val  Thr Leu Arg His Arg  Leu His Glu Tyr Cys  Gly Gly Gly
       2345                 2350                 2355
   Gly Gly  Ala Gly Gly Ser Gly  His Gly Thr Gly Ala  Gly Arg Lys
       2360                 2365                 2370
   Gly Leu  Leu Ser Gln Asp Asn  Leu Thr Ser Met Ser  Leu
       2375                 2380                 2385
<210> 58
   <211> 10966
   <212> DNA
   <213> Homo sapiens
<400> 58
   ggccgctctg gcccaagcgt cccctcgtct ctctgatctg gcccatccgg cttcggaggg     60
   aggcgagggt gggtgagcaa agggattggg tctgtggggt ccaggcccga acccctgaag    120
   acgggctccg cccccggcac ccgctcgcgc cccgcccccg gctggaggag tctctcctgg    180
   accatccgaa cctagcctgt cccggcccgc agcctctatg gaggctcctg ccgggccgta    240
   gagcccttcg ccccctgggg acccacccgt ctataaggtc cgtttggcct gcagcagcct    300
   gagtccgtaa tgctgggcac tgttcatggg atcggccccc tatggagccc tgtgtctata    360
   ggggactcct acggtcccta gggttcggcc ccgtccataa tgactccata tacagggcct 420
   tccatcgctc tatagggctc agccctcggc ttccagagcc tgtcagcagt ggccgtaccc 480
   ttcgccggga ctgccgggtc tccgggacct cttcgatcta caaggtcatg ttatgcctat 540
   agaggtcgca tttgcagggc ctcaccccgg gtagagggtc ctctccaggt ttttacggcc 600
   tgtccccgct ctaagggtca gtgcaggagg cggcgatggc cctgggcaag gttctggcca 660
   tggcactggt tttggccttg gccgtgctgg ggtcgctgtc ccctggggcc cgggcggggg 720
   actgcaaggg gcagcggcag gtgctgcggg aggcgccagg cttcgtgacg gatggtgcgg 780
   gcaactacag cgtcaatggc aactgcgagt ggctcatcga ggccccaagc ccccagcacc 840
   ggatcctgct ggacttcctt ttcctggaca cagagtgcac gtatgactac ctgttcgtgt 900
   atgacggtga ctccccgcga gggccgctgc ttgccagtct aagtgggagc acccgacctc 960
   cgcccatcga agcttcctca ggcaagatgc tgctgcacct cttcagtgat gccaactaca 1020
   acctgctggg ctttaacgcc tcattccgct tctccctgtg cccgggtggc tgccagagcc 1080
   acgggcagtg ccagccaccg ggtgtgtgtg cctgcgagcc gggctggggg ggtcctgact 1140
   gtggcctgca ggagtgctca gcctactgtg gcagccacgg cacctgcgcc tcgcccctgg 1200
   gaccatgccg ctgtgagcct ggcttcttgg gacgtgcctg tgacctgcac ctgtgggaga 1260
   accagggggc tgggtggtgg cacaacgtga gtgccaggga ccctgccttc tctgcccgta 1320
   ttggggcagc tggcgccttc ctgtccccac cagggctgct ggcagttttc ggaggccagg 1380
   acctcaacaa tgccctgggt gacctcgtcc tatacaactt ctccgccaac acctgggagt 1440
   cttgggacct gagtcctgcc ccggctgccc gtcactccca tgtggccgtg gcctgggccg 1500
   gctccctggt actgatgggt ggtgagctgg ctgacggctc gctcaccaac gacgtgtggg 1560
   ccttcagtcc actgggcagg ggccactggg agctcctggc accacctgcc tccagctcct 1620
   cggggccccc aggcctggca ggtcacgcgg ctgccctggt ggatgatgtc tggctatatg 1680
   tgtctggagg ccgcaccccg cacgacctct tctcctctgg cctcttccgt ttccgccttg 1740
   acagcaccag cgggggctat tgggagcagg tgattccggc aggcggacgg ccccctgctg 1800
   ccactggcca ctccatggtg ttccatgccc cctcccgtgc cctgctggtc catggtggac 1860
   accggccctc cactgcccgg ttctctgtgc gagtgaactc cactgagctt ttccacgtgg 1920
   atcggcatgt gtggacgacg ctgaaggggc gggatgggct tcagggccca agggagcgag 1980
   ccttccacac agccagtgtt ctgggcaatt acatggtggt ctatgggggc aatgtgcaca 2040
   cccattacca ggaggaaaag tgctacgaag atggcatctt cttctaccac cttggctgcc 2100
   atcaatgggt gtcaggagct gagcttgccc cgccaggaac ccctgagggc cgagcagcgc 2160
   ctcccagtgg tcggtactca catgtagctg cggtgcttgg tggcagcgtc ctgttggtgg 2220
   ctggggggta cagcggccgg ccccgtgggg acttgatggc gtacaaggtg cccccctttg 2280
   tgttccaggc acctgcccct gactaccact tggactactg ctccatgtac acagaccaca 2340
   gcgtctgctc ccgggacccg gaatgcagtt ggtgccaagg agcctgccaa gctgcacccc 2400
   ctcctgggac ccccttgggg gcttgtccag ccgccagctg cctgggcctg ggccgcctcc 2460
   tgggtgactg ccaggcctgc ctggccttca gcagccccac agcccctcca cggggacctg 2520
   gcaccctggg ctggtgcgtg cacaatgaga gctgcctccc taggcctgag caggcccgct 2580
   gccgagggga gcagatctca ggcactgtgg gctggtgggg gcctgcgcct gtcttcgtca 2640
   cgtccctgga ggcctgcgtc acccagagct tcctgcctgg cctgcacttg ctcacctttc 2700
   agcagccgcc caatacctcc cagcctgaca aggaggaggt ggggcgctgg gtggctcatc 2760
   aggagaagga gacgcggcgg ctgcagcgcc ctgggtctgc tcgcctcttc cctctgcctg 2820
   ggcgggacca caagtatgca gtagagatcc agggccagct caatggctcg gcaggccctg 2880
   ggcacagcga gctaactctg ctgtgggatc ggactggtgt gccaggaggc agcgagatct 2940
   ccttcttctt cctggagccc taccgctcgt cgtcctgcac ctcctattct tcctgcctgg 3000
   gctgcttggc agaccagggc tgtggctggt gcctgaccag tgccacctgc cacctgcgcc 3060
   agggcggagc ccattgcggg gatgacgggg ctggtgggtc cctgctggtg ctggtgccta 3120
   ccctctgccc actctgcgag gagcatcggg actgccacgc ctgcacccag gaccccttct 3180
   gtgagtggca tcagagcacc agccgcaaag gggacgcggc atgcagccgg cggggccggg 3240
   gtcggggtgc cctgaagagt ccagaggagt gtcccccgct ctgcagccag cgactgacct 3300
   gtgaggactg cctggccaac tctagccagt gcgcctggtg ccagtccacc cacacctgct 3360
   tcctgtttgc tgcctacttg gcccggtacc cacacggggg ctgtcgaggc tgggacgaca 3420
   gtgtacactc ggagccacgg tgccggagct gcgatggctt cctgacctgc catgagtgtc 3480
   tgcagagcca cgagtgtggc tggtgtggca atgaggacaa ccccacactg ggacggtgcc 3540
   tacaggggga cttctcaggg cccctcggtg ggggtaactg ctccctgtgg gtgggggagg 3600
   gcctggggct tcccgtggcc ctccctgccc gctgggcata cgcccgctgt cctgacgtgg 3660
   atgagtgtcg cctgggcctg gcccggtgcc acccgcgggc gacctgcctg aacacgcccc 3720
   tcagctacga gtgtcactgc cagcggggct accagggtga tggcatctca cactgcaacc 3780
   gcacgtgctt ggaggactgt ggccatggtg tgtgcagtgg ccccccggac tttacctgcg 3840
   tgtgtgacct aggctggaca tcagacctgc cccctcccac acccgccccg ggtccgccag 3900
   ccccccgctg ctcccgggac tgtggctgca gcttccacag ccactgccgc aagcggggcc 3960
   ctggcttctg cgacgagtgc caggactgga catgggggga gcactgcgaa cgatgccggc 4020
   ccggcagctt cggcaacgcc acaggctcta ggggctgccg gccctgccag tgcaacgggc 4080
   acggggaccc acgccgtggc cactgcgaca acctcagtgg gctctgcttc tgccaggacc 4140
   acaccgaggg tgcccactgc cagctctgct ccccaggcta ttatggggat ccccgggccg 4200
   gtggttcctg ctttcgggag tgtgggggtc gcgccctcct caccaacgtg tcctcagtgg 4260
   cactgggctc acgccgggtc ggggggctgc tgcctccagg tggcggggct gcaagagccg 4320
   ggcctggcct gtcctactgt gtgtgggttg tctcggccac tgaggagcta cagccctgtg 4380
   ctcccgggac cctctgtccc ccactcaccc tcaccttctc ccccgacagc agcaccccct 4440
   gcacgctgag ctacgtcctg gcgtttgatg gattcccacg cttcctggac actggtgttg 4500
   tccagtcgga ccgcagcctc atagctgcct tctgcggcca gcgacgggac aggcccctca 4560
   ctgttcaggc cctgtctggg ctgctcgtgc tgcactggga ggccaatggc tcctcatcct 4620
   ggggcttcaa tgcttcggtg ggctctgccc gctgtgggtc agggggcccc gggagctgtc 4680
   ccgtccccca ggaatgcgtg ccccaggacg gtgctgcagg tgcggggctc tgccgatgtc 4740
   ctcagggctg ggctggccca cactgccgca tggctctgtg tcctgagaac tgcaatgccc 4800
   acactggggc aggaacttgt aaccagagcc tgggtgtgtg catctgtgcc gagggcttcg 4860
   ggggccccga ctgcgccacc aagctggatg gcgggcagct ggtctgggag accctcatgg 4920
   acagccgcct ctcagccgac actgccagcc gcttcctgca ccgcctgggc cacaccatgg 4980
   tggatggacc cgatgccacc ttgtggatgt ttgggggcct gggcctgccc caggggctgc 5040
   tgggaaacct gtacaggtac tcagtgagtg agcggcggtg gacacagatg ctggcgggag 5100
   ccgaggacgg gggcccaggc ccatcgcccc gctccttcca tgcagccgca tatgtgcccg 5160
   ctggccgtgg tgccatgtat ctgctggggg gacttaccgc tggaggcgtc acccgtgatt 5220
   tctgggtcct caacctcacc accctgcaat ggcggcagga gaaggccccc cagaccgtgg 5280
   agctgccagc cgttgctggt cacaccctta ctgcccgccg aggcctgtct ctgctcctgg 5340
   tgggcggtta ctccccggaa aatggcttca accagcagct gctggagtac cagctggcaa 5400
   ccggcacctg ggtgtcagga gcccagagtg ggacaccccc cacaggtctc tatggtcact 5460
   ctgctgtcta ccacgaggcc accgactccc tctacgtgtt tggggggttc cgattccatg 5520
   tggagctggc ggccccatcc cccgagctct actccctgca ctgtcctgac cgcacctgga 5580
   gtctgctggc cccttctcag ggggcaaagc gagatcgtat gaggaatgtg cgtggctcat 5640
   ctcggggtct gggccaagtt cctggggagc agcctgggtc atgggggttc cgggaagtca 5700
   ggaagaagat ggctctgtgg gctgctcttg ctggtacagg aggtttcctg gaggaaatct 5760
   cacctcacct gaaggagccc cgcccccggc ttttccacgc ctcagccctg ttaggggaca 5820
   ccatggtggt tcttgggggg cgctcggacc ctgacgagtt cagcagcgac gttctgctct 5880
   accaggtcaa ctgcaatgcc tggcttctgc ccgacctcac ccgctcggcc tctgtggggc 5940
   ccccaatgga ggagtctgtg gcccatgctg tggcagcagt cgggagccgc ctgtatatct 6000
   ctgggggttt cgggggagtg gccctgggcc gcctgctggc actgaccctg ccccctgacc 6060
   cctgccgcct gctgtcctca cctgaagctt gtaaccagtc tggggcctgc acctggtgcc 6120
   atggggcctg cttgtccggg gatcaggccc acaggctggg ctgcgggggc tccccctgct 6180
   ccccaatgcc tcgctccccg gaggaatgtc gacgtctccg gacctgcagt gagtgcctgg 6240
   cccgccatcc tcggaccctg caacctggag atggagaggc gtccaccccc cgctgtaagt 6300
   ggtgtaccaa ctgccccgaa ggtgcttgca ttggacgcaa tgggtcctgc acctctgaga 6360
   atgactgtcg gatcaaccag cgagaggtct tctgggcagg gaactgctcc gaggctgcgt 6420
   gcggggctgc tgactgcgag cagtgcacgc gggagggcaa gtgcatgtgg acgcggcagt 6480
   tcaagaggac aggggagacc cgccgcatcc tctccgtgca gcccacctat gactggacgt 6540
   gcttcagcca ctctctgctg aatgtgtccc ccatgccggt ggaatcatca cccccactgc 6600
   cctgccccac cccttgtcac ctcctaccca actgtacctc ctgcctggac tctaagggag 6660
   cagatggggg ctggcagcac tgtgtttgga gcagcagcct gcagcagtgt ctgagccctt 6720
   cctacctgcc cctgcgatgt atggccggag gctgtgggcg gctgctccgg ggacctgaga 6780
   gctgctccct gggctgtgct caggcaactc agtgcgcctt gtgcctgcgg cgcccccatt 6840
   gcggctggtg tgcctggggg ggccaggatg ggggtggccg ctgcatggag ggtggactca 6900
   gcggcccccg tgatgggctg acatgtgggc gtccgggggc ctcctgggcc ttcctgtcct 6960
   gcccccctga ggacgagtgt gcaaacgggc accacgactg caacgagacg cagaattgcc 7020
   acgaccagcc ccacggctat gagtgcagct gcaagaccgg ctataccatg gacaacatga 7080
   cagggctgtg ccgccctgtg tgcgcccagg gctgcgtgaa cggctcatgt gtggagcccg 7140
   accactgccg ctgccacttt ggctttgtgg gccgcaactg ctccacggaa tgccgctgca 7200
   accgccacag tgaatgcgct ggtgttgggg cgcgtgacca ctgcttgctc tgccgcaacc 7260
   acaccaaggg cagccactgt gagcagtgcc tcccgctgtt tgtgggttca gctgtcggag 7320
   gcgggacctg ccggccctgc cacgcctttt gtcgtggaaa tagccacatc tgcatctcca 7380
   ggaaggagtt acaaatgtcc aagggagagc caaagaagta ctcactggac ccagaggaga 7440
   ttgaaaactg ggtgacagag ggtcctagtg aagacgaggc cgtgtgcgtg aactgccaga 7500
   ataacagcta tggggagaaa tgcgagagct gcctgcaggg ctacttcctc ctggacggga 7560
   agtgcaccaa atgccagtgt aatggccacg cggacacatg taacgagcag gatgggacgg 7620
   gctgtccatg tcagaataac acagagacgg gcacatgcca gggcagctcc cccagtgacc 7680
   gtcgagactg ctacaagtac cagtgcgcca agtgccggga atcatttcac gggagtccgc 7740
   tgggcggcca gcagtgctac cgcctcatct cggtggagca ggagtgctgc ctggacccca 7800
   cgtcccagac caactgcttc catgagccca aacgccgggc gctaggcccc ggccgcactg 7860
   tcctctttgg cgtgcagccc aaattcacca acgtggacat ccgcctgacg ctggacgtga 7920
   ccttcggggc cgtggacctc tatgtctcca cctcctatga caccttcgtg gtccgtgtgg 7980
   cccctgacac tggcgtccat actgtacaca tccagccacc cccagcccca ccacctccac 8040
   caccccctgc agatggtggg ccccgggggg ctggggatcc aggaggagca ggggccagca 8100
   gtgggccggg cgccccagca gagccacggg tacgggaggt atggccgcgg ggcctgatta 8160
   cctacgtgac ggtgacggag ccgtcggcag tgctggtggt ccgcggcgtg cgggaccggc 8220
   tggtcatcac ctacccacac gagcaccatg ccctcaagtc gagccgcttc tacctgctgc 8280
   tgctgggcgt gggagaccca agtgggcccg gcgccaacgg ctcagccgac tcgcagggcc 8340
   tgctcttctt ccggcaggac caggcccaca ttgacctgtt tgtcttcttc tccgtcttct 8400
   tctcctgctt cttcctcttc ctctcactct gtgtgctcct ctggaaggcc aagcaggctc 8460
   tggaccagcg gcaggagcag cgccggcact tgcaggagat gaccaagatg gccagccgcc 8520
   ccttcgccaa ggtcaccgtc tgcttcccac ctgaccctac tgccccggcc tccgcctgga 8580
   agccggctgg gctcccacct cccgccttcc gccgctctga gcccttcctg gcacccctgc 8640
   tgctgacagg ggccggtggg ccctggggac ccatgggagg gggctgctgc ccaccagcca 8700
   tccccgccac cactgctggg ctgcgagctg ggcccatcac tctcgagccc acagaagatg 8760
   gcatggctgg cgtggccaca ctgctgctcc agctgcctgg cgggccccat gcacccaacg 8820
   gcgcctgcct ggggtcagcc ctcgtcacac tgcggcacag gctgcacgag tactgtgggg 8880
   gtggtggggg tgctgggggc agtgggcatg ggactggtgc gggccggaag ggactgttga 8940
   gccaggacaa cctcaccagc atgtccctct gacatgccca gggttctcat ccacagcagc 9000
   tgggtcacct gatagggccg ccctggactt ggggtccctc cacctggggg cccctggaca 9060
   ctgtctactt ggagaccact ggcccccttc ccccagggtt gcccagatgg ggcctccttt 9120
   gttctgcatt cagcagctat ttatcgagta cctactctgt caggcactgt cataggcgtg 9180
   gggcaaagca ggaaccaaga gacgaggttc cctgatctca tgggacttag gttctggtga 9240
   agggagacaa tcagtgcaca tgcacacacc ccacacgcat acacacatga acacatgcac 9300
   atgtgcacac acaagtaaga tggcttcaga gagggagaag cactgtgagg cctccagagg 9360
   atgtggcagt gagggacgat ggggtgaagt cagctgggca ttcaaagaag ctagactgag 9420
   aacgcctgag aagaaccagc tacgggaaga gctttgggaa gcaaaggcag aggccctggg 9480
   gtgggagcag gcttgtttta ttggaaggac cagaaaactg gtaagtgtga cccagatcaa 9540
   gtgtgaggag atgaggctgg ggatagtcag gggctggatc acccagggcc ttgtgggccc 9600
   cacatagggt tttgggtttt attctcaggg caatgggaag ctgttggatg gtttgatgaa 9660
   ggggagtgac aggatccgat gtacctattt aagaatttaa gagggtcggg tgcggtggct    9720
   catgcctata atcctagcac tttgagaggc caaggtgggc tgatcacaag gtcaagagtt    9780
   cgtgaccagc ctggccaata tggtgaaacc ccatgtctac taaaaataca aaaattagct    9840
   gggcatggtg gcacacactt gtagtccgag ctactcggga ggctgaggca gaagaatcgc    9900
   ttgaacccag gaggcggagg ttgcagtgag ctcagattgc gtcactgcac tccaacctgg    9960
   gtgacagagc gagactccac ctcaaaaaaa aaaaaaaaat ttaagaggtc actcagttgt   10020
   gctgtggaga atggaccgga gggacaagag gggcagcagg gatggtgggc tggagtaggg   10080
   tgctggcaat gagggagtct ggctcagatg tgggatgtgt atggaagaat ataaatgatg   10140
   gtgtggatgt cagggtgagg gaggagacaa aaccacgatg acccctagct ttgtggcctg   10200
   aactgtgggt ggctgagggg atcgttaatt gaatggggca gactgaggct tgtgaggaag   10260
   atcagagtct ggttcttgac atgagatgcc cttcagacat ctcttcactc aggtccaact   10320
   agggatacag aaacactgaa tatttcaaca gcagaaattg aatgggggga ttgatagcgc   10380
   tggcgaggga agcagctgga aagagacaga tggcaccctg agacagccca gaggtgaata   10440
   ggacccccag gctgcaggga taaagctcag tggtggtgtt acctcaccgg ggaccagggt   10500
   cacacagcaa agctggaaca acagaggcgt gttgtggggg agcctcagag gggacaaaac   10560
   ctctgcctga gatcccaccc caggtgggca tgggggccac tgaggttggg gatgaaaatg   10620
   ccggtaccgt cagtgcacag ccctgttcca gacagtgctg cctggaagat ttctgggctc   10680
   tcctgaggcg ccaccccgca cctgagccac ctccttggac tcctgtcctc taccccttga   10740
   ggacctccct cccttctacc ctagctgtct tcttgaactt gggactctcc tttcccaaga   10800
   cttccatcac tagctcctgg agggactgga ctttgcatct tcccttcgcg tggagcctca   10860
   gtgtgagagg ccctagccaa tgcgtgcatg tcagaggtgg tggggaccac atcagaagaa   10920
   gaggggggtg atgaaattaa caaataaaaa gtatggggaa aaccca                 10966
<210> 59
   <211> 10966
   <212> DNA
   <213> Homo sapiens
<400> 59
<210> 60
   <211> 11167
   <212> DNA
   <213> Homo sapiens
<400> 60
<210> 61
   <211> 409
   <212> PRT
   <213> Homo sapiens
<400> 61
   Met Glu Glu Ser Trp Glu Ala Ala Pro Gly Gly Gln Ala Gly Ala Glu
   1               5                   10                  15
   Leu Pro Met Glu Pro Val Gly Ser Leu Val Pro Thr Leu Glu Gln Pro
               20                  25                  30
   Gln Val Pro Ala Lys Val Arg Gln Pro Glu Gly Pro Glu Ser Ser Pro
           35                  40                  45
   Ser Pro Ala Gly Ala Val Glu Lys Ala Ala Gly Ala Gly Leu Glu Pro
       50                  55                  60
   Ser Ser Lys Lys Lys Pro Pro Ser Pro Arg Pro Gly Ser Pro Arg Val
   65                  70                  75                  80
   Pro Pro Leu Ser Leu Gly Tyr Gly Val Cys Pro Glu Pro Pro Ser Pro
                   85                  90                  95
   Gly Pro Ala Leu Val Lys Leu Pro Arg Asn Gly Glu Ala Pro Gly Ala
               100                 105                 110
   Glu Pro Ala Pro Ser Ala Trp Ala Pro Met Glu Leu Gln Val Asp Val
           115                 120                 125
   Arg Val Lys Pro Val Gly Ala Ala Gly Gly Ser Ser Thr Pro Ser Pro
       130                 135                 140
   Arg Pro Ser Thr Arg Phe Leu Lys Val Pro Val Pro Glu Ser Pro Ala
   145                 150                 155                 160
   Phe Ser Arg His Ala Asp Pro Ala His Gln Leu Leu Leu Arg Ala Pro
                   165                 170                 175
   Ser Gln Gly Gly Thr Trp Gly Arg Arg Ser Pro Leu Ala Ala Ala Arg
               180                 185                 190
   Thr Glu Ser Gly Cys Asp Ala Glu Gly Arg Ala Ser Pro Ala Glu Gly
           195                 200                 205
   Ser Ala Gly Ser Pro Gly Ser Pro Thr Cys Cys Arg Cys Lys Glu Leu
       210                 215                 220
   Gly Leu Glu Lys Glu Asp Ala Ala Leu Leu Pro Arg Ala Gly Leu Asp
   225                 230                 235                 240
   Gly Asp Glu Lys Leu Pro Arg Ala Val Thr Leu Thr Gly Leu Pro Met
                   245                 250                 255
   Tyr Val Lys Ser Leu Tyr Trp Ala Leu Ala Phe Met Ala Val Leu Leu
               260                 265                 270
   Ala Val Ser Gly Val Val Ile Val Val Leu Ala Ser Arg Ala Gly Ala
           275                 280                 285
   Arg Cys Gln Gln Cys Pro Pro Gly Trp Val Leu Ser Glu Glu His Cys
       290                 295                 300
   Tyr Tyr Phe Ser Ala Glu Ala Gln Ala Trp Glu Ala Ser Gln Ala Phe
   305                 310                 315                 320
   Cys Ser Ala Tyr His Ala Thr Leu Pro Leu Leu Ser His Thr Gln Asp
                   325                 330                 335
   Phe Leu Gly Arg Tyr Pro Val Ser Arg His Ser Trp Val Gly Ala Trp
               340                 345                 350
   Arg Gly Pro Gln Gly Trp His Trp Ile Asp Glu Ala Pro Leu Pro Pro
           355                 360                 365
   Gln Leu Leu Pro Glu Asp Gly Glu Asp Asn Leu Asp Ile Asn Cys Gly
       370                 375                 380
   Ala Leu Glu Glu Gly Thr Leu Val Ala Ala Asn Cys Ser Thr Pro Arg
   385                 390                 395                 400
   Pro Trp Val Cys Ala Lys Gly Thr Gln
                   405
<210> 62
   <211> 314
   <212> PRT
   <213> Homo sapiens
<400> 62
   Met Glu Glu Ser Trp Glu Ala Ala Pro Gly Gly Gln Ala Gly Ala Glu
   1               5                   10                  15
   Leu Pro Met Glu Pro Val Gly Ser Leu Val Pro Thr Leu Glu Gln Pro
               20                  25                  30
   Gln Val Pro Ala Lys Val Arg Gln Pro Glu Gly Pro Glu Ser Ser Pro
           35                  40                  45
   Ser Pro Ala Gly Ala Val Glu Lys Ala Ala Gly Ala Gly Leu Glu Pro
       50                  55                  60
   Ser Ser Lys Lys Lys Pro Pro Ser Pro Arg Pro Gly Ser Pro Arg Val
   65                  70                  75                  80
   Pro Pro Leu Ser Leu Gly Tyr Gly Val Cys Pro Glu Pro Pro Ser Pro
                   85                  90                  95
   Gly Pro Ala Leu Val Lys Leu Pro Arg Asn Gly Glu Ala Pro Gly Ala
               100                 105                 110
   Glu Pro Ala Pro Ser Ala Trp Ala Pro Met Glu Leu Gln Val Asp Val
           115                 120                 125
   Arg Val Lys Pro Val Gly Ala Ala Gly Gly Ser Ser Thr Pro Ser Pro
       130                 135                 140
   Arg Pro Ser Thr Arg Phe Leu Lys Val Pro Val Pro Glu Ser Pro Ala
   145                 150                 155                 160
   Phe Ser Arg His Ala Asp Pro Ala His Gln Leu Leu Leu Arg Ala Pro
                   165                 170                 175
   Ser Gln Gly Gly Thr Trp Gly Arg Arg Ser Pro Leu Ala Ala Ala Arg
               180                 185                 190
   Thr Glu Ser Gly Cys Asp Ala Glu Gly Arg Ala Ser Pro Ala Glu Gly
           195                 200                 205
   Ser Ala Gly Ser Pro Gly Ser Pro Thr Cys Cys Arg Cys Lys Glu Leu
       210                 215                 220
   Gly Leu Glu Lys Glu Asp Ala Ala Leu Leu Pro Arg Ala Gly Leu Asp
   225                 230                 235                 240
   Gly Asp Glu Lys Leu Pro Arg Ala Val Thr Leu Thr Asp Ser Leu Arg
                   245                 250                 255
   Thr Ala Arg Thr Ile Trp Ile Ser Thr Val Gly Pro Trp Arg Lys Ala
               260                 265                 270
   Arg Trp Trp Leu Gln Thr Ala Ala Leu Gln Asp Pro Gly Ser Val Pro
           275                 280                 285
   Arg Gly Pro Ser Asp Leu Gly Ser Ala Trp Ser Ser Ala Cys Gln Ala
       290                 295                 300
   Asp Ala Ala Pro Pro Thr Gly Glu Ala Ser
   305                 310
<210> 63
   <211> 1544
   <212> DNA
   <213> Homo sapiens
<400> 63
   gagagcgaag ctcctctgca ctgggcccag gtgcgctcct cagcgtctcc gggtggcggg     60
   gcgcgcggga tggaggagtc ttgggaggct gcgcccggag gccaagccgg ggcagagctc    120
   ccaatggagc ccgtgggaag cctggtcccc acgctggagc agccgcaggt gcccgcgaag    180
   gtgcgacaac ctgaaggtcc cgaaagcagc ccaagtccgg ccggggccgt ggagaaggcg    240
   gcgggcgcag gcctggagcc ctcgagcaag aaaaagccgc cttcgcctcg ccccgggtcc    300
   ccgcgcgtgc cgccgctcag cctgggctac ggggtctgcc ccgagccgcc gtcaccgggc    360
   cctgccttgg tcaagctgcc ccggaatggc gaggcgcccg gggctgagcc tgcgcccagc    420
   gcctgggcgc ccatggagct gcaggtagat gtgcgcgtga agcccgtggg cgcggccggt    480
   ggcagcagca cgccatcgcc caggccctcc acgcgcttcc tcaaggtgcc ggtgcccgag    540
   tcccctgcct tctcccgcca cgcggacccg gcgcaccagc tcctgctgcg cgcaccatcc    600
   cagggcggca cgtggggccg ccgctcgccg ctggctgcag cccggacgga gagcggctgc    660
   gacgcagagg gccgggccag ccccgcggaa ggaagcgccg gctccccggg ctcccccacg    720
   tgctgccgct gcaaggagct ggggctggag aaggaggatg cggcgctgtt gccccgcgcg    780
   gggttggacg gcgacgagaa gctgccccgg gccgtaacgc ttacggggct acccatgtac    840
   gtgaagtccc tgtactgggc cctggcgttc atggctgtgc tcctggcagt ctctggggtt    900
   gtcattgtgg tcctggcctc aagagcagga gccagatgcc agcagtgccc cccaggctgg    960
   gtgttgtccg aggagcactg ttactacttc tctgcagaag cgcaggcctg ggaagccagc   1020
   caggctttct gctcagccta ccacgctacc ctccccctgc taagccacac ccaggacttc   1080
   ctgggcagat acccagtctc caggcactcc tgggtggggg cctggcgagg cccccagggc   1140
   tggcactgga tcgacgaggc cccactcccg ccccagctac tccctgagga cggcgaggac   1200
   aatctggata tcaactgtgg ggccctggag gaaggcacgc tggtggctgc aaactgcagc   1260
   actccaagac cctgggtctg tgccaagggg acccagtgat ctgggctctg cctggtcctc   1320
   agcctgccag gcagatgcag caccccctac aggggaggcc agttgagagc ttgggcagcc   1380
   tcttcctgga cccagttatc caggtcttca tgctctgctc aagggggcca catgagcgag   1440
   cctaggagct ggacttcaac ccaggaagat gcatccgagg gaaaggagat tttctatggc   1500
   ctcaggcctg agtgccaata ttagtctcca gcttctgtgg atga                     1544
<210> 64
   <211> 1192
   <212> DNA
   <213> Homo sapiens
<400> 64
   gagagcgaag ctcctctgca ctgggcccag gtgcgctcct cagcgtctcc gggtggcggg      60
   gcgcgcggga tggaggagtc ttgggaggct gcgcccggag gccaagccgg ggcagagctc     120
   ccaatggagc ccgtgggaag cctggtcccc acgctggagc agccgcaggt gcccgcgaag     180
   gtgcgacaac ctgaaggtcc cgaaagcagc ccaagtccgg ccggggccgt ggagaaggcg     240
   gcgggcgcag gcctggagcc ctcgagcaag aaaaagccgc cttcgcctcg ccccgggtcc     300
   ccgcgcgtgc cgccgctcag cctgggctac ggggtctgcc ccgagccgcc gtcaccgggc     360
   cctgccttgg tcaagctgcc ccggaatggc gaggcgcccg gggctgagcc tgcgcccagc     420
   gcctgggcgc ccatggagct gcaggtagat gtgcgcgtga agcccgtggg cgcggccggt     480
   ggcagcagca cgccatcgcc caggccctcc acgcgcttcc tcaaggtgcc ggtgcccgag     540
   tcccctgcct tctcccgcca cgcggacccg gcgcaccagc tcctgctgcg cgcaccatcc     600
   cagggcggca cgtggggccg ccgctcgccg ctggctgcag cccggacgga gagcggctgc     660
   gacgcagagg gccgggccag ccccgcggaa ggaagcgccg gctccccggg ctcccccacg     720
   tgctgccgct gcaaggagct ggggctggag aaggaggatg cggcgctgtt gccccgcgcg     780
   gggttggacg gcgacgagaa gctgccccgg gccgtaacgc ttacggactc cctgaggacg     840
   gcgaggacaa tctggatatc aactgtgggg ccctggagga aggcacgctg gtggctgcaa     900
   actgcagcac tccaagaccc tgggtctgtg ccaaggggac ccagtgatct gggctctgcc     960
   tggtcctcag cctgccaggc agatgcagca ccccctacag gggaggccag ttgagagctt    1020
   gggcagcctc ttcctggacc cagttatcca ggtcttcatg ctctgctcaa gggggccaca    1080
   tgagcgagcc taggagctgg acttcaaccc aggaagatgc atccgaggga aaggagattt    1140
   tctatggcct caggcctgag tgccaatatt agtctccagc ttctgtggat ga            1192
<210> 65
   <211> 518
   <212> PRT
   <213> Homo sapiens
<400> 65
   Met Leu Ala Ala Ala Ser Lys Tyr Arg His Gly Asn Met Val Phe Phe
   1               5                   10                  15
   Asp Val Leu Gly Leu Phe Val Ile Ala Tyr Pro Ser Arg Ile Gly Ser
               20                  25                  30
   Ile Ile Asn Tyr Met Val Val Met Gly Val Val Leu Tyr Leu Gly Lys
           35                  40                  45
   Lys Phe Leu Gln Pro Lys His Lys Thr Gly Asn Tyr Lys Lys Asp Phe
       50                  55                  60
   Leu Cys Gly Leu Gly Ile Thr Leu Ile Ser Trp Phe Thr Ser Leu Val
   65                  70                  75                  80
   Thr Val Leu Ile Ile Ala Val Phe Ile Ser Leu Ile Gly Gln Ser Leu
                   85                  90                  95
   Ser Trp Tyr Asn His Phe Tyr Val Ser Val Cys Leu Tyr Gly Thr Ala
               100                 105                 110
   Thr Val Ala Lys Ile Ile Leu Ile His Thr Leu Ala Lys Arg Phe Tyr
           115                 120                 125
   Tyr Met Asn Ala Ser Ala Gln Tyr Leu Gly Glu Val Phe Phe Asp Ile
       130                 135                 140
   Ser Leu Phe Val His Cys Cys Phe Leu Val Thr Leu Thr Tyr Gln Gly
   145                 150                 155                 160
   Leu Cys Ser Ala Phe Ile Ser Ala Val Trp Val Ala Phe Pro Leu Leu
                   165                 170                 175
   Thr Lys Leu Cys Val His Lys Asp Phe Lys Gln His Gly Ala Gln Gly
               180                 185                 190
   Lys Phe Ile Ala Phe Tyr Leu Leu Gly Met Phe Ile Pro Tyr Leu Tyr
           195                 200                 205
   Ala Leu Tyr Leu Ile Trp Ala Val Phe Glu Met Phe Thr Pro Ile Leu
       210                 215                 220
   Gly Arg Ser Gly Ser Glu Ile Pro Pro Asp Val Val Leu Ala Ser Ile
   225                 230                 235                 240
   Leu Ala Gly Cys Thr Met Ile Leu Ser Ser Tyr Phe Ile Asn Phe Ile
                   245                 250                 255
   Tyr Leu Ala Lys Ser Thr Lys Lys Thr Met Leu Thr Leu Thr Leu Val
               260                 265                 270
   Cys Ala Ile Thr Phe Leu Leu Val Cys Ser Gly Thr Phe Phe Pro Tyr
           275                 280                 285
   Ser Ser Asn Pro Ala Asn Pro Lys Pro Lys Arg Val Phe Leu Gln His
       290                 295                 300
   Met Thr Arg Thr Phe His Asp Leu Glu Gly Asn Ala Val Lys Arg Asp
   305                 310                 315                 320
   Ser Gly Ile Trp Ile Asn Gly Phe Asp Tyr Thr Gly Ile Ser His Ile
                   325                 330                 335
   Thr Pro His Ile Pro Glu Ile Asn Asp Ser Ile Arg Ala His Cys Glu
               340                 345                 350
   Glu Asn Ala Pro Leu Cys Gly Phe Pro Trp Tyr Leu Pro Val His Phe
           355                 360                 365
   Leu Ile Arg Lys Asn Trp Tyr Leu Pro Ala Pro Glu Val Ser Pro Arg
       370                 375                 380
   Asn Pro Pro His Phe Arg Leu Ile Ser Lys Glu Gln Thr Pro Trp Asp
   385                 390                 395                 400
   Ser Ile Lys Leu Thr Phe Glu Ala Thr Gly Pro Ser His Met Ser Phe
                   405                 410                 415
   Tyr Val Arg Ala His Lys Gly Ser Thr Leu Ser Gln Trp Ser Leu Gly
               420                 425                 430
   Asn Gly Thr Pro Val Thr Ser Lys Gly Gly Asp Tyr Phe Val Phe Tyr
           435                 440                 445
   Ser His Gly Leu Gln Ala Ser Ala Trp Gln Phe Trp Ile Glu Val Gln
       450                 455                 460
   Val Ser Glu Glu His Pro Glu Gly Met Val Thr Val Ala Ile Ala Ala
   465                 470                 475                 480
   His Tyr Leu Ser Gly Glu Asp Lys Arg Ser Pro Gln Leu Asp Ala Leu
                   485                 490                 495
   Lys Glu Lys Phe Pro Asp Trp Thr Phe Pro Ser Ala Trp Val Cys Thr
               500                 505                 510
   Tyr Asp Leu Phe Val Phe
           515
<210> 66
   <211> 904
   <212> PRT
   <213> Homo sapiens
<400> 66
   Met Glu Trp Gly Ser Glu Ser Ala Ala Val Arg Arg His Arg Val Gly
   1               5                   10                  15
   Val Glu Arg Arg Glu Gly Ala Ala Ala Ala Pro Pro Pro Glu Arg Glu
               20                  25                  30
   Ala Arg Ala Gln Glu Pro Leu Val Asp Gly Cys Ser Gly Gly Gly Arg
           35                  40                  45
   Thr Arg Lys Arg Ser Pro Gly Gly Ser Gly Gly Ala Ser Arg Gly Ala
       50                  55                  60
   Gly Thr Gly Leu Ser Glu Val Arg Ala Ala Leu Gly Leu Ala Leu Tyr
   65                  70                  75                  80
   Leu Ile Ala Leu Arg Thr Leu Val Gln Leu Ser Leu Gln Gln Leu Val
                   85                  90                  95
   Leu Arg Gly Ala Ala Gly His Arg Gly Glu Phe Asp Ala Leu Gln Ala
               100                 105                 110
   Arg Asp Tyr Leu Glu His Ile Thr Ser Ile Gly Pro Arg Thr Thr Gly
           115                 120                 125
   Ser Pro Glu Asn Glu Ile Leu Thr Val His Tyr Leu Leu Glu Gln Ile
       130                 135                 140
   Lys Leu Ile Glu Val Gln Ser Asn Ser Leu His Lys Ile Ser Val Asp
   145                 150                 155                 160
   Val Gln Arg Pro Thr Gly Ser Phe Ser Ile Asp Phe Leu Gly Gly Phe
                   165                 170                 175
   Thr Ser Tyr Tyr Asp Asn Ile Thr Asn Val Val Val Lys Leu Glu Pro
               180                 185                 190
   Arg Asp Gly Ala Gln His Ala Val Leu Ala Asn Cys His Phe Asp Ser
           195                 200                 205
   Val Ala Asn Ser Pro Gly Ala Ser Asp Asp Ala Val Ser Cys Ser Val
       210                 215                 220
   Met Leu Glu Val Leu Arg Val Leu Ser Thr Ser Ser Glu Ala Leu His
   225                 230                 235                 240
   His Ala Val Ile Phe Leu Phe Asn Gly Ala Glu Glu Asn Val Leu Gln
                   245                 250                 255
   Ala Ser His Gly Phe Ile Thr Gln His Pro Trp Ala Ser Leu Ile Arg
               260                 265                 270
   Ala Phe Ile Asn Leu Glu Ala Ala Gly Val Gly Gly Lys Glu Leu Val
               275                 280                 285
   Phe Gln Thr Gly Pro Glu Asn Pro Trp Leu Val Gln Ala Tyr Val Ser
           290                 295                 300
   Ala Ala Lys His Pro Phe Ala Ser Val Val Ala Gln Glu Val Phe Gln
   305                 310                 315                 320
   Ser Gly Ile Ile Pro Ser Asp Thr Asp Phe Arg Ile Tyr Arg Asp Phe
                   325                 330                 335
   Gly Asn Ile Pro Gly Ile Asp Leu Ala Phe Ile Glu Asn Gly Tyr Ile
               340                 345                 350
   Tyr His Thr Lys Tyr Asp Thr Ala Asp Arg Ile Leu Thr Asp Ser Ile
           355                 360                 365
   Gln Arg Ala Gly Asp Asn Ile Leu Ala Val Leu Lys His Leu Ala Thr
       370                 375                 380
   Ser Asp Met Leu Ala Ala Ala Ser Lys Tyr Arg His Gly Asn Met Val
   385                 390                 395                 400
   Phe Phe Asp Val Leu Gly Leu Phe Val Ile Ala Tyr Pro Ser Arg Ile
                   405                 410                 415
   Gly Ser Ile Ile Asn Tyr Met Val Val Met Gly Val Val Leu Tyr Leu
               420                 425                 430
   Gly Lys Lys Phe Leu Gln Pro Lys His Lys Thr Gly Asn Tyr Lys Lys
           435                 440                 445
   Asp Phe Leu Cys Gly Leu Gly Ile Thr Leu Ile Ser Trp Phe Thr Ser
       450                 455                 460
   Leu Val Thr Val Leu Ile Ile Ala Val Phe Ile Ser Leu Ile Gly Gln
   465                 470                 475                 480
   Ser Leu Ser Trp Tyr Asn His Phe Tyr Val Ser Val Cys Leu Tyr Gly
                   485                 490                 495
   Thr Ala Thr Val Ala Lys Ile Ile Leu Ile His Thr Leu Ala Lys Arg
               500                 505                 510
   Phe Tyr Tyr Met Asn Ala Ser Ala Gln Tyr Leu Gly Glu Val Phe Phe
           515                 520                 525
   Asp Ile Ser Leu Phe Val His Cys Cys Phe Leu Val Thr Leu Thr Tyr
       530                 535                 540
   Gln Gly Leu Cys Ser Ala Phe Ile Ser Ala Val Trp Val Ala Phe Pro
   545                 550                 555                 560
   Leu Leu Thr Lys Leu Cys Val His Lys Asp Phe Lys Gln His Gly Ala
                   565                 570                 575
   Gln Gly Lys Phe Ile Ala Phe Tyr Leu Leu Gly Met Phe Ile Pro Tyr
               580                 585                 590
   Leu Tyr Ala Leu Tyr Leu Ile Trp Ala Val Phe Glu Met Phe Thr Pro
           595                 600                 605
   Ile Leu Gly Arg Ser Gly Ser Glu Ile Pro Pro Asp Val Val Leu Ala
       610                 615                 620
   Ser Ile Leu Ala Gly Cys Thr Met Ile Leu Ser Ser Tyr Phe Ile Asn
   625                 630                 635                 640
   Phe Ile Tyr Leu Ala Lys Ser Thr Lys Lys Thr Met Leu Thr Leu Thr
                   645                 650                 655
   Leu Val Cys Ala Ile Thr Phe Leu Leu Val Cys Ser Gly Thr Phe Phe
               660                 665                 670
   Pro Tyr Ser Ser Asn Pro Ala Asn Pro Lys Pro Lys Arg Val Phe Leu
           675                 680                 685
   Gln His Met Thr Arg Thr Phe His Asp Leu Glu Gly Asn Ala Val Lys
       690                 695                 700
   Arg Asp Ser Gly Ile Trp Ile Asn Gly Phe Asp Tyr Thr Gly Ile Ser
   705                 710                 715                 720
   His Ile Thr Pro His Ile Pro Glu Ile Asn Asp Ser Ile Arg Ala His
                   725                 730                 735
   Cys Glu Glu Asn Ala Pro Leu Cys Gly Phe Pro Trp Tyr Leu Pro Val
               740                 745                 750
   His Phe Leu Ile Arg Lys Asn Trp Tyr Leu Pro Ala Pro Glu Val Ser
           755                 760                 765
   Pro Arg Asn Pro Pro His Phe Arg Leu Ile Ser Lys Glu Gln Thr Pro
       770                 775                 780
   Trp Asp Ser Ile Lys Leu Thr Phe Glu Ala Thr Gly Pro Ser His Met
   785                 790                 795                 800
   Ser Phe Tyr Val Arg Ala His Lys Gly Ser Thr Leu Ser Gln Trp Ser
                   805                 810                 815
   Leu Gly Asn Gly Thr Pro Val Thr Ser Lys Gly Gly Asp Tyr Phe Val
               820                 825                 830
   Phe Tyr Ser His Gly Leu Gln Ala Ser Ala Trp Gln Phe Trp Ile Glu
           835                 840                 845
   Val Gln Val Ser Glu Glu His Pro Glu Gly Met Val Thr Val Ala Ile
       850                 855                 860
   Ala Ala His Tyr Leu Ser Gly Glu Asp Lys Arg Ser Pro Gln Leu Asp
   865                 870                 875                 880
   Ala Leu Lys Glu Lys Phe Pro Asp Trp Thr Phe Pro Ser Ala Trp Val
                   885                 890                 895
   Cys Thr Tyr Asp Leu Phe Val Phe
               900
<210> 67
   <211> 419
   <212> PRT
   <213> Homo sapiens
<400> 67
   Met Val Val Met Gly Val Val Leu Tyr Leu Gly Lys Lys Phe Leu Gln
   1               5                   10                  15
   Pro Lys His Lys Thr Gly Asn Tyr Lys Lys Asp Phe Leu Cys Gly Leu
               20                  25                  30
   Gly Ile Thr Leu Ile Ser Trp Phe Thr Ser Leu Val Thr Val Leu Ile
           35                  40                  45
   Ile Ala Val Phe Ile Ser Leu Ile Gly Gln Ser Leu Ser Trp Tyr Asn
       50                  55                  60
   His Phe Tyr Val Ser Val Cys Leu Tyr Gly Thr Ala Thr Val Ala Lys
   65                  70                  75                  80
   Ile Ile Leu Ile His Thr Leu Ala Lys Arg Phe Tyr Tyr Met Asn Ala
                   85                  90                  95
   Ser Ala Gln Tyr Leu Gly Glu Val Phe Phe Asp Ile Ser Leu Phe Val
               100                 105                 110
   His Cys Cys Phe Leu Val Thr Leu Thr Tyr Gln Gly Leu Cys Ser Ala
           115                 120                 125
   Phe Ile Ser Ala Val Trp Val Ala Phe Pro Leu Leu Thr Lys Leu Cys
       130                 135                 140
   Val His Lys Asp Phe Lys Gln His Gly Ala Gln Gly Lys Phe Ile Ala
   145                 150                 155                 160
   Phe Tyr Leu Leu Gly Met Phe Ile Pro Tyr Leu Tyr Ala Leu Tyr Leu
                   165                 170                 175
   Ile Trp Ala Val Phe Glu Met Phe Thr Pro Ile Leu Gly Arg Ser Gly
               180                 185                 190
   Ser Glu Ile Pro Pro Asp Val Val Leu Ala Ser Ile Leu Ala Gly Cys
           195                 200                 205
   Thr Met Ile Leu Ser Ser Tyr Phe Ile Asn Phe Ile Tyr Leu Ala Lys
       210                 215                 220
   Ser Thr Lys Lys Thr Met Leu Thr Leu Thr Leu Val Cys Ala Ile Thr
   225                 230                 235                 240
   Phe Leu Leu Val Cys Ser Gly Thr Phe Phe Pro Tyr Ser Ser Asn Pro
                   245                 250                 255
   Ala Asn Pro Lys Pro Lys Arg Val Phe Leu Gln His Met Thr Arg Thr
               260                 265                 270
   Phe His Asp Leu Glu Gly Asn Ala Val Lys Arg Asp Ser Gly Ile Trp
           275                 280                 285
   Ile Asn Gly Phe Asp Tyr Thr Gly Ile Ser His Ile Thr Pro His Ile
       290                 295                 300
   Pro Glu Ile Asn Asp Ser Ile Arg Ala His Cys Glu Glu Asn Ala Pro
   305                 310                 315                 320
   Leu Cys Gly Phe Pro Trp Tyr Leu Pro Val His Phe Leu Ile Arg Lys
                   325                 330                 335
   Asn Trp Tyr Leu Pro Ala Pro Glu Val Ser Pro Arg Asn Pro Pro His
               340                 345                 350
   Phe Arg Leu Ile Ser Lys Glu Gln Thr Pro Trp Asp Ser Ile Lys Leu
           355                 360                 365
   Thr Phe Glu Ala Thr Ala Cys Leu Pro Ile Leu Gln Ile Leu Asp Leu
       370                 375                 380
   Pro Ala Ser Thr Ile Met Thr Lys Pro Tyr Val Leu Leu Cys Ser Ser
   385                 390                 395                 400
   Pro Gln Arg Val Asn Thr Phe Ser Val Val Ser Trp Gln Trp His Pro
                   405                 410                 415
   Ser His Lys
<210> 68
   <211> 4974
   <212> DNA
   <213> Homo sapiens
<400> 68
   ggcgcgggga ccgggctgtc tgaggtgcgc gccgcgctgg ggctcgcgct ctacctgatc     60
   gcgctgcgga cgctggtgca gctctcgctg cagcagctcg tgctacgcgg ggccgctgga    120
   caccgcgggg agttcgacgc gctccaagcc agggattatc ttgaacacat aacctccatt    180
   ggccccagga ctacaggaag tccagaaaat gaaattctga ccgtgcacta ccttttggaa    240
   cagattaaac tgattgaagt gcaaagcaac agccttcata agatttcagt agatgtacaa    300
   cggcccacag gctcttttag cattgatttc ttgggaggtt ttacaagcta ttatgacaac    360
   atcaccaatg ttgtggtaaa gctggaaccc agagatggag cccagcatgc tgtcttggct    420
   aattgtcatt ttgactcagt agcaaactca ccaggccagt catggtttca ttactcagca    480
   cccctgggct agcttgattc gtgcattcat taacctagag gcagcaggtg taggagggaa    540
   agaacttgta ttccaaacag gtcctgaaaa tccttggttg gttcaagctt atgtttcagc    600
   agctaaacac ccttttgctt ctgtggtggc tcaggaggtt tttcagagtg gaatcattcc    660
   ttcagatact gactttcgta tctacaggga ttttgggaac attccaggaa tagacttagc    720
   ttttattgag aatggataca tttatcacac caagtatgac acagcggaca gaattctaac    780
   agattccatt cagagagcag gtgacaacat tttagcagtt cttaagcatc tagctacatc    840
   tgatatgctg gctgctgctt ctaagtatcg acatggaaac atggtcttct ttgatgtgct    900
   gggcctgttt gtcattgcct acccctctcg tattggctca atcataaact acatggtggt    960
   aatgggtgtt gttttgtacc tgggcaaaaa atttttgcag cccaaacata agactggtaa   1020
   ctacaagaag gacttcttgt gtggacttgg catcactttg atcagctggt tcactagcct   1080
   tgttaccgtt ctcattatag cagtgttcat ctctcttatt ggacagtctc tctcatggta   1140
   taaccacttc tatgtctccg tttgtctgta tggaactgca actgtagcca aaataatact   1200
   tatacatact cttgcgaaaa gattttatta catgaatgcc agtgcccagt atctgggaga   1260
   agtatttttt gacatttcgc tgtttgtcca ttgctgtttt cttgttaccc tcacttacca   1320
   aggactttgc tcggcgttta ttagtgctgt ctgggtagca ttcccattgc tcacaaagct   1380
   ctgtgtgcat aaggacttca agcagcatgg tgcccaagga aaatttattg ctttttacct   1440
   tttggggatg tttattcctt atctttatgc attgtacctc atctgggcag tatttgagat   1500
   gtttacccct atcctcggga gaagtggttc tgaaatccca cctgatgttg tgctggcatc   1560
   cattttggct ggctgtacaa tgattctctc gtcctatttt attaacttca tctaccttgc   1620
   caagagcaca aaaaaaacca tgctaacttt aactttggta tgtgcaatta cattcctcct   1680
   tgtttgcagt ggaacatttt ttccatatag ctccaatcct gctaatccga agccaaagag   1740
   agtgtttctt cagcatatga ctagaacatt ccatgacttg gaaggaaatg cagttaaacg   1800
   ggactctgga atatggatca atgggtttga ttatactgga atttctcaca taacccctca   1860
   cattcctgag atcaatgata gtatccgagc tcactgtgag gagaatgcac ctctttgtgg   1920
   ttttccttgg tatcttccag tgcactttct gatcaggaaa aactggtatc ttcctgcccc   1980
   agaagtttct ccaagaaatc ctcctcattt ccgactcata tccaaagaac agacaccttg   2040
   ggattctata aaattgactt ttgaagcaac aggaccaagc catatgtcct tctatgttcg   2100
   agcccacaaa gggtcaacac tttctcagtg gtctcttggc aatggcaccc cagtcacaag   2160
   taaaggagga gactactttg tcttttactc ccatggactc caggcctctg catggcagtt   2220
   ctggatagaa gtgcaggttt cagaagaaca tcctgaagga atggtcaccg tggccattgc   2280
   tgcccactat ctgtctgggg aagacaagag atcccctcaa ctggatgctc tgaaggaaaa   2340
   gttcccagat tggacatttc cctctgcctg ggtgtgcacc tacgatctct ttgtatttta   2400
   atcttgtgga tgagctctaa gtacatgccc agtggatact ccatgtgaca tggtttctcc   2460
   ctatgttacg tggatgtttg taacgtaagt caatgaattt taatgatcat atgttcaaag   2520
   agctttctgg gttaacgctt ttcagggcca agcactataa gggtttagct gtggcgcagt   2580
   gatgcatggc ctgttgacac ttgaaaatgc cagtcttttg gcacttcagc acatgtgggt   2640
   actgccacta cacacacgtc attttatatg accttaagga caaagccaac aatccacttc   2700
   aatagctgcc cctttaggat caagaaagat gtacactgtc agagcattgt taatgagaca   2760
   aaagttgttt ccaatttaag ccccaaaacc atttgttgta ttagtggatg gtgggtaaaa   2820
   tatcattcac tgaggtaatg attccccttg agaatataac tctgtgtagg tcactggaaa   2880
   gtgattgcca tagggctggg agagaagcat tgcactcttg aggctgtagc ctgtgtcaag   2940
   ctgtttcttc aggcagcctc tcaaatgtgc tttgtctctc tgtgctgagg cctggaccct   3000
   gtgctgagct ggtgactcac tgtcctgaca agtggacaca cagatgcact gctgtgctgc   3060
   tttcctgagg tggttttcta tgcctgtttt cctctgaaac atgtctgtta cccctctcca   3120
   tcttaccaag ttgaaaaggg gaatatttgg ccacataccc ctctggtttt cgtaggttct   3180
   tttggttcag aatattgttt gtgccagtac atgaccttaa cttccttcct cagagcactg   3240
   agctgccatc tgggctattc tggggtagaa ggaaggctgg gagtggtggg aattttataa   3300
   atatttattc tcttttcttt gtttcatagg agtcttgtgt tatacaaggt tagtccttca   3360
   tggtataatc ttactgatgc actgggccta tctttttgtt ttccagccag ttgaatagat   3420
   tagtttttct cagtaactta ctatccagca gactggcttt cctgagactt gaggttgtgg   3480
   cttatactgg aatgagacca ctgtacgtgt aggtggttca gatcctgcgt aatggcagca   3540
   tgaggactta aaaggtggtt ttcattttga agatggctat gtagcttgta aggtgtatca   3600
   cagcagtacc tctcatggct ttttggttcc agcagtgagg gcattggtga gatcaatggt   3660
   aaactgtgca agctttcttt ttatcattag gaaatgtgaa acgttggaca aattttgagt   3720
   tttaacaagg acaaaaagtt gaaagaaaag gcacagttaa caaaaaaggg tggctagatt   3780
   tatcttgggt gatggaggaa atgagagagg aatgctcttg aaaggtggtc tgtggatctg   3840
   tctgaataga aagagcacag taagtatgca ttgccggaga aaacgtcctt gaagctgctt   3900
   gtctcatgtg tatgatgtgc tttttaaatc atgcccctcg ttgcctgcct aatctgtgac   3960
   tccctaaaaa ctaactgggc ccatgtagat ggggctgcaa ccagagctga ataacatgtt   4020
   aggctcacac atgcatcagc actgcacact ggaatcattg ctcttcctgg actttgtaga   4080
   aatcagtctc aagtgcttca agagtctggc tcctgctact tttatctgtc aggtagcaca   4140
   taaggtttgc agggtttata ttttgtatag aatcacagtt gtggagaaaa agtaataatt   4200
   tctcaatgaa ttttaaaaat gggcctattt tctatccccg tggttcatct gatataatta   4260
   gtgttccctg tgaattcccc ccctctatgg gaaggatgcc tttactcttt atcagtaata   4320
   aattatgact gttttcatat tgccttaggg ttatttccct gtgtaaacca ttgtcttttg   4380
   ttttggtttt ctttagcatt atgaagcttt ggtattgtac aaggtcagta gtaagatgct   4440
   cactagtctc agggcttgtg taatattctg ggaggtcatt taaatgccag aaatggtcaa   4500
   gcaattatac acagtattta tgactctgtt aagcataccg tttgtctgtc acattagtag   4560
   attctgagat taaaaaaaat ttttaaagag tgatcattta aataatttct aaaagggtct   4620
   tttcaagctc taacaaagtc actaacaaat gcattatttt ctacagaatt agatgttagt   4680
   agtacagtac tgcatattca gggaaaaagt gtgaggaatt gatttcaaaa tagttcgttc   4740
   ttgtgtttga cctaagaatg attgtcgcat gaagtgtttg tttttacagt ttagcatata   4800
   taaacaaaca tgataggatt ccttaagatg ttaccaccca gggggccaca agccagcctg   4860
   ctgtctcagg aagctgtaga aggagtgttt gtcaatttct tgtcactggt ttgctgactt   4920
   actgaggatt aattgttgcc ttacaatgtt actgaaataa actgtttaat atac         4974
<210> 69
   <211> 5338
   <212> DNA
   <213> Homo sapiens
<400> 69
   ggccggggct gtcgcgggtt ggggcggttg ggctggcagc tgaggctcgt ggccatggag      60
   tggggttctg agtcggctgc tgtgaggcgg caccgcgtcg gagtagagcg tcgagaggga     120
   gcggcggccg cgccaccgcc ggagagggag gcccgagcgc aggagcctct ggtggatggg     180
   tgcagcggcg gcgggaggac gcggaagagg agccccgggg gtagcggcgg cgcgagcagg     240
   ggcgcgggga ccgggctgtc tgaggtgcgc gccgcgctgg ggctcgcgct ctacctgatc     300
   gcgctgcgga cgctggtgca gctctcgctg cagcagctcg tgctacgcgg ggccgctgga     360
   caccgcgggg agttcgacgc gctccaagcc agggattatc ttgaacacat aacctccatt     420
   ggccccagga ctacaggaag tccagaaaat gaaattctga ccgtgcacta ccttttggaa     480
   cagattaaac tgattgaagt gcaaagcaac agccttcata agatttcagt agatgtacaa     540
   cggcccacag gctcttttag cattgatttc ttgggaggtt ttacaagcta ttatgacaac     600
   atcaccaatg ttgtggtaaa gctggaaccc agagatggag cccagcatgc tgtcttggct     660
   aattgtcatt ttgactcagt agcaaactca ccaggtgcca gtgatgatgc agttagctgc     720
   tcagtgatgc tggaagtcct tcgcgtcttg tcaacatctt cagaagcctt gcatcatgct     780
   gtcatatttc tctttaatgg tgctgaggaa aatgtcttgc aagccagtca tggtttcatt     840
   actcagcacc cctgggctag cttgattcgt gcattcatta acctagaggc agcaggtgta     900
   ggagggaaag aacttgtatt ccaaacaggt cctgaaaatc cttggttggt tcaagcttat     960
   gtttcagcag ctaaacaccc ttttgcttct gtggtggctc aggaggtttt tcagagtgga    1020
   atcattcctt cagatactga ctttcgtatc tacagggatt ttgggaacat tccaggaata    1080
   gacttagctt ttattgagaa tggatacatt tatcacacca agtatgacac agcggacaga    1140
   attctaacag attccattca gagagcaggt gacaacattt tagcagttct taagcatcta    1200
   gctacatctg atatgctggc tgctgcttct aagtatcgac atggaaacat ggtcttcttt    1260
   gatgtgctgg gcctgtttgt cattgcctac ccctctcgta ttggctcaat cataaactac    1320
   atggtggtaa tgggtgttgt tttgtacctg ggcaaaaaat ttttgcagcc caaacataag    1380
   actggtaact acaagaagga cttcttgtgt ggacttggca tcactttgat cagctggttc    1440
   actagccttg ttaccgttct cattatagca gtgttcatct ctcttattgg acagtctctc    1500
   tcatggtata accacttcta tgtctccgtt tgtctgtatg gaactgcaac tgtagccaaa   1560
   ataatactta tacatactct tgcgaaaaga ttttattaca tgaatgccag tgcccagtat   1620
   ctgggagaag tattttttga catttcgctg tttgtccatt gctgttttct tgttaccctc   1680
   acttaccaag gactttgctc ggcgtttatt agtgctgtct gggtagcatt cccattgctc   1740
   acaaagctct gtgtgcataa ggacttcaag cagcatggtg cccaaggaaa atttattgct   1800
   ttttaccttt tggggatgtt tattccttat ctttatgcat tgtacctcat ctgggcagta   1860
   tttgagatgt ttacccctat cctcgggaga agtggttctg aaatcccacc tgatgttgtg   1920
   ctggcatcca ttttggctgg ctgtacaatg attctctcgt cctattttat taacttcatc   1980
   taccttgcca agagcacaaa aaaaaccatg ctaactttaa ctttggtatg tgcaattaca   2040
   ttcctccttg tttgcagtgg aacatttttt ccatatagct ccaatcctgc taatccgaag   2100
   ccaaagagag tgtttcttca gcatatgact agaacattcc atgacttgga aggaaatgca   2160
   gttaaacggg actctggaat atggatcaat gggtttgatt atactggaat ttctcacata   2220
   acccctcaca ttcctgagat caatgatagt atccgagctc actgtgagga gaatgcacct   2280
   ctttgtggtt ttccttggta tcttccagtg cactttctga tcaggaaaaa ctggtatctt   2340
   cctgccccag aagtttctcc aagaaatcct cctcatttcc gactcatatc caaagaacag   2400
   acaccttggg attctataaa attgactttt gaagcaacag gaccaagcca tatgtccttc   2460
   tatgttcgag cccacaaagg gtcaacactt tctcagtggt ctcttggcaa tggcacccca   2520
   gtcacaagta aaggaggaga ctactttgtc ttttactccc atggactcca ggcctctgca   2580
   tggcagttct ggatagaagt gcaggtttca gaagaacatc ctgaaggaat ggtcaccgtg   2640
   gccattgctg cccactatct gtctggggaa gacaagagat cccctcaact ggatgctctg   2700
   aaggaaaagt tcccagattg gacatttccc tctgcctggg tgtgcaccta cgatctcttt   2760
   gtattttaat cttgtggatg agctctaagt acatgcccag tggatactcc atgtgacatg   2820
   gtttctccct atgttacgtg gatgtttgta acgtaagtca atgaatttta atgatcatat   2880
   gttcaaagag ctttctgggt taacgctttt cagggccaag cactataagg gtttagctgt   2940
   ggcgcagtga tgcatggcct gttgacactt gaaaatgcca gtcttttggc acttcagcac   3000
   atgtgggtac tgccactaca cacacgtcat tttatatgac cttaaggaca aagccaacaa   3060
   tccacttcaa tagctgcccc tttaggatca agaaagatgt acactgtcag agcattgtta   3120
   atgagacaaa agttgtttcc aatttaagcc ccaaaaccat ttgttgtatt agtggatggt   3180
   gggtaaaata tcattcactg aggtaatgat tccccttgag aatataactc tgtgtaggtc   3240
   actggaaagt gattgccata gggctgggag agaagcattg cactcttgag gctgtagcct   3300
   gtgtcaagct gtttcttcag gcagcctctc aaatgtgctt tgtctctctg tgctgaggcc   3360
   tggaccctgt gctgagctgg tgactcactg tcctgacaag tggacacaca gatgcactgc 3420
   tgtgctgctt tcctgaggtg gttttctatg cctgttttcc tctgaaacat gtctgttacc 3480
   cctctccatc ttaccaagtt gaaaagggga atatttggcc acatacccct ctggttttcg 3540
   taggttcttt tggttcagaa tattgtttgt gccagtacat gaccttaact tccttcctca 3600
   gagcactgag ctgccatctg ggctattctg gggtagaagg aaggctggga gtggtgggaa 3660
   ttttataaat atttattctc ttttctttgt ttcataggag tcttgtgtta tacaaggtta 3720
   gtccttcatg gtataatctt actgatgcac tgggcctatc tttttgtttt ccagccagtt 3780
   gaatagatta gtttttctca gtaacttact atccagcaga ctggctttcc tgagacttga 3840
   ggttgtggct tatactggaa tgagaccact gtacgtgtag gtggttcaga tcctgcgtaa 3900
   tggcagcatg aggacttaaa aggtggtttt cattttgaag atggctatgt agcttgtaag 3960
   gtgtatcaca gcagtacctc tcatggcttt ttggttccag cagtgagggc attggtgaga 4020
   tcaatggtaa actgtgcaag ctttcttttt atcattagga aatgtgaaac gttggacaaa 4080
   ttttgagttt taacaaggac aaaaagttga aagaaaaggc acagttaaca aaaaagggtg 4140
   gctagattta tcttgggtga tggaggaaat gagagaggaa tgctcttgaa aggtggtctg 4200
   tggatctgtc tgaatagaaa gagcacagta agtatgcatt gccggagaaa acgtccttga 4260
   agctgcttgt ctcatgtgta tgatgtgctt tttaaatcat gcccctcgtt gcctgcctaa 4320
   tctgtgactc cctaaaaact aactgggccc atgtagatgg ggctgcaacc agagctgaat 4380
   aacatgttag gctcacacat gcatcagcac tgcacactgg aatcattgct cttcctggac 4440
   tttgtagaaa tcagtctcaa gtgcttcaag agtctggctc ctgctacttt tatctgtcag 4500
   gtagcacata aggtttgcag ggtttatatt ttgtatagaa tcacagttgt ggagaaaaag 4560
   taataatttc tcaatgaatt ttaaaaatgg gcctattttc tatccccgtg gttcatctga 4620
   tataattagt gttccctgtg aattcccccc ctctatggga aggatgcctt tactctttat 4680
   cagtaataaa ttatgactgt tttcatattg ccttagggtt atttccctgt gtaaaccatt 4740
   gtcttttgtt ttggttttct ttagcattat gaagctttgg tattgtacaa ggtcagtagt 4800
   aagatgctca ctagtctcag ggcttgtgta atattctggg aggtcattta aatgccagaa 4860
   atggtcaagc aattatacac agtatttatg actctgttaa gcataccgtt tgtctgtcac 4920
   attagtagat tctgagatta aaaaaaattt ttaaagagtg atcatttaaa taatttctaa 4980
   aagggtcttt tcaagctcta acaaagtcac taacaaatgc attattttct acagaattag 5040
   atgttagtag tacagtactg catattcagg gaaaaagtgt gaggaattga tttcaaaata 5100
   gttcgttctt gtgtttgacc taagaatgat tgtcgcatga agtgtttgtt tttacagttt 5160
   agcatatata aacaaacatg ataggattcc ttaagatgtt accacccagg gggccacaag 5220
   ccagcctgct gtctcaggaa gctgtagaag gagtgtttgt caatttcttg tcactggttt 5280
   gctgacttac tgaggattaa ttgttgcctt acaatgttac tgaaataaac tgtttaat 5338
<210> 70
   <211> 5387
   <212> DNA
   <213> Homo sapiens
<400> 70
   ggccggggct gtcgcgggtt ggggcggttg ggctggcagc tgaggctcgt ggccatggag     60
   tggggttctg agtcggctgc tgtgaggcgg caccgcgtcg gagtagagcg tcgagaggga    120
   gcggcggccg cgccaccgcc ggagagggag gcccgagcgc aggagcctct ggtggatggg    180
   tgcagcggcg gcgggaggac gcggaagagg agccccgggg gtagcggcgg cgcgagcagg    240
   ggcgcgggga ccgggctgtc tgaggtgcgc gccgcgctgg ggctcgcgct ctacctgatc    300
   gcgctgcgga cgctggtgca gctctcgctg cagcagctcg tgctacgcgg ggccgctgga    360
   caccgcgggg agttcgacgc gctccaagcc agggattatc ttgaacacat aacctccatt    420
   ggccccagga ctacaggaag tccagaaaat gaaattctga ccgtgcacta ccttttggaa    480
   cagattaaac tgattgaagt gcaaagcaac agccttcata agatttcagt agatgtacaa    540
   cggcccacag gctcttttag cattgatttc ttgggaggtt ttacaagcta ttatgacaac    600
   atcaccaatg ttgtggtaaa gctggaaccc agagatggag cccagcatgc tgtcttggct    660
   aattgtcatt ttgactcagt agcaaactca ccaggtgcca gtgatgatgc agttagctgc    720
   tcagtgatgc tggaagtcct tcgcgtcttg tcaacatctt cagaagcctt gcatcatgct    780
   gtcatatttc tctttaatgg tgctgaggaa aatgtcttgc aagccagtca tggtttcatt    840
   actcagcacc cctgggctag cttgattcgt gcattcatta acctagaggc agcaggtgta    900
   ggagggaaag aacttgtatt ccaaacaggt cctgaaaatc cttggttggt tcaagcttat    960
   gtttcagcag ctaaacaccc ttttgcttct gtggtggctc aggaggtttt tcagagtgga   1020
   atcattcctt cagatactga ctttcgtatc tacagggatt ttgggaacat tccaggaata   1080
   gacttagctt ttattgagaa tggatacatt tatcacacca agtatgacac agcggacaga   1140
   attctaacag attccattca gagagcaggt gacaacattt tagcagttct taagcatcta   1200
   gctacatctg atatgctggc tgctgcttct aagtatcgac atggaaacat ggtcttcttt   1260
   gatgtgctgg gcctgtttgt cattgcctac ccctctcgta ttggctcaat cataaactac   1320
   atggtggtaa tgggtgttgt tttgtacctg ggcaaaaaat ttttgcagcc caaacataag   1380
   actggtaact acaagaagga cttcttgtgt ggacttggca tcactttgat cagctggttc   1440
   actagccttg ttaccgttct cattatagca gtgttcatct ctcttattgg acagtctctc   1500
   tcatggtata accacttcta tgtctccgtt tgtctgtatg gaactgcaac tgtagccaaa   1560
   ataatactta tacatactct tgcgaaaaga ttttattaca tgaatgccag tgcccagtat 1620
   ctgggagaag tattttttga catttcgctg tttgtccatt gctgttttct tgttaccctc 1680
   acttaccaag gactttgctc ggcgtttatt agtgctgtct gggtagcatt cccattgctc 1740
   acaaagctct gtgtgcataa ggacttcaag cagcatggtg cccaaggaaa atttattgct 1800
   ttttaccttt tggggatgtt tattccttat ctttatgcat tgtacctcat ctgggcagta 1860
   tttgagatgt ttacccctat cctcgggaga agtggttctg aaatcccacc tgatgttgtg 1920
   ctggcatcca ttttggctgg ctgtacaatg attctctcgt cctattttat taacttcatc 1980
   taccttgcca agagcacaaa aaaaaccatg ctaactttaa ctttggtatg tgcaattaca 2040
   ttcctccttg tttgcagtgg aacatttttt ccatatagct ccaatcctgc taatccgaag 2100
   ccaaagagag tgtttcttca gcatatgact agaacattcc atgacttgga aggaaatgca 2160
   gttaaacggg actctggaat atggatcaat gggtttgatt atactggaat ttctcacata 2220
   acccctcaca ttcctgagat caatgatagt atccgagctc actgtgagga gaatgcacct 2280
   ctttgtggtt ttccttggta tcttccagtg cactttctga tcaggaaaaa ctggtatctt 2340
   cctgccccag aagtttctcc aagaaatcct cctcatttcc gactcatatc caaagaacag 2400
   acaccttggg attctataaa attgactttt gaagcaacag cctgcctgcc tatccttcag 2460
   attttggact tgccagcctc aacaatcatg accaagccat atgtccttct atgttcgagc 2520
   ccacaaaggg tcaacacttt ctcagtggtc tcttggcaat ggcaccccag tcacaagtaa 2580
   aggaggagac tactttgtct tttactccca tggactccag gcctctgcat ggcagttctg 2640
   gatagaagtg caggtttcag aagaacatcc tgaaggaatg gtcaccgtgg ccattgctgc 2700
   ccactatctg tctggggaag acaagagatc ccctcaactg gatgctctga aggaaaagtt 2760
   cccagattgg acatttccct ctgcctgggt gtgcacctac gatctctttg tattttaatc 2820
   ttgtggatga gctctaagta catgcccagt ggatactcca tgtgacatgg tttctcccta 2880
   tgttacgtgg atgtttgtaa cgtaagtcaa tgaattttaa tgatcatatg ttcaaagagc 2940
   tttctgggtt aacgcttttc agggccaagc actataaggg tttagctgtg gcgcagtgat 3000
   gcatggcctg ttgacacttg aaaatgccag tcttttggca cttcagcaca tgtgggtact 3060
   gccactacac acacgtcatt ttatatgacc ttaaggacaa agccaacaat ccacttcaat 3120
   agctgcccct ttaggatcaa gaaagatgta cactgtcaga gcattgttaa tgagacaaaa 3180
   gttgtttcca atttaagccc caaaaccatt tgttgtatta gtggatggtg ggtaaaatat 3240
   cattcactga ggtaatgatt ccccttgaga atataactct gtgtaggtca ctggaaagtg 3300
   attgccatag ggctgggaga gaagcattgc actcttgagg ctgtagcctg tgtcaagctg 3360
   tttcttcagg cagcctctca aatgtgcttt gtctctctgt gctgaggcct ggaccctgtg 3420
   ctgagctggt gactcactgt cctgacaagt ggacacacag atgcactgct gtgctgcttt 3480
   cctgaggtgg ttttctatgc ctgttttcct ctgaaacatg tctgttaccc ctctccatct 3540
   taccaagttg aaaaggggaa tatttggcca catacccctc tggttttcgt aggttctttt 3600
   ggttcagaat attgtttgtg ccagtacatg accttaactt ccttcctcag agcactgagc 3660
   tgccatctgg gctattctgg ggtagaagga aggctgggag tggtgggaat tttataaata 3720
   tttattctct tttctttgtt tcataggagt cttgtgttat acaaggttag tccttcatgg 3780
   tataatctta ctgatgcact gggcctatct ttttgttttc cagccagttg aatagattag 3840
   tttttctcag taacttacta tccagcagac tggctttcct gagacttgag gttgtggctt 3900
   atactggaat gagaccactg tacgtgtagg tggttcagat cctgcgtaat ggcagcatga 3960
   ggacttaaaa ggtggttttc attttgaaga tggctatgta gcttgtaagg tgtatcacag 4020
   cagtacctct catggctttt tggttccagc agtgagggca ttggtgagat caatggtaaa 4080
   ctgtgcaagc tttcttttta tcattaggaa atgtgaaacg ttggacaaat tttgagtttt 4140
   aacaaggaca aaaagttgaa agaaaaggca cagttaacaa aaaagggtgg ctagatttat 4200
   cttgggtgat ggaggaaatg agagaggaat gctcttgaaa ggtggtctgt ggatctgtct 4260
   gaatagaaag agcacagtaa gtatgcattg ccggagaaaa cgtccttgaa gctgcttgtc 4320
   tcatgtgtat gatgtgcttt ttaaatcatg cccctcgttg cctgcctaat ctgtgactcc 4380
   ctaaaaacta actgggccca tgtagatggg gctgcaacca gagctgaata acatgttagg 4440
   ctcacacatg catcagcact gcacactgga atcattgctc ttcctggact ttgtagaaat 4500
   cagtctcaag tgcttcaaga gtctggctcc tgctactttt atctgtcagg tagcacataa 4560
   ggtttgcagg gtttatattt tgtatagaat cacagttgtg gagaaaaagt aataatttct 4620
   caatgaattt taaaaatggg cctattttct atccccgtgg ttcatctgat ataattagtg 4680
   ttccctgtga attccccccc tctatgggaa ggatgccttt actctttatc agtaataaat 4740
   tatgactgtt ttcatattgc cttagggtta tttccctgtg taaaccattg tcttttgttt 4800
   tggttttctt tagcattatg aagctttggt attgtacaag gtcagtagta agatgctcac 4860
   tagtctcagg gcttgtgtaa tattctggga ggtcatttaa atgccagaaa tggtcaagca 4920
   attatacaca gtatttatga ctctgttaag cataccgttt gtctgtcaca ttagtagatt 4980
   ctgagattaa aaaaaatttt taaagagtga tcatttaaat aatttctaaa agggtctttt 5040
   caagctctaa caaagtcact aacaaatgca ttattttcta cagaattaga tgttagtagt 5100
   acagtactgc atattcaggg aaaaagtgtg aggaattgat ttcaaaatag ttcgttcttg 5160
   tgtttgacct aagaatgatt gtcgcatgaa gtgtttgttt ttacagttta gcatatataa 5220
   acaaacatga taggattcct taagatgtta ccacccaggg ggccacaagc cagcctgctg 5280
   tctcaggaag ctgtagaagg agtgtttgtc aatttcttgt cactggtttg ctgacttact   5340
   gaggattaat tgttgcctta caatgttact gaaataaact gtttaat                 5387
<210> 71
   <211> 543
   <212> PRT
   <213> Homo sapiens
<400> 71
<210> 72
   <211> 938
   <212> PRT
   <213> Homo sapiens
<400> 72
   Met Pro Ala Leu Glu His Met Asn Gln Ile Leu His Ile Leu Phe Val
   1               5                   10                  15
   Phe Leu Pro Phe Leu Trp Ala Leu Gly Thr Leu Pro Pro Pro Asp Ala
               20                  25                  30
   Leu Leu Leu Trp Ala Met Glu Gln Val Leu Glu Phe Gly Leu Gly Gly
           35                  40                  45
   Ser Ser Met Ser Thr His Leu Arg Leu Leu Val Met Phe Ile Met Ser
       50                  55                  60
   Ala Gly Thr Ala Ile Ala Ser Tyr Phe Ile Pro Ser Thr Val Gly Val
   65                  70                  75                  80
   Val Leu Phe Met Thr Gly Phe Gly Phe Leu Leu Ser Leu Asn Leu Ser
                   85                  90                  95
   Asp Met Gly His Lys Ile Gly Thr Lys Ser Lys Asp Leu Pro Ser Gly
               100                 105                 110
   Pro Glu Lys His Phe Ser Trp Lys Glu Cys Leu Phe Tyr Ile Ile Ile
           115                 120                 125
   Leu Val Leu Ala Leu Leu Glu Thr Ser Leu Leu His His Phe Ala Gly
       130                 135                 140
   Phe Ser Gln Ile Ser Lys Ser Asn Ser Gln Ala Ile Val Gly Tyr Gly
   145                 150                 155                 160
   Leu Met Ile Leu Leu Ile Ile Leu Trp Ile Leu Arg Glu Ile Gln Ser
                   165                 170                 175
   Val Tyr Ile Ile Gly Ile Phe Arg Asn Pro Phe Tyr Pro Lys Asp Val
               180                 185                 190
   Gln Thr Val Thr Val Phe Phe Glu Lys Gln Thr Arg Leu Met Lys Ile
           195                 200                 205
   Gly Ile Val Arg Arg Ile Leu Leu Thr Leu Val Ser Pro Phe Ala Met
       210                 215                 220
   Ile Ala Phe Leu Ser Leu Asp Ser Ser Leu Gln Gly Leu His Ser Val
   225                 230                 235                 240
   Ser Val Cys Ile Gly Phe Thr Arg Ala Phe Arg Met Val Trp Gln Asn
                   245                 250                 255
   Thr Glu Asn Ala Leu Leu Glu Thr Val Ile Val Ser Thr Val His Leu
               260                 265                 270
   Ile Ser Ser Thr Asp Ile Trp Trp Asn Arg Ser Leu Asp Thr Gly Leu
           275                 280                 285
   Arg Leu Leu Leu Val Gly Ile Ile Arg Asp Arg Leu Ile Gln Phe Ile
       290                 295                 300
   Ser Lys Leu Gln Phe Ala Val Thr Val Leu Leu Thr Ser Trp Thr Glu
   305                 310                 315                 320
   Lys Lys Gln Arg Arg Lys Thr Thr Ala Thr Leu Cys Ile Leu Asn Ile
                   325                 330                 335
   Val Phe Ser Pro Phe Val Leu Val Ile Ile Val Phe Ser Thr Leu Leu
               340                 345                 350
   Ser Ser Pro Leu Leu Pro Leu Phe Thr Leu Pro Val Phe Leu Val Gly
           355                 360                 365
   Phe Pro Arg Pro Ile Gln Ser Trp Pro Gly Ala Ala Gly Thr Thr Ala
       370                 375                 380
   Cys Val Cys Ala Asp Thr Val Tyr Tyr Tyr Gln Met Val Pro Arg Leu
   385                 390                 395                 400
   Thr Ala Val Leu Gln Thr Ala Met Ala Ala Gly Ser Leu Gly Leu Leu
                   405                 410                 415
   Leu Pro Gly Ser His Tyr Leu Gly Arg Phe Gln Asp Arg Leu Met Trp
               420                 425                 430
   Ile Met Ile Leu Glu Cys Gly Tyr Thr Tyr Cys Ser Ile Asn Ile Lys
           435                 440                 445
   Gly Leu Glu Leu Gln Glu Thr Ser Cys His Thr Ala Glu Ala Arg Arg
       450                 455                 460
   Val Asp Glu Val Phe Glu Asp Ala Phe Glu Gln Glu Tyr Thr Arg Val
   465                 470                 475                 480
   Cys Ser Leu Asn Glu His Phe Gly Asn Val Leu Thr Pro Cys Thr Val
                   485                 490                 495
   Leu Pro Val Lys Leu Tyr Ser Asp Ala Arg Asn Val Leu Ser Gly Ile
               500                 505                 510
   Ile Asp Ser His Glu Asn Leu Lys Glu Phe Lys Gly Asp Leu Ile Lys
           515                 520                 525
   Val Leu Val Trp Ile Leu Val Gln Tyr Cys Ser Lys Arg Pro Gly Met
       530                 535                 540
   Lys Glu Asn Val His Asn Thr Glu Asn Lys Gly Lys Ala Pro Leu Met
   545                 550                 555                 560
   Leu Pro Ala Leu Asn Thr Leu Pro Pro Pro Lys Ser Pro Glu Asp Ile
                   565                 570                 575
   Asp Ser Leu Asn Ser Glu Thr Phe Asn Asp Trp Ser Asp Asp Asn Ile
               580                 585                 590
   Phe Asp Asp Glu Pro Thr Ile Lys Lys Val Ile Glu Glu Lys His Gln
           595                 600                 605
   Leu Lys Asp Leu Pro Gly Thr Asn Leu Phe Ile Pro Gly Ser Val Glu
       610                 615                 620
   Ser Gln Arg Val Gly Asp His Ser Thr Gly Thr Val Pro Glu Asn Asp
   625                 630                 635                 640
   Leu Tyr Lys Ala Val Leu Leu Gly Tyr Pro Ala Val Asp Lys Gly Lys
                   645                 650                 655
   Gln Glu Asp Met Pro Tyr Ile Pro Leu Met Glu Phe Ser Cys Ser His
               660                 665                 670
   Ser His Leu Val Cys Leu Pro Ala Glu Trp Arg Thr Ser Cys Met Pro
           675                 680                 685
   Ser Ser Lys Met Lys Glu Met Ser Ser Leu Phe Pro Glu Asp Trp Tyr
       690                 695                 700
   Gln Phe Val Leu Arg Gln Leu Glu Cys Tyr His Ser Glu Glu Lys Ala
   705                 710                 715                 720
   Ser Asn Val Leu Glu Glu Ile Ala Lys Asp Lys Val Leu Lys Asp Phe
                   725                 730                 735
   Tyr Val His Thr Val Met Thr Cys Tyr Phe Ser Leu Phe Gly Ile Asp
               740                 745                 750
   Asn Met Ala Pro Ser Pro Gly His Ile Leu Arg Val Tyr Gly Gly Val
           755                 760                 765
   Leu Pro Trp Ser Val Ala Leu Asp Trp Leu Thr Glu Lys Pro Glu Leu
       770                 775                 780
   Phe Gln Leu Ala Leu Lys Ala Phe Arg Tyr Thr Leu Lys Leu Met Ile
   785                 790                 795                 800
   Asp Lys Ala Ser Leu Gly Pro Ile Glu Asp Phe Arg Glu Leu Ile Lys
                   805                 810                 815
   Tyr Leu Glu Glu Tyr Glu Arg Asp Trp Tyr Ile Gly Leu Val Ser Asp
               820                 825                 830
   Glu Lys Trp Lys Glu Ala Ile Leu Gln Glu Lys Pro Tyr Leu Phe Ser
           835                 840                 845
   Leu Gly Tyr Asp Ser Asn Met Gly Ile Tyr Thr Gly Arg Val Leu Ser
       850                 855                 860
   Leu Gln Glu Leu Leu Ile Gln Val Gly Lys Leu Asn Pro Glu Ala Val
   865                 870                 875                 880
   Arg Gly Gln Trp Ala Asn Leu Ser Trp Glu Leu Leu Tyr Ala Thr Asn
                   885                 890                 895
   Asp Asp Glu Glu Arg Tyr Ser Ile Gln Ala His Pro Leu Leu Leu Arg
               900                 905                 910
   Asn Leu Thr Val Gln Ala Ala Glu Pro Pro Leu Gly Tyr Pro Ile Tyr
           915                 920                 925
   Ser Ser Lys Pro Leu His Ile His Leu Tyr
       930                 935
<210> 73
   <211> 230
   <212> PRT
   <213> Homo sapiens
<400> 73
   Met Ser Pro Asp Val Pro Leu Leu Asn Asp Tyr Lys Gln Asp Phe Phe
   1               5                   10                  15
   Leu Lys Arg Phe Pro Gln Thr Val Leu Gly Gly Pro Arg Phe Lys Leu
               20                  25                  30
   Gly Tyr Cys Ala Pro Pro Tyr Ile Tyr Val Asn Gln Ile Ile Leu Phe
           35                  40                  45
   Leu Met Pro Trp Val Trp Gly Gly Val Gly Thr Leu Leu Tyr Gln Leu
       50                  55                  60
   Gly Ile Leu Lys Asp Tyr Tyr Thr Ala Ala Leu Ser Gly Gly Leu Met
   65                  70                  75                  80
   Leu Phe Thr Ala Phe Val Ile Gln Phe Thr Ser Leu Tyr Ala Lys Asn
                   85                  90                  95
   Lys Ser Thr Thr Val Glu Arg Ile Leu Thr Thr Asp Ile Leu Ala Glu
               100                 105                 110
   Glu Asp Glu His Glu Phe Thr Ser Cys Thr Gly Ala Glu Thr Val Lys
           115                 120                 125
   Phe Leu Ile Pro Gly Lys Lys Tyr Val Ala Asn Thr Val Phe His Ser
       130                 135                 140
   Ile Leu Ala Gly Leu Ala Cys Gly Leu Gly Thr Trp Tyr Leu Leu Pro
   145                 150                 155                 160
   Asn Arg Ile Thr Leu Leu Tyr Gly Ser Thr Gly Gly Thr Ala Leu Leu
                   165                 170                 175
   Phe Phe Phe Gly Trp Met Thr Leu Cys Ile Ala Glu Tyr Ser Leu Ile
               180                 185                 190
   Val Asn Thr Ala Thr Glu Thr Ala Thr Phe Gln Thr Gln Asp Thr Tyr
           195                 200                 205
   Glu Ile Ile Pro Leu Met Arg Pro Leu Tyr Ile Phe Phe Phe Val Ser
       210                 215                 220
   Val Asp Leu Ala His Arg
   225                 230
<210> 74
   <211> 1172
   <212> PRT
   <213> Homo sapiens
<400> 74
   Met Ser Pro Asp Val Pro Leu Leu Asn Asp Tyr Lys Gln Asp Phe Phe
   1               5                   10                  15
   Leu Lys Arg Phe Pro Gln Thr Val Leu Gly Gly Pro Arg Phe Lys Leu
               20                  25                  30
   Gly Tyr Cys Ala Pro Pro Tyr Ile Tyr Val Asn Gln Ile Ile Leu Phe
           35                  40                  45
   Leu Met Pro Trp Val Trp Gly Gly Val Gly Thr Leu Leu Tyr Gln Leu
       50                  55                  60
   Gly Ile Leu Lys Asp Tyr Tyr Thr Ala Ala Leu Ser Gly Gly Leu Met
   65                  70                  75                  80
   Leu Phe Thr Ala Phe Val Ile Gln Phe Thr Ser Leu Tyr Ala Lys Asn
                   85                  90                  95
   Lys Ser Thr Thr Val Glu Arg Ile Leu Thr Thr Asp Ile Leu Ala Glu
               100                 105                 110
   Glu Asp Glu His Glu Phe Thr Ser Cys Thr Gly Ala Glu Thr Val Lys
           115                 120                 125
   Phe Leu Ile Pro Gly Lys Lys Tyr Val Ala Asn Thr Val Phe His Ser
       130                 135                 140
   Ile Leu Ala Gly Leu Ala Cys Gly Leu Gly Thr Trp Tyr Leu Leu Pro
   145                 150                 155                 160
   Asn Arg Ile Thr Leu Leu Tyr Gly Ser Thr Gly Gly Thr Ala Leu Leu
                   165                 170                 175
   Phe Phe Phe Gly Trp Met Thr Leu Cys Ile Ala Glu Tyr Ser Leu Ile
               180                 185                 190
   Val Asn Thr Ala Thr Glu Thr Ala Thr Phe Gln Thr Gln Asp Thr Tyr
           195                 200                 205
   Glu Ile Ile Pro Leu Met Arg Pro Leu Tyr Ile Phe Phe Phe Val Ser
       210                 215                 220
   Val Asp Leu Ala His Arg Phe Val Val Asn Met Pro Ala Leu Glu His
   225                 230                 235                 240
   Met Asn Gln Ile Leu His Ile Leu Phe Val Phe Leu Pro Phe Leu Trp
                   245                 250                 255
   Ala Leu Gly Thr Leu Pro Pro Pro Asp Ala Leu Leu Leu Trp Ala Met
               260                 265                 270
   Glu Gln Val Leu Glu Phe Gly Leu Gly Gly Ser Ser Met Ser Thr His
           275                 280                 285
   Leu Arg Leu Leu Val Met Phe Ile Met Ser Ala Gly Thr Ala Ile Ala
       290                 295                 300
   Ser Tyr Phe Ile Pro Ser Thr Val Gly Val Val Leu Phe Met Thr Gly
   305                 310                 315                 320
   Phe Gly Phe Leu Leu Ser Leu Asn Leu Ser Asp Met Gly His Lys Ile
                   325                 330                 335
   Gly Thr Lys Ser Lys Asp Leu Pro Ser Gly Pro Glu Lys His Phe Ser
               340                 345                 350
   Trp Lys Glu Cys Leu Phe Tyr Ile Ile Ile Leu Val Leu Ala Leu Leu
           355                 360                 365
   Glu Thr Ser Leu Leu His His Phe Ala Gly Phe Ser Gln Ile Ser Lys
       370                 375                 380
   Ser Asn Ser Gln Ala Ile Val Gly Tyr Gly Leu Met Ile Leu Leu Ile
   385                 390                 395                 400
   Ile Leu Trp Ile Leu Arg Glu Ile Gln Ser Val Tyr Ile Ile Gly Ile
                   405                 410                 415
   Phe Arg Asn Pro Phe Tyr Pro Lys Asp Val Gln Thr Val Thr Val Phe
               420                 425                 430
   Phe Glu Lys Gln Thr Arg Leu Met Lys Ile Gly Ile Val Arg Arg Ile
   435 440 445
   Leu Leu Thr Leu Val Ser Pro Phe Ala Met Ile Ala Phe Leu Ser Leu
   450 455 460
   Asp Ser Ser Leu Gln Gly Leu His Ser Val Ser Val Cys Ile Gly Phe
   465 470 475 480
   Thr Arg Ala Phe Arg Met Val Trp Gln Asn Thr Glu Asn Ala Leu Leu
   485 490 495
   Glu Thr Val Ile Val Ser Thr Val His Leu Ile Ser Ser Thr Asp Ile
   500 505 510
   Trp Trp Asn Arg Ser Leu Asp Thr Gly Leu Arg Leu Leu Leu Val Gly
   515 520 525
   Ile Ile Arg Asp Arg Leu Ile Gln Phe Ile Ser Lys Leu Gln Phe Ala
   530 535 540
   Val Thr Val Leu Leu Thr Ser Trp Thr Glu Lys Lys Gln Arg Arg Lys
   545 550 555 560
   Thr Thr Ala Thr Leu Cys Ile Leu Asn Ile Val Phe Ser Pro Phe Val
   565 570 575
   Leu Val Ile Ile Val Phe Ser Thr Leu Leu Ser Ser Pro Leu Leu Pro
   580 585 590
   Leu Phe Thr Leu Pro Val Phe Leu Val Gly Phe Pro Arg Pro Ile Gln
   595 600 605
   Ser Trp Pro Gly Ala Ala Gly Thr Thr Ala Cys Val Cys Ala Asp Thr
   610 615 620
   Val Tyr Tyr Tyr Gln Met Val Pro Arg Leu Thr Ala Val Leu Gln Thr
   625 630 635 640
   Ala Met Ala Ala Gly Ser Leu Gly Leu Leu Leu Pro Gly Ser His Tyr
   645 650 655
   Leu Gly Arg Phe Gln Asp Arg Leu Met Trp Ile Met Ile Leu Glu Cys
   660 665 670
   Gly Tyr Thr Tyr Cys Ser Ile Asn Ile Lys Gly Leu Glu Leu Gln Glu
   675 680 685
   Thr Ser Cys His Thr Ala Glu Ala Arg Arg Val Asp Glu Val Phe Glu
       690                 695                 700
   Asp Ala Phe Glu Gln Glu Tyr Thr Arg Val Cys Ser Leu Asn Glu His
   705                 710                 715                 720
   Phe Gly Asn Val Leu Thr Pro Cys Thr Val Leu Pro Val Lys Leu Tyr
                   725                 730                 735
   Ser Asp Ala Arg Asn Val Leu Ser Gly Ile Ile Asp Ser His Glu Asn
               740                 745                 750
   Leu Lys Glu Phe Lys Gly Asp Leu Ile Lys Val Leu Val Trp Ile Leu
           755                 760                 765
   Val Gln Tyr Cys Ser Lys Arg Pro Gly Met Lys Glu Asn Val His Asn
       770                 775                 780
   Thr Glu Asn Lys Gly Lys Ala Pro Leu Met Leu Pro Ala Leu Asn Thr
   785                 790                 795                 800
   Leu Pro Pro Pro Lys Ser Pro Glu Asp Ile Asp Ser Leu Asn Ser Glu
                   805                 810                 815
   Thr Phe Asn Asp Trp Ser Asp Asp Asn Ile Phe Asp Asp Glu Pro Thr
               820                 825                 830
   Ile Lys Lys Val Ile Glu Glu Lys His Gln Leu Lys Asp Leu Pro Gly
           835                 840                 845
   Thr Asn Leu Phe Ile Pro Gly Ser Val Glu Ser Gln Arg Val Gly Asp
       850                 855                 860
   His Ser Thr Gly Thr Val Pro Glu Asn Asp Leu Tyr Lys Ala Val Leu
   865                 870                 875                 880
   Leu Gly Tyr Pro Ala Val Asp Lys Gly Lys Gln Glu Asp Met Pro Tyr
                   885                 890                 895
   Ile Pro Leu Met Glu Phe Ser Cys Ser His Ser His Leu Val Cys Leu
               900                 905                 910
   Pro Ala Glu Trp Arg Thr Ser Cys Met Pro Ser Ser Lys Met Lys Glu
           915                 920                 925
   Met Ser Ser Leu Phe Pro Glu Asp Trp Tyr Gln Phe Val Leu Arg Gln
       930                 935                 940
   Leu Glu Cys Tyr His Ser Glu Glu Lys Ala Ser Asn Val Leu Glu Glu
   945                 950                 955                 960
   Ile Ala Lys Asp Lys Val Leu Lys Asp Phe Tyr Val His Thr Val Met
                   965                 970                 975
   Thr Cys Tyr Phe Ser Leu Phe Gly Ile Asp Asn Met Ala Pro Ser Pro
               980                 985                 990
   Gly His Ile Leu Arg Val Tyr Gly  Gly Val Leu Pro Trp  Ser Val Ala
           995                 1000                1005
   Leu Asp  Trp Leu Thr Glu Lys  Pro Glu Leu Phe Gln  Leu Ala Leu
       1010                1015                1020
   Lys Ala  Phe Arg Tyr Thr Leu  Lys Leu Met Ile Asp  Lys Ala Ser
       1025                1030                1035
   Leu Gly  Pro Ile Glu Asp Phe  Arg Glu Leu Ile Lys  Tyr Leu Glu
       1040                1045                1050
   Glu Tyr  Glu Arg Asp Trp Tyr  Ile Gly Leu Val Ser  Asp Glu Lys
       1055                1060                1065
   Trp Lys  Glu Ala Ile Leu Gln  Glu Lys Pro Tyr Leu  Phe Ser Leu
       1070                1075                1080
   Gly Tyr  Asp Ser Asn Met Gly  Ile Tyr Thr Gly Arg  Val Leu Ser
       1085                1090                1095
   Leu Gln  Glu Leu Leu Ile Gln  Val Gly Lys Leu Asn  Pro Glu Ala
       1100                1105                1110
   Val Arg  Gly Gln Trp Ala Asn  Leu Ser Trp Glu Leu  Leu Tyr Ala
       1115                1120                1125
   Thr Asn  Asp Asp Glu Glu Arg  Tyr Ser Ile Gln Ala  His Pro Leu
       1130                1135                1140
   Leu Leu  Arg Asn Leu Thr Val  Gln Ala Ala Glu Pro  Pro Leu Gly
       1145                1150                1155
   Tyr Pro  Ile Tyr Ser Ser Lys  Pro Leu His Ile His  Leu Tyr
       1160                1165                1170
<210> 75
   <211> 873
   <212> PRT
   <213> Homo sapiens
<400> 75
   Met Pro Ala Leu Glu His Met Asn Gln Ile Leu His Ile Leu Phe Val
   1               5                   10                  15
   Phe Leu Pro Phe Leu Trp Ala Leu Gly Thr Leu Pro Pro Pro Asp Ala
               20                  25                  30
   Leu Leu Leu Trp Ala Met Glu Gln Val Leu Glu Phe Gly Leu Gly Gly
           35                  40                  45
   Ser Ser Met Ser Thr His Leu Arg Leu Leu Val Met Phe Ile Met Ser
       50                  55                  60
   Ala Gly Thr Ala Ile Ala Ser Tyr Phe Ile Pro Ser Thr Val Gly Val
   65                  70                  75                  80
   Val Leu Phe Met Thr Gly Phe Gly Phe Leu Leu Ser Leu Asn Leu Ser
                   85                  90                  95
   Asp Met Gly His Lys Ile Gly Thr Lys Ser Lys Asp Leu Pro Ser Gly
               100                 105                 110
   Pro Glu Lys His Phe Ser Trp Lys Glu Cys Leu Phe Tyr Ile Ile Ile
           115                 120                 125
   Leu Val Leu Ala Leu Leu Glu Thr Ser Leu Leu His His Phe Ala Gly
       130                 135                 140
   Phe Ser Gln Ile Ser Lys Ser Asn Ser Gln Ala Ile Val Gly Tyr Gly
   145                 150                 155                 160
   Leu Met Ile Leu Leu Ile Ile Leu Trp Ile Leu Arg Glu Ile Gln Ser
                   165                 170                 175
   Val Tyr Ile Ile Gly Ile Phe Arg Asn Pro Phe Tyr Pro Lys Asp Val
               180                 185                 190
   Gln Thr Val Thr Val Phe Phe Glu Lys Gln Thr Arg Leu Met Lys Ile
           195                 200                 205
   Gly Ile Val Arg Arg Ile Leu Leu Thr Leu Val Ser Pro Phe Ala Met
       210                 215                 220
   Ile Ala Phe Leu Ser Leu Asp Ser Ser Leu Gln Gly Leu His Ser Val
   225                 230                 235                 240
   Ser Val Cys Ile Gly Phe Thr Arg Ala Phe Arg Met Val Trp Gln Asn
                   245                 250                 255
   Thr Glu Asn Ala Leu Leu Glu Thr Val Ile Val Ser Thr Val His Leu
               260                 265                 270
   Ile Ser Ser Thr Asp Ile Trp Trp Asn Arg Ser Leu Asp Thr Gly Leu
           275                 280                 285
   Arg Leu Leu Leu Val Gly Ile Ile Arg Asp Arg Leu Ile Gln Phe Ile
       290                 295                 300
   Ser Lys Leu Gln Phe Ala Val Thr Val Leu Leu Thr Ser Trp Thr Glu
   305                 310                 315                 320
   Lys Lys Gln Arg Arg Lys Thr Thr Ala Thr Leu Cys Ile Leu Asn Ile
                   325                 330                 335
   Val Phe Ser Pro Phe Val Leu Val Ile Ile Val Phe Ser Thr Leu Leu
               340                 345                 350
   Ser Ser Pro Leu Leu Pro Leu Phe Thr Leu Pro Val Phe Leu Val Gly
           355                 360                 365
   Phe Pro Arg Pro Ile Gln Ser Trp Pro Gly Ala Ala Gly Thr Thr Ala
       370                 375                 380
   Cys Val Cys Ala Asp Thr Val Tyr Tyr Tyr Gln Met Val Pro Arg Leu
   385                 390                 395                 400
   Thr Ala Val Leu Gln Thr Ala Met Ala Ala Gly Ser Leu Gly Leu Leu
                   405                 410                 415
   Leu Pro Gly Ser His Tyr Leu Gly Arg Phe Gln Asp Arg Leu Met Trp
               420                 425                 430
   Ile Met Ile Leu Glu Cys Gly Tyr Thr Tyr Cys Ser Ile Asn Ile Lys
           435                 440                 445
   Gly Leu Glu Leu Gln Glu Thr Ser Cys His Thr Ala Glu Ala Arg Arg
       450                 455                 460
   Val Asp Glu Val Phe Glu Asp Ala Phe Glu Gln Glu Tyr Thr Arg Val
   465                 470                 475                 480
   Cys Ser Leu Asn Glu His Phe Gly Asn Val Leu Thr Pro Cys Thr Val
                   485                 490                 495
   Leu Pro Val Lys Leu Tyr Ser Asp Ala Arg Asn Val Leu Ser Gly Ile
               500                 505                 510
   Ile Asp Ser His Glu Asn Leu Lys Glu Phe Lys Gly Asp Leu Ile Lys
           515                 520                 525
   Val Leu Val Trp Ile Leu Val Gln Tyr Cys Ser Lys Arg Pro Gly Met
       530                 535                 540
   Lys Glu Asn Val His Asn Thr Glu Asn Lys Gly Lys Ala Pro Leu Met
   545                 550                 555                 560
   Leu Pro Ala Leu Asn Thr Leu Pro Pro Pro Lys Ser Pro Glu Asp Ile
                   565                 570                 575
   Asp Ser Leu Asn Ser Glu Thr Phe Asn Asp Trp Ser Asp Asp Asn Ile
               580                 585                 590
   Phe Asp Asp Glu Pro Thr Ile Lys Lys Val Ile Glu Glu Lys His Gln
           595                 600                 605
   Leu Lys Asp Leu Pro Gly Thr Asn Leu Phe Ile Pro Gly Ser Val Glu
       610                 615                 620
   Ser Gln Arg Val Gly Asp His Ser Thr Gly Thr Val Pro Glu Asn Asp
   625                 630                 635                 640
   Leu Tyr Lys Ala Val Leu Leu Gly Tyr Pro Ala Val Asp Lys Gly Lys
                   645                 650                 655
   Gln Glu Asp Met Pro Tyr Ile Pro Leu Met Glu Phe Ser Cys Ser His
               660                 665                 670
   Ser His Leu Val Cys Leu Pro Ala Glu Trp Arg Thr Ser Cys Met Pro
           675                 680                 685
   Ser Ser Lys Met Lys Glu Met Ser Ser Leu Phe Pro Glu Asp Trp Tyr
       690                 695                 700
   Gln Phe Val Leu Arg Gln Leu Glu Cys Tyr His Ser Glu Glu Lys Ala
   705                 710                 715                 720
   Ser Asn Val Leu Glu Glu Ile Ala Lys Asp Lys Val Leu Lys Asp Phe
                   725                 730                 735
   Tyr Val His Thr Val Met Thr Cys Tyr Phe Ser Leu Phe Gly Ile Asp
               740                 745                 750
   Asn Met Ala Pro Ser Pro Gly His Ile Leu Arg Val Tyr Gly Gly Val
           755                 760                 765
   Leu Pro Trp Ser Val Ala Leu Asp Trp Leu Thr Glu Lys Pro Glu Leu
       770                 775                 780
   Phe Gln Leu Ala Leu Lys Ala Phe Arg Tyr Thr Leu Lys Leu Met Ile
   785                 790                 795                 800
   Asp Lys Ala Ser Leu Gly Pro Ile Glu Asp Phe Arg Glu Leu Ile Lys
                   805                 810                 815
   Tyr Leu Glu Glu Tyr Glu Arg Asp Trp Tyr Ile Gly Leu Val Ser Asp
               820                 825                 830
   Glu Lys Trp Lys Glu Ala Ile Leu Gln Glu Lys Pro Tyr Leu Phe Ser
           835                 840                 845
   Leu Gly Tyr Asp Ser Asn Met Pro Gly Pro Ala Leu Glu Ile Ser Arg
       850                 855                 860
   Val Asn Arg Asn Leu Trp Ser Gln Ile
   865                 870
<210> 76
   <211> 3927
   <212> DNA
   <213> Homo sapiens
<400> 76
   aacgacgctc ttgcgtaaag gcccggccca agggaacgtt cagggcgtct cggctttccc     60
   cgctgctgct tctgctaggc ccagtgcgag accagagcac gagcgactcc cgtcgtcccc    120
   ggccaggcag atgttggcct agtcctggcg cgaacgaagc gcgctatttc cctgcttcct    180
   ctaggccaag cctgctttac ggcagggccc gcctcgggag cgagcacaga ccggggcagc    240
   gaggccagcc aggcgccgac gaggtccccg aacgcgcacg cgctccgttc agctccgggt    300
   ggcggccgcc ggagtagacg ttagccatgg aaaccgagag ctggcccggg cggggccgcg    360
   gtgagctcgt tattcggccg ccgcagcttt tctgcctccg cattcgggca ctaaccaacc    420
   tcccggcggg agcgcccagc ccgagtttac ctgcaaaaat gcggtccctg ggatgccttc    480
   gcgtcttctc ttccctcggg tgacttgagg tttgtggtaa atatgccagc tctagaacac    540
   atgaatcaga ttttacacat cttgtttgta tttttaccct ttctgtgggc acttgggact    600
   ctgcccccac ccgatgcact tctcttatgg gcaatggagc aggttttaga gttcggcctt    660
   ggaggctcat ctatgtcaac ccacttacgg ttattagtaa tgttcatcat gtctgctgga    720
   acagctatag catcatattt cattccaagc actgttggtg tggttctttt catgactgga 780
   tttggtttct tgctgagtct gaacttaagt gatatgggtc acaaaattgg aaccaaatct 840
   aaggatttac ccagtggtcc ggaaaaacat ttttcatgga aggaatgcct tttctacatc 900
   attatattag tcttggctct tttagaaact agcttgcttc atcactttgc tggcttctca 960
   cagatttcta aaagcaattc ccaggctatt gtgggctatg gtttgatgat attacttata 1020
   atactgtgga tacttagaga aattcaaagc gtatatatca ttggaatttt ccgaaatccc 1080
   ttttatccga aggatgtgca aactgtgact gtattctttg agaagcaaac taggctcatg 1140
   aagattggta ttgtcagacg gattttgcta actttagtat caccttttgc catgatagca 1200
   tttctttcat tggacagttc cttacaaggg ctccactcag tgtctgtctg tattggattc 1260
   acaagagcct ttagaatggt atggcagaat acagaaaatg ctttattgga gacagtcatt 1320
   gtatcaacag tacacttgat ctccagtaca gacatatggt ggaacagaag cctggataca 1380
   ggactcagac tcttactggt tggtatcata cgtgatcgtt tgattcagtt catctctaaa 1440
   ttgcagtttg ccgtgactgt gcttttgaca tcatggacag agaaaaaaca acgtcgaaaa 1500
   acaactgcca ctttatgtat actcaacatt gtcttttctc cattcgtgtt ggtcatcata 1560
   gttttttcta cactactctc ttctccctta ctccctcttt tcacccttcc tgtgttcttg 1620
   gtggggtttc cccgacctat tcagagttgg ccaggagcag caggcaccac agcctgtgtg 1680
   tgtgcagata cagtgtacta ctaccaaatg gtgcccaggt tgactgctgt actgcagact 1740
   gcaatggcag ctggaagttt aggtctcctc ctacctggat ctcattactt gggccgtttt 1800
   caggatcgtt taatgtggat aatgattctg gaatgtggct atacttactg ctctattaac 1860
   attaaggggt tagaattgca ggaaacatcc tgtcatactg cagaagctcg cagagttgat 1920
   gaagtttttg aagatgcttt tgagcaagaa tacacaagag tatgttccct taatgaacac 1980
   tttggaaatg tcttgacacc ctgtactgtt ttgcctgtga aattgtattc tgatgccagg 2040
   aatgttctat caggcataat tgattctcat gaaaacttaa aagaatttaa aggtgacctc 2100
   attaaagtac ttgtgtggat acttgttcaa tactgctcca aaaggcctgg catgaaagag 2160
   aatgttcaca acactgaaaa taaagggaaa gcacctctaa tgttgcctgc tttgaacact 2220
   ttgccacctc ccaaatcccc agaagacata gacagtttaa attcagaaac ttttaatgac 2280
   tggtctgatg ataatatttt tgatgatgag ccaactatca aaaaagtaat agaagaaaaa 2340
   catcagttga aagatttgcc aggtacaaat ttgtttattc caggatcagt agaatcacag 2400
   agggttggtg atcattctac aggcactgtt cctgaaaacg atctttacaa agcagttcta 2460
   ttaggatacc ctgctgttga caaaggaaaa caagaggaca tgccatatat tcctctcatg 2520
   gagttcagtt gttcacattc tcacttagta tgcttacccg cagagtggag gactagctgt 2580
   atgcccagtt ccaaaatgaa ggagatgagc tcgttatttc cagaagactg gtaccaattt 2640
   gttctaaggc agttggaatg ttatcattca gaagagaagg cctcaaatgt actggaagaa 2700
   attgccaagg acaaagtttt aaaagacttt tatgttcata cagtaatgac ttgttatttt 2760
   agtttatttg gaatagacaa tatggctcct agtcctggtc atatattgag agtttacggt 2820
   ggtgttttgc cttggtctgt tgctttggac tggctcacag aaaagccaga actgtttcaa 2880
   ctagcactga aagcattcag gtatactctg aaactaatga ttgataaagc aagtttaggt 2940
   ccaatagaag actttagaga actgattaag taccttgaag aatatgaacg tgactggtac 3000
   attggtttgg tatctgatga aaagtggaag gaagcaattt tacaagaaaa gccatacttg 3060
   ttttctctgg ggtatgattc taatatggga atttacactg ggagagtgct tagccttcaa 3120
   gaattattga tccaagtggg aaagttaaat cctgaagctg ttagaggtca gtgggccaat 3180
   ctttcatggg aattacttta tgccacaaac gatgatgaag aacgttatag tatacaagct 3240
   catccactac ttttaagaaa tcttacggta caagcagcag aacctcccct gggatatccg 3300
   atttattctt caaaacctct ccacatacat ttgtattaga gctcattttg actgtaatgt 3360
   catcaaatgc aatgttttta ttttttcatc ctaaaaaagt aactgtgatt cttgtaactt 3420
   gaggacttct ccacaccccc attcagatgc ctgagaacag ctaagctccg taaagttggt 3480
   tctcttagcc atcttaatgg ttctaaaaaa cagcaaaaac atctttatgt ctaagataaa 3540
   agaactattt ggccaatatt tgtgccctct ggactttagt aggctttggt aaatgtgaga 3600
   aaacttttgt agaattatca tataatgaat tttgtaatgc tttcttaaat gtgttatagg 3660
   tgaattgcca tacaaagtta acagctatgt aatttttaca tacttaagag ataaacatat 3720
   cagtgttcta agtagtgata atggatcctg ttgaaggtta acataatgtg tatatatttg 3780
   tttgaaatat aatttatagt attttcaaat gtgctgattt attttgacat ctaatatctg 3840
   aatgtttttg tatcaagtag tttgttttca tagacttcaa ttcataaact ttaaaaaact 3900
   tttaataaaa tattttcctt ccttttc 3927
<210> 77
   <211> 3932
   <212> DNA
   <213> Homo sapiens
<400> 77
   aacgacgctc ttgcgtaaag gcccggccca agggaacgtt cagggcgtct cggctttccc 60
   cgctgctgct tctgctaggc ccagtgcgag accagagcac gagcgactcc cgtcgtcccc 120
   ggccaggcag atgttggcct agtcctggcg cgaacgaagc gcgctatttc cctgcttcct 180
   ctaggccaag cctgctttac ggcagggccc gcctcgggag cgagcacaga ccggggcagc 240
   gaggccagcc aggcgccgac gaggtccccg aacgcgcacg cgctccgttc agctccgggt 300
   ggcggccgcc ggagtagacg ttagccatgg aaaccgagag ctggcccggg cggggccgcg    360
   gtgagctcgt tattcggccg ccgcagcttt tctgcctccg cattcgggca ctaaccaacc    420
   tcccggcggg agcgcccagc ccgagtttac ctgcaaaaat gcggtccctg ggatgccttc    480
   gcgtcttctc ttccctcggg tgacttgagg tttgtggtaa atatgccagc tctagaacac    540
   atgaatcaga ttttacacat cttgtttgta tttttaccct ttctgtgggc acttgggact    600
   ctgcccccac ccgatgcact tctcttatgg gcaatggagc aggttttaga gttcggcctt    660
   ggaggctcat ctatgtcaac ccacttacgg ttattagtaa tgttcatcat gtctgctgga    720
   acagctatag catcatattt cattccaagc actgttggtg tggttctttt catgactgga    780
   tttggtttct tgctgagtct gaacttaagt gatatgggtc acaaaattgg aaccaaatct    840
   aaggatttac ccagtggtcc ggaaaaacat ttttcatgga aggaatgcct tttctacatc    900
   attatattag tcttggctct tttagaaact agcttgcttc atcactttgc tggcttctca    960
   cagatttcta aaagcaattc ccaggctatt gtgggctatg gtttgatgat attacttata   1020
   atactgtgga tacttagaga aattcaaagc gtatatatca ttggaatttt ccgaaatccc   1080
   ttttatccga aggatgtgca aactgtgact gtattctttg agaagcaaac taggctcatg   1140
   aagattggta ttgtcagacg gattttgcta actttagtat caccttttgc catgatagca   1200
   tttctttcat tggacagttc cttacaaggg ctccactcag tgtctgtctg tattggattc   1260
   acaagagcct ttagaatggt atggcagaat acagaaaatg ctttattgga gacagtcatt   1320
   gtatcaacag tacacttgat ctccagtaca gacatatggt ggaacagaag cctggataca   1380
   ggactcagac tcttactggt tggtatcata cgtgatcgtt tgattcagtt catctctaaa   1440
   ttgcagtttg ccgtgactgt gcttttgaca tcatggacag agaaaaaaca acgtcgaaaa   1500
   acaactgcca ctttatgtat actcaacatt gtcttttctc cattcgtgtt ggtcatcata   1560
   gttttttcta cactactctc ttctccctta ctccctcttt tcacccttcc tgtgttcttg   1620
   gtggggtttc cccgacctat tcagagttgg ccaggagcag caggcaccac agcctgtgtg   1680
   tgtgcagata cagtgtacta ctaccaaatg gtgcccaggt tgactgctgt actgcagact   1740
   gcaatggcag ctggaagttt aggtctcctc ctacctggat ctcattactt gggccgtttt   1800
   caggatcgtt taatgtggat aatgattctg gaatgtggct atacttactg ctctattaac   1860
   attaaggggt tagaattgca ggaaacatcc tgtcatactg cagaagctcg cagagttgat   1920
   gaagtttttg aagatgcttt tgagcaagaa tacacaagag tatgttccct taatgaacac   1980
   tttggaaatg tcttgacacc ctgtactgtt ttgcctgtga aattgtattc tgatgccagg   2040
   aatgttctat caggcataat tgattctcat gaaaacttaa aagaatttaa aggtgacctc   2100
   attaaagtac ttgtgtggat acttgttcaa tactgctcca aaaggcctgg catgaaagag   2160
   aatgttcaca acactgaaaa taaagggaaa gcacctctaa tgttgcctgc tttgaacact   2220
   ttgccacctc ccaaatcccc agaagacata gacagtttaa attcagaaac ttttaatgac   2280
   tggtctgatg ataatatttt tgatgatgag ccaactatca aaaaagtaat agaagaaaaa   2340
   catcagttga aagatttgcc aggtacaaat ttgtttattc caggatcagt agaatcacag   2400
   agggttggtg atcattctac aggcactgtt cctgaaaacg atctttacaa agcagttcta   2460
   ttaggatacc ctgctgttga caaaggaaaa caagaggaca tgccatatat tcctctcatg   2520
   gagttcagtt gttcacattc tcacttagta tgcttacccg cagagtggag gactagctgt   2580
   atgcccagtt ccaaaatgaa ggagatgagc tcgttatttc cagaagactg gtaccaattt   2640
   gttctaaggc agttggaatg ttatcattca gaagagaagg cctcaaatgt actggaagaa   2700
   attgccaagg acaaagtttt aaaagacttt tatgttcata cagtaatgac ttgttatttt   2760
   agtttatttg gaatagacaa tatggctcct agtcctggtc atatattgag agtttacggt   2820
   ggtgttttgc cttggtctgt tgctttggac tggctcacag aaaagccaga actgtttcaa   2880
   ctagcactga aagcattcag gtatactctg aaactaatga ttgataaagc aagtttaggt   2940
   ccaatagaag actttagaga actgattaag taccttgaag aatatgaacg tgactggtac   3000
   attggtttgg tatctgatga aaagtggaag gaagcaattt tacaagaaaa gccatacttg   3060
   ttttctctgg ggtatgattc taatatggga atttacactg ggagagtgct tagccttcaa   3120
   gaattattga tccaagtggg aaagttaaat cctgaagctg ttagaggtca gtgggccaat   3180
   ctttcatggg aattacttta tgccacaaac gatgatgaag aacgttatag tatacaagct   3240
   catccactac ttttaagaaa tcttacggta caagcagcag aacctcccct gggatatccg   3300
   atttattctt caaaacctct ccacatacat ttgtattaga gctcattttg actgtaatgt   3360
   catcaaatgc aatgttttta ttttttcatc ctaaaaaagt aactgtgatt cttgtaactt   3420
   gaggacttct ccacaccccc attcagatgc ctgagaacag ctaagctccg taaagttggt   3480
   tctcttagcc atcttaatgg ttctaaaaaa cagcaaaaac atctttatgt ctaagataaa   3540
   agaactattt ggccaatatt tgtgccctct ggactttagt aggctttggt aaatgtgaga   3600
   aaacttttgt agaattatca tataatgaat tttgtaatgc tttcttaaat gtgttatagg   3660
   tgaattgcca tacaaagtta acagctatgt aatttttaca tacttaagag ataaacatat   3720
   cagtgttcta agtagtgata atggatcctg ttgaaggtta acataatgtg tatatatttg   3780
   tttgaaatat aatttatagt attttcaaat gtgctgattt attttgacat ctaatatctg   3840
   aatgtttttg tatcaagtag tttgttttca tagacttcaa ttcataaact ttaaaaaact   3900
   tttaataaaa tattttcctt ccttttcaaa ta                                 3932
<210> 78
   <211> 4073
   <212> DNA
   <213> Homo sapiens
<400> 78
   tcttgcgtaa aggcccggcc caagggaacg ttcagggcgt ctcggctttc cccgctgctg     60
   cttctgctag gcccagtgcg agaccagagc acgagcgact cccgtcgtcc ccggccaggc    120
   agatgttggc ctagtcctgg cgcgaacgaa gcgcgctatt tccctgcttc ctctaggcca    180
   agcctgcttt acggcagggc ccgcctcggg agcgagcaca gaccggggca gcgaggccag    240
   ccaggcgccg acgaggtccc cgaacgcgca cgcgctccgt tcagctccgg gtggcggccg    300
   ccggagtaga cgttagccat ggaaaccgag agctggcccg ggcggggccg cggtgagctc    360
   gttattcggc cgccgcagct tttctgcctc cgcattcggg cactaaccaa cctcccggcg    420
   ggagcgccca gcccgagttt acctgcaaaa atgcggtccc tgggatgcct tcgcgtcttc    480
   tcttccctcg ggtgacttga gaaactgctg tgttacagaa aagcatgtga ctttcagaat    540
   aatcccgagt gaggatgagt ccagatgtgc ctctactgaa tgattacaag caggacttct    600
   ttctgaagcg ctttccacag actgttcttg gaggccctcg attcaaatta ggctattgtg    660
   cccctcctta catatatgtt aatcaaatta ttctttttct aatgccatgg gtttggggtg    720
   gagtcggaac acttttatac cagttaggca tcctgaaaga ctattataca gcagcacttt    780
   caggtggatt aatgcttttt actgcatttg tcatccagtt cacaagttta tacgccaaaa    840
   acaaatcaac aacagtagaa agaatactaa ccacggatat cttagcagag gaggatgagc    900
   atgaatttac cagttgtact ggtgctgaga ctgtcaaatt tctcattcct ggcaagaaat    960
   atgtagccaa tacagttttt cattctattc ttgctggatt agcgtgtggt cttggaacat   1020
   ggtatctgct cccaaataga ataaccttgc tgtatggcag tacaggaggc actgctctac   1080
   tattcttctt tggatggatg acactatgta tagcagaata ttctttaatt gtaaacacag   1140
   ctacagagac tgcgactttc caaacacagg atacttatga aattattcct cttatgagac   1200
   ctctttatat ttttttcttt gtttctgtgg atctggcaca caggtaaaaa cctaccaaat   1260
   actttgtaac taactttgtt tttaagtata cagagtaaga gagctttcct tttagtgtta   1320
   caaaaaaatg aatccatgga ttaaaaatca tcaaaccatt gggtgacagg ttattttgat   1380
   aattattctt ttaggattaa tctctgtaaa acatactaaa gcaatagtta aaacttatta   1440
   aagagttttt ttaaaaaacc ctttttgaga taaggaactt ttcaattttg tgtttcactt   1500
   taaataagga gctttgagtt tttaagatag cctggctaaa acctgtgtaa ggagatggaa   1560
   ctttcctgtg gggggaaaga agaaattaaa atttatacat ataaatatta tatacagatt   1620
   gaatgaattt aagacaaata caaaatttat ttctaatttt atgatagcaa caatagtaga   1680
   agtaatattg attttttaaa aaccaacttg ttacagaaga aaagtagaaa atagtttttt   1740
   taacagacat aattgttcac aaaattgttt gataacccct tttacttgcc ttttcaagtt 1800
   tgacttttct ttctccgtct ccgtagattg cagctttctt ttcttaggtt agtgtccagg 1860
   tagaaatgtt cagcatgtta tggactgaat atttgtgtct ctccagaatt catatattac 1920
   agtcttaaac cccaatgtga tggtattttg agatgaggcc tttgggaggt aattaggtca 1980
   tgaaggtggg tccttggtat gatgggacta gtccccttat gagaagaggt accagagagc 2040
   ttgatttgtc cctctctgtg ccatctaagg acacagcaaa aatgtggcca cctgcaagtc 2100
   aaagaagaga gctctcatca aaacctgacc atactggcag cctgatcatg gtctttcggc 2160
   cttcagaagt gtgagaaagt aaattgatgt tgtttaagcc actcagctta tggcattttt 2220
   ttatggcagc ccatgctgat taagattttg ctaccaagaa gtgggatgcc tttgtaccaa 2280
   atacctaaaa atgtggaaat ggctttgtaa ctgttggtaa tgggtagagg ctgaaagagg 2340
   tttttttgtt tgtttctttg ttttgttttg tttgagatgg agtcttcact ctcttgccca 2400
   ggctggagtg cagtggcaca atcttggccc actgcaacgt ccgcctcctg gcttcaagtg 2460
   atactcctcc ctgagcctcc tgagtagctg gaattacaga catgcgccac catgcccggc 2520
   taatttttat atttttaata gagacagggt ttcgccatgt tggccaggct ggtcccaaag 2580
   tcctgacctc aagtgatcca cccgcctcgg cctcccaaag ttctgggatt acaggcttga 2640
   gccatcacac ccagccaagg gttttgatgt gcatgctaga aatatggaca ttaagggtga 2700
   ttctgatgaa gtctgaagtc ccctgaaccc agaggaaagg cagtccttgt tacaaagcgt 2760
   caaagaactt agctgaactg tgtcctagtg ttttgtggag ccatgaagtt ggatacctac 2820
   gtaaggagag ttcaaagcag tgttgaaagg agcagttggc ttctcctgtt tctagtaaaa 2880
   tgtgaaagga gagagatgga ttgaagaagg gattgttaag caagagaagg aaccagaact 2940
   tgaagatttg gaaaattttc agcctggatt atccatattg taaaacatga gaaagcatat 3000
   tctgaagaga acaccaaaag tgtggggctg gactgtccct cagtaaagag cttttggcat 3060
   tatgtgagta gaaacactgc cagtttgaat tgaagtggtt ggagacagga agtaatgaag 3120
   gccgacagtt gaacttcttg gatttgacag gatgtaatga tagaactgtt cagctacaaa 3180
   agtgcagtat tcttcaagaa aaggggaaaa ttatgccaaa ggtgatttaa gggtcttgag 3240
   ggctaccacc tgtttcaaca agtcagctag cctctaccca aagcctcggg agcagaactg 3300
   aacttcagag ccacagaagc aggaccctca cctagagcac tggcggtgac ctgccacccc 3360
   agtggcctgg tgggcagagt atggaaccaa acagaattat tctcaagctc taagatctaa 3420
   tggaatttgc cttgctaggt tttgacttcc ttgggatcat cacccttttt ttcctgtttc 3480
   tcctattgga gtggggatgt ctcttctata cctgccctac ccttagattt tggaagcacg 3540
   taactcatct ggtttcacag attcacagct ggagaggaat tttgcctcag gatggattgt 3600
   acctaggtct catccatatc tgatttagat gagactttga attttagcca tcagagttaa   3660
   tgctagaatg agtgaagact ttgggggata tggggatgga atgaatgtct tttgcatttg   3720
   aatttggaaa ggacatgagt tttgaggggc cagggatgga atgttataga ctaattgtgt   3780
   tccctcaaaa atgtttatgt tgaagcccta acccccaatg tgttggtatt tggagatggg   3840
   cctctgggag gtagtttatg aaggtgagac cctagtctga taggattagt gcccttagga   3900
   gagatgccaa agagcttgat gtctctcttt ttgctacaaa aagacacagc aaaaaggcag   3960
   ccatgtgtaa gccaggaaga gagtcttcac cagaacctga ctatactggc agcctgatct   4020
   tgtacttgta gcccccagaa ctgttagaaa ataaatttct gttgtttaag cca          4073
<210> 79
   <211> 4666
   <212> DNA
   <213> Homo sapiens
<400> 79
   tcttgcgtaa aggcccggcc caagggaacg ttcagggcgt ctcggctttc cccgctgctg     60
   cttctgctag gcccagtgcg agaccagagc acgagcgact cccgtcgtcc ccggccaggc    120
   agatgttggc ctagtcctgg cgcgaacgaa gcgcgctatt tccctgcttc ctctaggcca    180
   agcctgcttt acggcagggc ccgcctcggg agcgagcaca gaccggggca gcgaggccag    240
   ccaggcgccg acgaggtccc cgaacgcgca cgcgctccgt tcagctccgg gtggcggccg    300
   ccggagtaga cgttagccat ggaaaccgag agctggcccg ggcggggccg cggtgagctc    360
   gttattcggc cgccgcagct tttctgcctc cgcattcggg cactaaccaa cctcccggcg    420
   ggagcgccca gcccgagttt acctgcaaaa atgcggtccc tgggatgcct tcgcgtcttc    480
   tcttccctcg ggtgacttga gaaactgctg tgttacagaa aagcatgtga ctttcagaat    540
   aatcccgagt gaggatgagt ccagatgtgc ctctactgaa tgattacaag caggacttct    600
   ttctgaagcg ctttccacag actgttcttg gaggccctcg attcaaatta ggctattgtg    660
   cccctcctta catatatgtt aatcaaatta ttctttttct aatgccatgg gtttggggtg    720
   gagtcggaac acttttatac cagttaggca tcctgaaaga ctattataca gcagcacttt    780
   caggtggatt aatgcttttt actgcatttg tcatccagtt cacaagttta tacgccaaaa    840
   acaaatcaac aacagtagaa agaatactaa ccacggatat cttagcagag gaggatgagc    900
   atgaatttac cagttgtact ggtgctgaga ctgtcaaatt tctcattcct ggcaagaaat    960
   atgtagccaa tacagttttt cattctattc ttgctggatt agcgtgtggt cttggaacat   1020
   ggtatctgct cccaaataga ataaccttgc tgtatggcag tacaggaggc actgctctac   1080
   tattcttctt tggatggatg acactatgta tagcagaata ttctttaatt gtaaacacag   1140
   ctacagagac tgcgactttc caaacacagg atacttatga aattattcct cttatgagac   1200
   ctctttatat ttttttcttt gtttctgtgg atctggcaca caggtttgtg gtaaatatgc 1260
   cagctctaga acacatgaat cagattttac acatcttgtt tgtattttta ccctttctgt 1320
   gggcacttgg gactctgccc ccacccgatg cacttctctt atgggcaatg gagcaggttt 1380
   tagagttcgg ccttggaggc tcatctatgt caacccactt acggttatta gtaatgttca 1440
   tcatgtctgc tggaacagct atagcatcat atttcattcc aagcactgtt ggtgtggttc 1500
   ttttcatgac tggatttggt ttcttgctga gtctgaactt aagtgatatg ggtcacaaaa 1560
   ttggaaccaa atctaaggat ttacccagtg gtccggaaaa acatttttca tggaaggaat 1620
   gccttttcta catcattata ttagtcttgg ctcttttaga aactagcttg cttcatcact 1680
   ttgctggctt ctcacagatt tctaaaagca attcccaggc tattgtgggc tatggtttga 1740
   tgatattact tataatactg tggatactta gagaaattca aagcgtatat atcattggaa 1800
   ttttccgaaa tcccttttat ccgaaggatg tgcaaactgt gactgtattc tttgagaagc 1860
   aaactaggct catgaagatt ggtattgtca gacggatttt gctaacttta gtatcacctt 1920
   ttgccatgat agcatttctt tcattggaca gttccttaca agggctccac tcagtgtctg 1980
   tctgtattgg attcacaaga gcctttagaa tggtatggca gaatacagaa aatgctttat 2040
   tggagacagt cattgtatca acagtacact tgatctccag tacagacata tggtggaaca 2100
   gaagcctgga tacaggactc agactcttac tggttggtat catacgtgat cgtttgattc 2160
   agttcatctc taaattgcag tttgccgtga ctgtgctttt gacatcatgg acagagaaaa 2220
   aacaacgtcg aaaaacaact gccactttat gtatactcaa cattgtcttt tctccattcg 2280
   tgttggtcat catagttttt tctacactac tctcttctcc cttactccct cttttcaccc 2340
   ttcctgtgtt cttggtgggg tttccccgac ctattcagag ttggccagga gcagcaggca 2400
   ccacagcctg tgtgtgtgca gatacagtgt actactacca aatggtgccc aggttgactg 2460
   ctgtactgca gactgcaatg gcagctggaa gtttaggtct cctcctacct ggatctcatt 2520
   acttgggccg ttttcaggat cgtttaatgt ggataatgat tctggaatgt ggctatactt 2580
   actgctctat taacattaag ggttagaatt tgcaggaaac atcctgtcat actgcagaag 2640
   ctcgcagagt tgatgaagtt tttgaagatg cttttgagca agaatacaca agagtatgtt 2700
   cccttaatga acactttgga aatgtcttga caccctgtac tgttttgcct gtgaaattgt 2760
   attctgatgc caggaatgtt ctatcaggca taattgattc tcatgaaaac ttaaaagaat 2820
   ttaaaggtga cctcattaaa gtacttgtgt ggatacttgt tcaatactgc tccaaaaggc 2880
   ctggcatgaa agagaatgtt cacaacactg aaaataaagg gaaagcacct ctaatgttgc 2940
   ctgctttgaa cactttgcca cctcccaaat ccccagaaga catagacagt ttaaattcag 3000
   aaacttttaa tgactggtct gatgataata tttttgatga tgagccaact atcaaaaaag 3060
   taatagaaga aaaacatcag ttgaaagatt tgccaggtac aaatttgttt attccaggat 3120
   cagtagaatc acagagggtt ggtgatcatt ctacaggcac tgttcctgaa aacgatcttt 3180
   acaaagcagt tctattagga taccctgctg ttgacaaagg aaaacaagag gacatgccat 3240
   atattcctct catggagttc agttgttcac attctcactt agtatgctta cccgcagagt 3300
   ggaggactag ctgtatgccc agttccaaaa tgaaggagat gagctcgtta tttccagaag 3360
   actggtacca atttgttcta aggcagttgg aatgttatca ttcagaagag aaggcctcaa 3420
   atgtactgga agaaattgcc aaggacaaag ttttaaaaga cttttatgtt catacagtaa 3480
   tgacttgtta ttttagttta tttggaatag acaatatggc tcctagtcct ggtcatatat 3540
   tgagagttta cggtggtgtt ttgccttggt ctgttgcttt ggactggctc acagaaaagc 3600
   cagaactgtt tcaactagca ctgaaagcat tcaggtatac tctgaaacta atgattgata 3660
   aagcaagttt aggtccaata gaagacttta gagaactgat taagtacctt gaagaatatg 3720
   aacgtgactg gtacattggt ttggtatctg atgaaaagtg gaaggaagca attttacaag 3780
   aaaagccata cttgttttct ctggggtatg attctaatat gggaatttac actgggagag 3840
   tgcttagcct tcaagaatta ttgatccaag tgggaaagtt aaatcctgaa gctgttagag 3900
   gtcagtgggc caatctttca tgggaattac tttatgccac aaacgatgat gaagaacgtt 3960
   atagtataca agctcatcca ctacttttaa gaaatcttac ggtacaagca gcagaacctc 4020
   ccctgggata tccgatttat tcttcaaaac ctctccacat acatttgtat tagagctcat 4080
   tttgactgta atgtcatcaa atgcaatgtt tttatttttt catcctaaaa aagtaactgt 4140
   gattcttgta acttgaggac ttctccacac ccccattcag atgcctgaga acagctaagc 4200
   tccgtaaagt tggttctctt agccatctta atggttctaa aaaacagcaa aaacatcttt 4260
   atgtctaaga taaaagaact atttggccaa tatttgtgcc ctctggactt tagtaggctt 4320
   tggtaaatgt gagaaaactt ttgtagaatt atcatataat gaattttgta atgctttctt 4380
   aaatgtgtta taggtgaatt gccatacaaa gttaacagct atgtaatttt tacatactta 4440
   agagataaac atatcagtgt tctaagtagt gataatggat cctgttgaag gttaacataa 4500
   tgtgtatata tttgtttgaa atataattta tagtattttc aaatgtgctg atttattttg 4560
   acatctaata tctgaatgtt tttgtatcaa gtagtttgtt ttcatagact tcaattcata 4620
   aactttaaaa aacttttaat aaaatatttt ccttcctttt caaata 4666
<210> 80
   <211> 3513
   <212> DNA
   <213> Homo sapiens
<400> 80
   aaaggcccgg cccaagggaa cgttcagggc gtctcggctt tccccgctgc tgcttctgct 60
   aggcccagtg cgagaccaga gcacgagcga ctcccgtcgt ccccggccag gcagatgttg 120
   gcctagtcct ggcgcgaacg aagcgcgcta tttccctgct tcctctaggc caagcctgct 180
   ttacggcagg gcccgcctcg ggagcgagca cagaccgggg cagcgaggcc agccaggcgc 240
   cgacgaggtc cccgaacgcg cacgcgctcc gttcagctcc gggtggcggc cgccggagta 300
   gacgttagcc atggaaaccg agagctggcc cgggcggggc cgcggtgagc tcgttattcg 360
   gccgccgcag cttttctgcc tccgcattcg ggcactaacc aacctcccgg cgggagcgcc 420
   cagcccgagt ttacctgcaa aaatgcggtc cctgggatgc cttcgcgtct tctcttccct 480
   cgggtgactt gaggtttgtg gtaaatatgc cagctctaga acacatgaat cagattttac 540
   acatcttgtt tgtattttta ccctttctgt gggcacttgg gactctgccc ccacccgatg 600
   cacttctctt atgggcaatg gagcaggttt tagagttcgg ccttggaggc tcatctatgt 660
   caacccactt acggttatta gtaatgttca tcatgtctgc tggaacagct atagcatcat 720
   atttcattcc aagcactgtt ggtgtggttc ttttcatgac tggatttggt ttcttgctga 780
   gtctgaactt aagtgatatg ggtcacaaaa ttggaaccaa atctaaggat ttacccagtg 840
   gtccggaaaa acatttttca tggaaggaat gccttttcta catcattata ttagtcttgg 900
   ctcttttaga aactagcttg cttcatcact ttgctggctt ctcacagatt tctaaaagca 960
   attcccaggc tattgtgggc tatggtttga tgatattact tataatactg tggatactta 1020
   gagaaattca aagcgtatat atcattggaa ttttccgaaa tcccttttat ccgaaggatg 1080
   tgcaaactgt gactgtattc tttgagaagc aaactaggct catgaagatt ggtattgtca 1140
   gacggatttt gctaacttta gtatcacctt ttgccatgat agcatttctt tcattggaca 1200
   gttccttaca agggctccac tcagtgtctg tctgtattgg attcacaaga gcctttagaa 1260
   tggtatggca gaatacagaa aatgctttat tggagacagt cattgtatca acagtacact 1320
   tgatctccag tacagacata tggtggaaca gaagcctgga tacaggactc agactcttac 1380
   tggttggtat catacgtgat cgtttgattc agttcatctc taaattgcag tttgccgtga 1440
   ctgtgctttt gacatcatgg acagagaaaa aacaacgtcg aaaaacaact gccactttat 1500
   gtatactcaa cattgtcttt tctccattcg tgttggtcat catagttttt tctacactac 1560
   tctcttctcc cttactccct cttttcaccc ttcctgtgtt cttggtgggg tttccccgac 1620
   ctattcagag ttggccagga gcagcaggca ccacagcctg tgtgtgtgca gatacagtgt 1680
   actactacca aatggtgccc aggttgactg ctgtactgca gactgcaatg gcagctggaa 1740
   gtttaggtct cctcctacct ggatctcatt acttgggccg ttttcaggat cgtttaatgt 1800
   ggataatgat tctggaatgt ggctatactt actgctctat taacattaag gggttagaat 1860
   tgcaggaaac atcctgtcat actgcagaag ctcgcagagt tgatgaagtt tttgaagatg 1920
   cttttgagca agaatacaca agagtatgtt cccttaatga acactttgga aatgtcttga   1980
   caccctgtac tgttttgcct gtgaaattgt attctgatgc caggaatgtt ctatcaggca   2040
   taattgattc tcatgaaaac ttaaaagaat ttaaaggtga cctcattaaa gtacttgtgt   2100
   ggatacttgt tcaatactgc tccaaaaggc ctggcatgaa agagaatgtt cacaacactg   2160
   aaaataaagg gaaagcacct ctaatgttgc ctgctttgaa cactttgcca cctcccaaat   2220
   ccccagaaga catagacagt ttaaattcag aaacttttaa tgactggtct gatgataata   2280
   tttttgatga tgagccaact atcaaaaaag taatagaaga aaaacatcag ttgaaagatt   2340
   tgccaggtac aaatttgttt attccaggat cagtagaatc acagagggtt ggtgatcatt   2400
   ctacaggcac tgttcctgaa aacgatcttt acaaagcagt tctattagga taccctgctg   2460
   ttgacaaagg aaaacaagag gacatgccat atattcctct catggagttc agttgttcac   2520
   attctcactt agtatgctta cccgcagagt ggaggactag ctgtatgccc agttccaaaa   2580
   tgaaggagat gagctcgtta tttccagaag actggtacca atttgttcta aggcagttgg   2640
   aatgttatca ttcagaagag aaggcctcaa atgtactgga agaaattgcc aaggacaaag   2700
   ttttaaaaga cttttatgtt catacagtaa tgacttgtta ttttagttta tttggaatag   2760
   acaatatggc tcctagtcct ggtcatatat tgagagttta cggtggtgtt ttgccttggt   2820
   ctgttgcttt ggactggctc acagaaaagc cagaactgtt tcaactagca ctgaaagcat   2880
   tcaggtatac tctgaaacta atgattgata aagcaagttt aggtccaata gaagacttta   2940
   gagaactgat taagtacctt gaagaatatg aacgtgactg gtacattggt ttggtatctg   3000
   atgaaaagtg gaaggaagca attttacaag aaaagccata cttgttttct ctggggtatg   3060
   attctaatat gccaggacca gccttggaga taagcagagt gaacagaaat ttatggtctc   3120
   agatttaaga aaaacaaaat tctttcttgc ttcttaaatc atactccatc ccattggctt   3180
   gcaaacatgc tgacactcct cataatttca ctcttcataa accaaagcat aaggtcagag   3240
   gagaacttga catattagaa cacttaggca ttgaaagtgg ttagtctaac taacccattg   3300
   aagttttgga gaacctggga ctcaaatttt ggaagatgtg acagatgata tgttaacata   3360
   cattgcaccg aggctgaagt gggaggattg cttgagactg cctggaaggc agaagttgca   3420
   gtgagccgag actgatggtg tcactgcact ccagcctggg caacagagca agaccctgtc   3480
   ttaaaaaaac aaaacaaaca aacaaaagaa acc                                3513
<210> 81
   <211> 21
   <212> DNA
   <213> Homo sapiens
<400> 81
   cgaggacaat ctggatatca a 21
<210> 82
   <211> 21
   <212> DNA
   <213> Homo sapiens
<400> 82
   ctggagccct cgagcaagaa a 21
<210> 83
   <211> 21
   <212> DNA
   <213> Homo sapiens
<400> 83
   cccgtggttc atctgatata a 21
<210> 84
   <211> 21
   <212> DNA
   <213> Homo sapiens
<400> 84
   aaggactttg ctcggcgttt a 21
<210> 85
   <211> 21
   <212> DNA
   <213> Homo sapiens
<400> 85
   tacgtggatg tttgtaacgt a 21
<210> 86
   <211> 21
   <212> DNA
   <213> Homo sapiens
<400> 86
   ctcgtattgg ctcaatcata a 21